# EUROPEAN PATENT SPECIFICATION

(11) **EP 4 175 642 B1**
(45) Date of publication and mention of the grant of the patent: **18.11.2026**
(21) Application number: 21831949.9
(22) Date of filing: 01.07.2021
(51) Int. Cl.: A61K 31/53, C07D 237/14, C07D 253/07, A61P 25/28, C07D 401/04, C07D 401/12, C07D 401/14, C07D 403/12

(54) **NLRP-3 MODULATING COMPOUNDS AND PHARMACEUTICAL COMPOSITIONS THEREOF**
NLRP-3 MODULIERENDE VERBDINUNGEN UND DEREN PHARMAZEUTISCHE ZUSAMMENSETZUNGEN
COMPOSÉS MODULATEURS DE NLRP-3 ET COMPOSITIONS PHARMACEUTIQUES LES COMPRENANT

(30) Priority: 02.07.2020 US 202063047811 P
(43) Date of publication of application: 10.05.2023
(73) Proprietor: Tenvie Therapeutics, Inc., Brisbane, CA 94005 (US)
(72) Inventor: CRAIG, II Robert A., San Francisco, CA 94080 (US); DE VICENTE FIDALGO, Javier, San Francisco, CA 94080 (US); ESTRADA, Anthony A., San Francisco, CA 94080 (US); FOX, Brian M., San Francisco, CA 94080 (US); HU, Cheng, San Francisco, CA 94080 (US); LEXA, Katrina W., San Francisco, CA 94080 (US); NILEWSKI, Lizanne G., San Francisco, CA 94080 (US); OSIPOV, Maksim, San Francisco, CA 94080 (US); THOTTUMKARA, Arun, San Francisco, CA 94080 (US)
(74) Representative: Marks & Clerk LLP
(86) International application number: PCT/US2021/040145
(87) International publication number: WO 2022/006433

(56) References cited:
- WO-A1-2019/034686
- WO-A1-2019/043610
- WO-A1-2020/097265
- KR-A- 20190 032 790
- US-A1- 2005 192 279
- US-A1- 2019 256 474
- US-B2- 8 524 712

## Description

### FIELD

The present disclosure relates generally to small molecule modulators of NLR Family Pyrin Domain Containing 3 (NLRP3), and the small molecule modulators for use as therapeutic agents.

### BACKGROUND

Inhibition of NLRP3 activation has been shown to result in potent therapeutic effects in animal models of inflammatory diseases. Modulators of NLRP3, inhibitors in particular, have broad therapeutic potential in a wide array of auto-inflammatory and chronic inflammatory diseases that either require better treatment options or for which no adequate therapies exist. Therapies targeting NLRP3-dependent cytokines are already approved for therapeutic use; however, they have notable disadvantages relative to direct NLRP3 antagonists. WO2019043610A1 discloses heterocyclic compounds which are NLRP3 modulators and their use in the treatment of disease states mediated by NLRP3. KR20190032790A discloses the use of 2-cyclohexylimino-6-metyl-6,7-dihydro-5H-benzo[1,3]oxathiol-4-one as an NLRP3 inhibitor. WO2019034686A1 discloses sulfonamide carboxamide compounds and the use of such compounds in the treatment and prevention of medical disorders and diseases by the inhibition of NLRP3. There remains a strong impetus for the discovery and clinical development of molecules that antagonize NLRP3.

### DESCRIPTION

Provided herein are compounds, or a pharmaceutically acceptable salt, isotopically enriched analog, stereoisomer, or mixture of stereoisomers, for use in treating and/or preventing diseases mediated, at least in part, by NLRP3.

**In** some embodiments, provided are compounds for use in modulating the activity of NLRP3. In some embodiments, the compounds are for use in inhibiting the activation of NLRP3.

**In** another embodiment, provided is a pharmaceutical composition comprising a compound as described herein, or a pharmaceutically acceptable salt, isotopically enriched analog, stereoisomer, or mixture of stereoisomers, and a pharmaceutically acceptable carrier.

**In** another embodiment, provided is a pharmaceutical composition comprising a compound as described herein, or a pharmaceutically acceptable salt, isotopically enriched analog, stereoisomer, or mixture of stereoisomers, for use in a method for treating a disease or condition mediated, at least in part, by NLRP3, the method comprising administering an effective amount of the pharmaceutical composition.

In another embodiment, provided is a pharmaceutical composition comprising a compound as described herein, or a pharmaceutically acceptable salt, isotopically enriched analog, stereoisomer, or mixture of stereoisomers, for use in a method for treating a disease or condition mediated, at least in part, by TNF-α, the method comprising administering an effective amount of the pharmaceutical composition. In some embodiments the administration is to a subject resistant to treatment with an anti-TNF-α agent. In some embodiments, the disease is a gut disease or condition. In some embodiments the disease or condition is inflammatory bowel disease, Crohn's disease, or ulcerative colitis.

The disclosure also provides compositions, including pharmaceutical compositions, kits that include the compounds, or a pharmaceutically acceptable salt, isotopically enriched analog, stereoisomer, or mixture of stereoisomers, methods of using (or administering) and making the compounds, or a pharmaceutically acceptable salt, isotopically enriched analog, stereoisomer, or mixture of stereoisomers, and intermediates thereof.

The disclosure further provides compounds, or a pharmaceutically acceptable salt, isotopically enriched analog, stereoisomer, or mixture of stereoisomers, or compositions thereof for use in a method of treating a disease, disorder, or condition that is mediated, at least in part, by NLRP3.

The description herein sets forth exemplary embodiments of the present technology. It should be recognized, however, that such description is not intended as a limitation on the scope of the present disclosure but is instead provided as a description of exemplary embodiments.

### 1. Definitions

As used in the present specification, the following words, phrases and symbols are generally intended to have the meanings as set forth below, except to the extent that the context in which they are used indicates otherwise.

A dash ("-") that is not between two letters or symbols is used to indicate a point of attachment for a substituent. For example, -C(O)NH₂ is attached through the carbon atom. A dash at the front or end of a chemical group is a matter of convenience; chemical groups may be depicted with or without one or more dashes without losing their ordinary meaning. A wavy line or a dashed line drawn through a line in a structure indicates a specified point of attachment of a group. Unless chemically or structurally required, no directionality or stereochemistry is indicated or implied by the order in which a chemical group is written or named.

The prefix "Cᵤ₋ᵥ" indicates that the following group has from u to v carbon atoms. For example, "C₁₋₆ alkyl" indicates that the alkyl group has from 1 to 6 carbon atoms.

Reference to "about" a value or parameter herein includes (and describes) embodiments that are directed to that value or parameter per se. In certain embodiments, the term "about" includes the indicated amount ± 10%. In other embodiments, the term "about" includes the indicated amount ± 5%. In certain other embodiments, the term "about" includes the indicated amount ± 1%. Also, to the term "about X" includes description of "X". Also, the singular forms "a" and "the" include plural references unless the context clearly dictates otherwise. Thus, e.g., reference to "the compound" includes a plurality of such compounds and reference to "the assay" includes reference to one or more assays and equivalents thereof known to those skilled in the art.

"Alkyl" refers to an unbranched or branched saturated hydrocarbon chain. As used herein, alkyl has 1 to 20 carbon atoms (i.e., C₁₋₂₀ alkyl), 1 to 12 carbon atoms (i.e., C₁₋₁₂ alkyl), 1 to 8 carbon atoms (i.e., C₁₋₈ alkyl), 1 to 6 carbon atoms (i.e., C₁₋₆ alkyl) or 1 to 4 carbon atoms (i.e., C₁₋₄ alkyl). Examples of alkyl groups include, e.g., methyl, ethyl, propyl, isopropyl, n-butyl, sec-butyl, iso-butyl, tert-butyl, pentyl, 2-pentyl, isopentyl, neopentyl, hexyl, 2-hexyl, 3-hexyl, and 3-methylpentyl. When an alkyl residue having a specific number of carbons is named by chemical name or identified by molecular formula, all positional isomers having that number of carbons may be encompassed; thus, for example, "butyl" includes n-butyl (i.e., -(CH₂)₃CH₃), sec-butyl (i.e., -CH(CH₃)CH₂CH₃), isobutyl (i.e., -CH₂CH(CH₃)₂), and tert-butyl (i.e., -C(CH₃)₃); and "propyl" includes n-propyl (i.e., -(CH₂)₂CH₃) and isopropyl (i.e., -CH(CH₃)₂).

Certain commonly used alternative chemical names may be used. For example, a divalent group such as a divalent "alkyl" group, a divalent "aryl" group, a divalent heteroaryl group, etc., may also be referred to as an "alkylene" group or an "alkylenyl" group (for example, methylenyl, ethylenyl, and propylenyl), an "arylene" group or an "arylenyl" group (for example, phenylenyl or napthylenyl, or quinolinyl for heteroarylene), respectively. Also, unless indicated explicitly otherwise, where combinations of groups are referred to herein as one moiety, e.g., arylalkyl or aralkyl, the last mentioned group contains the atom by which the moiety is attached to the rest of the molecule.

"Alkenyl" refers to an alkyl group containing at least one (e.g., 1-3 or 1) carbon-carbon double bond and having from 2 to 20 carbon atoms (i.e., C₂₋₂₀ alkenyl), 2 to 12 carbon atoms (i.e., C₂₋₁₂ alkenyl), 2 to 8 carbon atoms (i.e., C₂₋₈ alkenyl), 2 to 6 carbon atoms (i.e., C₂₋₆ alkenyl), or 2 to 4 carbon atoms (i.e., C₂₋₄ alkenyl). Examples of alkenyl groups include, e.g., ethenyl, propenyl, butadienyl (including 1,2-butadienyl and 1,3-butadienyl).

"Alkynyl" refers to an alkyl group containing at least one (e.g., 1-3 or 1) carbon-carbon triple bond and having from 2 to 20 carbon atoms (i.e., C₂₋₂₀ alkynyl), 2 to 12 carbon atoms (i.e., C₂₋₁₂ alkynyl), 2 to 8 carbon atoms (i.e., C₂₋₈ alkynyl), 2 to 6 carbon atoms (i.e., C₂₋₆ alkynyl), or 2 to 4 carbon atoms (i.e., C₂₋₄ alkynyl). The term "alkynyl" also includes those groups having one triple bond and one double bond.

"Alkoxy" refers to the group "alkyl-O-". Examples of alkoxy groups include, e.g., methoxy, ethoxy, n-propoxy, iso-propoxy, n-butoxy, tert-butoxy, sec-butoxy, n-pentoxy, n-hexoxy, and 1,2-dimethylbutoxy.

"Alkoxyalkyl" refers to the group "alkyl-O-alkyl".

"Alkylthio" refers to the group "alkyl-S-". "Alkylsulfinyl" refers to the group "alkyl-S(O)-". "Alkylsulfonyl" refers to the group "alkyl-S(O)₂-". "Alkylsulfonylalkyl" refers to -alkyl-S(O)₂-alkyl.

"Acyl" refers to a group -C(O)R^{y}, wherein R^{y} is hydrogen, alkyl, alkenyl, alkynyl, cycloalkyl, heterocyclyl, aryl, heteroalkyl, or heteroaryl; each of which may be optionally substituted, as defined herein. Examples of acyl include, e.g., formyl, acetyl, cyclohexylcarbonyl, cyclohexylmethyl-carbonyl, and benzoyl.

"Amido" refers to both a "C-amido" group which refers to the group -C(O)NR^{y}R^{z} and an "N-amido" group which refers to the group -NR^{y}C(O)R^{z}, wherein R^{y} and R^{z} are independently hydrogen, alkyl, alkenyl, alkynyl, cycloalkyl, heterocyclyl, aryl, heteroalkyl, or heteroaryl; each of which may be optionally substituted, as defined herein, or R^{y} and R^{z} are taken together to form a cycloalkyl or heterocyclyl; each of which may be optionally substituted, as defined herein.

"Amino" refers to the group -NR^{y}R^{z} wherein R^{y} and R^{z} are independently hydrogen, alkyl, alkenyl, alkynyl, cycloalkyl, heterocyclyl, aryl, heteroalkyl, or heteroaryl; each of which may be optionally substituted, as defined herein.

"Amidino" refers to -C(NR^{y})(NR^{z}₂), wherein R^{y} and R^{z} are independently hydrogen, alkyl, alkenyl, alkynyl, cycloalkyl, heterocyclyl, aryl, heteroalkyl, or heteroaryl; each of which may be optionally substituted, as defined herein.

"Aryl" refers to an aromatic carbocyclic group having a single ring (e.g., monocyclic) or multiple rings (e.g., bicyclic or tricyclic) including fused systems. As used herein, aryl has 6 to 20 ring carbon atoms (i.e., C₆₋₂₀ aryl), 6 to 12 carbon ring atoms (i.e., C₆₋₁₂ aryl), or 6 to 10 carbon ring atoms (i.e., C₆₋₁₀ aryl). Examples of aryl groups include, e.g., phenyl, naphthyl, fluorenyl, and anthryl. Aryl, however, does not encompass or overlap in any way with heteroaryl defined below. If one or more aryl groups are fused with a heteroaryl, the resulting ring system is heteroaryl. If one or more aryl groups are fused with a heterocyclyl, the resulting ring system is heterocyclyl. If one or more aryl groups are fused with a cycloalkyl, the resulting ring system is cycloalkyl.

"Arylalkyl" or "Aralkyl" refers to the group "aryl-alkyl-".

"Carbamoyl" refers to both an "O-carbamoyl" group which refers to the group -O-C(O)NR^{y}R^{z} and an "N-carbamoyl" group which refers to the group -NR^{y}C(O)OR^{z}, wherein R^{y} and R^{z} are independently hydrogen, alkyl, alkenyl, alkynyl, cycloalkyl, heterocyclyl, aryl, heteroalkyl, or heteroaryl; each of which may be optionally substituted, as defined herein.

"Carboxyl ester" or "ester" refer to both -OC(O)R^{x} and -C(O)OR^{x}, wherein R^{x} is alkyl, alkenyl, alkynyl, cycloalkyl, heterocyclyl, aryl, heteroalkyl, or heteroaryl; each of which may be optionally substituted, as defined herein.

"Cyanoalkyl" refers to refers to an alkyl group as defined above, wherein one or more (e.g., 1 or 2) hydrogen atoms are replaced by a cyano (-CN) group.

"Cycloalkyl" refers to a saturated or partially unsaturated cyclic alkyl group having a single ring or multiple rings including fused, bridged, and spiro ring systems. The term "cycloalkyl" includes cycloalkenyl groups (i.e., the cyclic group having at least one double bond) and carbocyclic fused ring systems having at least one sp³ carbon atom (i.e., at least one non-aromatic ring). As used herein, cycloalkyl has from 3 to 20 ring carbon atoms (i.e., C₃₋₂₀ cycloalkyl), 3 to 14 ring carbon atoms (i.e., C₃₋₁₂ cycloalkyl), 3 to 12 ring carbon atoms (i.e., C₃₋₁₂ cycloalkyl), 3 to 10 ring carbon atoms (i.e., C₃₋₁₀ cycloalkyl), 3 to 8 ring carbon atoms (i.e., C₃₋₈ cycloalkyl), or 3 to 6 ring carbon atoms (i.e., C₃₋₆ cycloalkyl). Monocyclic groups include, for example, cyclopropyl, cyclobutyl, cyclopentyl, cyclohexyl, cycloheptyl, and cyclooctyl. Polycyclic groups include, for example, bicyclo[2.2.1]heptanyl, bicyclo[2.2.2]octanyl, adamantyl, norbornyl, decalinyl, 7,7-dimethyl-bicyclo[2.2.1]heptanyl, and the like. Further, the term cycloalkyl is intended to encompass any non-aromatic ring which may be fused to an aryl ring, regardless of the attachment to the remainder of the molecule. Still further, cycloalkyl also includes "spirocycloalkyl" when there are two positions for substitution on the same carbon atom, for example spiro[2.5]octanyl, spiro[4.5]decanyl, or spiro[5.5]undecanyl.

"Cycloalkylalkyl" refers to the group "cycloalkyl-alkyl-".

"Imino" refers to a group -C(NR^{y})R^{z}, wherein R^{y} and R^{z} are each independently hydrogen, alkyl, alkenyl, alkynyl, cycloalkyl, heterocyclyl, aryl, heteroalkyl, or heteroaryl; each of which may be optionally substituted, as defined herein.

"Imido" refers to a group -C(O)NR^{y}C(O)R^{z}, wherein R^{y} and R^{z} are each independently hydrogen, alkyl, alkenyl, alkynyl, cycloalkyl, heterocyclyl, aryl, heteroalkyl, or heteroaryl; each of which may be optionally substituted, as defined herein.

"Halogen" or "halo" refers to atoms occupying group VIIA of the periodic table, such as fluoro, chloro, bromo, or iodo.

"Haloalkyl" refers to an unbranched or branched alkyl group as defined above, wherein one or more (e.g., 1 to 6 or 1 to 3) hydrogen atoms are replaced by a halogen. For example, where a residue is substituted with more than one halogen, it may be referred to by using a prefix corresponding to the number of halogen moieties attached. Dihaloalkyl and trihaloalkyl refer to alkyl substituted with two ("di") or three ("tri") halo groups, which may be, but are not necessarily, the same halogen. Examples of haloalkyl include, e.g., trifluoromethyl, difluoromethyl, fluoromethyl, trichloromethyl, 2,2,2-trifluoroethyl, 1,2-difluoroethyl, 3-bromo-2-fluoropropyl, 1,2-dibromoethyl, and the like.

"Haloalkoxy" refers to an alkoxy group as defined above, wherein one or more (e.g., 1 to 6 or 1 to 3) hydrogen atoms are replaced by a halogen.

"Haloalkoxyalkyl" refers to an alkoxyalkyl group as defined above, wherein one or more (e.g., 1 to 6 or 1 to 3) hydrogen atoms are replaced by a halogen.

"Hydroxyalkyl" refers to an alkyl group as defined above, wherein one or more (e.g., 1 to 6 or 1 to 3) hydrogen atoms are replaced by a hydroxy group.

"Heteroalkyl" refers to an alkyl group in which one or more of the carbon atoms (and any associated hydrogen atoms), excluding any terminal carbon atom(s), are each independently replaced with the same or different heteroatomic group, provided the point of attachment to the remainder of the molecule is through a carbon atom. The term "heteroalkyl" includes unbranched or branched saturated chain having carbon and heteroatoms. By way of example, 1, 2 or 3 carbon atoms may be independently replaced with the same or different heteroatomic group. Heteroatomic groups include, but are not limited to, -NR^{y}-, -O-, -S-, -S(O)-, -S(O)₂-, and the like, wherein R^{y} is hydrogen, alkyl, alkenyl, alkynyl, cycloalkyl, heterocyclyl, aryl, heteroalkyl, or heteroaryl; each of which may be optionally substituted, as defined herein. Examples of heteroalkyl groups include, e.g., ethers (e.g., -CH₂OCH₃, -CH(CH₃)OCH₃, -CH₂CH₂OCH₃, -CH₂CH₂OCH₂CH₂OCH₃, etc.), thioethers (e.g., -CH₂SCH₃, -CH(CH₃)SCH₃, -CH₂CH₂SCH₃,-CH₂CH₂SCH₂CH₂SCH₃, etc.), sulfones (e.g., -CH₂S(O)₂CH₃, -CH(CH₃)S(O)₂CH₃, -CH₂CH₂S(O)₂CH₃, -CH₂CH₂S(O)₂CH₂CH₂OCH₃, etc.), and amines (e.g., -CH₂NR^{y}CH₃, -CH(CH₃)NR^{y}CH₃, -CH₂CH₂NR^{y}CH₃, -CH₂CH₂NR^{y}CH₂CH₂NR^{y}CH₃, etc., where R^{y} is hydrogen, alkyl, alkenyl, alkynyl, cycloalkyl, heterocyclyl, aryl, heteroalkyl, or heteroaryl; each of which may be optionally substituted, as defined herein). As used herein, heteroalkyl includes 2 to 10 carbon atoms, 2 to 8 carbon atoms, or 2 to 4 carbon atoms; and 1 to 3 heteroatoms, 1 to 2 heteroatoms, or 1 heteroatom.

"Heteroaryl" refers to an aromatic group having a single ring, multiple rings or multiple fused rings, with one or more ring heteroatoms independently selected from nitrogen, oxygen, and sulfur. As used herein, heteroaryl includes 1 to 20 ring carbon atoms (i.e., C₁₋₂₀ heteroaryl), 3 to 12 ring carbon atoms (i.e., C₃₋₁₂ heteroaryl), or 3 to 8 carbon ring atoms (i.e., C₃₋₈ heteroaryl), and 1 to 5 ring heteroatoms, 1 to 4 ring heteroatoms, 1 to 3 ring heteroatoms, 1 to 2 ring heteroatoms, or 1 ring heteroatom independently selected from nitrogen, oxygen, and sulfur. In certain instances, heteroaryl includes 5-10 membered ring systems, 5-7 membered ring systems, or 5-6 membered ring systems, each independently having 1 to 4 ring heteroatoms, 1 to 3 ring heteroatoms, 1 to 2 ring heteroatoms, or 1 ring heteroatom independently selected from nitrogen, oxygen, and sulfur. Examples of heteroaryl groups include, e.g., acridinyl, benzimidazolyl, benzothiazolyl, benzindolyl, benzofuranyl, benzothiazolyl, benzothiadiazolyl, benzonaphthofuranyl, benzoxazolyl, benzothienyl (benzothiophenyl), benzotriazolyl, benzo[4,6]imidazo[1,2-a]pyridyl, carbazolyl, cinnolinyl, dibenzofuranyl, dibenzothiophenyl, furanyl, isothiazolyl, imidazolyl, indazolyl, indolyl, indazolyl, isoindolyl, isoquinolyl, isoxazolyl, naphthyridinyl, oxadiazolyl, oxazolyl, 1-oxidopyridinyl, 1-oxidopyrimidinyl, 1-oxidopyrazinyl, 1-oxidopyridazinyl, phenazinyl, phthalazinyl, pteridinyl, purinyl, pyrrolyl, pyrazolyl, pyridinyl, pyrazinyl, pyrimidinyl, pyridazinyl, quinazolinyl, quinoxalinyl, quinolinyl, quinuclidinyl, isoquinolinyl, thiazolyl, thiadiazolyl, triazolyl, tetrazolyl, and triazinyl. Examples of the fused-heteroaryl rings include, but are not limited to, benzo[d]thiazolyl, quinolinyl, isoquinolinyl, benzo[b]thiophenyl, indazolyl, benzo[d]imidazolyl, pyrazolo[1,5-a]pyridinyl, and imidazo[1,5-a]pyridinyl, where the heteroaryl can be bound *via* either ring of the fused system. Any aromatic ring, having a single or multiple fused rings, containing at least one heteroatom, is considered a heteroaryl regardless of the attachment to the remainder of the molecule (i.e., through any one of the fused rings). Heteroaryl does not encompass or overlap with aryl as defined above.

"Heteroarylalkyl" refers to the group "heteroaryl-alkyl-".

"Heterocyclyl" refers to a saturated or partially unsaturated cyclic alkyl group, with one or more ring heteroatoms independently selected from nitrogen, oxygen, and sulfur. The term "heterocyclyl" includes heterocycloalkenyl groups (i.e., the heterocyclyl group having at least one double bond), bridged-heterocyclyl groups, fused-heterocyclyl groups, and spiro-heterocyclyl groups. A heterocyclyl may be a single ring or multiple rings wherein the multiple rings may be fused, bridged, or spiro, and may comprise one or more (e.g., 1 to 3) oxo (=O) or N-oxide (-O⁻) moieties. Any non-aromatic ring containing at least one heteroatom is considered a heterocyclyl, regardless of the attachment (i.e., can be bound through a carbon atom or a heteroatom). Further, the term heterocyclyl is intended to encompass any non-aromatic ring containing at least one heteroatom, which ring may be fused to an aryl or heteroaryl ring, regardless of the attachment to the remainder of the molecule. As used herein, heterocyclyl has 2 to 20 ring carbon atoms (i.e., C₂₋₂₀ heterocyclyl), 2 to 12 ring carbon atoms (i.e., C₂₋₁₂ heterocyclyl), 2 to 10 ring carbon atoms (i.e., C₂₋₁₀ heterocyclyl), 2 to 8 ring carbon atoms (i.e., C₂₋₈ heterocyclyl), 3 to 12 ring carbon atoms (i.e., C₃₋₁₂ heterocyclyl), 3 to 8 ring carbon atoms (i.e., C₃₋₈ heterocyclyl), or 3 to 6 ring carbon atoms (i.e., C₃₋₆ heterocyclyl); having 1 to 5 ring heteroatoms, 1 to 4 ring heteroatoms, 1 to 3 ring heteroatoms, 1 to 2 ring heteroatoms, or 1 ring heteroatom independently selected from nitrogen, sulfur, or oxygen. Examples of heterocyclyl groups include, e.g., azetidinyl, azepinyl, benzodioxolyl, benzo[b][1,4]dioxepinyl, 1,4-benzodioxanyl, benzopyranyl, benzodioxinyl, benzopyranonyl, benzofuranonyl, dioxolanyl, dihydropyranyl, hydropyranyl, thienyl[1,3]dithianyl, decahydroisoquinolyl, furanonyl, imidazolinyl, imidazolidinyl, indolinyl, indolizinyl, isoindolinyl, isothiazolidinyl, isoxazolidinyl, morpholinyl, octahydroindolyl, octahydroisoindolyl, 2-oxopiperazinyl, 2-oxopiperidinyl, 2-oxopyrrolidinyl, oxazolidinyl, oxiranyl, oxetanyl, phenothiazinyl, phenoxazinyl, piperidinyl, piperazinyl, 4-piperidonyl, pyrrolidinyl, pyrazolidinyl, quinuclidinyl, thiazolidinyl, tetrahydrofuryl, tetrahydropyranyl, trithianyl, tetrahydroquinolinyl, thiophenyl (i.e., thienyl), thiomorpholinyl, thiamorpholinyl, 1-oxo-thiomorpholinyl, and 1,1-dioxo-thiomorpholinyl. The term "heterocyclyl" also includes "spiroheterocyclyl" when there are two positions for substitution on the same carbon atom. Examples of the spiro-heterocyclyl rings include, e.g., bicyclic and tricyclic ring systems, such as oxabicyclo[2.2.2]octanyl, 2-oxa-7-azaspiro[3.5]nonanyl, 2-oxa-6-azaspiro[3.4]octanyl, and 6-oxa-1-azaspiro[3.3]heptanyl. Examples of the fused-heterocyclyl rings include, but are not limited to, 1,2,3,4-tetrahydroisoquinolinyl, 4,5,6,7-tetrahydrothieno[2,3-c]pyridinyl, indolinyl, and isoindolinyl, where the heterocyclyl can be bound *via* either ring of the fused system.

"Heterocyclylalkyl" refers to the group "heterocyclyl-alkyl-."

"Oxime" refers to the group -CR^{y}(=NOH) wherein R^{y} is hydrogen, alkyl, alkenyl, alkynyl, cycloalkyl, heterocyclyl, aryl, heteroalkyl, or heteroaryl; each of which may be optionally substituted, as defined herein.

"Sulfonyl" refers to the group -S(O)₂R^{y}, where R^{y} is hydrogen, alkyl, alkenyl, alkynyl, cycloalkyl, heterocyclyl, aryl, heteroalkyl, or heteroaryl; each of which may be optionally substituted, as defined herein. Examples of sulfonyl are methylsulfonyl, ethylsulfonyl, phenylsulfonyl, and toluenesulfonyl.

"Sulfinyl" refers to the group -S(O)R^{y}, where R^{y} is hydrogen, alkyl, alkenyl, alkynyl, cycloalkyl, heterocyclyl, aryl, heteroalkyl, or heteroaryl; each of which may be optionally substituted, as defined herein. Examples of sulfinyl are methylsulfinyl, ethylsulfinyl, phenylsulfinyl, and toluenesulfinyl.

"Sulfonamido" refers to the groups -SO₂NR^{y}R^{z} and -NR^{y}SO₂R^{z}, where R^{y} and R^{z} are each independently hydrogen, alkyl, alkenyl, alkynyl, cycloalkyl, heterocyclyl, aryl, heteroalkyl, or heteroaryl; each of which may be optionally substituted, as defined herein.

The terms "optional" or "optionally" means that the subsequently described event or circumstance may or may not occur and that the description includes instances where said event or circumstance occurs and instances in which it does not. Also, the term "optionally substituted" refers to any one or more (e.g., 1 to 5 or 1 to 3) hydrogen atoms on the designated atom or group may or may not be replaced by a moiety other than hydrogen.

The term "substituted" used herein means any of the above groups (i.e., alkyl, alkenyl, alkynyl, alkylene, alkoxy, haloalkyl, haloalkoxy, cycloalkyl, aryl, heterocyclyl, heteroaryl, and/or heteroalkyl) wherein at least one (e.g., 1 to 5 or 1 to 3) hydrogen atom is replaced by a bond to a non-hydrogen atom such as, but not limited to alkyl, alkenyl, alkynyl, alkoxy, alkylthio, acyl, amido, amino, amidino, aryl, aralkyl, azido, carbamoyl, carboxyl, carboxyl ester, cyano, cycloalkyl, cycloalkylalkyl, guanadino, halo, haloalkyl, haloalkoxy, hydroxyalkyl, heteroalkyl, heteroaryl, heteroarylalkyl, heterocyclyl, heterocyclylalkyl, -NHNH₂, =NNH₂, imino, imido, hydroxy, oxo, oxime, nitro, sulfonyl, sulfinyl, alkylsulfonyl, alkylsulfinyl, thiocyanate, -S(O)OH, -S(O)₂OH, sulfonamido, thiol, thioxo, N-oxide, or -Si(R^{y})₃, wherein each R^{y} is independently hydrogen, alkyl, alkenyl, alkynyl, heteroalkyl, cycloalkyl, aryl, heteroaryl, or heterocyclyl.

**In** certain embodiments, "substituted" includes any of the above alkyl, alkenyl, alkynyl, cycloalkyl, heterocyclyl, aryl, or heteroaryl groups in which one or more (e.g., 1 to 5 or 1 to 3) hydrogen atoms are independently replaced with deuterium, halo, cyano, nitro, azido, oxo, alkyl, alkenyl, alkynyl, haloalkyl, cycloalkyl, heterocyclyl, aryl, heteroaryl, -NR^{g}R^{h}', -NR^{g}C(O)R^{h}, -NR^{g}C(O)NR^{g}R^{h}, -NR^{g}C(O)OR^{h}, -NR^{g}S(O)₁₋₂R^{h}, -C(O)R^{g}, -C(O)OR^{g}, -OC(O)OR^{g}, -OC(O)R^{g}, -C(O)NR^{g}R^{h}, -OC(O)NR^{g}R^{h}, -OR^{g}, -SR^{g}, -S(O)R^{g}, -S(O)₂R^{g}, -OS(O)₁₋₂R^{g}, -S(O)₁₋₂OR^{g}, -NR^{g}S(O)₁₋₂NR^{g}R^{h}, =NSO₂R^{g}, =NOR^{g}, -S(O)₁₋₂NR^{g}R^{h}, -SF₅, -SCF₃, or -OCF₃. In certain embodiments, "substituted" also means any of the above groups in which one or more (e.g., 1 to 5 or 1 to 3) hydrogen atoms are replaced with -C(O)R^{g}, -C(O)OR^{g}, -C(O)NR^{g}R^{h}, -CH₂SO₂R^{g}, or -CH₂SO₂NR^{g}R^{h}. In the foregoing, R^{g} and R^{h} are the same or different and independently hydrogen, alkyl, alkenyl, alkynyl, alkoxy, thioalkyl, aryl, aralkyl, cycloalkyl, cycloalkylalkyl, haloalkyl, heterocyclyl, heterocyclylalkyl, heteroaryl, and/or heteroarylalkyl. In certain embodiments, "substituted" also means any of the above groups in which one or more (e.g., 1 to 5 or 1 to 3) hydrogen atoms are replaced by a bond to an amino, cyano, hydroxy, imino, nitro, oxo, thioxo, halo, alkyl, alkoxy, alkylamino, thioalkyl, aryl, aralkyl, cycloalkyl, cycloalkylalkyl, haloalkyl, heterocyclyl, N-heterocyclyl, heterocyclylalkyl, heteroaryl, and/or heteroarylalkyl, or two of R^{g} and R^{h} and Rⁱ are taken together with the atoms to which they are attached to form a heterocyclyl ring optionally substituted with oxo, halo, or alkyl optionally substituted with oxo, halo, amino, hydroxy, or alkoxy.

Polymers or similar indefinite structures arrived at by defining substituents with further substituents appended ad infinitum (e.g., a substituted aryl having a substituted alkyl which is itself substituted with a substituted aryl group, which is further substituted by a substituted heteroalkyl group, etc.) are not intended for inclusion herein. Unless otherwise noted, the maximum number of serial substitutions in compounds described herein is three. For example, serial substitutions of substituted aryl groups with two other substituted aryl groups are limited to ((substituted aryl)substituted aryl) substituted aryl. Similarly, the above definitions are not intended to include impermissible substitution patterns (e.g., methyl substituted with 5 fluorines or heteroaryl groups having two adjacent oxygen ring atoms). Such impermissible substitution patterns are well known to the skilled artisan. When used to modify a chemical group, the term "substituted" may describe other chemical groups defined herein.

**In** certain embodiments, as used herein, the phrase "one or more" refers to one to five. In certain embodiments, as used herein, the phrase "one or more" refers to one to three.

Any compound or structure given herein, is also intended to represent unlabeled forms as well as isotopically labeled forms of the compounds. These forms of compounds may also be referred to as "isotopically enriched analogs." Isotopically labeled compounds have structures depicted herein, except that one or more atoms are replaced by an atom having a selected atomic mass or mass number. Examples of isotopes that can be incorporated into the disclosed compounds include isotopes of hydrogen, carbon, nitrogen, oxygen, phosphorous, fluorine, chlorine, and iodine, such as ²H, ³H, ¹¹C, ¹³C, ¹⁴C, ¹³N, ¹⁵N*,* ¹⁵O, ¹⁷O, ¹⁸O, ³¹P, ³²P, ³⁵S, ¹⁸F, ³⁶Cl, ¹²³I, and ¹²⁵I, respectively. Various isotopically labeled compounds of the present disclosure, for example those into which radioactive isotopes such as ³H and ¹⁴C are incorporated. Such isotopically labelled compounds may be useful in metabolic studies, reaction kinetic studies, detection or imaging techniques, such as positron emission tomography (PET) or single-photon emission computed tomography (SPECT) including drug or substrate tissue distribution assays or in radioactive treatment of patients.

The term "isotopically enriched analogs" includes "deuterated analogs" of compounds described herein in which one or more hydrogens is/are replaced by deuterium, such as a hydrogen on a carbon atom. Such compounds exhibit increased resistance to metabolism and are thus useful for increasing the half-life of any compound when administered to a mammal, particularly a human. See, for example, Foster, "Deuterium Isotope Effects in Studies of Drug Metabolism," Trends Pharmacol. Sci. 5(12):524-527 (1984). Such compounds are synthesized by means well known in the art, for example by employing starting materials in which one or more hydrogens have been replaced by deuterium.

Deuterium labelled or substituted therapeutic compounds of the disclosure may have improved DMPK (drug metabolism and pharmacokinetics) properties, relating to distribution, metabolism, and excretion (ADME). Substitution with heavier isotopes such as deuterium may afford certain therapeutic advantages resulting from greater metabolic stability, for example increased *in vivo* half-life, reduced dosage requirements, and/or an improvement in therapeutic index. An ¹⁸F, ³H, ¹¹C labeled compound may be useful for PET or SPECT or other imaging studies. Isotopically labeled compounds of this disclosure can generally be prepared by carrying out the procedures disclosed in the schemes or in the examples and preparations described below by substituting a readily available isotopically labeled reagent for a non-isotopically labeled reagent. It is understood that deuterium in this context is regarded as a substituent in a compound described herein.

The concentration of such a heavier isotope, specifically deuterium, may be defined by an isotopic enrichment factor. In the compounds of this disclosure any atom not specifically designated as a particular isotope is meant to represent any stable isotope of that atom. Unless otherwise stated, when a position is designated specifically as "H" or "hydrogen", the position is understood to have hydrogen at its natural abundance isotopic composition. Accordingly, in the compounds of this disclosure any atom specifically designated as a deuterium (D) is meant to represent deuterium.

In many cases, the compounds of this disclosure are capable of forming acid and/or base salts by virtue of the presence of amino, and/or carboxyl groups, or groups similar thereto.

Provided are also or a pharmaceutically acceptable salt, isotopically enriched analog, deuterated analog, stereoisomer, and mixture of stereoisomers of the compounds described herein. "Pharmaceutically acceptable" or "physiologically acceptable" refer to compounds, salts, compositions, dosage forms, and other materials which are useful in preparing a pharmaceutical composition that is suitable for veterinary or human pharmaceutical use.

The term "pharmaceutically acceptable salt" of a given compound refers to salts that retain the biological effectiveness and properties of the given compound and which are not biologically or otherwise undesirable. "Pharmaceutically acceptable salts" or "physiologically acceptable salts" include, for example, salts with inorganic acids, and salts with an organic acid. In addition, if the compounds described herein are obtained as an acid addition salt, the free base can be obtained by basifying a solution of the acid salt. Conversely, if the product is a free base, an addition salt, particularly a pharmaceutically acceptable addition salt, may be produced by dissolving the free base in a suitable organic solvent and treating the solution with an acid, in accordance with conventional procedures for preparing acid addition salts from base compounds. Those skilled in the art will recognize various synthetic methodologies that may be used to prepare nontoxic pharmaceutically acceptable addition salts. Pharmaceutically acceptable acid addition salts may be prepared from inorganic or organic acids. Salts derived from inorganic acids include, e.g., hydrochloric acid, hydrobromic acid, sulfuric acid, nitric acid, phosphoric acid, and the like. Salts derived from organic acids include, e.g., acetic acid, propionic acid, gluconic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluene-sulfonic acid, salicylic acid, and the like. Likewise, pharmaceutically acceptable base addition salts can be prepared from inorganic or organic bases. Salts derived from inorganic bases include, by way of example only, sodium, potassium, lithium, aluminum, ammonium, calcium, and magnesium salts. Salts derived from organic bases include, but are not limited to, salts of primary, secondary, and tertiary amines, such as alkyl amines (i.e., NH₂(alkyl)), dialkyl amines (i.e., HN(alkyl)₂), trialkyl amines (i.e., N(alkyl)₃), substituted alkyl amines (i.e., NH₂(substituted alkyl)), di(substituted alkyl) amines (i.e., HN(substituted alkyl)₂), tri(substituted alkyl) amines (i.e., N(substituted alkyl)₃), alkenyl amines (i.e., NH₂(alkenyl)), dialkenyl amines (i.e., HN(alkenyl)₂), trialkenyl amines (i.e., N(alkenyl)₃), substituted alkenyl amines (i.e., NH₂(substituted alkenyl)), di(substituted alkenyl) amines (i.e., HN(substituted alkenyl)₂), tri(substituted alkenyl) amines (i.e., N(substituted alkenyl)₃, mono-, di- or tri- cycloalkyl amines (i.e., NH₂(cycloalkyl), HN(cycloalkyl)₂, N(cycloalkyl)₃), mono-, di- or tri-arylamines (i.e., NH₂(aryl), HN(aryl)₂, N(aryl)₃), or mixed amines, etc. Specific examples of suitable amines include, by way of example only, isopropylamine, trimethyl amine, diethyl amine, tri(iso-propyl) amine, tri(n-propyl) amine, ethanolamine, 2-dimethylaminoethanol, piperazine, piperidine, morpholine, N-ethylpiperidine, and the like.

Some of the compounds exist as tautomers. Tautomers are in equilibrium with one another. For example, amide containing compounds may exist in equilibrium with imidic acid tautomers. Regardless of which tautomer is shown and regardless of the nature of the equilibrium among tautomers, the compounds are understood by one of ordinary skill in the art to comprise both amide and imidic acid tautomers. Thus, the amide containing compounds are understood to include their imidic acid tautomers. Likewise, the imidic acid containing compounds are understood to include their amide tautomers.

The compounds of the disclosure, or their pharmaceutically acceptable salts include an asymmetric center and may thus give rise to enantiomers, diastereomers, and other stereoisomeric forms that may be defined, in terms of absolute stereochemistry, as (R)- or (S)- or, as (D)- or (L)- for amino acids. The present disclosure is meant to include all such possible isomers, as well as their racemic and optically pure forms. Optically active (+) and (-), *(R)-* and (S)-, or (D)- and (L)- isomers may be prepared using chiral synthons or chiral reagents, or resolved using conventional techniques, for example, chromatography and/or fractional crystallization. Conventional techniques for the preparation/isolation of individual enantiomers include chiral synthesis from a suitable optically pure precursor or resolution of the racemate (or the racemate of a salt or derivative) using, for example, chiral high pressure liquid chromatography (HPLC). When the compounds described herein contain olefinic double bonds or other centers of geometric asymmetry, and unless specified otherwise, it is intended that the compounds include both E and Z geometric isomers.

A "stereoisomer" refers to a compound made up of the same atoms bonded by the same bonds but having different three-dimensional structures, which are not interchangeable. The present disclosure contemplates various stereoisomers, or mixtures thereof, and includes "enantiomers," which refers to two stereoisomers whose molecules are nonsuperimposeable mirror images of one another.

"Diastereomers" are stereoisomers that have at least two asymmetric atoms, but which are not mirror-images of each other.

Relative centers of the compounds as depicted herein are indicated graphically using the "thick bond" style (bold or parallel lines) and absolute stereochemistry is depicted using wedge bonds (bold or parallel lines).

### 2. Compounds

Provided herein are compounds that are modulators of NLRP3. In certain embodiments, provided is a compound of Formula I: 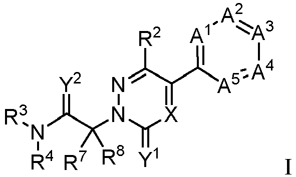 or a pharmaceutically acceptable salt, isotopically enriched analog, stereoisomer, or mixture of stereoisomers, wherein:
each of A¹, A², A³, A⁴, and A⁵ is independently N or CR¹;
X is N or CR⁵;
each of Y¹ and Y² is independently O or S;
each R' is independently hydrogen, halo, cyano, hydroxy, -SH, -NH₂, -NO₂, -SF₅, C₁₋₆ alkyl, C₂₋₆ alkenyl, C₂₋₆ alkynyl, C₁₋₆ haloalkyl, C₂₋₆ heteroalkyl, C₃₋₁₀ cycloalkyl, heterocyclyl, aryl, heteroaryl, -N(R¹¹)₂, -OR¹¹, -C(O)R¹¹, -C(O)OR¹¹, -S(O)₀₋₂R¹¹, -NR¹¹S(O)₀₋₂-R¹¹, -S(O)₀₋₂N(R¹¹)₂, -NR¹¹S(O)₀₋₂N(R¹¹)₂, -NR¹¹C(O)N(R¹¹)₂, -C(O)N(R¹¹)₂, -NR¹¹C(O)R¹¹, -OC(O)N(R¹¹)₂, or -NR¹¹C(O)OR¹¹; wherein each C₁₋₆alkyl, C₂₋₆ alkenyl, C₂₋₆alkynyl, C₁₋₆ haloalkyl, C₂₋₆ heteroalkyl, C₃₋₁₀ cycloalkyl, heterocyclyl, aryl, or heteroaryl is independently optionally substituted with one to five Z¹; and wherein any two adjacent R¹ groups can join to form a C₃₋₁₀ cycloalkyl, heterocyclyl, aryl, or heteroaryl ring, which C₃₋₁₀ cycloalkyl, heterocyclyl, aryl, or heteroaryl ring may further be independently optionally substituted with one to five Z¹;
R² is -C(R⁶)₂R¹⁰, -OR⁹, -N(R⁶)(R⁹), -SR⁹, -S(O)R⁹, -S(O)₂R⁹, -OC(O)N(R⁶)(R⁹), -NR⁶C(O)OR⁹, -NR⁶C(O)R⁹, C₃₋₁₀ cycloalkyl, heterocyclyl, or halo; wherein the C₃₋₁₀ cycloalkyl or heterocyclyl is independently optionally substituted with one to five Z¹;
R³ is hydrogen, C₁₋₆ alkyl, C₂₋₆ alkenyl, C₂₋₆ alkynyl, C₁₋₆ haloalkyl, C₁₋₆ alkoxy, C₂₋₆ heteroalkyl, C₃₋₁₀ cycloalkyl, heterocyclyl, aryl, or heteroaryl; wherein the C₁₋₆ alkyl, C₂₋₆ alkenyl, C₂₋₆ alkynyl, C₁₋₆ haloalkyl, C₁₋₆ alkoxy, C₂₋₆ heteroalkyl, C₃₋₁₀ cycloalkyl, heterocyclyl, aryl, or heteroaryl is independently optionally substituted with one to five Z¹;
R⁴ is hydrogen, C₁₋₆ alkyl, C₂₋₆ alkenyl, C₂₋₆ alkynyl, C₁₋₆ haloalkyl, C₁₋₆ alkoxy, C₂₋₆ heteroalkyl, C₃₋₁₀ cycloalkyl, heterocyclyl, aryl, or heteroaryl; wherein the C₁₋₆ alkyl, C₂₋₆ alkenyl, C₂₋₆ alkynyl, C₁₋₆ haloalkyl, C₁₋₆ alkoxy, C₂₋₆ heteroalkyl, C₃₋₁₀ cycloalkyl, heterocyclyl, aryl, or heteroaryl is independently optionally substituted with one to five Z¹;
or R³ and R⁴ join to form a heterocyclyl or heteroaryl ring; wherein the heterocyclyl or heteroaryl ring may further be independently optionally substituted with one to five Z¹;
R⁵ is hydrogen, halo, C₁₋₆ alkyl, C₁₋₆ haloalkyl, C₁₋₆ alkoxy, C₁₋₆ haloalkoxy, C₃₋₁₀ cycloalkyl, or heterocyclyl; wherein the C₁₋₆ alkyl, C₁₋₆ haloalkyl, C₁₋₆ alkoxy, C₁₋₆ haloalkoxy, C₃₋₁₀ cycloalkyl, or heterocyclyl is independently optionally substituted with one to five Z¹;
each R⁶ is independently hydrogen, halo, cyano, hydroxy, C₁₋₆ alkyl, C₂₋₆ alkenyl, C₂₋₆ alkynyl, C₁₋₆ haloalkyl, C₁₋₆ alkoxy, C₁₋₆ haloalkoxy, C₂₋₆ heteroalkyl, C₃₋₁₀ cycloalkyl, heterocyclyl, aryl, or heteroaryl;
R⁷ is hydrogen, halo, cyano, hydroxy, C₁₋₆ alkyl, C₂₋₆ alkenyl, C₂₋₆ alkynyl, C₁₋₆ haloalkyl, C₁₋₆ alkoxy, C₁₋₆ haloalkoxy, C₂₋₆ heteroalkyl, C₃₋₁₀ cycloalkyl, or heterocyclyl;
R⁸ is hydrogen, halo, cyano, hydroxy, C₁₋₆ alkyl, C₂₋₆ alkenyl, C₂₋₆ alkynyl, C₁₋₆ haloalkyl, C₁₋₆ alkoxy, C₁₋₆ haloalkoxy, C₂₋₆ heteroalkyl, C₃₋₁₀ cycloalkyl, or heterocyclyl;
or R⁷ and R⁸ join to form a C₃₋₁₀ cycloalkyl or heterocyclyl ring; wherein the C₃₋₁₀ cycloalkyl or heterocyclyl ring may further be independently optionally substituted with one to five Z^{1a};
R⁹ is C₁₋₆ alkyl, C₂₋₆ alkenyl, C₂₋₆ alkynyl, C₁₋₆ haloalkyl, C₂₋₆ heteroalkyl, C₁₋₆ alkoxy, C₁₋₆ haloalkoxy, C₃₋₁₀ cycloalkyl, heterocyclyl, aryl, or heteroaryl; wherein the C₁₋₆ alkyl, C₂₋₆ alkenyl, C₂₋₆ alkynyl, C₁₋₆ haloalkyl, C₂₋₆ heteroalkyl, C₁₋₆ alkoxy, C₁₋₆ haloalkoxy, C₃₋₁₀ cycloalkyl, heterocyclyl, aryl, or heteroaryl is independently optionally substituted with one to five Z¹; or
R¹⁰ is hydrogen, halo, hydroxy, C₁₋₆ alkyl, C₂₋₆ alkenyl, C₂₋₆ alkynyl, C₁₋₆ haloalkyl, C₂₋₆ heteroalkyl, C₃₋₁₀ cycloalkyl, heterocyclyl, aryl, or heteroaryl; wherein the C₁₋₆ alkyl, C₂₋₆ alkenyl, C₂₋₆ alkynyl, C₁₋₆ haloalkyl, C₂₋₆ heteroalkyl, C₃₋₁₀ cycloalkyl, heterocyclyl, aryl, or heteroaryl is independently optionally substituted with one to five Z¹;
each Z¹ is independently halo, cyano, hydroxy, -SH, -NH₂, -NO₂, -SF₅, C₁₋₆ alkyl, C₂₋₆ alkenyl, C₂₋₆ alkynyl, C₁₋₆ haloalkyl, C₂₋₆ heteroalkyl, C₃₋₁₀ cycloalkyl, heterocyclyl, aryl, heteroaryl, -N(R¹¹)₂, -OR¹¹, -C(O)R¹¹, -C(O)OR¹¹, -S(O)₀₋₂R¹¹, -NR¹¹S(O)₀₋₂-R¹¹, -S(O)₀₋₂N(R¹¹)₂, -NR¹¹S(O)₀₋₂N(R¹¹)₂, -NR¹¹C(O)N(R¹¹)₂, -C(O)N(R¹¹)₂, -NR¹¹C(O)R¹¹, -OC(O)N(R¹¹)₂, or -NR¹¹C(O)OR¹¹; wherein each C₁₋₆ alkyl, C₂₋₆ alkenyl, C₂₋₆ alkynyl, C₁₋₆ haloalkyl, C₂₋₆ heteroalkyl, C₃₋₁₀ cycloalkyl, heterocyclyl, aryl, or heteroaryl is independently optionally substituted with one to five Z^{1a};
each R¹¹ is independently hydrogen, C₁₋₆ alkyl, C₂₋₆ alkenyl, C₂₋₆ alkynyl, C₁₋₆ haloalkyl, C₂₋₆ heteroalkyl, C₃₋₁₀ cycloalkyl, heterocyclyl, aryl, or heteroaryl; wherein each C₁₋₆ alkyl, C₂₋₆ alkenyl, C₂₋₆ alkynyl, C₁₋₆ haloalkyl, C₂₋₆ heteroalkyl, C₃₋₁₀ cycloalkyl, heterocyclyl, aryl, or heteroaryl of R¹¹ is independently optionally substituted with one to five Z^{1a};
each Z^{1a} is independently hydroxy, halo, cyano, hydroxy, -SH, -NH₂, -NO₂, -SF₅, C₁₋₆ alkyl, C₂₋₆ alkenyl, C₂₋₆ alkynyl, C₁₋₆ haloalkyl, C₁₋₆ alkoxy, C₁₋₆ haloalkoxy, C₃₋₁₀ cycloalkyl, heterocyclyl, aryl, heteroaryl, -N(R¹²)₂, -OR¹², -C(O)R¹², -C(O)OR¹², -S(O)₀₋₂R¹², -NR¹²S(O)₀₋₂-R¹², -S(O)₀₋₂N(R¹²)₂, -NR¹²S(O)₀₋₂N(R¹²)₂, -NR¹²C(O)N(R¹²)₂, -C(O)N(R¹²)₂, -NR¹²C(O)R¹², -OC(O)N(R¹²)₂, or -NR¹²C(O)OR¹²; wherein each C₁₋₆ alkyl, C₂₋₆ alkenyl, C₂₋₆ alkynyl, C₁₋₆ haloalkyl, C₁₋₆ alkoxy, C₁₋₆ haloalkoxy, C₃₋₁₀ cycloalkyl, heterocyclyl, aryl, or heteroaryl is independently optionally substituted with one to five Z^{1b};
each R¹² is independently hydrogen, C₁₋₆ alkyl, C₂₋₆ alkenyl, C₂₋₆ alkynyl, C₁₋₆ haloalkyl, C₂₋₆ heteroalkyl, C₃₋₁₀ cycloalkyl, heterocyclyl, aryl, or heteroaryl; wherein each C₁₋₆ alkyl, C₂₋₆ alkenyl, C₂₋₆ alkynyl, C₁₋₆ haloalkyl, C₂₋₆ heteroalkyl, C₃₋₁₀ cycloalkyl, heterocyclyl, aryl, or heteroaryl of R¹² is independently optionally substituted with one to five Z^{1b};
each Z^{1b} is independently halo, cyano, hydroxy, -SH, -NH₂, -NO₂, -SF₅, C₁₋₆ alkyl, C₂₋₆ alkenyl, C₂₋₆ alkynyl, C₁₋₆ haloalkyl, C₁₋₆ alkoxy, C₂₋₆ heteroalkyl, C₁₋₆ haloalkoxy, C₃₋₁₀ cycloalkyl, heterocyclyl, aryl, heteroaryl, -L-C₁₋₆ alkyl, -L-C₂₋₆ alkenyl, -L-C₂₋₆ alkynyl, -L-C₁₋₆ haloalkyl, -L-C₃₋₁₀ cycloalkyl, -L-heterocyclyl, -L-aryl, or -L-heteroaryl; and
each L is independently -O-, -NH-, -S-, -S(O)-, -S(O)₂-, -N(C₁₋₆ alkyl)-, -N(C₂₋₆ alkenyl)-, -N(C₂₋₆ alkynyl)-, -N(C₁₋₆ haloalkyl)-, -N(C₃₋₁₀ cycloalkyl)-, -N(heterocyclyl)-, -N(aryl)-, -N(heteroaryl)-, -C(O)-, -C(O)O-, -C(O)NH-, -C(O)N(C₁₋₆ alkyl)-, -C(O)N(C₂₋₆ alkenyl)-, -C(O)N(C₂₋₆ alkynyl)-, -C(O)N(C₁₋₆ haloalkyl)-, -C(O)N(C₃₋₁₀ cycloalkyl)-, -C(O)N(heterocyclyl)-, -C(O)N(aryl)-, -C(O)N(heteroaryl)-, -NHC(O)-, -NHC(O)O-, -NHC(O)NH-, -NHS(O)-, or -S(O)₂NH-;
wherein each C₁₋₆ alkyl, C₂₋₆ alkenyl, C₂₋₆ alkynyl, C₁₋₆ haloalkyl, C₁₋₆ alkoxy, C₁₋₆ haloalkoxy, C₃₋₁₀ cycloalkyl, heterocyclyl, aryl, and heteroaryl of Z^{1b} and L is further independently optionally substituted with one to five hydroxy, halo, cyano, hydroxy, -SH, -NH₂, -NO₂, -SF₅, C₁₋₆ alkyl, C₂₋₆ alkenyl, C₂₋₆ alkynyl, C₁₋₆ haloalkyl, C₁₋₆ alkoxy, C₁₋₆ haloalkoxy, C₃₋₁₀ cycloalkyl, heterocyclyl, aryl, or heteroaryl.

In certain embodiments, the compound is not N-(4-bromophenyl)-2-[3-methyl-6-oxo-4-phenylpyridazin-1(6H)-yl]acetamide, N-(4-bromophenyl)-5-[(3-methoxyphenyl)methyl]-3-methyl-6-oxo-4-phenyl-1(6H)-pyridazineacetamide, 4-[[2-[4-(2-acetyl-5-chlorophenyl)-3-methoxy-6-oxo-1(6H)-pyridazinyl]-4-methyl-1-oxopentyl]amino]-benzoic acid 1,1-dimethylethyl ester, or 4-[[2-[4-(2-acetyl-5-chlorophenyl)-3-methoxy-6-oxo-1(6H)-pyridazinyl]-4-methyl-1-oxopentyl]amino]-benzoic acid.

In certain embodiments, Y¹ is O. In certain embodiments, Y¹ is S.

In certain embodiments, Y² is O. In certain embodiments, Y² is S.

In certain embodiments, Y¹ is O and Y² is O. In certain embodiments, Y¹ is O and Y² is S.

In certain embodiments, Y¹ is S and Y² is O. In certain embodiments, Y¹ is S and Y² is S.

In certain embodiments, provided is a compound of Formula IA: 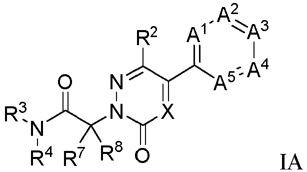 or a pharmaceutically acceptable salt, isotopically enriched analog, stereoisomer, or mixture of stereoisomers, wherein:
each of A¹, A², A³, A⁴, and A⁵ is independently N or CR¹;
X is N or CR⁵;
each R¹ is independently hydrogen, halo, cyano, hydroxy, -SH, -NH₂, -NO₂, -SF₅, C₁₋₆ alkyl, C₂₋₆ alkenyl, C₂₋₆ alkynyl, C₁₋₆ haloalkyl, C₂₋₆ heteroalkyl, C₃₋₁₀ cycloalkyl, heterocyclyl, aryl, heteroaryl, -N(R¹¹)₂, -OR¹¹, -C(O)R¹¹, -C(O)OR¹¹, -S(O)₀₋₂R¹¹, -NR¹¹S(O)₀₋₂-R¹¹, -S(O)₀₋₂N(R¹¹)₂, -NR¹¹S(O)₀₋₂N(R¹¹)₂, -NR¹¹C(O)N(R¹¹)_{2,} -C(O)N(R¹¹)₂, -NR¹¹C(O)R¹¹, -OC(O)N(R¹¹)₂, or -NR¹¹C(O)OR¹¹; wherein any two adjacent R¹ groups can join to form a C₃₋₁₀ cycloalkyl, heterocyclyl, aryl, or heteroaryl ring;
R² is -C(R⁶)₂R¹⁰, -OR⁹, -N(R⁶)(R⁹), -SR⁹, -S(O)R⁹, -S(O)₂R⁹, -OC(O)N(R⁶)(R⁹), -NR⁶C(O)OR⁹, -NR⁶C(O)R⁹, C₃₋₁₀ cycloalkyl, heterocyclyl, or halo;
R³ is hydrogen, C₁₋₆ alkyl, C₂₋₆ alkenyl, C₂₋₆ alkynyl, C₁₋₆ haloalkyl, C₁₋₆ alkoxy, C₂₋₆ heteroalkyl, C₃₋₁₀ cycloalkyl, heterocyclyl, aryl, or heteroaryl;
R⁴ is hydrogen, C₁₋₆ alkyl, C₂₋₆ alkenyl, C₂₋₆ alkynyl, C₁₋₆ haloalkyl, C₁₋₆ alkoxy, C₂₋₆ heteroalkyl, C₃₋₁₀ cycloalkyl, heterocyclyl, aryl, or heteroaryl;
or R³ and R⁴ join to form a heterocyclyl or heteroaryl ring;
R⁵ is hydrogen, halo, C₁₋₆ alkyl, C₁₋₆haloalkyl, C₁₋₆alkoxy, C₁₋₆ haloalkoxy, C₃₋₁₀ cycloalkyl, or heterocyclyl;
each R⁶ is independently hydrogen, halo, cyano, hydroxy, C₁₋₆ alkyl, C₂₋₆ alkenyl, C₂₋₆ alkynyl, C₁₋₆ haloalkyl, C₁₋₆ alkoxy, C₁₋₆ haloalkoxy, C₂₋₆ heteroalkyl, C₃₋₁₀ cycloalkyl, heterocyclyl, aryl, or heteroaryl;
R⁷ is hydrogen, halo, cyano, hydroxy, C₁₋₆ alkyl, C₂₋₆ alkenyl, C₂₋₆ alkynyl, C₁₋₆ haloalkyl, C₁₋₆ alkoxy, C₁₋₆ haloalkoxy, C₂₋₆ heteroalkyl, C₃₋₁₀ cycloalkyl, or heterocyclyl;
R⁸ is hydrogen, halo, cyano, hydroxy, C₁₋₆ alkyl, C₂₋₆ alkenyl, C₂₋₆ alkynyl, C₁₋₆ haloalkyl, C₁₋₆ alkoxy, C₁₋₆ haloalkoxy, C₂₋₆ heteroalkyl, C₃₋₁₀ cycloalkyl, or heterocyclyl;
or R⁷ and R⁸ join to form a C₃₋₁₀ cycloalkyl or heterocyclyl ring;
R⁹ is C₁₋₆ alkyl, C₂₋₆ alkenyl, C₂₋₆ alkynyl, C₁₋₆ haloalkyl, C₂₋₆ heteroalkyl, C₁₋₆ alkoxy, C₁₋₆ haloalkoxy, C₃₋₁₀ cycloalkyl, heterocyclyl, aryl, or heteroaryl; or
R¹⁰ is hydrogen, halo, hydroxy, C₁₋₆ alkyl, C₂₋₆ alkenyl, C₂₋₆ alkynyl, C₁₋₆ haloalkyl, C₂₋₆ heteroalkyl, C₃₋₁₀ cycloalkyl, heterocyclyl, aryl, or heteroaryl;
each R¹¹ is independently hydrogen, C₁₋₆ alkyl, C₂₋₆ alkenyl, C₂₋₆ alkynyl, C₁₋₆ haloalkyl, C₂₋₆ heteroalkyl, C₃₋₁₀ cycloalkyl, heterocyclyl, aryl, or heteroaryl;
wherein each alkyl, alkenyl, alkynyl, haloalkyl, heteroalkyl, alkoxy, haloalkoxy cycloalkyl, heterocyclyl, aryl, and heteroaryl of the above listed substituents is independently optionally substituted.

**In** certain embodiments, provided is a compound of Formula IA: 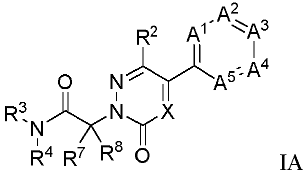 or a pharmaceutically acceptable salt, isotopically enriched analog, stereoisomer, or mixture of stereoisomers, wherein:
each of A¹, A², A³, A⁴, and A⁵ is independently N or CR¹;
X is N or CR⁵;
each R' is independently hydrogen, halo, cyano, hydroxy, -SH, -NH₂, -NO₂, -SF₅, C₁₋₆ alkyl, C₂₋₆ alkenyl, C₂₋₆ alkynyl, C₁₋₆ haloalkyl, C₂₋₆ heteroalkyl, C₃₋₁₀ cycloalkyl, heterocyclyl, aryl, heteroaryl, -N(R¹¹)₂, -OR¹¹, -C(O)R¹¹, -C(O)OR¹¹, -S(O)₀₋₂R¹¹, -NR¹¹S(O)₀₋₂-R¹¹, -S(O)₀₋₂N(R¹¹)₂, -NR¹¹S(O)₀₋₂N(R¹¹)₂, -NR¹¹C(O)N(R¹¹)₂, -C(O)N(R¹¹)₂, -NR¹¹C(O)R¹¹, -OC(O)N(R¹¹)₂, or -NR¹¹C(O)OR¹¹; wherein each C₁₋₆ alkyl, C₂₋₆ alkenyl, C₂₋₆ alkynyl, C₁₋₆ haloalkyl, C₂₋₆ heteroalkyl, C₃₋₁₀ cycloalkyl, heterocyclyl, aryl, or heteroaryl is independently optionally substituted with one to five Z¹; and wherein any two adjacent R¹ groups can join to form a C₃₋₁₀ cycloalkyl, heterocyclyl, aryl, or heteroaryl ring, which C₃₋₁₀ cycloalkyl, heterocyclyl, aryl, or heteroaryl ring may further be independently optionally substituted with one to five Z¹;
R² is -C(R⁶)₂R¹⁰, -OR⁹, -N(R⁶)(R⁹), -SR⁹, -S(O)R⁹, -S(O)₂R⁹, -OC(O)N(R⁶)(R⁹), -NR⁶C(O)OR⁹, -NR⁶C(O)R⁹, C₃₋₁₀ cycloalkyl, heterocyclyl, or halo; wherein the C₃₋₁₀ cycloalkyl or heterocyclyl is independently optionally substituted with one to five Z¹;
R³ is hydrogen, C₁₋₆ alkyl, C₂₋₆ alkenyl, C₂₋₆ alkynyl, C₁₋₆ haloalkyl, C₁₋₆ alkoxy, C₂₋₆ heteroalkyl, C₃₋₁₀ cycloalkyl, heterocyclyl, aryl, or heteroaryl; wherein the C₁₋₆ alkyl, C₂₋₆ alkenyl, C₂₋₆ alkynyl, C₁₋₆ **haloalkyl,** C₁₋₆ alkoxy, C₂₋₆ heteroalkyl, C₃₋₁₀ cycloalkyl, heterocyclyl, aryl, or heteroaryl is independently optionally substituted with one to five Z¹;
R⁴ is hydrogen, C₁₋₆ alkyl, C₂₋₆ alkenyl, C₂₋₆ alkynyl, C₁₋₆ haloalkyl, C₁₋₆ alkoxy, C₂₋₆ heteroalkyl, C₃₋₁₀ cycloalkyl, heterocyclyl, aryl, or heteroaryl; wherein the C₁₋₆ alkyl, C₂₋₆ alkenyl, C₂₋₆ alkynyl, C₁₋₆ haloalkyl, C₁₋₆ alkoxy, C₂₋₆ heteroalkyl, C₃₋₁₀ cycloalkyl, heterocyclyl, aryl, or heteroaryl is independently optionally substituted with one to five Z¹;
or R³ and R⁴ join to form a heterocyclyl or heteroaryl ring; wherein the heterocyclyl or heteroaryl ring may further be independently optionally substituted with one to five Z¹;
R⁵ is hydrogen, halo, C₁₋₆ alkyl, C₁₋₆ haloalkyl, C₁₋₆ alkoxy, C₁₋₆ haloalkoxy, C₃₋₁₀ cycloalkyl, or heterocyclyl; wherein the C₁₋₆ alkyl, C₁₋₆ haloalkyl, C₁₋₆ alkoxy, C₁₋₆ haloalkoxy, C₃₋₁₀ cycloalkyl, or heterocyclyl is independently optionally substituted with one to five Z¹;
each R⁶ is independently hydrogen, halo, cyano, hydroxy, C₁₋₆ alkyl, C₂₋₆ alkenyl, C₂₋₆ alkynyl, C₁₋₆ haloalkyl, C₁₋₆ alkoxy, C₁₋₆ haloalkoxy, C₂₋₆ heteroalkyl, C₃₋₁₀ cycloalkyl, heterocyclyl, aryl, or heteroaryl;
R⁷ is hydrogen, halo, cyano, hydroxy, C₁₋₆ alkyl, C₂₋₆ alkenyl, C₂₋₆ alkynyl, C₁₋₆ haloalkyl, C₁₋₆ alkoxy, C₁₋₆ haloalkoxy, C₂₋₆ heteroalkyl, C₃₋₁₀ cycloalkyl, or heterocyclyl;
R⁸ is hydrogen, halo, cyano, hydroxy, C₁₋₆ alkyl, C₂₋₆ alkenyl, C₂₋₆ alkynyl, C₁₋₆ haloalkyl, C₁₋₆ alkoxy, C₁₋₆ haloalkoxy, C₂₋₆ heteroalkyl, C₃₋₁₀ cycloalkyl, or heterocyclyl;
or R⁷ and R⁸ join to form a C₃₋₁₀ cycloalkyl or heterocyclyl ring; wherein the C₃₋₁₀ cycloalkyl or heterocyclyl ring may further be independently optionally substituted with one to five Z^{1a};
R⁹ is C₁₋₆ alkyl, C₂₋₆ alkenyl, C₂₋₆ alkynyl, C₁₋₆ haloalkyl, C₂₋₆ heteroalkyl, C₁₋₆ alkoxy, C₁₋₆ haloalkoxy, C₃₋₁₀ cycloalkyl, heterocyclyl, aryl, or heteroaryl; wherein the C₁₋₆ alkyl, C₂₋₆ alkenyl, C₂₋₆ alkynyl, C₁₋₆ haloalkyl, C₂₋₆ heteroalkyl, C₁₋₆ alkoxy, C₁₋₆ haloalkoxy, C₃₋₁₀ cycloalkyl, heterocyclyl, aryl, or heteroaryl is independently optionally substituted with one to five Z¹; or
R¹⁰ is hydrogen, halo, hydroxy, C₁₋₆ alkyl, C₂₋₆ alkenyl, C₂₋₆ alkynyl, C₁₋₆ haloalkyl, C₂₋₆ heteroalkyl, C₃₋₁₀ cycloalkyl, heterocyclyl, aryl, or heteroaryl; wherein the C₁₋₆ alkyl, C₂₋₆ alkenyl, C₂₋₆ alkynyl, C₁₋₆ haloalkyl, C₂₋₆ heteroalkyl, C₃₋₁₀ cycloalkyl, heterocyclyl, aryl, or heteroaryl is independently optionally substituted with one to five Z¹;
each Z¹ is independently halo, cyano, hydroxy, -SH, -NH₂, -NO₂, -SF₅, C₁₋₆ alkyl, C₂₋₆ alkenyl, C₂₋₆ alkynyl, C₁₋₆ haloalkyl, C₂₋₆ heteroalkyl, C₃₋₁₀ cycloalkyl, heterocyclyl, aryl, heteroaryl, -N(R¹¹)₂, -OR¹¹, -C(O)R¹¹, -C(O)OR¹¹, -S(O)₀₋₂R¹¹, -NR¹¹S(O)₀₋₂-R¹¹, -S(O)₀₋₂N(R¹¹)₂, -NR¹¹S(O)₀₋₂N(R¹¹)₂, -NR¹¹C(O)N(R¹¹)₂, -C(O)N(R¹¹)₂, -NR¹¹C(O)R¹¹, -OC(O)N(R¹¹)₂, or -NR¹¹C(O)OR¹¹; wherein each C₁₋₆ alkyl, C₂₋₆ alkenyl, C₂₋₆ alkynyl, C₁₋₆ haloalkyl, C₂₋₆ heteroalkyl, C₃₋₁₀ cycloalkyl, heterocyclyl, aryl, or heteroaryl is independently optionally substituted with one to five Z^{1a};
each R¹¹ is independently hydrogen, C₁₋₆ alkyl, C₂₋₆ alkenyl, C₂₋₆ alkynyl, C₁₋₆ haloalkyl, C₂₋₆ heteroalkyl, C₃₋₁₀ cycloalkyl, heterocyclyl, aryl, or heteroaryl; wherein each C₁₋₆ alkyl, C₂₋₆ alkenyl, C₂₋₆ alkynyl, C₁₋₆ haloalkyl, C₂₋₆ heteroalkyl, C₃₋₁₀ cycloalkyl, heterocyclyl, aryl, or heteroaryl of R¹¹ is independently optionally substituted with one to five Z^{1a};
each Z^{1a} is independently hydroxy, halo, cyano, hydroxy, -SH, -NH₂, -NO₂, -SF₅, C₁₋₆ alkyl, C₂₋₆ alkenyl, C₂₋₆ alkynyl, C₁₋₆ haloalkyl, C₁₋₆ alkoxy, C₁₋₆ haloalkoxy, C₃₋₁₀ cycloalkyl, heterocyclyl, aryl, heteroaryl, -N(R¹²)₂, -OR¹², -C(O)R¹², -C(O)OR¹², -S(O)₀₋₂R¹², -NR¹²S(O)₀₋₂-R¹², -S(O)₀₋₂N(R¹²)₂, -NR¹²S(O)₀₋₂N(R¹²)₂, -NR¹²C(O)N(R¹²)₂, -C(O)N(R¹²)₂, -NR¹²C(O)R¹², -OC(O)N(R¹²)₂, or -NR¹²C(O)OR¹²; wherein each C₁₋₆ alkyl, C₂₋₆ alkenyl, C₂₋₆ alkynyl, C₁₋₆ haloalkyl, C₁₋₆ alkoxy, C₁₋₆ haloalkoxy, C₃₋₁₀ cycloalkyl, heterocyclyl, aryl, or heteroaryl is independently optionally substituted with one to five Z^{1b};
each R¹² is independently hydrogen, C₁₋₆ alkyl, C₂₋₆ alkenyl, C₂₋₆ alkynyl, C₁₋₆ haloalkyl, C₂₋₆ heteroalkyl, C₃₋₁₀ cycloalkyl, heterocyclyl, aryl, or heteroaryl; wherein each C₁₋₆ alkyl, C₂₋₆ alkenyl, C₂₋₆ alkynyl, C₁₋₆ haloalkyl, C₂₋₆ heteroalkyl, C₃₋₁₀ cycloalkyl, heterocyclyl, aryl, or heteroaryl of R¹² is independently optionally substituted with one to five Z^{1b};
each Z^{1b} is independently halo, cyano, hydroxy, -SH, -NH₂, -NO₂, -SF₅, C₁₋₆ alkyl, C₂₋₆ alkenyl, C₂₋₆ alkynyl, C₁₋₆ haloalkyl, C₁₋₆ alkoxy, C₂₋₆ heteroalkyl, C₁₋₆ haloalkoxy, C₃₋₁₀ cycloalkyl, heterocyclyl, aryl, heteroaryl, -L-C₁₋₆ alkyl, -L-C₂₋₆ alkenyl, -L-C₂₋₆ alkynyl, -L-C₁₋₆ haloalkyl, -L-C₃₋₁₀ cycloalkyl, -L-heterocyclyl, -L-aryl, or -L-heteroaryl; and
each L is independently -O-, -NH-, -S-, -S(O)-, -S(O)₂-, -N(C₁₋₆ alkyl)-, -N(C₂₋₆ alkenyl)-, -N(C₂₋₆ alkynyl)-, -N(C₁₋₆ haloalkyl)-, -N(C₃₋₁₀ cycloalkyl)-, -N(heterocyclyl)-, -N(aryl)-, -N(heteroaryl)-, -C(O)-, -C(O)O-, -C(O)NH-, -C(O)N(C₁₋₆ alkyl)-, -C(O)N(C₂₋₆ alkenyl)-, -C(O)N(C₂₋₆ alkynyl)-, -C(O)N(C₁₋₆ haloalkyl)-, -C(O)N(C₃₋₁₀ cycloalkyl)-, -C(O)N(heterocyclyl)-, -C(O)N(aryl)-, -C(O)N(heteroaryl)-, -NHC(O)-, -NHC(O)O-, -NHC(O)NH-, -NHS(O)-, or -S(O)₂NH-;
wherein each C₁₋₆ alkyl, C₂₋₆ alkenyl, C₂₋₆ alkynyl, C₁₋₆ haloalkyl, C₁₋₆ alkoxy, C₁₋₆ haloalkoxy, C₃₋₁₀ cycloalkyl, heterocyclyl, aryl, and heteroaryl of Z^{1b} and L is further independently optionally substituted with one to five hydroxy, halo, cyano, hydroxy, -SH, -NH₂, -NO₂, -SF₅, C₁₋₆ alkyl, C₂₋₆ alkenyl, C₂₋₆ alkynyl, C₁₋₆ haloalkyl, C₁₋₆ alkoxy, C₁₋₆ haloalkoxy, C₃₋₁₀ cycloalkyl, heterocyclyl, aryl, or heteroaryl.

In certain embodiments, the compound is not N-(4-bromophenyl)-2-[3-methyl-6-oxo-4-phenylpyridazin-1(6H)-yl]acetamide:

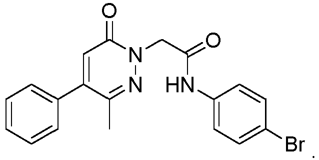

In certain embodiments, the compound is not N-(4-bromophenyl)-5-[(3-methoxyphenyl)methyl]-3-methyl-6-oxo-4-phenyl-1(6H)-pyridazineacetamide:

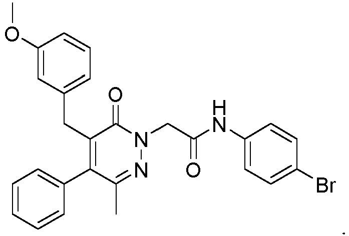

In certain embodiments, the compound is not 4-[[2-[4-(2-acetyl-5-chlorophenyl)-3-methoxy-6-oxo-1(6H)-pyridazinyl]-4-methyl-1-oxopentyl]amino]-benzoic acid 1,1-dimethylethyl ester:

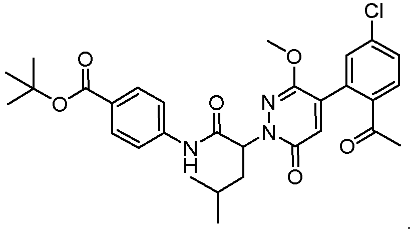

In certain embodiments, the compound is not 4-[[2-[4-(2-acetyl-5-chlorophenyl)-3-methoxy-6-oxo-1(6H)-pyridazinyl]-4-methyl-1-oxopentyl]amino]-benzoic acid:

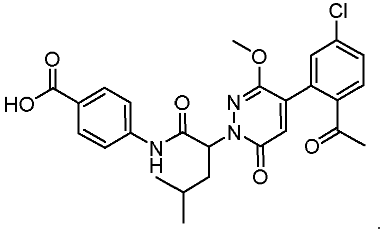

In certain embodiments, each of A¹, A², A³, A⁴, and A⁵ is independently CR¹.

In certain embodiments, one of A¹, A², A³, A⁴, and A⁵ is N and the remaining A¹, A², A³, A⁴, and A⁵ are independently CR¹. In certain embodiments, A¹ is N and the remaining A², A³, A⁴, and A⁵ are independently CR¹. In certain embodiments, A² is N and the remaining A¹, A³, A⁴, and A⁵ are independently CR¹. In certain embodiments, A³ is N and the remaining A¹, A², A⁴, and A⁵ are independently CR¹.

In certain embodiments, two of A¹, A², A³, A⁴, and A⁵ are N and the remaining A¹, A², A³, A⁴, and A⁵ are independently CR¹.

In certain embodiments, X is N.

In certain embodiments, X is CR⁵.

In certain embodiments, R⁵ is hydrogen. In certain embodiments, R⁵ is hydrogen, C₁₋₆ alkyl, or C₁₋₆ haloalkyl.

In certain embodiments, each R' is independently hydrogen, halo, cyano, C₁₋₆ alkyl, C₁₋₆ alkoxy, C₁₋₆ haloalkyl, or C₃₋₁₀ cycloalkyl; wherein the C₃₋₁₀ cycloalkyl may further be independently optionally substituted with one to five Z¹, or any two adjacent R¹ groups can join to form an aryl or heteroaryl ring. In certain embodiments, each R¹ is independently hydrogen, halo, cyano, C₁₋₆ alkyl, C₁₋₆ alkoxy, C₁₋₆ haloalkyl, or C₃₋₁₀ cycloalkyl; wherein the C₃₋₁₀ cycloalkyl may further be independently optionally substituted with one to five halo, or any two adjacent R¹ groups can join to form an aryl or heteroaryl ring. In certain embodiments, each R¹ is independently hydrogen, halo, cyano, C₁₋₆ alkyl, C₁₋₆ alkoxy, or C₁₋₆ haloalkyl.

In certain embodiments, each R¹ is independently hydrogen, fluoro, chloro, cyano, -CH₃, -OCH₃, -OCH₂CH₃, -CF₃, cyclopropyl, 2,2-difluorocyclopropyl, or cyclobutyl. In certain embodiments, each R¹ is independently hydrogen, fluoro, chloro, cyano, -CH₃, -OCH₃, or -CF₃.

In certain embodiments, each R¹ is independently hydrogen or any two adjacent R¹ groups can join to form an aryl or heteroaryl ring. In certain embodiments, each R¹ is independently hydrogen or any two adjacent R¹ groups can join to form an aryl ring. In certain embodiments, each R¹ is independently hydrogen or any two adjacent R¹ groups can join to form a phenyl ring. In certain embodiments, each R¹ is independently hydrogen or any two adjacent R¹ groups can join to form a heteroaryl ring. In certain embodiments, each R¹ is independently hydrogen or any two adjacent R¹ groups can join to form a pyridyl ring.

In certain embodiments, at least one R¹ is other than hydrogen.

In certain embodiments, each of A¹, A², A³, A⁴, and A⁵ is independently CR¹, wherein at least one R¹ is other than hydrogen. In certain embodiments, each of A¹, A², A³, A⁴, and A⁵ is independently CR¹, wherein at least two R¹ are other than hydrogen. In certain embodiments, each of A¹, A², A³, A⁴, and A⁵ is independently CR¹, and at least two R¹ are independently halo.

In certain embodiments, R² is -C(R⁶)₂R¹⁰, -OR⁹, C₃₋₁₀ cycloalkyl, or halo; wherein the C₃₋₁₀ cycloalkyl is independently optionally substituted with one to five Z¹. In certain embodiments, R² is -C(R⁶)₂R¹⁰, -OR⁹, C₃₋₁₀ cycloalkyl, or halo; wherein the C₃₋₁₀ cycloalkyl is independently optionally substituted with one to five halo. In certain embodiments, R² is -C(R⁶)₂R¹⁰, -OR⁹, C₃₋₁₀ cycloalkyl, or halo; wherein the C₃₋₁₀ cycloalkyl is independently optionally substituted with one to five halo; at least one R⁶ is hydrogen; R¹⁰ is halo, C₁₋₆ alkyl, or C₁₋₆ haloalkyl; and R⁹ is C₁₋₆ alkyl.

In certain embodiments, R² is -C(R⁶)₂R¹⁰ or -OR⁹. In certain embodiments, R² is -C(R⁶)₂R¹⁰. In certain embodiments, R² is -OR⁹. In certain embodiments, R² is C₃₋₁₀ cycloalkyl; wherein the C₃₋₁₀ cycloalkyl is independently optionally substituted with one to five halo. In certain embodiments, R² is halo. In certain embodiments, R² is isopropyl.

In certain embodiments, R³ is C₃₋₁₀ cycloalkyl, heterocyclyl or heteroaryl; wherein the C₃₋₁₀ cycloalkyl, heterocyclyl or heteroaryl is independently optionally substituted with one to five Z¹.

In certain embodiments, R³ is C₃₋₁₀ cycloalkyl, heterocyclyl, or heteroaryl; wherein the C₃₋₁₀ cycloalkyl, heterocyclyl, or heteroaryl is independently optionally substituted with one to five halo, hydroxy, C₁₋₆ alkyl, or C₃₋₁₀ cycloalkyl.

In certain embodiments, R³ is 5-fluoropyrimidin-4-yl, 1-cyclobutylpiperidin-3-yl, 1-ethylpiperidin-3-yl, 1-cyclopropylpiperidin-3-yl, 3-fluoropyridin-2-yl, 5-fluoropyrimidin-2-yl, 3,5-difluoropyridin-2-yl, or 3-hydroxy-3-methylcyclobutyl. In certain embodiments, R³ is 5-fluoropyrimidin-4-yl, 1-cyclobutylpiperidin-3-yl, or 3-hydroxy-3-methylcyclobutyl.

In certain embodiments, R⁴ is hydrogen.

In certain embodiments, R² is -C(R⁶)₂R¹⁰; and at least one R⁶ is hydrogen. In certain embodiments, R² is -C(R⁶)₂R¹⁰; and one R⁶ is hydrogen.

In certain embodiments, R² is -C(R⁶)₂R¹⁰; and R¹⁰ is halo, C₁₋₆ alkyl, or C₁₋₆ haloalkyl. In certain embodiments, R² is -C(R⁶)₂R¹⁰; and R¹⁰ is C₁₋₆ alkyl. In certain embodiments, R² is -C(R⁶)₂R¹⁰; at least one R⁶ is hydrogen, and R¹⁰ is C₁₋₆ alkyl.

In certain embodiments, R² is -OR⁹; and R⁹ is C₁₋₆ alkyl. In certain embodiments, R² is -OR⁹; and R⁹ is C₁₋₂ alkyl.

In certain embodiments, R⁷ and R⁸ join to form a C₃₋₁₀ cycloalkyl. In certain embodiments, R⁷ is hydrogen. In certain embodiments, R⁸ is hydrogen. In certain embodiments, R⁷ and R⁸ are hydrogen; or R⁷ and R⁸ join to form a C₃₋₁₀ cycloalkyl.

In certain embodiments, each Z¹ is independently halo, cyano, hydroxy, -SH, -NH₂, -NO₂, -SF₅, C₁₋₆ alkyl, C₂₋₆ alkenyl, C₂₋₆ alkynyl, C₁₋₆ haloalkyl, C₂₋₆ heteroalkyl, C₃₋₁₀ cycloalkyl, heterocyclyl, aryl, heteroaryl, -N(R¹¹)₂, -OR¹¹, -C(O)R¹¹, -C(O)OR¹¹, -S(O)₀₋₂R¹¹, -NR¹¹S(O)₀₋₂-R¹¹, -S(O)₀₋₂N(R¹¹)₂, -NR¹¹S(O)₀₋₂N(R¹¹)₂, -NR¹¹C(O)N(R¹¹)₂, -C(O)N(R¹¹)₂, -NR¹¹C(O)R¹¹, -OC(O)N(R¹¹)₂, or -NR¹¹C(O)OR¹¹.

In certain embodiments, each Z¹ is independently halo, cyano, hydroxy, C₁₋₆ alkyl, or C₃₋₁₀ cycloalkyl.

In certain embodiments, each R¹¹ is independently hydrogen, C₁₋₆ alkyl, C₂₋₆ alkenyl, C₂₋₆ alkynyl, C₁₋₆ haloalkyl, C₂₋₆ heteroalkyl, C₃₋₁₀ cycloalkyl, heterocyclyl, aryl, or heteroaryl.

In certain embodiments, each R¹¹ is independently hydrogen, C₁₋₆ alkyl, C₁₋₆ haloalkyl, C₂₋₆ heteroalkyl, C₃₋₁₀ cycloalkyl, heterocyclyl, aryl, or heteroaryl. In certain embodiments, each R¹¹ is independently hydrogen or C₁₋₆ alkyl. In certain embodiments, each R¹¹ is hydrogen.

In certain embodiments, each R¹² is independently hydrogen, C₁₋₆ alkyl, C₂₋₆ alkenyl, C₂₋₆ alkynyl, C₁₋₆ haloalkyl, C₂₋₆ heteroalkyl, C₃₋₁₀ cycloalkyl, heterocyclyl, aryl, or heteroaryl.

In certain embodiments, each R¹² is independently hydrogen, C₁₋₆ alkyl, C₁₋₆ haloalkyl, C₂₋₆ heteroalkyl, C₃₋₁₀ cycloalkyl, heterocyclyl, aryl, or heteroaryl. In certain embodiments, each R¹² is independently hydrogen or C₁₋₆ alkyl. In certain embodiments, each R¹² is hydrogen.

In certain embodiments, each of A¹, A², A³, A⁴, and A⁵ is independently CR¹; each R¹ is independently hydrogen, halo, cyano, C₁₋₆ alkyl, C₁₋₆ alkoxy, C₁₋₆ haloalkyl, or C₃₋₁₀ cycloalkyl, or any two adjacent R¹ groups can join to form a aryl or heteroaryl ring; X is CR⁵; R² is -C(R⁶)₂R¹⁰, -OR⁹, C₃₋₁₀ cycloalkyl, or halo; wherein the C₃₋₁₀ cycloalkyl is independently optionally substituted with one to five Z¹; R³ is C₃₋₁₀ cycloalkyl, heterocyclyl, or heteroaryl; wherein the C₃₋₁₀ cycloalkyl, heterocyclyl, or heteroaryl is independently optionally substituted with one to five halo, hydroxy, C₁₋₆ alkyl, or C₃₋₁₀ cycloalkyl; R⁴ is hydrogen; R⁵ is hydrogen, C₁₋₆ alkyl, or C₁₋₆ haloalkyl; at least one R⁶ is hydrogen; R⁷ and R⁸ are hydrogen, or R⁷ and R⁸ join to form a C₃₋₁₀ cycloalkyl; R⁹ is C₁₋₆ alkyl; and R¹⁰ is halo, C₁₋₆ alkyl, or C₁₋₆ haloalkyl.

In certain embodiments, one of A¹, A², A³, A⁴, and A⁵ is N and the remaining A¹, A², A³, A⁴, and A⁵ are independently CR¹; each R¹ is independently hydrogen, halo, cyano, C₁₋₆ alkyl, C₁₋₆ alkoxy, C₁₋₆ haloalkyl, or C₃₋₁₀ cycloalkyl, or any two adjacent R¹ groups can join to form a aryl or heteroaryl ring; X is CR⁵; R² is -C(R⁶)₂R¹⁰, -OR⁹, C₃₋₁₀ cycloalkyl, or halo; wherein the C₃₋₁₀ cycloalkyl is independently optionally substituted with one to five Z¹; R³ is C₃₋₁₀ cycloalkyl, heterocyclyl, or heteroaryl; wherein the C₃₋₁₀ cycloalkyl, heterocyclyl, or heteroaryl is independently optionally substituted with one to five halo, hydroxy, C₁₋₆ alkyl, or C₃₋₁₀ cycloalkyl; R⁴ is hydrogen; R⁵ is hydrogen, C₁₋₆ alkyl, or C₁₋₆ haloalkyl; at least one R⁶ is hydrogen; R⁷ and R⁸ are hydrogen, or R⁷ and R⁸ join to form a C₃₋₁₀ cycloalkyl; R⁹ is C₁₋₆ alkyl; and R¹⁰ is halo, C₁₋₆ alkyl, or C₁₋₆ haloalkyl.

In certain embodiments, two of A¹, A², A³, A⁴, and A⁵ are N and the remaining A¹, A², A³, A⁴, and A⁵ are independently CR¹; each R¹ is independently hydrogen, halo, cyano, C₁₋₆ alkyl, C₁₋₆ alkoxy, C₁₋₆ haloalkyl, or C₃₋₁₀ cycloalkyl, or any two adjacent R¹ groups can join to form a aryl or heteroaryl ring; X is CR⁵; R² is -C(R⁶)₂R¹⁰, -OR⁹, C₃₋₁₀ cycloalkyl, or halo; wherein the C₃₋₁₀ cycloalkyl is independently optionally substituted with one to five Z¹; R³ is C₃₋₁₀ cycloalkyl, heterocyclyl, or heteroaryl; wherein the C₃₋₁₀ cycloalkyl, heterocyclyl, or heteroaryl is independently optionally substituted with one to five halo, hydroxy, C₁₋₆ alkyl, or C₃₋₁₀ cycloalkyl; R⁴ is hydrogen; R⁵ is hydrogen, C₁₋₆ alkyl, or C₁₋₆ haloalkyl; at least one R⁶ is hydrogen; R⁷ and R⁸ are hydrogen; R⁹ is C₁₋₆ alkyl; and R¹⁰ is halo, C₁₋₆ alkyl, or C₁₋₆ haloalkyl.

In certain embodiments, each of A¹, A², A³, A⁴, and A⁵ is independently CR¹; each R¹ is independently hydrogen, halo, cyano, C₁₋₆ alkyl, C₁₋₆ alkoxy, C₁₋₆ haloalkyl, or C₃₋₁₀ cycloalkyl, or any two adjacent R¹ groups can join to form a aryl or heteroaryl ring; X is N; R² is -C(R⁶)₂R¹⁰, -OR⁹, C₃₋₁₀ cycloalkyl, or halo; wherein the C₃₋₁₀ cycloalkyl is independently optionally substituted with one to five Z¹; R³ is C₃₋₁₀ cycloalkyl, heterocyclyl, or heteroaryl; wherein the C₃₋₁₀ cycloalkyl, heterocyclyl, or heteroaryl is independently optionally substituted with one to five halo, hydroxy, C₁₋₆ alkyl, or C₃₋₁₀ cycloalkyl; R⁴ is hydrogen; at least one R⁶ is hydrogen; R⁷ and R⁸ are hydrogen, or R⁷ and R⁸ join to form a C₃₋₁₀ cycloalkyl; R⁹ is C₁₋₆ alkyl; and R¹⁰ is halo, C₁₋₆ alkyl, or C₁₋₆ haloalkyl.

In certain embodiments, one of A¹, A², A³, A⁴, and A⁵ is N and the remaining A¹, A², A³, A⁴, and A⁵ are independently CR¹; each R¹ is independently hydrogen, halo, cyano, C₁₋₆ alkyl, C₁₋₆ alkoxy, C₁₋₆ haloalkyl, or C₃₋₁₀ cycloalkyl, or any two adjacent R¹ groups can join to form a aryl or heteroaryl ring; X is N; R² is -C(R⁶)₂R¹⁰, -OR⁹, C₃₋₁₀ cycloalkyl, or halo; wherein the C₃₋₁₀ cycloalkyl is independently optionally substituted with one to five Z¹; R³ is C₃₋₁₀ cycloalkyl, heterocyclyl, or heteroaryl; wherein the C₃₋₁₀ cycloalkyl, heterocyclyl, or heteroaryl is independently optionally substituted with one to five halo, hydroxy, C₁₋₆ alkyl, or C₃₋₁₀ cycloalkyl; R⁴ is hydrogen; at least one R⁶ is hydrogen; R⁷ and R⁸ are hydrogen, or R⁷ and R⁸ join to form a C₃₋₁₀ cycloalkyl; R⁹ is C₁₋₆ alkyl; and R¹⁰ is halo, C₁₋₆ alkyl, or C₁₋₆ haloalkyl.

In certain embodiments, two of A¹, A², A³, A⁴, and A⁵ are N and the remaining A¹, A², A³, A⁴, and A⁵ are independently CR¹; each R¹ is independently hydrogen, halo, cyano, C₁₋₆ alkyl, C₁₋₆ alkoxy, C₁₋₆ haloalkyl, or C₃₋₁₀ cycloalkyl, or any two adjacent R¹ groups can join to form a aryl or heteroaryl ring; X is N; R² is -C(R⁶)₂R¹⁰, -OR⁹, C₃₋₁₀ cycloalkyl, or halo; wherein the C₃₋₁₀ cycloalkyl is independently optionally substituted with one to five Z¹; R³ is C₃₋₁₀ cycloalkyl, heterocyclyl, or heteroaryl; wherein the C₃₋₁₀ cycloalkyl, heterocyclyl, or heteroaryl is independently optionally substituted with one to five halo, hydroxy, C₁₋₆ alkyl, or C₃₋₁₀ cycloalkyl; R⁴ is hydrogen; at least one R⁶ is hydrogen; R⁷ and R⁸ are hydrogen, or R⁷ and R⁸ join to form a C₃₋₁₀ cycloalkyl; R⁹ is C₁₋₆ alkyl; and R¹⁰ is halo, C₁₋₆ alkyl, or C₁₋₆ haloalkyl.

In certain embodiments, each of A¹, A², A³, A⁴, and A⁵ is independently CR¹; each R¹ is independently hydrogen, halo, cyano, C₁₋₆ alkyl, C₁₋₆ alkoxy, or C₁₋₆ haloalkyl; X is CR⁵; R² is -C(R⁶)₂R¹⁰ or -OR⁹; R³ is C₃₋₁₀ cycloalkyl, heterocyclyl, or heteroaryl; wherein the C₃₋₁₀ cycloalkyl, heterocyclyl, or heteroaryl is independently optionally substituted with one to five halo, hydroxy, C₁₋₆ alkyl, or C₃₋₁₀ cycloalkyl; R⁴ is hydrogen; R⁵ is hydrogen; at least one R⁶ is hydrogen; R⁷ and R⁸ are hydrogen; R⁹ is C₁₋₆ alkyl; and R¹⁰ is C₁₋₆ alkyl.

In certain embodiments, each of A¹, A², A³, A⁴, and A⁵ is independently CR¹; X is CR⁵; R¹ is independently hydrogen, fluoro, chloro, cyano, -CH₃, -OCH₃, or -CF₃; R² is -C(R⁶)₂R¹⁰ or -OR⁹; R³ is C₃₋₁₀ cycloalkyl, heterocyclyl, or heteroaryl; wherein the C₃₋₁₀ cycloalkyl, heterocyclyl, or heteroaryl is independently optionally substituted with one to five halo, hydroxy, C₁₋₆ alkyl, or C₃₋₁₀ cycloalkyl; R⁴ is hydrogen; R⁵ is hydrogen; at least one R⁶ is hydrogen; R⁷ and R⁸ are hydrogen; R⁹ is C₁₋₆ alkyl; and R¹⁰ is C₁₋₆ alkyl.

In certain embodiments, one of A¹, A², A³, A⁴, and A⁵ is N and the remaining A¹, A², A³, A⁴, and A⁵ are independently CR¹; each R¹ is independently hydrogen, halo, cyano, C₁₋₆ alkyl, C₁₋₆ alkoxy, or C₁₋₆ haloalkyl; X is CR⁵; R² is -C(R⁶)₂R¹⁰ or -OR⁹; R³ is C₃₋₁₀ cycloalkyl, heterocyclyl, or heteroaryl; wherein the C₃₋₁₀ cycloalkyl, heterocyclyl, or heteroaryl is independently optionally substituted with one to five halo, hydroxy, C₁₋₆ alkyl, or C₃₋₁₀ cycloalkyl; R⁴ is hydrogen; R⁵ is hydrogen; at least one R⁶ is hydrogen; R⁷ and R⁸ are hydrogen; R⁹ is C₁₋₆ alkyl; and R¹⁰ is C₁₋₆ alkyl.

In certain embodiments, one of A¹, A², A³, A⁴, and A⁵ is N and the remaining A¹, A², A³, A⁴, and A⁵ are independently CR¹; X is CR⁵; R¹ is independently hydrogen, fluoro, chloro, cyano, -CH₃, -OCH₃, or -CF₃; R² is -C(R⁶)₂R¹⁰ or -OR⁹; R³ is C₃₋₁₀ cycloalkyl, heterocyclyl, or heteroaryl; wherein the C₃₋₁₀ cycloalkyl, heterocyclyl, or heteroaryl is independently optionally substituted with one to five halo, hydroxy, C₁₋₆ alkyl, or C₃₋₁₀ cycloalkyl; R⁴ is hydrogen; R⁵ is hydrogen; at least one R⁶ is hydrogen; R⁷ and R⁸ are hydrogen; R⁹ is C₁₋₆ alkyl; and R¹⁰ is C₁₋₆ alkyl.

In certain embodiments, two of A¹, A², A³, A⁴, and A⁵ are N and the remaining A¹, A², A³, A⁴, and A⁵ are independently CR¹; each R¹ is independently hydrogen, halo, cyano, C₁₋₆ alkyl, C₁₋₆ alkoxy, or C₁₋₆ haloalkyl; X is CR⁵; R¹ is independently hydrogen, fluoro, chloro, cyano, -CH₃, -OCH₃, or -CF₃; R² is -C(R⁶)₂R¹⁰ or -OR⁹; R³ is C₃₋₁₀ cycloalkyl, heterocyclyl, or heteroaryl; wherein the C₃₋₁₀ cycloalkyl, heterocyclyl, or heteroaryl is independently optionally substituted with one to five halo, hydroxy, C₁₋₆ alkyl, or C₃₋₁₀ cycloalkyl; R⁴ is hydrogen; R⁵ is hydrogen; at least one R⁶ is hydrogen; R⁷ and R⁸ are hydrogen; R⁹ is C₁₋₆ alkyl; and R¹⁰ is C₁₋₆ alkyl.

In certain embodiments, two of A¹, A², A³, A⁴, and A⁵ are N and the remaining A¹, A², A³, A⁴, and A⁵ are independently CR¹; X is CR⁵; R¹ is independently hydrogen, fluoro, chloro, cyano, -CH₃, -OCH₃, or -CF₃; R² is -C(R⁶)₂R¹⁰ or -OR⁹; R³ is C₃₋₁₀ cycloalkyl, heterocyclyl, or heteroaryl; wherein the C₃₋₁₀ cycloalkyl, heterocyclyl, or heteroaryl is independently optionally substituted with one to five halo, hydroxy, C₁₋₆ alkyl, or C₃₋₁₀ cycloalkyl; R⁴ is hydrogen; R⁵ is hydrogen; at least one R⁶ is hydrogen; R⁷ and R⁸ are hydrogen; R⁹ is C₁₋₆ alkyl; and R¹⁰ is C₁₋₆ alkyl.

In certain embodiments, each of A¹, A², A³, A⁴, and A⁵ is independently CR¹; each R¹ is independently hydrogen, halo, cyano, C₁₋₆ alkyl, C₁₋₆ alkoxy, or C₁₋₆ haloalkyl; X is N; R² is -C(R⁶)₂R¹⁰ or -OR⁹; R³ is C₃₋₁₀ cycloalkyl, heterocyclyl, or heteroaryl; wherein the C₃₋₁₀ cycloalkyl, heterocyclyl, or heteroaryl is independently optionally substituted with one to five halo, hydroxy, C₁₋₆ alkyl, or C₃₋₁₀ cycloalkyl; R⁴ is hydrogen; at least one R⁶ is hydrogen; R⁷ and R⁸ are hydrogen; R⁹ is C₁₋₆ alkyl; and R¹⁰ is C₁₋₆ alkyl.

In certain embodiments, each of A¹, A², A³, A⁴, and A⁵ is independently CR¹; X is N; R¹ is independently hydrogen, fluoro, chloro, cyano, -CH₃, -OCH₃, or -CF₃; R² is -C(R⁶)₂R¹⁰ or -OR⁹; R³ is C₃₋₁₀ cycloalkyl, heterocyclyl, or heteroaryl; wherein the C₃₋₁₀ cycloalkyl, heterocyclyl, or heteroaryl is independently optionally substituted with one to five halo, hydroxy, C₁₋₆ alkyl, or C₃₋₁₀ cycloalkyl; R⁴ is hydrogen; at least one R⁶ is hydrogen; R⁷ and R⁸ are hydrogen; R⁹ is C₁₋₆ alkyl; and R¹⁰ is C₁₋₆ alkyl.

In certain embodiments, one of A¹, A², A³, A⁴, and A⁵ is N and the remaining A¹, A², A³, A⁴, and A⁵ are independently CR¹; each R¹ is independently hydrogen, halo, cyano, C₁₋₆ alkyl, C₁₋₆ alkoxy, or C₁₋₆ haloalkyl; X is N; R² is -C(R⁶)₂R¹⁰ or -OR⁹; R³ is C₃₋₁₀ cycloalkyl, heterocyclyl, or heteroaryl; wherein the C₃₋₁₀ cycloalkyl, heterocyclyl, or heteroaryl is independently optionally substituted with one to five halo, hydroxy, C₁₋₆ alkyl, or C₃₋₁₀ cycloalkyl; R⁴ is hydrogen; at least one R⁶ is hydrogen; R⁷ and R⁸ are hydrogen; R⁹ is C₁₋₆ alkyl; and R¹⁰ is C₁₋₆ alkyl.

In certain embodiments, one of A¹, A², A³, A⁴, and A⁵ is N and the remaining A¹, A², A³, A⁴, and A⁵ are independently CR¹; X is N; R¹ is independently hydrogen, fluoro, chloro, cyano, -CH₃, -OCH₃, or -CF₃; R² is -C(R⁶)₂R¹⁰ or -OR⁹; R³ is C₃₋₁₀ cycloalkyl, heterocyclyl, or heteroaryl; wherein the C₃₋₁₀ cycloalkyl, heterocyclyl, or heteroaryl is independently optionally substituted with one to five halo, hydroxy, C₁₋₆ alkyl, or C₃₋₁₀ cycloalkyl; R⁴ is hydrogen; at least one R⁶ is hydrogen; R⁷ and R⁸ are hydrogen; R⁹ is C₁₋₆ alkyl; and R¹⁰ is C₁₋₆ alkyl.

In certain embodiments, two of A¹, A², A³, A⁴, and A⁵ are N and the remaining A¹, A², A³, A⁴, and A⁵ are independently CR¹; each R¹ is independently hydrogen, halo, cyano, C₁₋₆ alkyl, C₁₋₆ alkoxy, or C₁₋₆ haloalkyl; X is N; R² is -C(R⁶)₂R¹⁰ or -OR⁹; R³ is C₃₋₁₀ cycloalkyl, heterocyclyl, or heteroaryl; wherein the C₃₋₁₀ cycloalkyl, heterocyclyl, or heteroaryl is independently optionally substituted with one to five halo, hydroxy, C₁₋₆ alkyl, or C₃₋₁₀ cycloalkyl; R⁴ is hydrogen; at least one R⁶ is hydrogen; R⁷ and R⁸ are hydrogen; R⁹ is C₁₋₆ alkyl; and R¹⁰ is C₁₋₆ alkyl.

In certain embodiments, two of A¹, A², A³, A⁴, and A⁵ are N and the remaining A¹, A², A³, A⁴, and A⁵ are independently CR¹; X is N; R¹ is independently hydrogen, fluoro, chloro, cyano, -CH₃, -OCH₃, or -CF₃;R² is -C(R⁶)₂R¹⁰ or -OR⁹; R³ is C₃₋₁₀ cycloalkyl, heterocyclyl, or heteroaryl; wherein the C₃₋₁₀ cycloalkyl, heterocyclyl, or heteroaryl is independently optionally substituted with one to five halo, hydroxy, C₁₋₆ alkyl, or C₃₋₁₀ cycloalkyl; R⁴ is hydrogen; at least one R⁶ is hydrogen; R⁷ and R⁸ are hydrogen; R⁹ is C₁₋₆ alkyl; and R¹⁰ is C₁₋₆ alkyl.

In certain embodiments, provided is a compound selected from Table 1, or a pharmaceutically acceptable salt, isotopically enriched analog, stereoisomer, or a mixture of stereoisomers thereof:

**Table 1**

| **Ex.** | **Structure** |
|---|---|
| 1 | 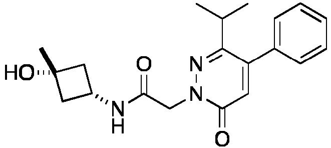 |
| 2 | 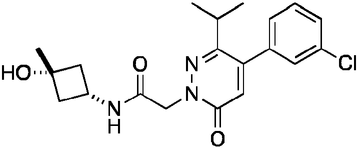 |
| 3 | 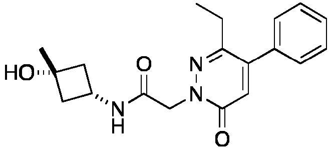 |
| 4 | 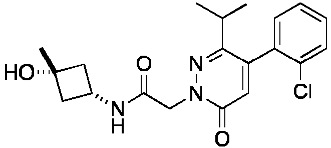 |
| 5 | 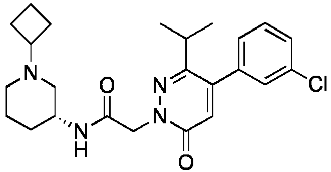 |
| 6 | 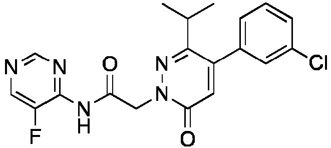 |
| 7 | 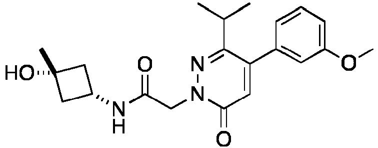 |
| 8 | 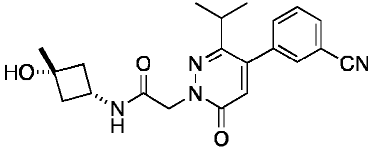 |
| 9 | 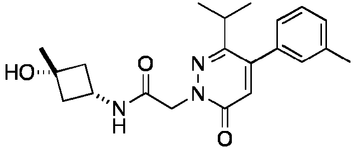 |
| 10 | 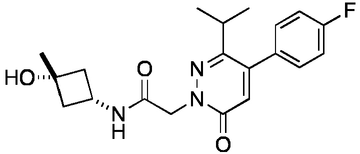 |
| 11 | 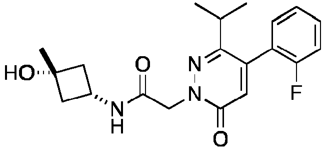 |
| 12 | 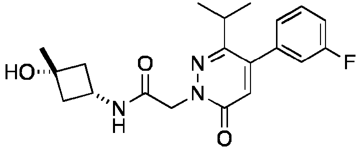 |
| 13 | 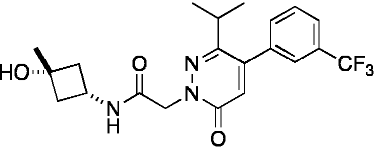 |
| 14 | 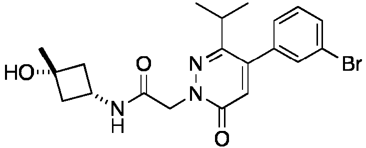 |
| 15 | 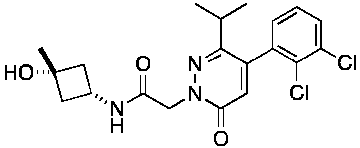 |
| 16 | 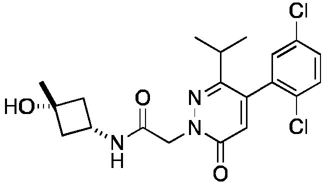 |
| 17 | 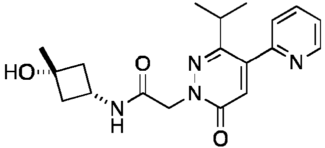 |
| 18 | 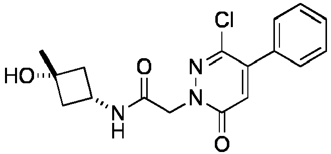 |
| 19 | 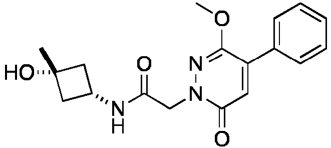 |
| 20 | 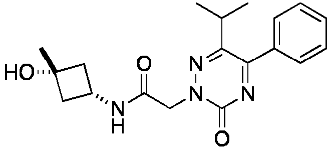 |
| 21 | 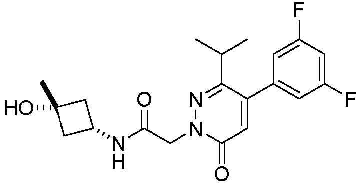 |
| 22 | 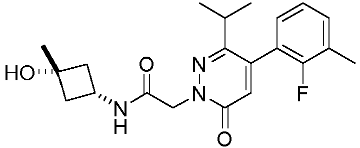 |
| 23 | 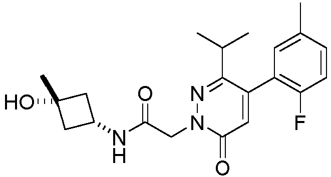 |
| 24 | 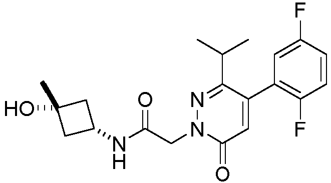 |
| 25 | 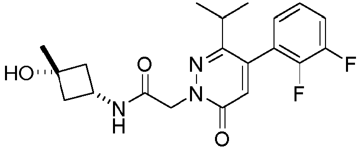 |
| 26 | 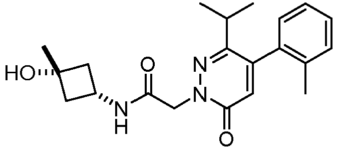 |
| 27 | 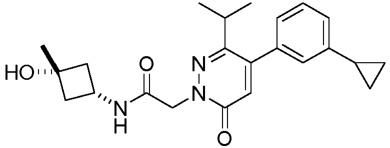 |
| 28 | 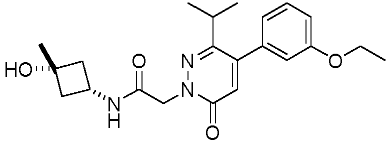 |
| 29 | 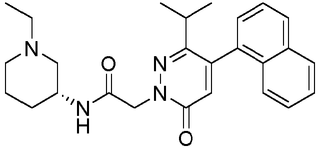 |
| 30 | 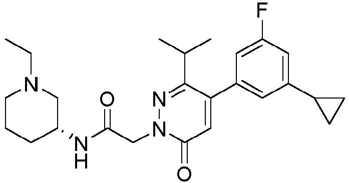 |
| 31 | 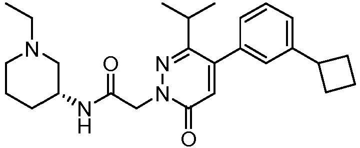 |
| 32 | 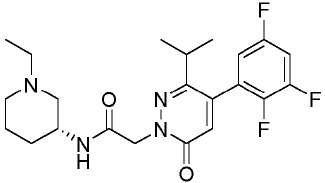 |
| 33 | 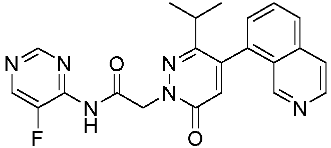 |
| 34 | 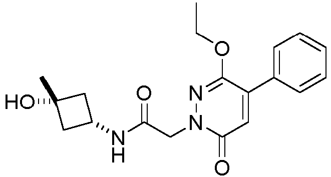 |
| 35 | 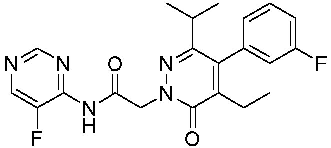 |
| 36 | 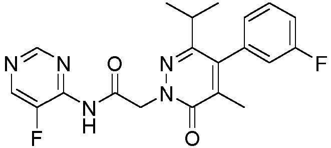 |
| 37 | 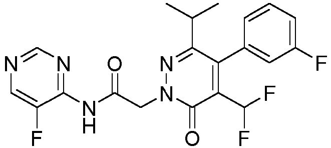 |
| 38 | 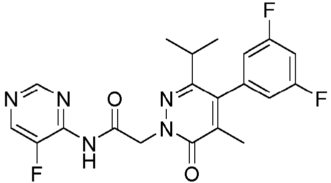 |
| 39 | 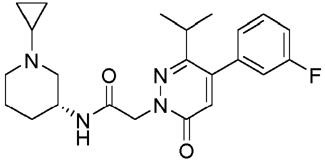 |
| 40 | 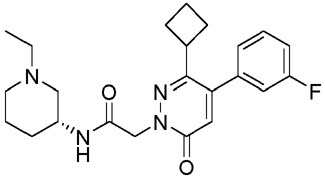 |
| 41 | 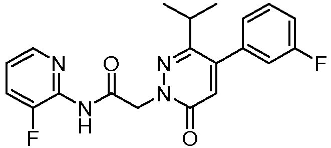 |
| 42 | 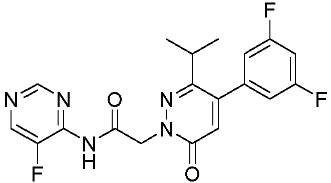 |
| 43 | 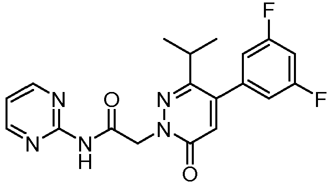 |
| 44 | 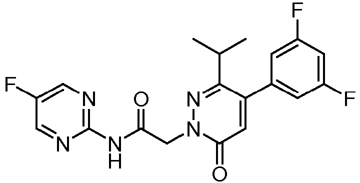 |
| 45 | 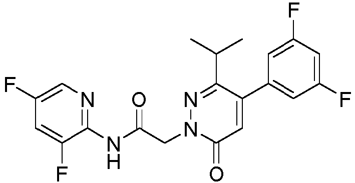 |
| 46 | 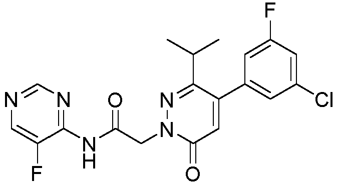 |
| 47 | 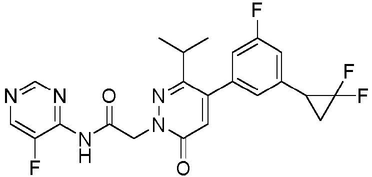 |
| 48 | 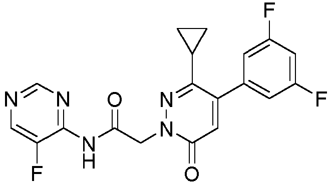 |
| 49 | 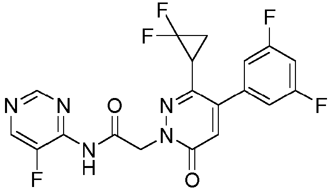 |
| 50 | 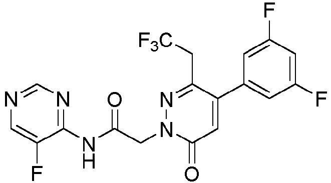 |
| 51 | 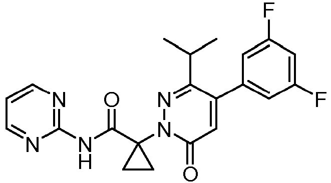 |
| 52 | 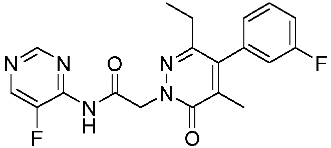 |
| 53 | 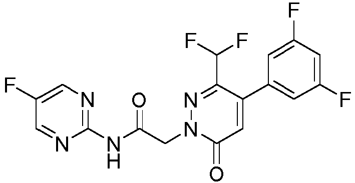 |
| 54 | 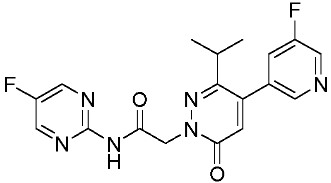 |
| 55 | 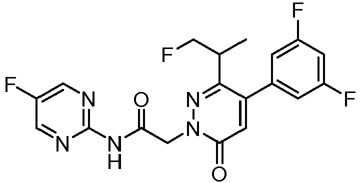 |
| 56 | 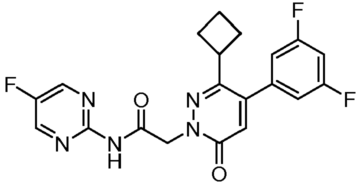 |
| 57 | 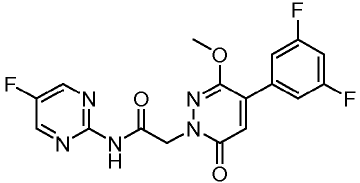 |

In certain embodiments, provided is a compound selected from Table 2 or a pharmaceutically acceptable salt, isotopically enriched analog, stereoisomer, or a mixture of stereoisomers thereof:

**Table 2**

| **Structure** |
|---|
| 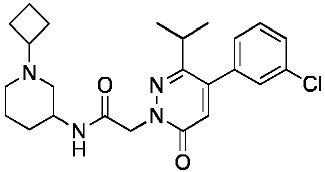 |
| 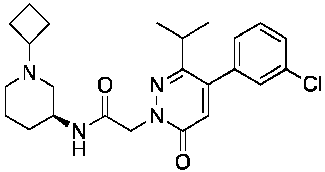 |
| 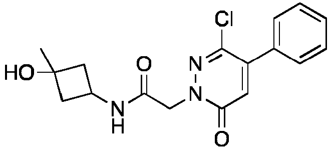 |
| 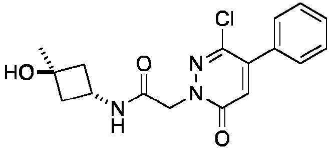 |
| 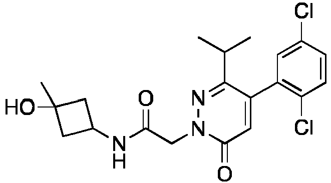 |
| 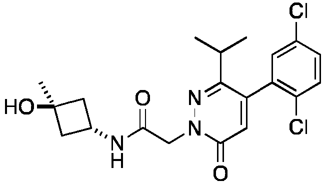 |
| 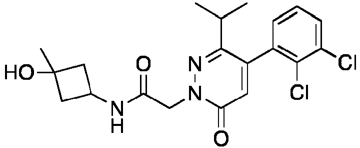 |
| 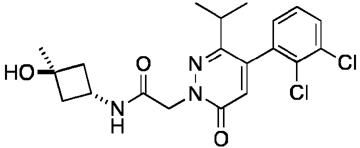 |
| 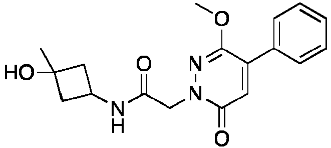 |
| 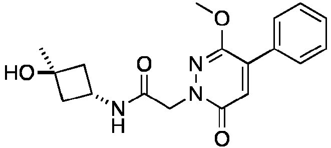 |
| 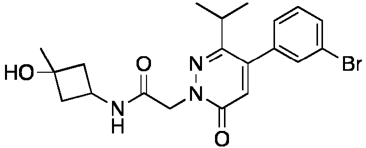 |
| 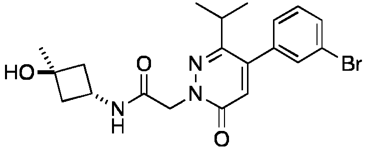 |
| 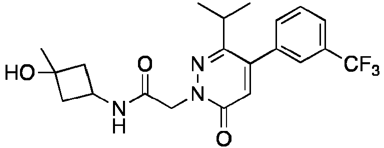 |
| 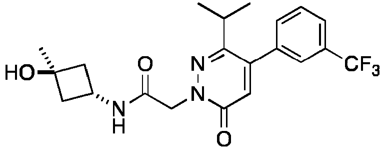 |
| 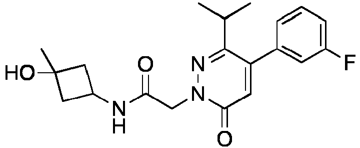 |
| 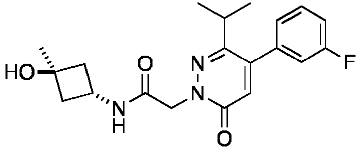 |
| 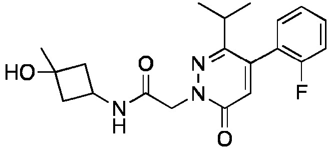 |
| 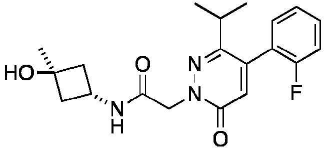 |
| 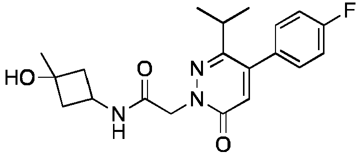 |
| 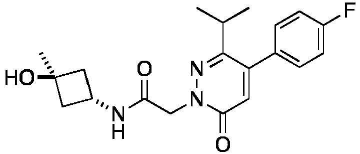 |
| 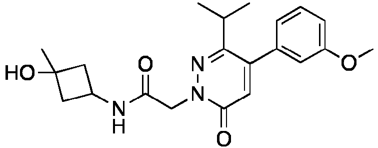 |
| 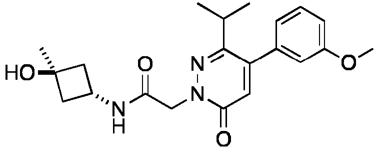 |
| 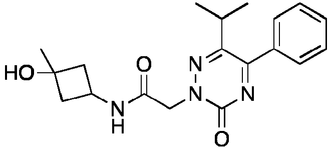 |
| 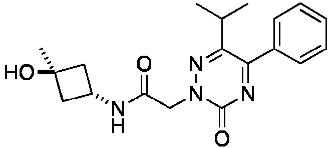 |
| 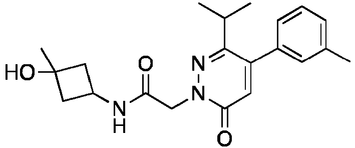 |
| 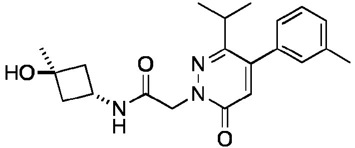 |
| 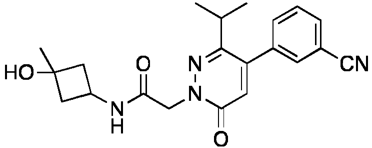 |
| 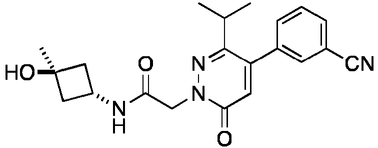 |
| 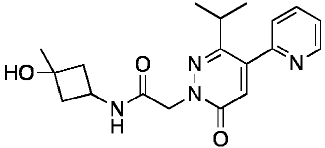 |
| 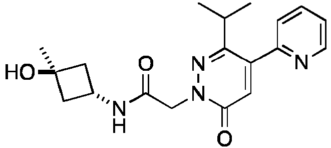 |
| 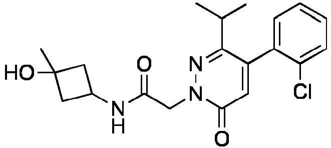 |
| 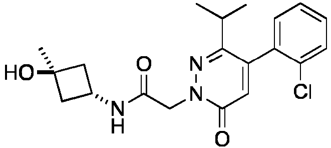 |
| 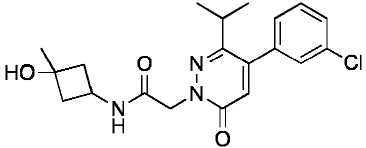 |
| 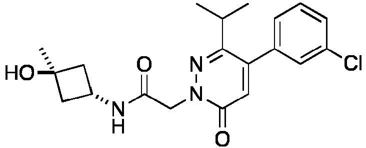 |
| 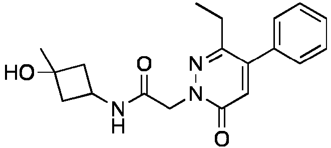 |
| 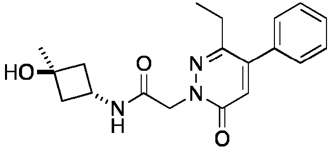 |
| 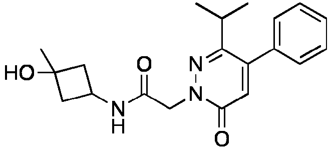 |
| 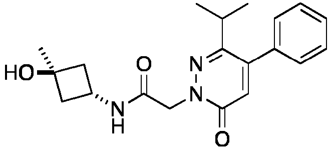 |
| 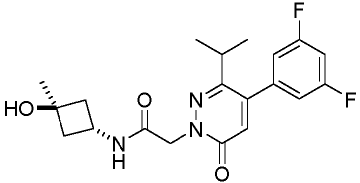 |
| 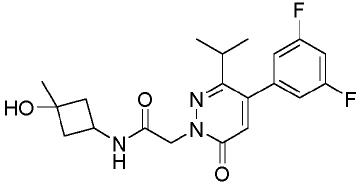 |
| 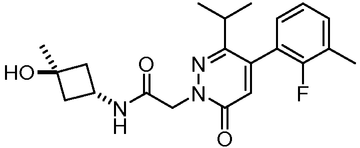 |
| 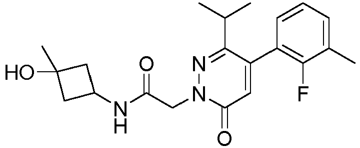 |
| 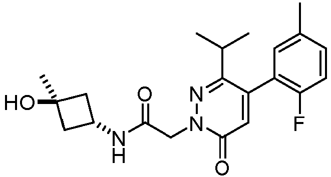 |
| 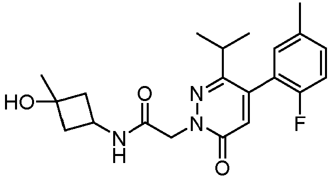 |
| 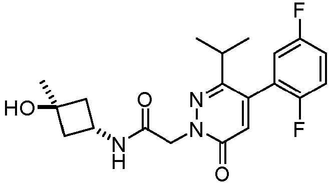 |
| 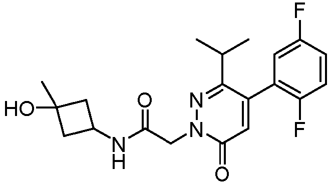 |
| 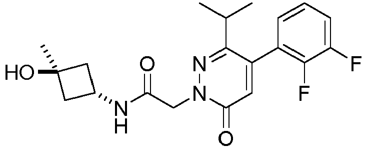 |
| 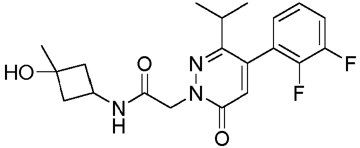 |
| 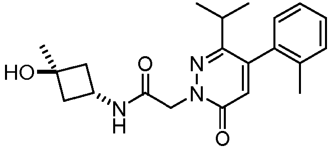 |
| 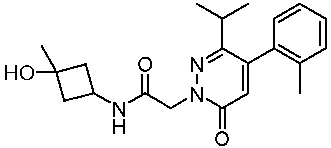 |
| 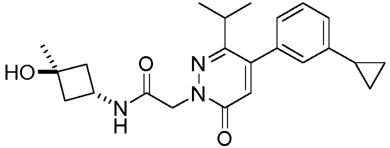 |
| 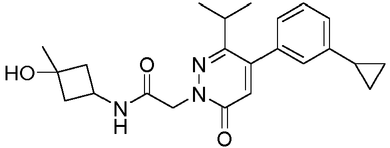 |
| 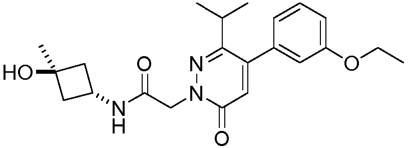 |
| 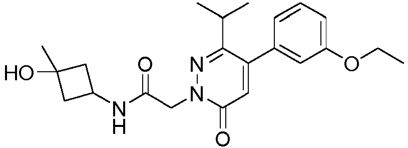 |
| 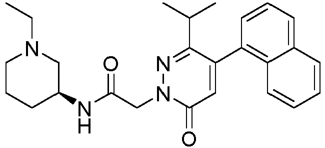 |
| 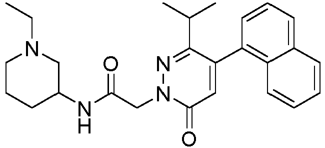 |
| 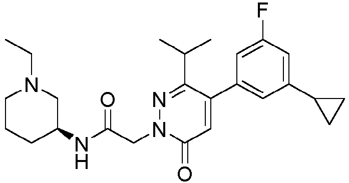 |
| 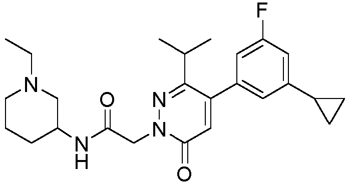 |
| 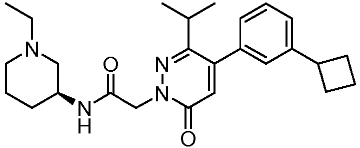 |
| 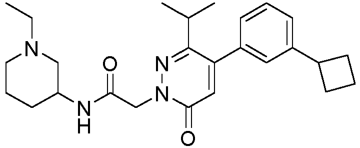 |
| 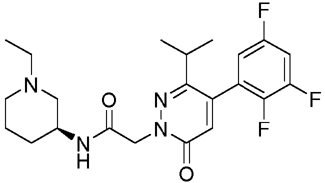 |
| 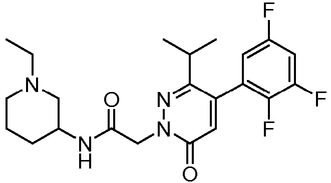 |
| 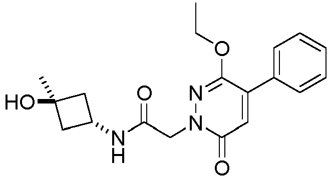 |
| 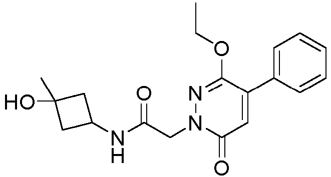 |
| 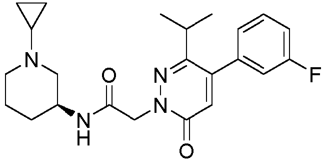 |
| 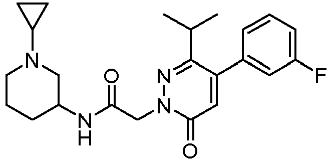 |
| 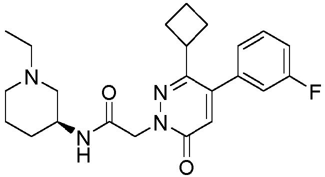 |
| 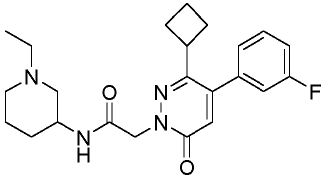 |
| 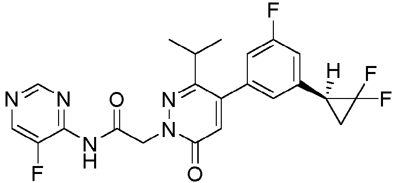 |
| 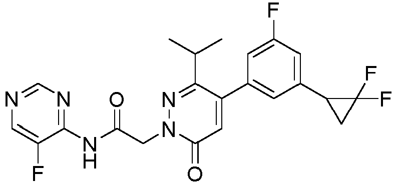 |
| 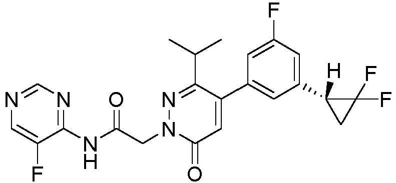 |
| 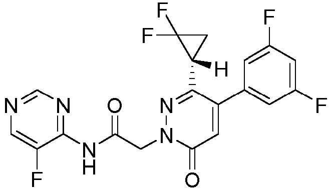 |
| 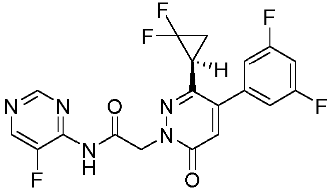 |
| 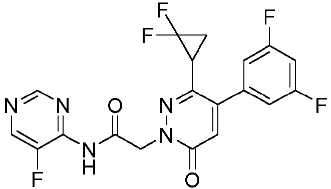 |
| 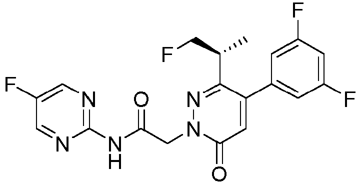 |
| 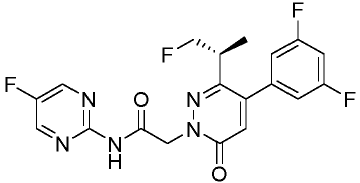 |
| 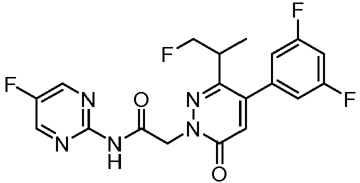 |

### 3. Methods

"Treatment" or "treating" is an approach for obtaining beneficial or desired results including clinical results. Beneficial or desired clinical results may include one or more of the following: a) inhibiting the disease or condition (e.g., decreasing one or more symptoms resulting from the disease or condition, and/or diminishing the extent of the disease or condition); b) slowing or arresting the development of one or more clinical symptoms associated with the disease or condition (e.g., stabilizing the disease or condition, preventing or delaying the worsening or progression of the disease or condition, and/or preventing or delaying the spread (e.g., metastasis) of the disease or condition); and/or c) relieving the disease, that is, causing the regression of clinical symptoms (e.g., ameliorating the disease state, providing partial or total remission of the disease or condition, enhancing effect of another medication, delaying the progression of the disease, increasing the quality of life, and/or prolonging survival.

"Prevention" or "preventing" means any treatment of a disease or condition that causes the clinical symptoms of the disease or condition not to develop. Compounds may, in some embodiments, be administered to a subject (including a human) who is at risk or has a family history of the disease or condition.

"Subject" refers to an animal, such as a mammal (including a human), that has been or will be the object of treatment, observation or experiment. The methods described herein may be useful in human therapy, and/or veterinary applications. In some embodiments, the subject is a mammal. In certain embodiments, the subject is a human.

The term "therapeutically effective amount" or "effective amount" of a compound described herein or a pharmaceutically acceptable salt, isotopically enriched analog, stereoisomer, or mixture of stereoisomers means an amount sufficient to effect treatment when administered to a subject, to provide a therapeutic benefit such as amelioration of symptoms or slowing of disease progression. For example, a therapeutically effective amount may be an amount sufficient to decrease a symptom of a disease or condition of as described herein. The therapeutically effective amount may vary depending on the subject, and disease or condition being treated, the weight and age of the subject, the severity of the disease or condition, and the manner of administering, which can readily be determined by one of ordinary skill in the art.

The methods described herein may be applied to cell populations *in vivo* or *ex vivo. "In vivo"* means within a living individual, as within an animal or human. In this context, the methods described herein may be used therapeutically in an individual. *"Ex vivo"* means outside of a living individual. Examples of ex vivo cell populations include in vitro cell cultures and biological samples including fluid or tissue samples obtained from individuals. Such samples may be obtained by methods well known in the art. Exemplary biological fluid samples include blood, cerebrospinal fluid, urine, and saliva. In this context, the compounds and compositions described herein may be used for a variety of purposes, including therapeutic and experimental purposes. For example, the compounds and compositions described herein may be used *ex vivo* to determine the optimal schedule and/or dosing of administration of a compound of the present disclosure for a given indication, cell type, individual, and other parameters. Information gleaned from such use may be used for experimental purposes or in the clinic to set protocols for *in vivo* treatment. Other *ex vivo* uses for which the compounds and compositions described herein may be suited are described below or will become apparent to those skilled in the art. The selected compounds may be further characterized to examine the safety or tolerance dosage in human or non-human subjects. Such properties may be examined using commonly known methods to those skilled in the art.

In certain embodiments, provided are compounds, or a pharmaceutically acceptable salt, isotopically enriched analog, stereoisomer, or mixture of stereoisomers, for use in modulating the activity of NLR Family Pyrin Domain Containing 3 (NLRP3). In certain embodiments, the compounds provided herein, or a pharmaceutically acceptable salt, isotopically enriched analog, stereoisomer, or mixture of stereoisomers, are for use in inhibiting the activation of NLRP3.

NLR proteins are involved in the immune system, helping to start and regulate the immune system's response to injury, toxins, or invasion by microorganisms. NLRP3 (also known as cryopyrin, NALP3, LRR and PYD domains-containing protein 3), is a protein encoded by the NLRP3 gene (also known as CIAS1). Once activated, NLRP3 molecules assemble, along with other proteins, into inflammasomes. The activation of NLRP3 by cellular stress leads to inflammasome activation and downstream proteolytic events, including the formation of active proinflammatory cytokines such as interleukin (IL)-1β and IL-18 which are then secreted. Among other cytokines, IL-1β and IL-18 are known mediators of inflammation, e.g., artery wall inflammation, atherosclerosis and the aging process.

In certain embodiments, provided is a compound disclosed herein, or a pharmaceutically acceptable salt, isotopically enriched analog, stereoisomer, or mixture of stereoisomers for use in a method of inhibiting inflammasome (e.g., the NLRP3 inflammasome) activity comprising contacting a cell with an effective amount of the compound. The inhibiting can be *in vitro* or *in vivo.*

In certain embodiments, provided is a compound as disclosed herein, or a pharmaceutically acceptable salt, isotopically enriched analog, stereoisomer, or mixture of stereoisomers, for use in inhibiting inflammasome (e.g., the NLRP3 inflammasome) activity (e.g., *in vitro* or *in vivo*).

Chronic inflammation responses have been associated with various types of cancer. During malignant transformation or cancer therapy, inflammasomes may become activated in response to certain signals; and IL-Iβ expression is elevated in a variety of cancers (e.g., breast, prostate, colon, lung, head and neck cancers, melanomas, etc.), where patients with IL-Iβ producing tumors generally have a worse prognosis.

In certain embodiments, provided is a compound disclosed herein, or a pharmaceutically acceptable salt, isotopically enriched analog, stereoisomer, or mixture of stereoisomers for use in a method for treating a disease or condition mediated, at least in part, by NLRP3, comprising administering an effective amount of the compound disclosed herein to a subject in need thereof.

In certain embodiments, provided is a compound disclosed herein, or a pharmaceutically acceptable salt, isotopically enriched analog, stereoisomer, or mixture of stereoisomers for use in a method for treating a disease or condition selected from an autoinflammatory disorder, an autoimmune disorder, a neurodegenerative disease or cancer, comprising administering to a subject in need thereof a therapeutically effective amount of the compound.

In certain embodiments, provided is a compound as disclosed herein, or a pharmaceutically acceptable salt, isotopically enriched analog, stereoisomer, or mixture of stereoisomers, for use in treating an autoinflammatory disorder, an autoimmune disorder, a neurodegenerative disease or cancer in a subject in need thereof.

In certain embodiments, provided is a compound disclosed herein, or a pharmaceutically acceptable salt, isotopically enriched analog, stereoisomer, or mixture of stereoisomers for use in a method for treating inflammation, an auto-immune disease, cancer, an infection, a central nervous system disease, a metabolic disease, a cardiovascular disease, a respiratory disease, a liver disease, a renal disease, an ocular disease, a skin disease, a lymphatic condition, a psychological disorder, graft versus host disease, allodynia, or any disease where an individual has been determined to carry a germline or somatic non-silent mutation in NLRP3, comprising administering to a subject in need thereof a therapeutically effective amount of the compound.

In certain embodiments, the disease or condition may be a disease or condition of the immune system, the cardiovascular system, the endocrine system, the gastrointestinal tract, the renal system, the hepatic system, the metabolic system, the respiratory system, the central nervous system, may be a cancer or other malignancy, and/or may be caused by or associated with a pathogen. It will be appreciated that these general embodiments defined according to broad categories of diseases, disorders and conditions are not mutually exclusive.

In certain embodiments, the disease or condition includes, inflammation, including inflammation occurring as a result of an inflammatory disorder, e.g. an autoinflammatory disease, inflammation occurring as a symptom of a non-inflammatory disorder, inflammation occurring as a result of infection, or inflammation secondary to trauma, injury or autoimmunity; auto-immune diseases such as acute disseminated encephalitis, Addison's disease, ankylosing spondylitis, antiphospholipid antibody syndrome (APS), anti-synthetase syndrome, aplastic anemia, autoimmune adrenalitis, autoimmune hepatitis, autoimmune oophoritis, autoimmune polyglandular failure, autoimmune thyroiditis, Coeliac disease, Crohn's disease, type 1 diabetes (T1D), Goodpasture's syndrome, Graves' disease, Guillain-Barré syndrome (GBS), Hashimoto's disease, idiopathic thrombocytopenic purpura, Kawasaki's disease, lupus erythematosus including systemic lupus erythematosus (SLE), multiple sclerosis (MS) including primary progressive multiple sclerosis (PPMS), secondary progressive multiple sclerosis (SPMS) and relapsing remitting multiple sclerosis (RRMS), myasthenia gravis, opsoclonus myoclonus syndrome (OMS), optic neuritis, Ord's thyroiditis, pemphigus, pernicious anemia, polyarthritis, primary biliary cirrhosis, rheumatoid arthritis (RA), psoriatic arthritis, juvenile idiopathic arthritis or Still's disease, refractory gouty arthritis, Reiter's syndrome, Sjögren's syndrome, systemic sclerosis a systemic connective tissue disorder, Takayasu's arteritis, temporal arteritis, warm autoimmune hemolytic anemia, Wegener's granulomatosis, alopecia universalis, Behçet's disease, Chagas' disease, dysautonomia, endometriosis, hidradenitis suppurativa (HS), interstitial cystitis, neuromyotonia, psoriasis, sarcoidosis, scleroderma, ulcerative colitis, Schnitzler syndrome, macrophage activation syndrome, Blau syndrome, vitiligo or vulvodynia; cancer including lung cancer, pancreatic cancer, gastric cancer, myelodysplastic syndrome, leukemia including acute lymphocytic leukemia (ALL) and acute myeloid leukemia (AML), adrenal cancer, anal cancer, basal and squamous cell skin cancer, bile duct cancer, bladder cancer, bone cancer, brain and spinal cord tumors, breast cancer, cervical cancer, chronic lymphocytic leukemia (CLL), chronic myeloid leukemia (CML), chronic myelomonocytic leukemia (CMML), colorectal cancer, endometrial cancer, oesophagus cancer, Ewing family of tumors, eye cancer, gallbladder cancer, gastrointestinal carcinoid tumors, gastrointestinal stromal tumor (GIST), gestational trophoblastic disease, glioma, Hodgkin lymphoma, Kaposi sarcoma, kidney cancer, laryngeal and hypopharyngeal cancer, liver cancer, lung carcinoid tumor, lymphoma including cutaneous T cell lymphoma, malignant mesothelioma, melanoma skin cancer, Merkel cell skin cancer, multiple myeloma, nasal cavity and paranasal sinuses cancer, nasopharyngeal cancer, neuroblastoma, non-Hodgkin lymphoma, non-small cell lung cancer, oral cavity and oropharyngeal cancer, osteosarcoma, ovarian cancer, penile cancer, pituitary tumors, prostate cancer, retinoblastoma, rhabdomyosarcoma, salivary gland cancer, skin cancer, small cell lung cancer, small intestine cancer, soft tissue sarcoma, stomach cancer, testicular cancer, thymus cancer, thyroid cancer including anaplastic thyroid cancer, uterine sarcoma, vaginal cancer, vulvar cancer, Waldenstrom macroglobulinemia, and Wilms tumor; infections including viral infections (e.g. from influenza virus, human immunodeficiency virus (HIV), alphavirus (such as Chikungunya and Ross River virus), flaviviruses (such as Dengue virus and Zika virus), herpes viruses (such as Epstein Barr Virus, cytomegalovirus, Varicella-zoster virus, and KSHV), poxviruses (such as vaccinia virus (Modified vaccinia virus Ankara) and Myxoma virus), adenoviruses (such as Adenovirus 5), or papillomavirus), bacterial infections (e.g. from Staphylococcus aureus, Helicobacter pylori, Bacillus anthracis, Bordatella pertussis, Burkholderia pseudomallei, Corynebacterium diptheriae, Clostridium tetani, Clostridium botulinum, Streptococcus pneumoniae, Streptococcus pyogenes, Listeria monocytogenes, Hemophilus influenzae, Pasteurella multicida, Shigella dysenteriae, Mycobacterium tuberculosis, Mycobacterium leprae, Mycoplasma pneumoniae, Mycoplasma hominis, Neisseria meningitidis, Neisseria gonorrhoeae, Rickettsia rickettsii, Legionella pneumophila, Klebsiella pneumoniae, Pseudomonas aeruginosa, Propionibacterium acnes, Treponema pallidum, Chlamydia trachomatis, Vibrio cholerae, Salmonella typhimurium, Salmonella typhi, Borrelia burgdorferi or Yersinia pestis), fungal infections (e.g. from Candida or Aspergillus species), protozoan infections (e.g. from Plasmodium, Babesia, Giardia, Entamoeba, Leishmania or Trypanosomes), helminth infections (e.g. from schistosoma, roundworms, tapeworms or flukes) and prion infections; central nervous system diseases such as Parkinson's disease, Alzheimer's disease, dementia, motor neuron disease, Huntington's disease, cerebral malaria, brain injury from pneumococcal meningitis, intracranial aneurysms, traumatic brain injury, and amyotrophic lateral sclerosis; metabolic diseases such as type 2 diabetes (T2D), atherosclerosis, obesity, gout, and pseudo-gout; cardiovascular diseases such as hypertension, ischemia, reperfusion injury including post-MI ischemic reperfusion injury, stroke including ischemic stroke, transient ischemic attack, myocardial infarction including recurrent myocardial infarction, heart failure including congestive heart failure and heart failure with preserved ejection fraction, embolism, aneurysms including abdominal aortic aneurysm, and pericarditis including Dressler's syndrome; respiratory diseases including chronic obstructive pulmonary disorder (COPD), asthma such as allergic asthma and steroid-resistant asthma, asbestosis, silicosis, nanoparticle induced inflammation, cystic fibrosis and idiopathic pulmonary fibrosis; liver diseases including non-alcoholic fatty liver disease (NAFLD) and non-alcoholic steatohepatitis (NASH) including advanced fibrosis stages F3 and F4; alcoholic fatty liver disease (AFLD), and alcoholic steatohepatitis (ASH); renal diseases including chronic kidney disease, oxalate nephropathy, nephrocalcinosis, glomerulonephritis, and diabetic nephropathy; ocular diseases including those of the ocular epithelium, age-related macular degeneration (AMD) (dry and wet), uveitis, corneal infection, diabetic retinopathy, optic nerve damage, dry eye, and glaucoma; skin diseases including dermatitis such as contact dermatitis and atopic dermatitis, contact hypersensitivity, sunburn, skin lesions, hidradenitis suppurativa (HS), other cyst-causing skin diseases, and acne conglobata; lymphatic conditions such as lymphangitis and Castleman's disease; psychological disorders such as depression and psychological stress; graft versus host disease; allodynia including mechanical allodynia; and any disease where an individual has been determined to carry a germline or somatic non-silent mutation in NLRP3.

In certain embodiments, the disease, disorder or condition is an autoinflammatory disease such as cryopyrin-associated periodic syndromes (CAPS), Muckle-Wells syndrome (MWS), familial cold autoinflammatory syndrome (FCAS), familial Mediterranean fever (FMF), neonatal onset multisystem inflammatory disease (NOMID), tumor necrosis factor (TNF) receptor-associated periodic syndrome (TRAPS), hyperimmunoglobulinemia D and periodic fever syndrome (HIDS), deficiency of interleukin 1 receptor antagonist (DIRA), Majeed syndrome, pyogenic arthritis, pyoderma gangrenosum and acne syndrome (PAPA), adult-onset Still's disease (AOSD), haploinsufficiency of A20 (HA20), pediatric granulomatous arthritis (PGA), PLCG2-associated antibody deficiency and immune dysregulation (PLAID), PLCG2-associated autoinflammatory, antibody deficiency and immune dysregulation (APLAID), or sideroblastic anemia with B-cell immunodeficiency, periodic fevers and developmental delay (SIFD).

In certain embodiments, provided is a compound disclosed herein, or a pharmaceutically acceptable salt, isotopically enriched analog, stereoisomer, or mixture of stereoisomers for use in a method for treating a disease or condition selected from an autoinflammatory disorder and/or an autoimmune disorder selected from cryopyrin-associated autoinflammatory syndrome (CAPS; e.g., familial cold autoinflammatory syndrome (FCAS)), Muckle-Wells syndrome (MWS), chronic infantile neurological cutaneous and articular (CINCA) syndrome, neonatal-onset multisystem inflammatory disease (NOMID), familial Mediterranean fever and nonalcoholic fatty liver disease (NAFLD), non-alcoholic steatohepatitis (NASH), gout, rheumatoid arthritis, osteoarthritis, Crohn's disease, chronic obstructive pulmonary disease (COPD), chronic kidney disease (CKD), fibrosis, obesity, type 2 diabetes, and multiple sclerosis and neuroinflammation occurring in protein misfolding diseases (e.g., Prion diseases) comprising administering to a subject in need thereof a therapeutically effective amount of the compound.

In certain embodiments, provided is a compound disclosed herein, or a pharmaceutically acceptable salt, isotopically enriched analog, stereoisomer, or mixture of stereoisomers for use in a method for treating a disease or condition selected from cryopyrin-associated periodic syndromes (CAPS), Muckle-Wells syndrome (MWS), familial cold autoinflammatory syndrome (FCAS), neonatal onset multisystem inflammatory disease (NOMID), familial Mediterranean fever (FMF), pyogenic arthritis, pyoderma gangrenosum and acne syndrome (PAPA); hyperimmunoglobulinemia D and periodic fever syndrome (HIDS), tumor necrosis factor (TNF) receptor-associated periodic syndrome (TRAPS), systemic juvenile idiopathic arthritis, adult-onset Still's disease (AOSD), relapsing polychondritis, Schnitzler's syndrome, Sweet's syndrome, Behcet's disease, anti-synthetase syndrome, deficiency of interleukin 1 receptor antagonist (DIRA), and haploinsufficiency of A20 (HA20) comprising administering to a subject in need thereof a therapeutically effective amount of the compound.

In certain embodiments, provided is a compound disclosed herein, or a pharmaceutically acceptable salt, isotopically enriched analog, stereoisomer, or mixture of stereoisomers for use in a method for treating a disease or condition selected from Alzheimer's disease, atherosclerosis, asthma, allergic airway inflammation, cryopyrin-associated periodic syndromes, gout, inflammatory bowel disease and related disorders, nonalcoholic fatty liver disease (NAFLD), nonalcoholic steatohepatitis (NASH), hypertension, myocardial infarction, multiple sclerosis, experimental autoimmune encephalitis, oxalate-induced nephropathy, hyperinflammation following influenza infection, graft-versus-host disease, stroke, silicosis, type 1 diabetes, obesity-induced inflammation or insulin resistance, rheumatoid arthritis, myelodysplastic syndrome, contact hypersensitivity, joint inflammation triggered by chikungunya virus, or traumatic brain injury comprising administering to a subject in need thereof a therapeutically effective amount of the compound.

In certain embodiments, provided is a compound disclosed herein or a pharmaceutically acceptable salt, isotopically enriched analog, stereoisomer, or mixture of stereoisomers for use in a method for treating a disease or condition that is mediated, at least in part, by TNF-α. In certain embodiments, the disease or condition is resistant to treatment with an anti-TNF-α agent. In some embodiments, the disease is a gut disease or condition. In some embodiments the disease or condition is inflammatory bowel disease, Crohn's disease, or ulcerative colitis. In some embodiments, a compound disclosed herein or a pharmaceutically acceptable salt, isotopically enriched analog, stereoisomer, or mixture of stereoisomers is for use by administeration in combination with an anti-TNF-α agent. In some embodiments, the anti-TNF-α agent is Infliximab, Etanercept, Certolizumab pegol, Golimumab, or Adalimumab.

In certain embodiments, the disease or condition is an autoinflammatory disorder, an autoimmune disorder, a neurodegenerative disease, or cancer.

In certain embodiments, the disease or condition is an autoinflammatory disorder and/or an autoimmune disorder.

In certain embodiments, the disease or condition is a neurodegenerative disease.

In certain embodiments, the disease or condition is Parkinson's disease or Alzheimer's disease.

In certain embodiments, provided is a method for treating cancer, comprising administering an effective amount of a compound disclosed herein, or a pharmaceutically acceptable salt, isotopically enriched analog, stereoisomer, or mixture of stereoisomers, to a subject in need thereof.

In certain embodiments, the cancer is metastasizing cancer, gastrointestinal cancer, skin cancer, non-small-cell lung carcinoma, or colorectal adenocarcinoma.

In certain embodiments, provided is a compound as disclosed herein, or a pharmaceutically acceptable salt, isotopically enriched analog, stereoisomer, or mixture of stereoisomers for use in treating a neurodegenerative disease (e.g., Parkinson's disease or Alzheimer's disease) in a subject in need thereof.

In certain embodiments, provided is a compound as disclosed herein, or a pharmaceutically acceptable salt, isotopically enriched analog, stereoisomer, or mixture of stereoisomers, for use in treating cancer in a subject in need thereof.

In certain embodiments, a compound as disclosed herein, or a pharmaceutically acceptable salt, isotopically enriched analog, stereoisomer, or mixture of stereoisomers, may be for use by administration alone as a sole therapy or can be administered in addition with one or more other substances and/or treatments. Such conjoint treatment may be achieved by way of the simultaneous, sequential or separate administration of the individual components of the treatment.

For example, therapeutic effectiveness may be enhanced by administration of an adjuvant (i.e., by itself the adjuvant may only have minimal therapeutic benefit, but in combination with another therapeutic agent, the overall therapeutic benefit to the individual is enhanced). Alternatively, by way of example only, the benefit experienced by an individual may be increased by administering compound as disclosed herein, or a pharmaceutically acceptable salt, isotopically enriched analog, stereoisomer, or mixture of stereoisomers, with another therapeutic agent (which also includes a therapeutic regimen) that also has therapeutic benefit.

Other embodiments include use of the presently disclosed compounds in therapy.

### 4. Kits

Provided herein are also kits that include a compound of the disclosure, or a pharmaceutically acceptable salt, isotopically enriched analog, stereoisomer, or mixture of stereoisomers, and suitable packaging. In certain embodiments, a kit further includes instructions for use. In one aspect, a kit includes a compound of the disclosure, or a pharmaceutically acceptable salt, isotopically enriched analog, stereoisomer, or mixture of stereoisomers, and a label and/or instructions for use of the compounds in the treatment of the indications, including the diseases or conditions, described herein.

Provided herein are also articles of manufacture that include a compound described herein or a pharmaceutically acceptable salt, isotopically enriched analog, stereoisomer, or mixture of stereoisomers in a suitable container. The container may be a vial, jar, ampoule, preloaded syringe, or intravenous bag.

### 5. Pharmaceutical Compositions and Modes of Administration

Compounds provided herein are usually administered in the form of pharmaceutical compositions. Thus, provided herein are also pharmaceutical compositions that contain one or more of the compounds described herein, or a pharmaceutically acceptable salt, stereoisomer, or mixture of stereoisomers, and one or more pharmaceutically acceptable vehicles selected from carriers, adjuvants, and excipients. Suitable pharmaceutically acceptable vehicles may include, for example, inert solid diluents and fillers, diluents, including sterile aqueous solution and various organic solvents, permeation enhancers, solubilizers, and adjuvants. Such compositions are prepared in a manner well known in the pharmaceutical art. See, e.g., Remington's Pharmaceutical Sciences, Mace Publishing Co., Philadelphia, Pa. 17th Ed. (1985); and Modern Pharmaceutics, Marcel Dekker, Inc. 3rd Ed. (G.S. Banker & C.T. Rhodes, Eds.).

The pharmaceutical compositions may be for use by administeration in either single or multiple doses. The pharmaceutical composition may be for use administration by various methods including, for example, rectal, buccal, intranasal, and transdermal routes. In certain embodiments, the pharmaceutical composition may be for use by administeration by intra-arterial injection, intravenously, intraperitoneally, parenterally, intramuscularly, subcutaneously, orally, topically, or as an inhalant.

One mode for administration is parenteral, for example, by injection. The forms in which the pharmaceutical compositions described herein may be incorporated for administration by injection include, for example, aqueous or oil suspensions, or emulsions, with sesame oil, corn oil, cottonseed oil, or peanut oil, as well as elixirs, mannitol, dextrose, or a sterile aqueous solution, and similar pharmaceutical vehicles.

Oral administration may be another route for administration of the compounds described herein. Administration may be via, for example, capsule or enteric coated tablets. In making the pharmaceutical compositions that include at least one compound described herein or a pharmaceutically acceptable salt, isotopically enriched analog, stereoisomer, or mixture of stereoisomers, the active ingredient is usually diluted by an excipient and/or enclosed within such a carrier that can be in the form of a capsule, sachet, paper or other container. When the excipient serves as a diluent, it can be in the form of a solid, semisolid, or liquid material, which acts as a vehicle, carrier or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing, for example, up to 10% by weight of the active compound, soft and hard gelatin capsules, sterile injectable solutions, and sterile packaged powders.

Some examples of suitable excipients include, e.g., lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, sterile water, syrup, and methyl cellulose. The formulations can additionally include lubricating agents such as talc, magnesium stearate, and mineral oil; wetting agents; emulsifying and suspending agents; preserving agents such as methyl and propylhydroxybenzoates; sweetening agents; and flavoring agents.

The compositions that include at least one compound described herein or a pharmaceutically acceptable salt, isotopically enriched analog, stereoisomer, or mixture of stereoisomers can be formulated so as to provide quick, sustained or delayed release of the active ingredient after administration to the subject by employing procedures known in the art. Controlled release drug delivery systems for oral administration include osmotic pump systems and dissolutional systems containing polymer-coated reservoirs or drug-polymer matrix formulations. Another formulation for use in the methods disclosed herein employ transdermal delivery devices ("patches"). Such transdermal patches may be used to provide continuous or discontinuous infusion of the compounds described herein in controlled amounts. The construction and use of transdermal patches for the delivery of pharmaceutical agents is well known in the art. Such patches may be constructed for continuous, pulsatile, or on demand delivery of pharmaceutical agents.

For preparing solid compositions such as tablets, the principal active ingredient may be mixed with a pharmaceutical excipient to form a solid preformulation composition containing a homogeneous mixture of a compound described herein or a pharmaceutically acceptable salt, isotopically enriched analog, stereoisomer, or mixture of stereoisomers. When referring to these preformulation compositions as homogeneous, the active ingredient may be dispersed evenly throughout the composition so that the composition may be readily subdivided into equally effective unit dosage forms such as tablets, pills, and capsules.

The tablets or pills of the compounds described herein may be coated or otherwise compounded to provide a dosage form affording the advantage of prolonged action, or to protect from the acid conditions of the stomach. For example, the tablet or pill can include an inner dosage and an outer dosage component, the latter being in the form of an envelope over the former. The two components can be separated by an enteric layer that serves to resist disintegration in the stomach and permit the inner component to pass intact into the duodenum or to be delayed in release. A variety of materials can be used for such enteric layers or coatings, such materials including a number of polymeric acids and mixtures of polymeric acids with such materials as shellac, cetyl alcohol, and cellulose acetate.

Compositions for inhalation or insufflation may include solutions and suspensions in pharmaceutically acceptable, aqueous or organic solvents, or mixtures thereof, and powders. The liquid or solid compositions may contain suitable pharmaceutically acceptable excipients as described herein. In some embodiments, the compositions are administered by the oral or nasal respiratory route for local or systemic effect. In other embodiments, compositions in pharmaceutically acceptable solvents may be nebulized by use of inert gases. Nebulized solutions may be inhaled directly from the nebulizing device or the nebulizing device may be attached to a facemask tent, or intermittent positive pressure breathing machine. Solution, suspension, or powder compositions may be administered, preferably orally or nasally, from devices that deliver the formulation in an appropriate manner.

### 6. Dosing

The specific dose level of a compound of the present application for any particular subject will depend upon a variety of factors including the activity of the specific compound employed, the age, body weight, general health, sex, diet, time of administration, route of administration, and rate of excretion, drug combination and the severity of the particular disease in the subject undergoing therapy. For example, a dosage may be expressed as a number of milligrams of a compound described herein per kilogram of the subject's body weight (mg/kg). Dosages of between about 0.1 and 150 mg/kg may be appropriate. In some embodiments, about 0.1 and 100 mg/kg may be appropriate. In other embodiments a dosage of between 0.5 and 60 mg/kg may be appropriate. In some embodiments, a dosage of from about 0.0001 to about 100 mg per kg of body weight per day, from about 0.001 to about 50 mg of compound per kg of body weight, or from about 0.01 to about 10 mg of compound per kg of body weight may be appropriate. Normalizing according to the subject's body weight is particularly useful when adjusting dosages between subjects of widely disparate size, such as occurs when using the drug in both children and adult humans or when converting an effective dosage in a non-human subject such as dog to a dosage suitable for a human subject.

### 7. Synthesis of the Compounds

The compounds may be prepared using the methods disclosed herein and routine modifications thereof, which will be apparent given the disclosure herein and methods well known in the art. Conventional and well-known synthetic methods may be used in addition to the teachings herein. The synthesis of typical compounds described herein may be accomplished as described in the following examples. If available, reagents and starting materials may be purchased commercially, e.g., from Sigma Aldrich or other chemical suppliers.

It will be appreciated that where typical or preferred process conditions (i.e., reaction temperatures, times, mole ratios of reactants, solvents, pressures, etc.) are given, other process conditions can also be used unless otherwise stated. Optimum reaction conditions may vary with the particular reactants or solvent used, but such conditions can be determined by one skilled in the art by routine optimization procedures.

Additionally, conventional protecting groups ("PG") may be necessary to prevent certain functional groups from undergoing undesired reactions. Suitable protecting groups for various functional groups as well as suitable conditions for protecting and deprotecting particular functional groups are well known in the art. For example, numerous protecting groups are described in Wuts, P. G. M., Greene, T. W., & Greene, T. W. (2006). Greene's protective groups in organic synthesis. Hoboken, N.J., Wiley-Interscience, and references cited therein. For example, protecting groups for alcohols, such as hydroxy, include silyl ethers (including trimethylsilyl (TMS), tert-butyldimethylsilyl (TBDMS), tri-iso-propylsilyloxymethyl (TOM), and triisopropylsilyl (TIPS) ethers), which can be removed by acid or fluoride ion, such as NaF, TBAF (tetra-n-butylammonium fluoride), HF-Py, or HF-NEt₃. Other protecting groups for alcohols include acetyl, removed by acid or base, benzoyl, removed by acid or base, benzyl, removed by hydrogenation, methoxyethoxymethyl ether, removed by acid, dimethoxytrityl, removed by acid, methoxymethyl ether, removed by acid, tetrahydropyranyl or tetrahydrofuranyl, removed by acid, and trityl, removed by acid. Examples of protecting groups for amines include carbobenzyloxy, removed by hydrogenolysis p-methoxybenzyl carbonyl, removed by hydrogenolysis, tert-butyloxycarbonyl, removed by concentrated strong acid (such as HCl or CF₃COOH), or by heating to greater than about 80 °C, 9-fluorenylmethyloxycarbonyl, removed by base, such as piperidine, acetyl, removed by treatment with a base, benzoyl, removed by treatment with a base, benzyl, removed by hydrogenolysis, carbamate group, removed by acid and mild heating, p-methoxybenzyl, removed by hydrogenolysis, 3,4-dimethoxybenzyl, removed by hydrogenolysis, p-methoxyphenyl, removed by ammonium cerium(IV) nitrate, tosyl, removed by concentrated acid (such as HBr or H₂SO₄) and strong reducing agents (sodium in liquid ammonia or sodium naphthalenide), troc (trichloroethyl chloroformate), removed by Zn insertion in the presence of acetic acid, and sulfonamides (Nosyl & Nps), removed by samarium iodide or tributyltin hydride.

Furthermore, the compounds of this disclosure may contain one or more chiral centers. Accordingly, if desired, such compounds can be prepared or isolated as pure stereoisomers, i.e., as individual enantiomers or diastereomers or as stereoisomer-enriched mixtures. All such stereoisomers (and enriched mixtures) are included within the scope of this disclosure, unless otherwise indicated. Pure stereoisomers (or enriched mixtures) may be prepared using, for example, optically active starting materials or stereoselective reagents well-known in the art. Alternatively, racemic mixtures of such compounds can be separated using, for example, chiral column chromatography, chiral resolving agents, and the like.

The starting materials for the following reactions are generally known compounds or can be prepared by known procedures or obvious modifications thereof. For example, many of the starting materials are available from commercial suppliers such as Aldrich Chemical Co. (Milwaukee, Wisconsin, USA), Bachem (Torrance, California, USA), Emka-Chemce or Sigma (St. Louis, Missouri, USA). Others may be prepared by procedures or obvious modifications thereof, described in standard reference texts such as Fieser and Fieser's Reagents for Organic Synthesis, Volumes 1-15 (John Wiley, and Sons, 1991), Rodd's Chemistry of Carbon Compounds, Volumes 1-5, and Supplementals (Elsevier Science Publishers, 1989) organic Reactions, Volumes 1-40 (John Wiley, and Sons, 1991), March's Advanced Organic Chemistry, (John Wiley, and Sons, 5th Edition, 2001), and Larock's Comprehensive Organic Transformations (VCH Publishers Inc., 1989).

### General Synthesis

Scheme I illustrates a general methods which can be employed for the synthesis of compounds described herein, where each of X, Y¹, Y², A¹-A⁵, R², R³, R⁴, R⁷, and R⁸ are independently as defined herein, each R⁵⁰ is independently C₁₋₆ alkyl or two R⁵⁰ together with the atoms to which they are attached form a ring, and each LG is a leaving group (e.g., halo). It should be understood that derivatization of any one or more of compounds I-1, I-3, and I-5, or any product obtained by the process outlined in Scheme I, can be performed to provide various compounds of Formula I.

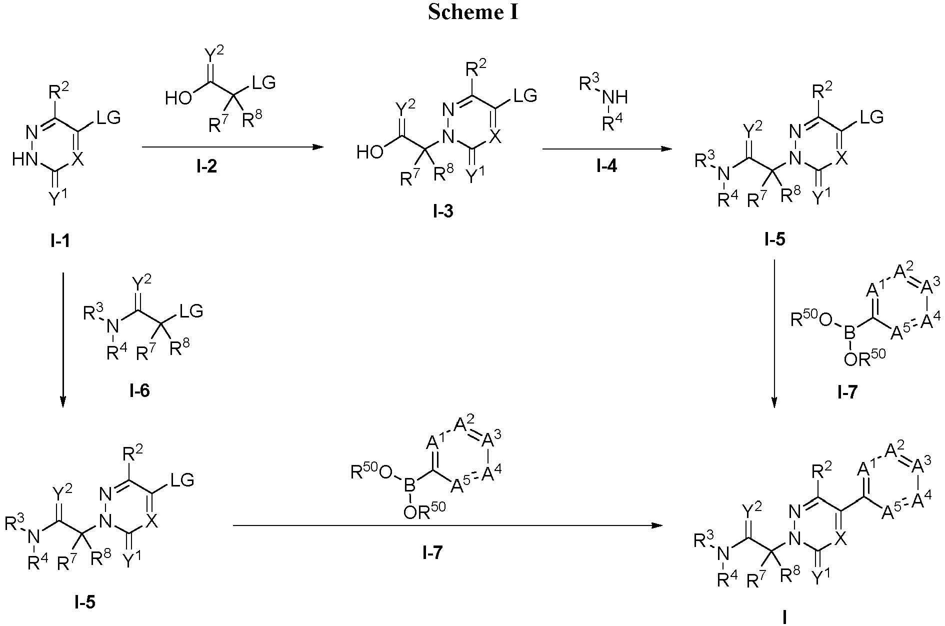

In Scheme I, coupling of compound I-1 with compound I-2 provides compound I-3, which upon reaction with an appropriately substituted amine I-4 under amide bond forming reaction conditions, yields Compound I-5. Alternatively, compound I-5 can be prepared from compound I-1 by coupling with compound I-6. Compounds of formula I are provided by contacting compound I-5 with appropriately substituted compound I-7 under standard metal-catalyzed coupling conditions.

Alternatively, as shown in Scheme II, compounds of formula I can be provided by first coupling compound I-1 with compound I-7 under standard metal-catalyzed coupling conditions to provide compound II-1, followed by either compounds I-2 then I-4 or compound I-6 under similar reaction conditions described above.

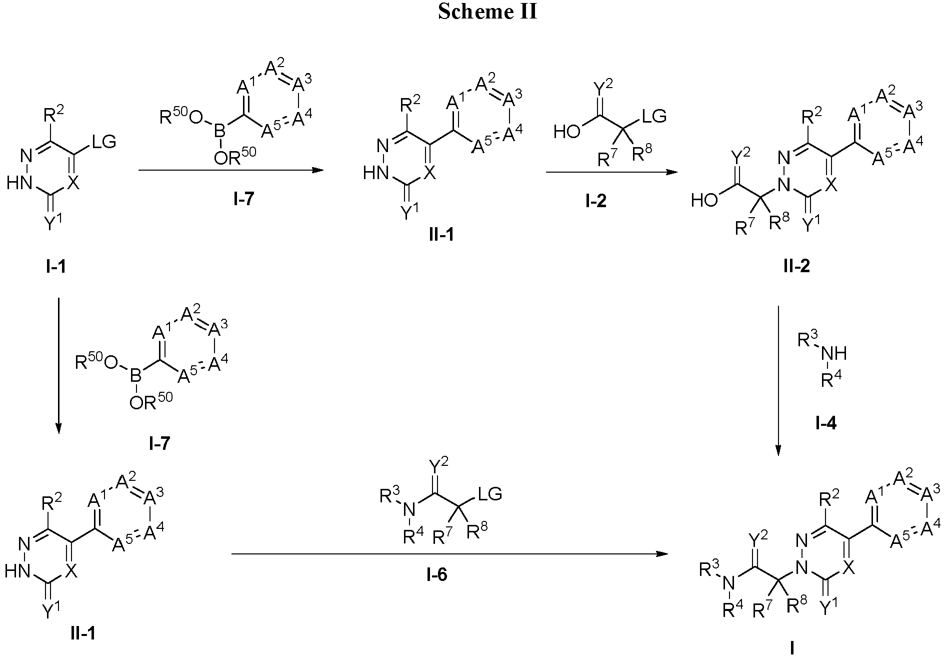

Appropriate starting materials and reagents can be purchased or prepared by methods known to one of skill in the art. Upon each reaction completion, each of the intermediate or final compounds can be recovered, and optionally purified, by conventional techniques such as neutralization, extraction, precipitation, chromatography, filtration and the like.

In some embodiments, the various substituents of compounds I-1, I-2, I-3, I-4, I-5, I-6, I-7, and II-1 as used in Schemes I and II are as defined for Formula I. However, derivatization of compounds I-1, I-2, I-3, I-4, I-5, I-6, I-7, and II-1 provides various compounds of Formula I.

For example, starting from compound II-1 where R² is halo, derivatization at R² can be performed via functional group interconversion to provide various starting materials for use in the schemes above (see, e.g., Scheme III). Such methods are known to one of skill in the art.

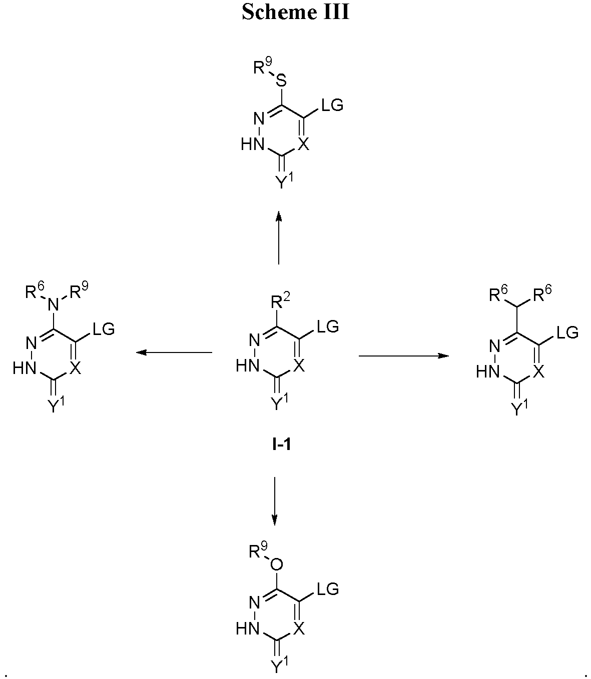

In certain embodiments, provided is a process for preparing a compound of Formula I, comprising contacting a compound of Formula I-5 with a compound of Formula I-7, under conditions suitable to provide a compound of Formula I.

In certain embodiments, provided is a process for preparing a compound of Formula I, comprising:
contacting a compound of Formula I-3 with a compound of Formula I-4, under conditions suitable to provide a compound of Formula I-5; and
contacting a compound of Formula I-5 with a compound of Formula I-7, under conditions suitable to provide a compound of Formula I.

In certain embodiments, provided is a process for preparing a compound of Formula I, comprising:
contacting a compound of Formula I-1 with a compound of Formula I-2, under conditions suitable to provide a compound of Formula I-3;
contacting a compound of Formula I-3 with a compound of Formula I-4, under conditions suitable to provide a compound of Formula I-5; and
contacting a compound of Formula I-5 with a compound of Formula I-7, under conditions suitable to provide a compound of Formula I.

In certain embodiments, provided is a process for preparing a compound of Formula I, comprising:
contacting a compound of Formula I-1 with a compound of Formula I-6, under conditions suitable to provide a compound of Formula I-5; and
contacting a compound of Formula I-5 with a compound of Formula I-7, under conditions suitable to provide a compound of Formula I.

In certain embodiments, provided is a process for preparing a compound of Formula I, comprising contacting a compound of Formula II-1 with a compound of Formula I-6, under conditions suitable to provide a compound of Formula I.

In certain embodiments, provided is a process for preparing a compound of Formula I, comprising:
contacting a compound of Formula II-1 with a compound of Formula I-2, under conditions suitable to provide a compound of Formula II-2; and
contacting a compound of Formula II-2 with a compound of Formula I-4, under conditions suitable to provide a compound of Formula I.

In certain embodiments, provided is a process for preparing a compound of Formula I, comprising:
contacting a compound of Formula I-1 with a compound of Formula I-7, under conditions suitable to provide a compound of Formula II-1; and
contacting a compound of Formula II-1 with a compound of Formula I-6, under conditions suitable to provide a compound of Formula I.

In certain embodiments, provided is a process for preparing a compound of Formula I, comprising:
contacting a compound of Formula I-1 with a compound of Formula I-7, under conditions suitable to provide a compound of Formula II-1;
contacting a compound of Formula II-1 with a compound of Formula I-2, under conditions suitable to provide a compound of Formula II-2; and
contacting a compound of Formula II-2 with a compound of Formula I-4, under conditions suitable to provide a compound of Formula I.

### EXAMPLES

The following examples are included to demonstrate specific embodiments of the disclosure. It should be appreciated by those of skill in the art that the techniques disclosed in the examples which follow represent techniques to function well in the practice of the disclosure, and thus can be considered to constitute specific modes for its practice. However, those of skill in the art should, in light of the present disclosure, appreciate that many changes can be made in the specific embodiments which are disclosed and still obtain a like or similar result.

### General Experimental Methods

All solvents used were commercially available and were used without further purification. Reactions were typically run using anhydrous solvents under an inert atmosphere of nitrogen.

**NMR Spectroscopy:** ¹H Nuclear magnetic resonance (NMR) spectroscopy was carried out using a Bruker Avance III equipped with a BBFO 300 MHz probe operating at 300 MHz or one of the following instruments: a Bruker Avance 400 instrument equipped with probe DUAL 400 MHz S1, a Bruker Avance 400 instrument equipped with probe 6 S1 400 MHz 5mm ¹H-¹³C ID, a Bruker Avance III 400 instrument with nanobay equipped with probe Broadband BBFO 5 mm direct, a Bruker Mercury Plus 400 NMR spectrometer equipped with a Bruker 400 BBO probe operating at 400 MHz. All deuterated solvents contained typically 0.03% to 0.05% v/v tetramethylsilane, which was used as the reference signal (set at δ 0.00 for both ¹H and ¹³C). In certain cases, ¹H Nuclear magnetic resonance (NMR) spectroscopy was carried out using a Bruker Advance 400 instrument operating at 400 MHz using the stated solvent at around room temperature unless otherwise stated. In all cases, NMR data were consistent with the proposed structures. Characteristic chemical shifts (δ) are given in parts-per-million using conventional abbreviations for designation of major peaks: e.g. s, singlet; d, doublet; t, triplet; q, quartet; dd, doublet of doublets; dt, doublet of triplets; br, broad.

**Thin Layer Chromatography:** Where thin layer chromatography (TLC) has been used it refers to silica gel TLC using silica gel F254 (Merck) plates, Rf is the distance travelled by the compound divided by the distance travelled by the solvent on a TLC plate. Column chromatography was performed using an automatic flash chromatography system over silica gel cartridges or in the case of reverse phase chromatography over C18 cartridges. Alternatively, thin layer chromatography (TLC) was performed on Alugram^{®} (Silica gel 60 F254) from Mancherey-Nagel and UV was typically used to visualize the spots. Additional visualization methods were also employed in some cases. In these cases the TLC plate was developed with iodine (generated by adding approximately 1 g of I₂ to 10 g silica gel and thoroughly mixing), ninhydrin (available commercially from Aldrich), or Magic Stain (generated by thoroughly mixing 25 g (NH₄)₆Mo₇O₂₄.4H₂O, 5 g (NH₄)₂Ce(IV)(NO₃)₆ in 450 mL water and 50 mL concentrated H₂SO₄) to visualize the compound.

**Liquid Chromatography-Mass Spectrometry and HPLC Analysis:** HPLC analysis was performed on Shimadzu 20AB HPLC system with a photodiode array detector and Luna-C18(2) 2.0×50 mm, 5 µm column at a flow rate of 1.2 mL/min with a gradient solvent Mobile phase A (MPA, H₂O+0.037 % (v/v) TFA): Mobile phase B (MPB, ACN+0.018 % (v/v) TFA) (0.01 min, 10% MPB; 4 min, 80% MPB; 4,9 min, 80% MPB; 4.92 min, 10% MPB; 5.5 min, 10% MPB). LCMS was detected under 220 and 254 nm or used evaporative light scattering (ELSD) detection as well as positive electrospray ionization (MS). Semi-preparative HPLC was performed by either acidic or neutral conditions. Acidic: Luna C18 100 × 30 mm, 5 µm; MPA: HCl/H₂O=0.04%, or formic acid/H₂O=0.2% (v/v); MPB: ACN. Neutral: Waters Xbridge 150 × 25, 5 µm; MPA: 10 mM NH₄HCO₃ in H₂O; MPB: ACN. Gradient for both conditions: 10% of MPB to 80% of MPB over 12 min at a flow rate of 20 mL/min, then 100% MPB over 2 min, 10% MPB over 2 min, UV detector. SFC analysis was performed on Thar analytical SFC system with a UV/Vis detector and series of chiral columns including AD, AS-H, OJ, OD, AY and IC, 4.6 × 100 mm, 3 µm column at a flow rate of 4 mL/min with a gradient solvent Mobile phase A (MPA, CO₂): Mobile phase B (MPB, MeOH+0.05 % (v/v) IPAm) (0.01 min, 10% MPB; 3 min, 40% MPB; 3.5 min, 40% MPB; 3.56-5 min, 10% MPB). SFC preparative was performed on Thar 80 preparative SFC system with a UV/Vis detector and series of chiral preparative columns including AD-H, AS-H, OJ-H, OD-H, AY-H and IC-H, 30×250 mm, 5 µm column at a flow rate of 65 mL/min with a gradient solvent Mobile phase A (MPA, CO₂): Mobile phase B (MPB, MeOH+0.1 % (v/v) NH₃H₂O) (0.01 min, 10% MPB; 5 min, 40% MPB; 6 min, 40% MPB; 6.1-10 min, 10% MPB). LC-MS data were also collected using an UPLC-MS Acquity^{™} system equipped with PDA detector and coupled to a Waters single quadrupole mass spectrometer operating in alternated positive and negative electrospray ionization mode. The column used was a Cortecs UPLC C18, 1.6 µm, 2.1 × 50 mm. A linear gradient was applied, starting at 95% A (A: 0.1% formic acid in water) and ending at 95% B (B: 0.1% formic acid in MeCN) over 2.0 min with a total run time of 2.5 min. The column temperature was at 40 °C with the flow rate of 0.8 mL/min.

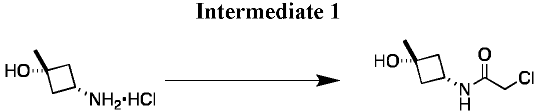

**2-chloro-*N*-(*cis*-3-hydroxy-3-methylcyclobutyl)acetamide**: To a solution of *cis*-3-amino-1-methylcyclobutanol HCl salt (1.1 g, 7.99 mmol) in DCM (15 mL) was added DMF (2 mL) and *N-*methylmorpholine (2.43 g, 24.0 mmol). To the reaction mixture was added a solution of 2-chloroacetyl chloride (903 mg, 7.99 mmol) in DCM (2 mL) dropwise at -78 °C. The reaction mixture was stirred at 20 °C for 2 h. The reaction mixture was then concentrated under reduced pressure. The crude residue was purified by silica gel chromatography. ¹H NMR (400 MHz, CDCl₃): δ 6.81 (br s, 1H), 4.10-3.96 (m, 3H), 2.59-2.48 (m, 2H), 2.14-2.04 (m, 2H), 1.39 (s, 3H).

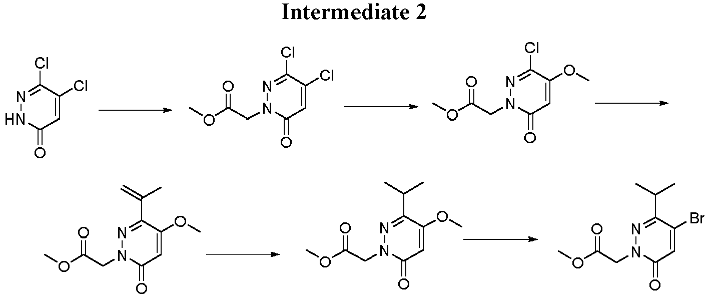

**Methyl 2-(3,4-dichloro-6-oxopyridazin-1(6*H*)-yl)acetate**: To a solution of 5,6-dichloropyridazin-3(2*H*)-one (40 g, 242 mmol) and methyl 2-bromoacetate (37.8 g, 247 mmol) in DMF (500 mL) was added Cs₂CO₃ (79 g, 242 mmol). The reaction mixture was stirred at 15 °C for 1 h. The reaction mixture was poured onto water (2000 mL) and extracted with EtOAc (3 × 700 mL). The combined organic layers were washed with brine (700 mL), dried over anhydrous Na₂SO₄, filtered, and concentrated under reduced pressure to provide a residue that was used directly without further purification. ¹H NMR (400 MHz, CDCl₃): δ 7.13 (s, 1H), 4.83 (s, 2H), 3.79 (s, 3H).

**Methyl 2-(3-chloro-4-methoxy-6-oxopyridazin-1(6*H*)-yl)acetate**: To a solution of methyl 2-(3,4-dichloro-6-oxopyridazin-1(6*H*)-yl)acetate (58 g, 245 mmol) in MeOH (300 mL) was added MeONa (5 M in MeOH, 53.8 mL) at 0 °C. The reaction mixture was stirred at 15 °C for 1 h. The reaction mixture was poured into saturated NH₄Cl (300 mL) and concentrated under reduced pressure. The remaining aqueous solution was extracted with EtOAc (3 × 200 mL). The combined organics were washed with brine (100 mL), dried over anhydrous Na₂SO₄, filtered, and concentrated under reduced pressure. The crude residue was triturated with MTBE (500 mL) and then filtered to provide the desired product. ¹H NMR (400 MHz, CDCl₃): δ 6.22 (s, 1H), 4.81 (s, 2H), 3.90 (s, 3H), 3.78 (s, 3H).

**Methyl 2-(4-methoxy-6-oxo**-**3-(prop-1-en-2-yl)pyridazin-1(6*H*)-yl)acetate**: To a mixture of methyl 2-(3-chloro-4-methoxy-6-oxopyridazin-1(6*H*)-yl)acetate (47.6 g, 205 mmol), 2-isopropenyl-4,4,5,5-tetramethyl-1,3,2-dioxaborolane (51.5 g, 307 mmol) and Cs₂CO₃ (133 g, 409 mmol) in 1,4-dioxane (600 mL) and H₂O (150 mL) was added Pd(dppf)Cl₂ (7.49 g, 10.2 mmol). The reaction mixture was stirred at 110 °C for 16 h. The reaction mixture was quenched by addition of water (1000 mL) and extracted with DCM (3 × 400 mL). The combined organic layers were washed with brine (500 mL), dried over anhydrous Na₂SO₄, filtered, and concentrated under reduced pressure. The crude residue was purified by silica gel column chromatography. LCMS: *m*/*z* = 239.2 [M+H]⁺. ¹H NMR (400 MHz, CDCl₃): δ 6.18 (s, 1H), 5.64 (dd, *J* = 0.8, 1.5 Hz, 1H), 5.41 (quin, *J* = 1.6 Hz, 1H), 4.85 (s, 2H), 3.84 (s, 3H), 3.77 (s, 3H), 2.08 - 2.00 (m, 3H).

**Methyl 2-(3-isopropyl-4-methoxy-6-oxopyridazin-1(6*H*)-yl)acetate**: To a solution of methyl 2-(4-methoxy-6-oxo-3-(prop-1-en-2-yl)pyridazin-1(6*H*)-yl)acetate (15 g, 63.0 mmol) in MeOH (150 mL) was added Pd (5 g, 10 wt % on carbon). The suspension was degassed under vacuum and purged with H₂ three times. The reaction mixture was stirred at 30 °C under an atmosphere of H₂ for 2 h. The reaction mixture was filtered and the filtrate was concentrated under reduced pressure. The resultant residue was used directly. LCMS: *m*/*z* = 241.2 [M+H]⁺. ¹H NMR (400 MHz, CDCl₃): δ 6.13 (s, 1H), 4.93-4.72 (m, 2H), 3.82 (s, 3H), 3.77 (s, 3H), 3.13 (spt, *J* = 6.8 Hz, 1H), 1.18 (d, *J* = 6.8 Hz, 6H).

**Methyl 2-(4-bromo-3-isopropyl-6-oxopyridazin-1(6*H*)-yl)acetate**: To a solution of methyl 2-(3-isopropyl-4-methoxy-6-oxopyridazin-1(6*H*)-yl)acetate (18.8 g, 78.3 mmol) in 1,4-dioxane (200 mL) was added POBr₃ (44.9 g, 157 mmol). The reaction mixture was stirred at 110 °C for 1 h. The reaction mixture was poured onto ice-water (300 mL) and extracted with DCM (3 × 100 mL). The combined organic layers were washed with brine (100 mL), dried over anhydrous Na₂SO₄, filtered, and concentrated under reduced pressure. The crude residue was purified by silica gel column chromatography. LCMS: *m*/*z* = 289.1, 291.1 [M+H]⁺. ¹H NMR (400 MHz, CDCl₃): δ 7.27 (s, 1H), 4.85 (s, 2H), 3.79 (s, 3H), 3.27 (spt, *J* = 6.8 Hz, 1H), 1.24 (d, *J* = 6.8 Hz, 6H).

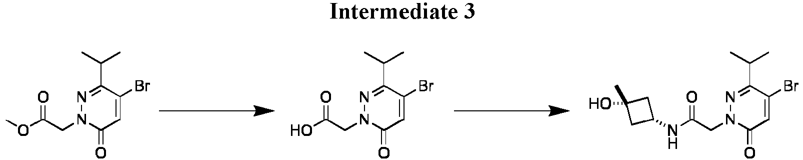

**2-(4-Bromo-3-isopropyl-6-oxopyridazin-1(6*H*)-yl) acetic acid:** To a solution of methyl 2-(4-bromo-3-isopropyl-6-oxo-pyridazin-1-yl) acetate (5.8 g, 20.1 mmol) in THF (60 mL) and H₂O (15 mL) was added LiOH•H₂O (1.68 g, 40.1 mmol). The reaction mixture was stirred at 20 °C for 1 h. The reaction mixture was poured into H₂O (100 mL) and washed with MTBE (3 × 40 mL). The aqueous layer was then adjusted to pH = 3 by addition of aqueous HCl (2 N) and extracted with EtOAc (3 × 40 mL). The combined EtOAc layers were washed with brine (40 mL), dried over anhydrous Na₂SO₄, filtered, and concentrated under reduced pressure to provide a residue that was used directly. LCMS: *m*/*z* = 275.2, 277.2 [M+H]⁺. ¹H NMR (400 MHz, CDCl₃): δ 9.40 (br s, 1H), 7.35 (s, 1H), 4.89 (s, 2H), 3.27 (m, 1H), 1.24 (d, *J* = 6.8 Hz, 6H).

**2-(4-Bromo-3-isopropyl-6-oxopyridazin-1(6*H*)-yl)-*N*-(*cis***-**3-hydroxy-3-methylcyclobutyl)acetamide:** To a solution of 2-(4-bromo-3-isopropyl-6-oxo-pyridazin-1(6*H*)-yl)acetic acid (2.0 g, 7.27 mmol) in DMF (30 mL) were added HATU (4.15 g, 10.9 mmol) and DIPEA (3.76 g, 29.1 mmol). The reaction mixture was stirred at 20 °C for 0.5 h and then *cis*-3-amino-1-methylcyclobutanol HCl salt (1.65 g, 11.96 mmol) was added. The resulting reaction mixture was stirred at 20 °C for 1 h. The reaction mixture was quenched by the addition of water (150 mL) and extracted with EtOAc (3 × 50 mL). The combined organic layers were washed with brine (50 mL), dried over anhydrous Na₂SO₄, filtered, and concentrated under reduced pressure. The crude residue was purified by reverse-phase HPLC. LCMS: *m*/*z* = 358.1, 360.1 [M+H]⁺. ¹H NMR (400 MHz, CDCl₃) δ 7.29 (s, 1H), 6.60 (br d, *J* = 7.2 Hz, 1H), 4.74 (s, 2H), 3.98 (m, 1H), 3.22-3.20 (m, 1H), 3.28 (m, 1H), 2.57-2.46 (m, 2H), 2.09-1.96 (m, 2H), 1.37 (s, 3H), 1.25 (d, *J* = 6.8 Hz, 6H).

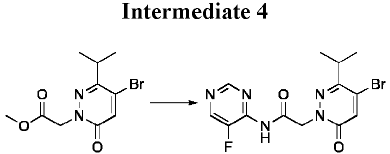

**2-(4-Bromo-3-isopropyl-6-oxopyridazin-1(6*H*)-yl)-*N*-(5-fluoropyrimidin-4-yl)acetamide:** To a solution of methyl 2-(4-bromo-3-isopropyl-6-oxo-pyridazin-1-yl)acetate (3.0 g, 10.4 mmol) and 5-fluoropyrimidin-4-amine (3.5 g, 31.1 mmol) in toluene (50 mL) and THF (50 mL) was added AlMe₃ (2 M in toluene, 15.6 mL). The reaction mixture was stirred at 110 °C for 12 h. The reaction mixture was diluted with water (120 mL) and extracted with EtOAc (3 × 40 mL). The combined organic layers were washed with brine (40 mL), dried over anhydrous Na₂SO₄, filtered, and concentrated under reduced pressure. The crude residue was purified silica gel column chromatography and by reverse-phase preparative HPLC. LCMS: *m*/*z* = 370.0, 372.0 [M+H]⁺.

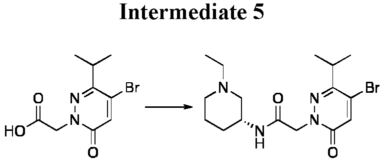

**(*R*)-2-(4-Bromo-3-isopropyl-6-oxopyridazin-1(6*H*)-yl)-*N*-(1-ethylpiperidin-3-yl)acetamide:** To a solution of 2-(4-bromo-3-isopropyl-6-oxopyridazin-1(6*H*)-yl)acetic acid (700 mg, 2.54 mmol) in DMF (8 mL) were added (R)-1-ethylpiperidin-3-amine hydrochloride (563 mg, 2.80 mmol), DIPEA (1.32 g, 10.2 mmol) and HATU (1.94 g, 5.09 mmol). The reaction mixture was stirred at 20 °C for 2 h. The reaction mixture was filtered, and the filtrate was concentrated under reduced pressure to give a residue that was purified by reverse-phase preparative HPLC. LCMS: *m*/*z* = 385.0, 387.0 [M+H]⁺.

### Example 1

### N-(cis-3-hydroxy-3-methylcyclobutyl)-2-(3-isopropyl-6-oxo-4-phenylpyridazin-1(6H)-yl)acetamide

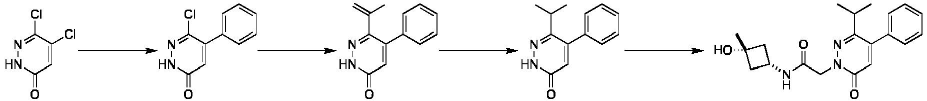

**6-Chloro-5-phenylpyridazin-3(2*H*)-one:** To a mixture of 5,6-dichloropyridazin-3(2H)-one (500 mg, 3.03 mmol), phenylboronic acid (296 mg, 2.42 mmol), and K₂CO₃ (838 mg, 6.06 mmol) in H₂O (1 mL) and 1,4-dioxane (10 mL) was added Pd(dppf)Cl₂ (222 mg, 0.30 mmol) The reaction mixture was stirred at 100 °C for 3 h. The reaction mixture was filtered. The filtrate was poured into H₂O (20 mL) and extracted with EtOAc (3 × 10 mL). The combined organic layers were washed with brine (2 × 10 mL), dried over anhydrous Na₂SO₄, filtered, and concentrated under reduced pressure. The crude residue was purified by preparative TLC. LCMS: *m*/*z* = 207.0 [M+H]⁺. ¹H NMR (400 MHz, CDCl₃): δ 12.49 (br s, 1H), 7.63-7.41 (m, 5H), 6.98 (s, 1H).

**5-Phenyl-6-(prop-1-en-2-yl)pyridazin-3(2*H*)-one:** To a solution of 6-chloro-5-phenylpyridazin-3(2H)-one (220 mg, 1.06 mmol) and 4,4,5,5-tetramethyl-2-(prop-1-en-2-yl)-1,3,2-dioxaborolane (267 mg, 1.59 mmol) in 1,4-dioxane (5 mL) and H₂O (0.5 mL) were added Pd(dppf)Cl₂ (78 mg, 0.11 mmol) and K₂CO₃ (293 mg, 2.12 mmol). The reaction mixture was stirred at 100 °C for 16 h. The reaction mixture was filtered. The filtrate was poured into H₂O (10 mL) and extracted with EtOAc (3 × 5 mL). The combined organic layers were washed with brine (2 × 5 mL), dried over anhydrous Na₂SO₄, filtered, and concentrated under reduced pressure. The crude residue was purified by preparative TLC. LCMS: *m*/*z* = 213.1 [M+H]⁺. ¹H NMR (400 MHz, CDCl₃): δ 7.46-7.33 (m, 5H), 6.87 (s, 1H), 5.23-5.17 (m, 1H), 4.96 (s, 1H), 1.80 (s, 3H).

**6-Isopropyl-5-phenylpyridazin-3(2*H*)-one:** To a solution of 5-phenyl-6-(prop-1-en-2-yl)pyridazin-3(2*H*)-one (150 mg, 0.71 mmol) in MeOH (10 mL) was added Pd (70 mg, 10 wt % on carbon). The suspension was degassed under vacuum and purged with H₂ three times. The reaction mixture was stirred under H₂ (15 psi) at 15 °C for 2 h. The reaction mixture was filtered. The filtrate was concentrated under reduced pressure and the residue was used directly. LCMS: *m*/*z* = 215.1 [M+H]⁺. ¹H NMR (400 MHz, CDCl₃): δ 7.51-7.43 (m, 3H), 7.32-7.26 (m, 2H), 6.77 (s, 1H), 3.04-2.95 (m, 1H), 1.10 (d, *J=* 6.8 Hz, 6H).

***N*-(*cis*-3-hydroxy-3-methylcyclobutyl)-2-(3-isopropyl-6-oxo-4-phenylpyridazin-1(6*H*)-yl)acetamide:** To a solution of 6-isopropyl-5-phenylpyridazin-3(2H)-one (40 mg, 0.19 mmol) and 2-chloro-*N*-(*cis*-3-hydroxy-3-methylcyclobutyl)acetamide (33 mg, 0.19 mmol) in CH₃CN (2 mL) was added Cs₂CO₃ (91 mg, 0.28 mmol). The reaction mixture was stirred at 90 °C for 1 h. The reaction mixture was filtered. The filtrate was poured into H₂O (10 mL) and extracted with EtOAc (3 × 5 mL). The combined organic layers were washed with brine (2 × 5 mL), dried over anhydrous Na₂SO₄, filtered, and concentrated under reduced pressure. The crude residue was purified by reverse-phase HPLC. LCMS: *m*/*z* = 356.2 [M+H]⁺. ¹H NMR (400 MHz, CDCl₃): δ 7.55-7.40 (m, 3H), 7.32-7.26 (m, 2H), 7.08 (br d, *J* = 7.2 Hz, 1H), 6.77 (s, 1H), 4.85 (s, 2H), 4.18-3.91 (m, 1H), 3.06-2.95 (m, 1H), 2.89 (s, 1H), 2.55-2.49 (m, 2H), 2.07 (br t, *J =* 10 Hz, 2H), 1.36 (s, 3H), 1.10 (d, *J =* 6.4 Hz, 6H).

### Example 2

The following compound was, or can be, made via similar procedures as those described above.

| Ex. | Structure | Name | NMR | LCMS *m*/*z* |
|---|---|---|---|---|
| 2 | 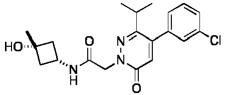 | 2-(4-(3-chlorophenyl)-3-isopropyl-6-oxopyridazin-1(6*H*)-yl)-*N*-(*cis*-3-hydroxy-3-methylcyclobutyl)ace tamide | ¹H NMR (400 MHz, CDCl₃): δ 7.39-7.49 (m, 2H), 7.31 (s, 1H), 7.20 (d, *J =* 7.2 Hz, 1H), 6.77 (s, 1H), 6.74 (br d, *J =* 7.8 Hz, 1H), 4.81-4.85 (m, 2H), 4.01 (m, 1H), 2.90-2.99 (m, 1H), 2.50-2.59 (m, 2H), 2.02-2.11 (m, 2H), 1.46-1.77 (m, 1H), 1.39 (s, 3H), 1.12 (d, *J =* 6.8 Hz, 6H). | 390.2 [M+H]⁺ |

### Example 3

### 2-(3-Ethyl-6-oxo-4-phenylpyridazin-1(6H)-yl)-N-(cis-3-hydroxy-3-methylcyclobutyl)acetamide

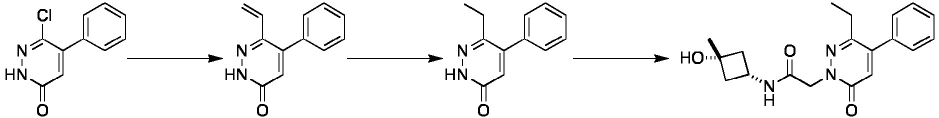

**5-Phenyl-6-vinylpyridazin-3(2*H*)-one:** To a solution of 6-chloro-5-phenylpyridazin-3(2*H*)-one (230 mg, 1.11 mmol) and potassium trifluoro(vinyl)borate (1.49 g, 11.13 mmol) in 1,4-dioxane (10 mL) and H₂O (1 mL) were added Pd(dppf)Cl₂ (81 mg, 0.11 mmol) and Cs₂CO₃ (725 mg, 2.23 mmol). The reaction mixture was stirred at 100 °C for 3 h. The reaction mixture was filtered. The filtrate was poured into H₂O (10 mL) and extracted with EtOAc (3 × 5 mL). The combined organic layers were washed with brine (2 × 5 mL), dried over anhydrous Na₂SO₄, filtered, and concentrated under reduced pressure. The crude residue was purified by preparative TLC. LCMS: *m*/*z* = 199.1 [M+H]⁺.

**6-Ethyl-5-phenylpyridazin-3(2*H*)-one:** To a solution of 5-phenyl-6-vinylpyridazin-3(2*H*)-one (130 mg, 0.66 mmol) in MeOH (10 mL) was added Pd (70 mg, 10 wt % on carbon). The suspension was degassed under vacuum and purged with H₂ three times. The reaction mixture was stirred at 15 °C under an atmosphere of H₂ (15 psi) for 2 h. The reaction mixture was filtered and the filtrate was concentrated under reduced pressure to provide a residue that was used directly. LCMS: *m*/*z* = 201.1 [M+H]⁺. ¹H NMR (400 MHz, DMSO): δ 12.93 (br s, 1H), 7.49-7.45 (m, 3H), 7.43-7.39 (m, 2H), 6.62 (s, 1H), 2.48-2.44 (m, 2H), 0.95 (t, *J=* 7.6 Hz, 3H).

**2-(3-Ethyl-6-oxo-4-phenylpyridazin-1(6*H*)-yl)-*N*-(*cis*-3-hydroxy-3-methylcyclobutyl)acetamide:** To a solution of 6-ethyl-5-phenylpyridazin-3(2*H*)-one (50 mg, 0.25 mmol) and 2-chloro-*N*-(*cis*-3-hydroxy-3-methylcyclobutyl)acetamide (45 mg, 0.25 mmol) in CH₃CN (3 mL) was added Cs₂CO₃ (122 mg, 0.37 mmol). The reaction mixture was stirred at 90 °C for 1 h. The reaction mixture was poured into H₂O (10 mL) and extracted with EtOAc (3 × 5 mL). The combined organic layers were washed with brine (2 × 5 mL), dried over anhydrous Na₂SO₄, filtered, and concentrated under reduced pressure. The crude residue was purified by reverse-phase HPLC. LCMS: *m*/*z* = 342.2 [M+H]⁺. ¹H NMR (400 MHz, CDCl₃): δ 7.56-7.42 (m, 3H), 7.31 (m, 2H), 6.90 (br d, *J= 7.6* Hz, 1H), 6.80 (s, 1H), 4.84 (s, 2H), 4.13-3.84 (m, 1H), 2.65-2.47 (m, 4H), 2.41 (br s, 1H), 2.08 (br t, *J=* 10.4 Hz, 2H), 1.38 (s, 3H), 1.07 (t, *J* = *7.6* Hz, 3H).

### Example 4

### 2-(4-(2-Chlorophenyl)-3-isopropyl-6-oxopyridazin-1(6H)-yl)-N-(cis-3-hydroxy-3-methylcyclobutyl)acetamide

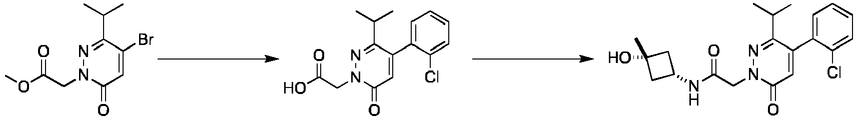

**2-(4-(2-chlorophenyl)-3-isopropyl-6-oxopyridazin-1(6*H*)-yl)acetic acid:** To a solution of methyl 2-(4-bromo-3-isopropyl-6-oxopyridazin-1(6*H*)-yl)acetate (100 mg, 0.35 mmol) and (2-chlorophenyl)boronic acid (59 mg, 0.38 mmol) in 1,4-dioxane (2 mL) and H₂O (0.5 mL) were added Pd(dppf)Cl₂ (13 mg, 0.017 mmol) and Cs₂CO₃ (225 mg, 0.69 mmol). The reaction mixture was stirred for 3 h at 100 °C. The reaction mixture was concentrated under reduced pressure. The residue was dissolved in water (5 mL) and washed with MTBE (2 × 3 mL). The aqueous phase was then adjusted to pH = 3 by the addition of aqueous HCl (3 N). The aqueous phase was extracted with EtOAc (2 × 3 mL). The combined organics were washed with brine (5 mL), dried over anhydrous Na₂SO₄, filtered, and concentrated under reduced pressure to provide a residue that was used directly as a mixture of methyl 2-(4-(2-chlorophenyl)-3-isopropyl-6-oxopyridazin-1(6*H*)-yl)acetic acid and 2-(4-bromo-3-isopropyl-6-oxopyridazin-1(6H)-yl)acetic acid (1: 1 molar ratio).

**2-(4-(2-Chlorophenyl)-3-isopropyl-6-oxopyridazin-1(6*H*)-yl)-*N*-(*cis*-3-hydroxy-3-methylcyclobutyl)acetamide:** To a mixture of methyl 2-(4-(2-chlorophenyl)-3-isopropyl-6-oxopyridazin-1(6*H*)-yl)acetic acid and 2-(4-bromo-3-isopropyl-6-oxopyridazin-1(6H)-yl)acetic acid (100 mg, 1:1 molar ratio) and *cis*-3-amino-1-methyl-cyclobutanol HCl salt (47 mg, 0.34 mmol) in DMF (3 mL) were added DIPEA (121 mg, 0.94 mmol) and HATU (178 mg, 0.47 mmol). The reaction mixture was stirred at 20 °C for 1 h. The reaction mixture was diluted with water (5 mL) and extracted with EtOAc (2 × 3 mL). The combined organics were washed with brine (3 mL), dried over anhydrous Na₂SO₄, filtered, and concentrated under reduced pressure. The crude residue was purified by preparative TLC and then further purified by reverse-phase HPLC. LCMS: *m*/*z* = 390.0 [M+H]⁺. ¹H-NMR (400 MHz, CDCl₃): δ 7.53-7.49 (m, 1H), 7.46-7.36 (m, 2H), 7.25 (m, 1H), 6.79-6.77 (m, 2H), 4.96-4.74 (m, 2H), 4.06-3.96 (m, 1H), 2.75-2.62 (m, 1H), 2.59-2.49 (m, 2H), 2.12-1.97 (m, 3H), 1.39 (s, 3H), 1.20 (d, *J* = 6.8 Hz, 3H), 1.00 (d, *J* = 6.8 Hz, 3H).

### Examples 5-6

The following compounds were, or can be, made via similar procedures as those described above.

| **Ex.** | **Structure** | **Name** | **NMR** | **LCMS *m*/*z*** |
|---|---|---|---|---|
| 5 | 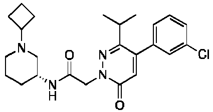 | (*R*)-2-(4-(3-chlorophenyl)-3-isopropyl-6-oxopyridazin-1(6*H*)-yl)-*N*-(1-cyclobutylpiperidin-3-yl)acetamide | ¹H NMR (400 MHz, CDCl₃): δ 7.47-7.38 (m, 2H), 7.29 (m, 1H), 7.18 (td, *J* = 1.6, 7.2 Hz, 1H), 6.77 (s, 1H), 6.60 (br s, 1H), 4.94-4.77 (m, 2H), 4.08 (br s, 1H), 3.00-2.90 (m, 1H), 2.70-2.63 (m, 1H), 2.44-2.22 (m, 3H), 2.07-1.91 (m, 3H), 1.84-1.47 (m, 8H), 1.14 (d, *J* = 1.2 Hz, 3H), 1.12 (d, *J =* 1.2 Hz, 3H). | 443.1 [M+H]⁺ |
| 6 | 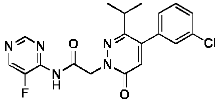 | 2-(4-(3-chlorophenyl)-3-isopropyl-6-oxopyridazin-1(6*H*)-yl)-*N*-(5-fluoropyrimidin-4-yl)acetamide | ¹H NMR (400 MHz, CDCl₃): δ 9.03 (br s, 1H), 8.79 (s, 1H), 8.52-8.50 (m, 1H), 7.49-7.39 (m, 2H), 7.32 (s, 1H), 7.21-7.19 (m, 1H), 6.83 (s, 1H), 5.40 (s, 2H), 3.03-2.88 (m, 1H), 1.12 (d, *J =* 6.8 Hz, 6H). | 402.0 [M+H]⁺ |

### Example 7

### N-(cis-3-hydroxy-3-methylcyclobutyl)-2-(3-isopropyl-4-(3-methoxyphenyl)-6-oxopyridazin-1(6H)-yl)acetamide

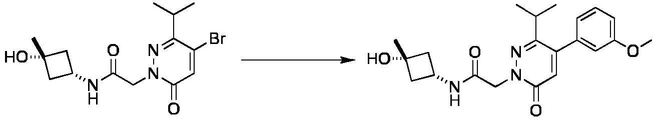

To a mixture of 2-(4-bromo-3-isopropyl-6-oxopyridazin-1(6*H*)-yl)-*N*-(*cis*-3-hydroxy-3-methylcyclobutyl)acetamide (80 mg, 0.22 mmol) and (3-methoxyphenyl)boronic acid (41 mg, 0.27 mmol) in 1,4-dioxane (2 mL) and H₂O (0.5 mL) were added K₂CO₃ (62 mg, 0.44 mmol) and Pd(dppf)Cl₂ (16 mg, 0.022 mmol). The reaction mixture was stirred at 100 °C for 2 h. The reaction mixture was poured into water (10 mL) and extracted with EtOAc (3 × 5 mL). The combined organics were dried over anhydrous Na₂SO₄, filtered, and concentrated under reduced pressure. The crude residue was purified by reverse-phase HPLC. LCMS: *m*/*z* = 386.1 [M+H]⁺. ¹H NMR (400 MHz, CDCl₃): δ 7.39 (m, 1H), 7.00 (m, 1H), 6.88 (m, 1H), 6.84-6.76 (m, 3H), 4.83 (s, 2H), 4.01 (m, 1H), 3.86 (s, 3H), 3.10-2.95 (m, 1H), 2.63-2.42 (m, 2H), 2.13-1.99 (m, 3H), 1.39 (s, 3H), 1.12 (d, *J =* 7.2 Hz, 6H).

### Examples 8-16

The following compounds were, or can be, made via similar procedures as those described above.

| **Ex.** | **Structure** | **Name** | **NMR** | **LCMS *m*/*z*** |
|---|---|---|---|---|
| 8 | 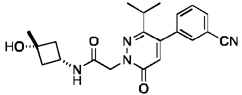 | 2-(4-(3-cyanophenyl)-3-isopropyl-6-oxopyridazin-1(6*H*)-yl)-*N*-(*cis*-3-hydroxy-3-methylcyclobutyl)acet amide | ¹H NMR (400 MHz, CDCl₃): δ 7.79 (d, *J* = 7.6 Hz, 1H), 7.67-7.60 (m, 2H), 7.59-7.53 (m, 1H), 6.78 (s, 1H), 6.70 (br d, *J* = 7.2 Hz, 1H), 4.84 (s, 2H), 4.06-3.96 (m, 1H), 2.93-2.82 (m, 1H), 2.60-2.48 (m, 2H), 2.15 (s, 1H), 2.07 (br t, *J* = 10.4 Hz, 2H), 1.39 (s, 3H), 1.12 (d, *J =* 6.8 Hz, 6H). | 381.2 [M+H]⁺ |
| 9 | 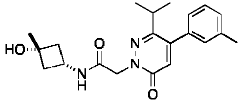 | *N*-(*cis*-3-hydroxy-3-methylcyclobutyl)-2-(3-isopropyl-6-oxo-4-(*m*-tolyl)pyridazin-1(6*H*)-yl)acetamide | ¹H NMR (400 MHz, CDCl₃): δ 7.39-7.31 (m, 1H), 7.28 (br s, 1H), 7.15-7.04 (m, 2H), 6.92 (br d, *J =* 7.2 Hz, 1H), 6.76 (s, 1H), 4.84 (s, 2H), 4.06-3.96 (m, 1H), 3.05-2.98 (m, 1H), 2.60-2.48 (m, 2H), 2.42 (s, 4H), 2.06 (br t, *J* = 10.0 Hz, 2H), 1.38 (s, 3H), 1.11 (d, *J =* 6.8 Hz, 6H). | 370.2 [M+H]⁺ |
| 10 | 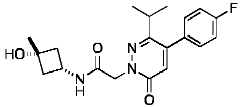 | 2-(4-(4-fluorophenyl)-3-isopropyl-6-oxopyridazin-1(6*H*)-yl)-*N*-(*cis*-3-hydroxy-3-methylcyclobutyl)acet amide | ¹H NMR (400 MHz, CDCl₃): δ 7.31-7.27 (m, 2H), 7.20-7.13 (m, 2H), 6.79-6.71 (m, 2H), 4.82 (s, 2H), 4.06-3.96 (m, 1H), 3.04-2.89 (m, 1H), 2.59-2.49 (m, 2H), 2.12-1.97 (m, 3H), 1.38 (s, 3H), 1.10 (d, *J =* 6.8 Hz, 6H). | 374.1 [M+H]⁺ |
| 11 | 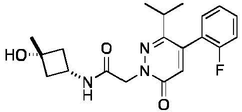 | 2-(4-(2-fluorophenyl)-3-isopropyl-6-oxopyridazin-1(6*H*)-yl)-*N*-(*cis*-3-hydroxy-3-methylcyclobutyl)acet amide | ¹H NMR (400 MHz, CDCl₃): δ 7.54-7.44 (m, 1H), 7.31-7.27 (m, 1H), 7.27-7.24 (m, 1H), 7.24-7.16 (m, 1H), 6.82 (s, 2H), 4.84 (s, 2H), 4.06-3.96 (m, 1H), 2.87-2.73 (m, 1H), 2.60-2.50 (m, 2H), 2.19 (br s, 1H), 2.11-2.02 (m, 2H), 1.38 (s, 3H), 1.11 (br d, *J* = 6.4 Hz, 6H). | 374.1 [M+H]⁺ |
| 12 | 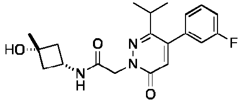 | 2-(4-(3-fluorophenyl)-3-isopropyl-6-oxopyridazin-1(6*H*)-yl)-*N*-(*cis*-3-hydroxy-3-methylcyclobutyl)acet amide | ¹H NMR (400 MHz, CDCl₃): δ 7.46 (dt, *J=* 5.6, 8.0 Hz, 1H), 7.21-7.15 (m, 1H), 7.09 (td, *J* = 1.2, 7.6 Hz, 1H), 7.05-7.00 (m, 1H), 6.78 (s, 1H), 6.74 (br d, *J=* 7.2 Hz, 1H), 4.83 (s, 2H), 4.06-3.96 (m, 1H), 3.03-2.91 (m, 1H), 2.59-2.49 (m, 2H), 2.11-2.01 (m, 3H), 1.39 (s, 3H), 1.12 (d, *J =* 6.8 Hz, 6H). | 374.1 [M+H]⁺ |
| 13 | 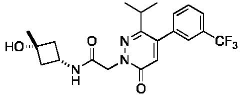 | *N*-(*cis*-3-hydroxy-3-methylcyclobutyl)-2-(3-isopropyl-6-oxo-4-(3-(trifluoromethyl)pheny l)pyridazin-1(6*H*)-yl)acetamide | ¹H NMR (400 MHz, CDCl₃): δ 7.75 (br d, *J*= 8 Hz, 1H), 7.63 (t, *J* = 7.6 Hz, 1H), 7.58 (s, 1H), 7.51 (br d, *J* = 7.6 Hz, 1H), 6.86-6.69 (m, 2H), 4.84 (s, 2H), 4.05-3.97 (m, 1H), 2.92-2.87 (m, 1H), 2.60-2.47 (m, 2H), 2.23 (br s, 1H), 2.07 (br t, *J =* 10.4 Hz, 2H), 1.38 (s, 3H), 1.12 (d, *J =* 6.8 Hz, 6H). | 424.1 [M+H]⁺ |
| 14 | 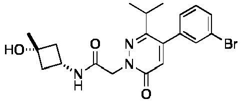 | 2-(4-(3-bromophenyl)-3-isopropyl-6-oxopyridazin-1(6*H*)-yl)-*N*-(*cis*-3-hydroxy-3-methylcyclobutyl)acet amide | ¹H NMR (400 MHz, CDCl₃): δ 7.62 (d, *J* = 8.4 Hz, 1H), 7.47 (s, 1H), 7.36 (t, *J* = 7.6 Hz, 1H), 7.24 (d, *J* = 8.0 Hz, 1H), 6.80-6.69 (m, 2H), 4.83 (s, 2H), 4.06-3.96 (m, 1H), 2.98-2.93 (m, 1H), 2.61-2.49 (m, 2H), 2.14-1.97 (m, 3H), 1.39 (s, 3H), 1.12 (d, *J =* 6.8 Hz, 6H). | 434.1 [M+H]⁺ |
| 15 | 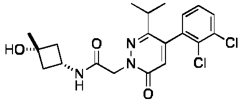 | 2-(4-(2,3-dichlorophenyl)-3-isopropyl-6-oxopyridazin-1(6H)-yl)-*N*-(*cis*-3-hydroxy-3-methylcyclobutyl)acet amide | ¹H NMR (400 MHz, CDCl₃): δ 7.59 (d, *J* = 7.2 Hz, 1H), 7.34 (t, *J =* 8.0 Hz, 1H), 7.18 (d, *J =* 8.0 Hz, 1H), 6.79-6.64 (m, 2H), 4.98-4.69 (m, 2H), 4.06-3.96 (m, 1H), 2.69-2.48 (m, 3H), 2.06 (br dd, *J =* 8.8, 11.6 Hz, 3H), 1.39 (s, 3H), 1.21 (d, *J* = 6.8 Hz, 3H), 1.00 (d, *J* = 6.8 Hz, 3H). | 424.0 [M+H]⁺ |
| 16 | 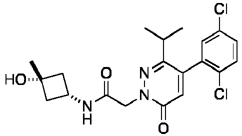 | 2-(4-(2,5-dichlorophenyl)-3-isopropyl-6-oxopyridazin-1(6H)-yl)-N-(*cis*-3-hydroxy-3-methylcyclobutyl)acet amide | ¹H NMR (400 MHz, CDCl₃): δ 7.47-7.38 (m, 2H), 7.28 (br s, 1H), 6.81-6.64 (m, 2H), 5.02-4.66 (m, 2H), 4.06-3.96 (m, 1H), 2.67-2.57 (m, 1H), 2.59-2.50 (m, 2H), 2.13-1.96 (m, 3H), 1.39 (s, 3H), 1.20 (d, *J=* 6.8 Hz, 3H), 1.04 (d, *J=* 6.8 Hz, 3H). | 424.0 [M+H]⁺ |

### Example 17

### N-(cis-3-hydroxy-3-methylcyclobutyl)-2-(3-isopropyl-6-oxo-4-(pyridin-2-yl)pyridazin-1(6H)-yl)acetamide

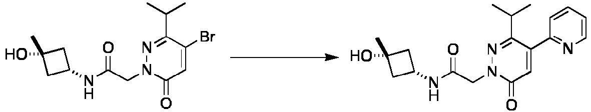

To a solution of 2-(4-bromo-3-isopropyl-6-oxopyridazin-1(6*H*)-yl)-*N*-(*cis*-3-hydroxy-3-methylcyclobutyl)acetamide (100 mg, 0.30 mmol) and tributyl(2-pyridyl)stannane (154 mg, 0.42 mmol) in DMF (2 mL) was added Pd(PPh₃)₂Cl₂ (20 mg, 0.03 mmol). The reaction mixture was stirred at 100 °C for 16 h. The reaction mixture was poured into water (15 mL) and extracted with EtOAc (3 × 5 mL). The combined organics were washed with brine (5 mL), dried over anhydrous Na₂SO₄, filtered, and concentrated under reduced pressure. The crude residue was purified by reverse-phase HPLC. LCMS: *m*/*z* = 357.1 [M+H]⁺. ¹H NMR (400 MHz, CDCl₃): δ 8.72-8.71 (m, 1H), 7.87-7.83 (m, 1H), 7.44-7.38 (m, 2H), 6.93-6.91 (br d, *J* = 7.6 Hz, 1H), 6.89 (s, 1H), 4.85 (s, 2H), 4.03-3.97 (m, 1H), 3.31-3.25 (m, 1H), 2.63 (s, 1H), 2.54-2.49 (m, 2H), 2.07-2.02 (m, 2H), 1.36 (s, 3H), 1.12 (d, *J* = 6.8 Hz, 6H).

### Example 18

### 2-(3-chloro-6-oxo-4-phenylpyridazin-1(6H)-yl)-N-(cis-3-hydroxy-3-methylcyclobutyl)acetamide

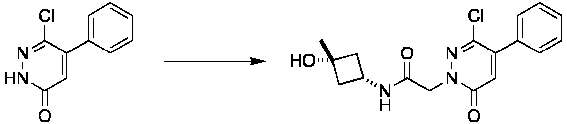

To a mixture of 6-chloro-5-phenylpyridazin-3(2*H*)-one (50 mg, 0.24 mmol) and Cs₂CO₃ (118 mg, 0.36 mmol) in CH₃CN (1 mL) was added 2-chloro-*N*-(*cis*-3-hydroxy-3-methylcyclobutyl)acetamide (45 mg, 0.25 mmol). The reaction mixture was stirred at 90 °C for 1 h. The reaction mixture was concentrated. The crude residue was purified by reverse-phase HPLC. LCMS: *m*/*z* = 348.1 [M+H]⁺. ¹H-NMR (400 MHz, CDCl₃): δ 7.53-7.48 (m, 3H), 7.47-7.41 (m, 2H), 6.96 (s, 1H), 6.50 (br d, *J =* 6.8 Hz, 1H), 4.79 (s, 2H), 4.16-3.96 (m, 1H), 2.76-2.46 (m, 2H), 2.20 (br s, 1H), 2.17-1.91 (m, 2H), 1.39 (s, 3H).

### Example 19

### N-(cis-3-hydroxy-3-methylcyclobutyl)-2-(3-methoxy-6-oxo-4-phenylpyridazin-1(6H)-yl)acetamide

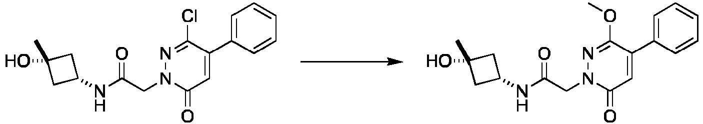

To a solution of 2-(3-chloro-6-oxo-4-phenylpyridazin-1(6*H*)-yl)-*N*-(*cis*-3-hydroxy-3-methylcyclobutyl)acetamide (50 mg, 0.14 mmol) in MeOH (1 mL) was added a solution of NaOMe (0.06 mL, 5 M in MeOH). The reaction mixture was stirred at 80 °C for 2 h. The reaction mixture was filtered and the filtrate was directly purified by reverse-phase HPLC. LCMS: *m*/*z* = 344.2 [M+H]⁺. ¹H-NMR (400 MHz, CDCl₃): δ 7.57-7.52 (m, 2H), 7.49-7.45 (m, 3H), 6.97 (s, 1H), 6.84 (br d, *J* = 6.8 Hz, 1H), 4.72 (s, 2H), 4.07-3.94 (m, 1H), 3.89 (s, 3H), 2.60-2.48 (m, 2H), 2.14-2.01 (m, 3H), 1.38 (s, 3H).

### Example 20

### N-(cis-3-hydroxy-3-methylcyclobutyl)-2-(6-isopropyl-3-oxo-5-phenyl-1,2,4-triazin-2(3H)-yl)acetamide

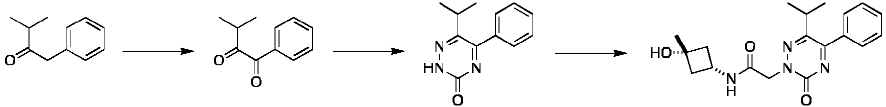

**3-Methyl-1-phenylbutane-1,2-dione:** To a solution of 3-methyl-1-phenylbutan-2-one (500 mg, 3.08 mmol) in 1,4-dioxane (10 mL) was added SeO₂ (684 mg, 6.16 mmol). The reaction mixture was stirred at 100 °C for 15 h. The reaction mixture was poured into water (30 mL) and extracted with EtOAc (3 × 10 mL). The combined organics were washed with brine (10 mL), dried over anhydrous Na₂SO₄, filtered, and concentrated under reduced pressure. The crude residue was purified by silica gel chromatography. ¹H NMR (400 MHz, CDCl₃): δ 7.97-7.91 (m, 2H), 7.69-7.62 (m, 1H), 7.55-7.48 (m, 2H), 3.36 (m, 1H), 1.21 (d, *J=* 7.2 Hz, 6H).

**6-Isopropyl-5-phenyl-1,2,4-triazin-3(2*H*)-one:** To a solution of 3-methyl-1-phenylbutane-1,2-dione (100 mg, 0.57 mmol) in AcOH (1 mL) were added *N*-aminourea HCl salt (69 mg, 0.62 mmol) and NaOAc (51 mg, 0.62 mmol). The reaction mixture was stirred at 120 °C for 15 h. The reaction mixture was poured into water (8 mL) and extracted with EtOAc (3 × 5 mL). The combined organics were washed with brine (5 mL), dried over anhydrous Na₂SO₄, filtered, and concentrated under reduced pressure to provide a residue that was used directly. ¹H NMR (400 MHz, CDCl₃): δ 12.07 (br s, 1H), 7.65-7.59 (m, 2H), 7.58-7.48 (m, 3H), 3.28 (m, 1H), 1.17 (d, *J=* 7.2 Hz, 6H).

***N*-(*cis*-3-hydroxy-3-methylcyclobutyl)-2-(6-isopropyl-3-oxo-5-phenyl-1,2,4-triazin-2(3*H*)-yl)acetamide:** To a solution of 6-isopropyl-5-phenyl-1,2,4-triazin-3(2H)-one (80 mg, 0.37 mmol) and 2-chloro-*N*-(*cis*-3-hydroxy-3-methylcyclobutyl)acetamide (66 mg, 0.37 mmol) in DMF (2 mL) was added Cs₂CO₃ (145 mg, 0.45 mmol). The reaction mixture was stirred at 90 °C for 1 h. The reaction mixture was filtered and the filtrate was directly purified by reverse-phase HPLC. LCMS: *m*/*z* = 357.1 [M+H]⁺. ¹H NMR (400 MHz, CDCl₃): δ 7.64-7.45 (m, 6H), 4.84 (s, 2H), 4.00 (m, 1H), 3.43 (br s, 1H), 3.24 (m, 1H), 2.47 (m, 2H), 2.13-2.06 (m, 2H), 1.32 (s, 3H), 1.15 (d, *J* = 7.2 Hz, 6H).

### Examples 21-28

The following compounds were, or can be, made via similar procedures as those described above.

| **Ex.** | **Structure** | **Name** | **NMR** | **LCMS *m*/*z*** |
|---|---|---|---|---|
| 21 | 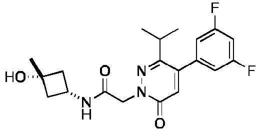 | 2-[4-(3,5-difluorophenyl)-6-oxo-3-propan-2-ylpyridazin-1-yl]-N-(*cis*-3-hydroxy-3-methylcyclobutyl)aceta mide | ¹H-NMR (400 MHz; CDCl₃): δ 6.95-6.86 (m, 2H), 6.85-6.81 (m, 2H), 6.77 (s, 1H), 4.83 (s, 2H), 4.06-3.96 (m, 1H), 2.97-2.87 (m, 1H), 2.56-2.48 (m, 2H), 2.09-2.04 (m, 2H), 1.37 (s, 3H), 1.11 (d, *J =* 6.8 Hz, 6H) | 392.9 [M+H]⁺ |
| 22 | 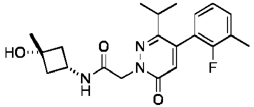 | 2-[4-(2-fluoro-3-methylphenyl)-6-oxo-3-propan-2-ylpyridazin-1-yl]-N-(*cis*-3-hydroxy-3-methylcyclobutyl)aceta mide | ¹H-NMR (400 MHz; CDCl₃): δ 7.32-7.27 (m, 1H), 7.13 (t, *J* = 7.6 Hz, 1H), 7.05-7.02 (m, 1H), 6.98-6.96 (m, 1H), 6.79 (s, 1H), 4.83 (s, 2H), 4.07-3.93 (m, 1H), 2.84-2.72 (m, 1H), 2.56-2.47 (m, 2H), 2.33 (d, *J* = 1.7 Hz, 3H), 2.08-2.04 (m, 2H), 1.36 (s, 3H), 1.09-1.09 (m, 6H) | 388.8 [M+H]⁺ |
| 23 | 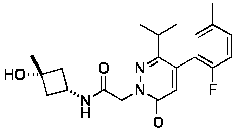 | 2-[4-(2-fluoro-5-methylphenyl)-6-oxo-3-propan-2-ylpyridazin-1-yl]-N-(*cis-*3-hydroxy-3-methylcyclobutyl)aceta mide | ¹H-NMR (400 MHz; CDCl₃): δ 7.25-7.20 (m, 1H), 7.09-7.00 (m, 3H), 6.80 (s, 1H), 4.84 (s, 2H), 4.06-3.95 (m, 1H), 2.83-2.76 (m, 1H), 2.53-2.48 (m, 2H), 2.36 (s, 3H), 2.10-2.03 (m, 2H), 1.35 (s, 3H), 1.09 (d, *J* = 6.2 Hz, 6H) | 388.8 [M+H]⁺ |
| 24 | 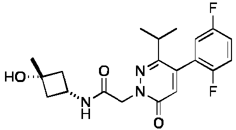 | 2-[4-(2,5-difluorophenyl)-6-oxo-3-propan-2-ylpyridazin-1-yl]-N-(*cis*-3-hydroxy-3-methylcyclobutyl)aceta mide | ¹H-NMR (400 MHz; CDCl₃): δ 7.15 (td, *J =* 6.2, 1.5 Hz, 2H), 7.00-6.96 (m, 1H), 6.95-6.91 (m, 1H), 6.81 (s, 1H), 4.83 (s, 2H), 4.06-3.96 (m, 1H), 2.82-2.72 (m, 1H), 2.56-2.46 (m, 2H), 2.16 (br s, 1H), 2.09-2.04 (m, 2H), 1.36 (s, 3H), 1.11 (d, *J* = 6.7 Hz, 6H) | 392.9 [M+H]⁺ |
| 25 | 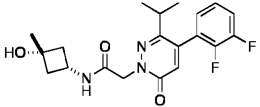 | 2-[4-(2,3-difluorophenyl)-6-oxo-3-propan-2-ylpyridazin-1-yl]-N-(*cis*-3-hydroxy-3-methylcyclobutyl)aceta mide | ¹H-NMR (400 MHz; CDCl₃): δ 7.33-7.27 (m, 1H), 7.24-7.21 (m, 1H), 7.01 (ddt, *J=* 8.4, 5.1, 1.6 Hz, 1H), 6.82 (s, 1H), 6.77 (d, *J =* 7.1 Hz, 1H), 4.83 (s, 2H), 4.06-3.96 (m, 1H), 2.80-2.73 (m, 1H), 2.56-2.51 (m, 2H), 2.08-2.01 (m, 2H), 1.70 (br s, 1H), 1.37 (s, 3H), 1.11 (d, *J* = 6.5 Hz, 6H) | 392.8 [M+H]⁺ |
| 26 | 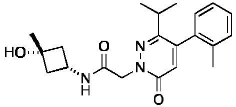 | 2-[4-(2-methylphenyl)-6-oxo-3-propan-2-ylpyridazin-1-yl] ]-N-( *cis*-3-hydroxy-3-methylcyclobutyl)aceta mide | ¹H-NMR (400 MHz; CDCl₃): δ 7.37-7.33 (m, 1H), 7.32-7.27 (m, 2H), 7.11-7.08 (m, 1H), 6.89-6.85 (m, 1H), 6.72 (s, 1H), 4.92-4.74 (m, 2H), 4.06-3.95 (m, 1H), 2.65-2.58 (m, 1H), 2.56-2.51 (m, 2H), 2.14 (s, 3H), 2.08-2.03 (m, 2H), 1.63 (br s, 1H), 1.37 (s, 3H), 1.07 (dd, *J* = 41.1, 6.7 Hz, 6H) | 370.9 [M+H]⁺ |
| 27 | 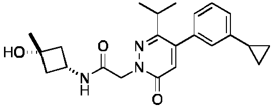 | 2-[4-(3-cyclopropylphenyl)-6-oxo-3-propan-2-ylpyridazin-1-yl]-N-( *cis*-3-hydroxy-3-methylcyclobutyl)aceta mide | ¹H NMR (400 MHz, CDCl₃): δ 7.41-7.31 (m, 1H), 7.18-6.96 (m, 3H), 6.77 (s, 2H), 4.83 (s, 2H), 4.10-3.95 (m, 1H), 3.00 (quin, *J =* 6.8 Hz, 1H), 2.60-2.47 (m, 2H), 2.09-2.03 (m, 2H), 2.02 (s, 1H), 1.99-1.90 (m, 1H), 1.39 (s, 3H), 1.11 (d, *J* = 6.8 Hz, 6H), 1.07-1.01 (m, 2H), 0.78-0.65 (m, 2H) | 396.1 [M+H]⁺ |
| 28 | 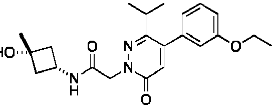 | 2-[4-(3-ethoxyphenyl)-6-oxo-3-propan-2-ylpyridazin-1-yl]-N-( *cis*-3-hydroxy-3-methylcyclobutyl)aceta mide | ¹H NMR (400 MHz, CDCl₃): δ 7.36 (t, *J =* 8.0 Hz, 1H), 6.98 (dd, *J* = 2.0, 8.4 Hz, 1H), 6.93 (br d, *J* = 6.8 Hz, 1H), 6.85 (br d, *J* = 7.6 Hz, 1H), 6.79 (br d, *J* = 11.6 Hz, 2H), 4.83 (s, 2H), 4.07 (m, 2H), 4.03-3.95 (m, 1H), 2.96-3.11 (m, 1H), 2.53 (m, 2H), 2.08 (m, 2H), 1.45 (t, *J=* 6.8 Hz, 3H), 1.37 (s, 3H), 1.11 (d, *J* = 6.8 Hz, 6H) | 400.1 [M+H]⁺ |

### Example 29

### N-[(3R)-1-ethylpiperidin-3-yl]-2-(4-naphthalen-1-yl-6-oxo-3-propan-2-ylpyridazin-1-yl)acetamide

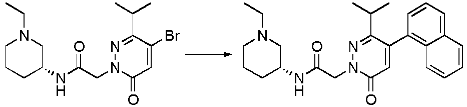

To a mixture of 2-(4-bromo-3-isopropyl-6-oxo-pyridazin-1-yl)-N-[rac-(3R)-1-ethyl-3-piperidyl]acetamide (18 mg, 0.05 mmol) and 1-naphthylboronic acid (16 mg, 0.09 mmol) in 1,4-dioxane (1 mL) were added K₂CO₃ (62 mg, 0.44 mmol) in water (0.14 mL) and Pd(PPh₃)₄ (11 mg, 0.01 mmol). The reaction mixture was stirred at 80 °C for 20 h. The reaction mixture was poured into water (10 mL) and extracted with EtOAc (3 × 5 mL). The combined organics were washed with brine (10 mL), dried over anhydrous Na₂SO₄, filtered, and concentrated under reduced pressure. The crude residue was purified by reverse-phase HPLC. LCMS: *m*/*z* = 433.9 [M+H]⁺. ¹H NMR (400 MHz, CDCl₃): δ 7.93 (dd, *J* = 7.7, 6.4 Hz, 2H), 7.56-7.52 (m, 2H), 7.50-7.46 (m, 2H), 7.34 (dt, *J* = 7.0, 1.2 Hz, 1H), 7.06 (s, 1H), 6.86 (d, *J* = 0.4 Hz, 1H), 4.99-4.87 (m, 2H), 4.21-4.17 (m, 1H), 2.72-2.60 (m, 2H), 2.57-2.43 (m, 4H), 2.27-2.20 (m, 1H), 1.86-1.53 (m, 4H), 1.10-1.06 (m, 3H), 1.04-0.96 (m, 6H).

### Examples 30-32

The following compounds were, or can be, made via similar procedures as those described above using the appropriate reagents.

| **Ex.** | **Structure** | **Name** | **NMR** | **LCMS *m*/*z*** |
|---|---|---|---|---|
| 30 | 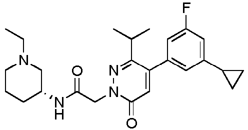 | 2-[4-(3-cyclopropyl-5-fluorophenyl)-6-oxo-3-propan-2-ylpyridazin-1-yl]-N-[(3R)-1-ethylpiperidin-3-yl]acetamide | ¹H NMR (400 MHz, CDCl₃): δ 6.80 (dt, *J =* 9.8, 1.9 Hz, 1H), 6.77-6.74 (m, 2H), 6.73 (s, 1H), 5.29 (s, 1H), 4.89-4.80 (m, 2H), 4.16-4.12 (m, 1H), 2.99-2.88 (m, 1H), 2.67-2.48 (m, 2H), 2.50-2.37 (m, 3H), 2.26-2.19 (m, 1H), 1.95-1.88 (m, 1H), 1.78-1.53 (m, 4H), 1.10 (dd, *J =* 6.8, 1.7 Hz, 6H), 1.08-1.05 (m, 3H), 1.05-1.01 (m, 2H), 0.75-0.68 (m, 2H) | 441.7 [M+H]⁺ |
| 31 | 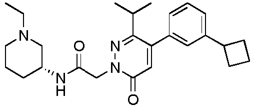 | 2-[4-(3-cyclobutylphenyl)-6-oxo-3-propan-2-ylpyridazin-1-yl]-N-[(3R)-1-ethylpiperidin-3-yl]acetamide | ¹H NMR (400 MHz, CDCl₃): δ 7.36 (t, *J* = 7.6 Hz, 1H), 7.28 (s, 1H), 7.09-7.06 (m, 2H), 6.96 (s, 1H), 6.75 (s, 1H), 4.89-4.80 (m, 2H), 4.15-4.09 (m, 1H), 3.63-3.52 (m, 1H), 3.03-2.93 (m, 1H), 2.62-2.48 (m, 2H), 2.45-2.33 (m, 6H), 2.13-2.02 (m, 3H), 1.89-1.83 (m, 1H), 1.75-1.65 (m, 2H), 1.61-1.53 (m, 2H), 1.09 (dd, *J=* 6.8, 2.1 Hz, 6H), 1.03 (t, *J=* 7.2 Hz, 3H) | 437.7 [M+H]⁺ |
| 32 | 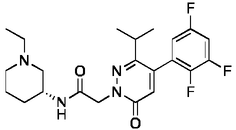 | N-[(3R)-1-ethylpiperidin-3-yl]-2-[6-oxo-3-propan-2-yl-4-(2,3,5-trifluorophenyl)pyrid azin-1-yl]acetamide | | 437.7 [M+H]⁺ |

### Example 33

### N-(5-fluoropyrimidin-4-yl)-2-(4-isoquinolin-8-yl-6-oxo-3-propan-2-ylpyridazin-1-yl)acetamide

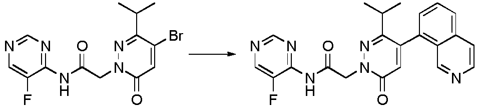

To a mixture of 2-(4-bromo-3-isopropyl-6-oxo-pyridazin-1-yl)-N-(5-fluoropyrimidin-4-yl)acetamide (39 mg, 0.11 mmol) and 8-isoquinolylboronic acid (36 mg, 0.21 mmol) in 1,4-dioxane (1.5 mL) were added K₂CO₃ (58 mg, 0.42 mmol) in water (0.13 mL) and Pd(PPh₃)₄ (18 mg, 0.15 mmol). The reaction mixture was stirred at 80 °C for 20 h. The reaction mixture was poured into water (10 mL) and extracted with EtOAc (3 × 5 mL). The combined organics were washed with brine (10 mL), dried over anhydrous Na₂SO₄, filtered, and concentrated under reduced pressure. The crude residue was purified by reverse-phase HPLC. LCMS: *m*/*z* = 419.6 [M+H]⁺. ¹H NMR (400 MHz, CDCl₃): δ 9.21 (s, 1H), 9.03 (s, 1H), 8.79 (d, *J* = 2.1 Hz, 1H), 8.64 (d, *J* = 5.8 Hz, 1H), 8.52 (d, *J* = 2.4 Hz, 1H), 7.99 (d, *J =* 8.3 Hz, 1H), 7.87-7.81 (m, 2H), 7.53 (dd, *J =* 7.0, 0.9 Hz, 1H), 6.95 (s, 1H), 5.60-5.39 (m, 2H), 2.56-2.49 (m, 1H), 1.04 (dd, *J =* 14.3, 6.8 Hz, 6H).

### Example 34

### 2-(3-ethoxy-6-oxo-4-phenylpyridazin-1(6H)-yl)-N-(cis-3-hydroxy-3-methylcyclobutyl)acetamide

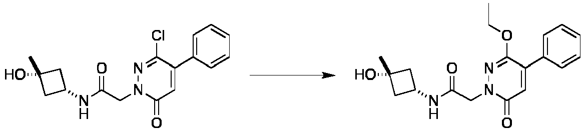

A mixture of NaH (14 mg, 0.35 mmol, 60% purity) in EtOH (1 mL) was stirred at 20 °C for 15 min. To this solution was added 2-(3-chloro-6-oxo-4-phenylpyridazin-1(6*H*)-yl)-*N*-(*cis*-3-hydroxy-3-methylcyclobutyl)acetamide (40 mg, 0.11 mmol) and the reaction mixture was stirred at 80 °C for 2 h. The reaction mixture was concentrated under reduced pressure and purified directly by reverse-phase preparative HPLC. LCMS: *m*/*z* = 358.2 [M+H]⁺. ¹H NMR (400 MHz, CDCl₃): δ 7.60-7.53 (m, 2H), 7.47 (m, 3H), 6.96 (s, 1H), 6.94-6.84 (m, 1H), 4.70 (s, 2H), 4.27 (m, 2H), 4.05-3.91 (m, 1H), 2.60-2.45 (m, 2H), 2.17-1.99 (m, 2H), 1.42-1.26 (m, 6H).

### Example 35

### 2-[5-ethyl-4-(3-fluorophenyl)-6-oxo-3-propan-2-ylpyridazin-1-yl]-N-(5-fluoropyrimidin-4-yl)acetamide

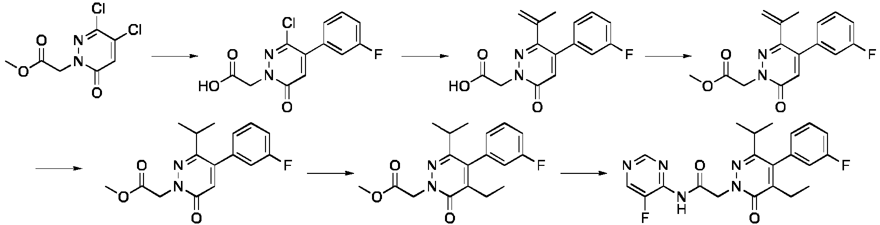

**2-(3-chloro-4-(3-fluorophenyl)-6-oxopyridazin-1(6*H*)-yl)acetic** acid: To a solution of methyl 2-(3,4-dichloro-6-oxopyridazin-1(6*H*)-yl)acetate (20 g, 59.1 mmol, 70% purity) in 1,4-dioxane (200 mL) and water (40 mL) were added (3-fluorophenyl)boronic acid (12.4 g, 88.6 mmol), Na₂CO₃ (12.5 g, 118 mmol) and Pd(PPh₃)₄ (3.4 g, 2.9 mmol). The mixture was stirred at 100 °C for 16 h. The mixture was poured into water (200 mL) and adjusted pH = 9 by NaHCO₃. The mixture extracted with EtOAc (150 mL). The aqueous phase was adjusted pH = 5 by aq. HCl (3 M) and extracted EtOAc (3 × 70 mL). The combined organic layers were washed with brine (200 mL), dried over anhydrous Na₂SO₄, filtered, and concentrated under reduced pressure. The crude residue was used directly. LCMS: *m*/*z* = 283.1, 285.1 [M+H]⁺.

**2-(4-(3-fluorophenyl)-6-oxo-3-(prop-1-en-2-yl)pyridazin-1(6*H*)-yl)acetic acid:** To a solution of 2-(3-chloro-4-(3-fluorophenyl)-6-oxopyridazin-1(6*H*)-yl)acetic acid (16 g, 56 mmol) in 1,4-dioxane (160 mL) and water (40 mL) were added 4,4,5,5-tetramethyl-2-(prop-1-en-2-yl)-1,3,2-dioxaborolane (47 g, 283 mmol), Cs₂CO₃ (74 g, 226 mmol), and Pd(dppf)Cl₂ (4.2 g, 5.7 mmol). The mixture was stirred at 110 °C for 16 h. The mixture was poured into water (200 mL) and extracted with EtOAc (150 mL). The aqueous phase was adjusted pH = 5 by aq. HCl (3 M) and extracted EtOAc (3 × 70 mL). The combined organic layers were washed with brine (200 mL), dried over anhydrous Na₂SO₄, filtered, and concentrated under reduced pressure. The crude residue was used directly. LCMS: *m*/*z* = 289.2 [M+H]⁺.

**methyl 2-(4-(3-fluorophenyl)-6-oxo-3-(prop-1-en-2-yl)pyridazin-1(6*H*)-yl)acetate:** To a solution of 2-(4-(3-fluorophenyl)-6-oxo-3-(prop-1-en-2-yl)pyridazin-1(6*H*)-yl)acetic acid (16 g, 55 mmol) in MeCN (160 mL) was added (diazomethyl)trimethylsilane (2 M in n-hexane, 55.5 mL) at 0 °C. The mixture was stirred at 25 °C for 16 h. The mixture was added AcOH to adjust pH = 6. The mixture was concentrated under reduced pressure and purified by silica gel column chromatography. LCMS: *m*/*z* = 303.3 [M+H] ⁺.

**methyl 2-(4-(3-fluorophenyl)-3-isopropyl-6-oxopyridazin-1(6*H*)-yl)acetate:** To a solution of methyl 2-(4-(3-fluorophenyl)-6-oxo-3-(prop-1-en-2-yl)pyridazin-1(6*H*)-yl)acetate (10 g, 33 mmol) in EtOAc (70 mL) was added Pd/C (2 g, 10% purity). The mixture was stirred at 20 °C for 2 h under H₂ (15 psi). The mixture was filtered and concentrated under reduced pressure. The crude residue was used directly. LCMS: *m*/*z =* 305.1 [M+H]⁺.

**methyl 2-(5-ethyl-4-(3-fluorophenyl)-3-isopropyl-6-oxopyridazin-1(6*H*)-yl)acetate:** To a solution of methyl 2-(4-(3-fluorophenyl)-3-isopropyl-6-oxopyridazin-1(6*H*)-yl)acetate (500 mg, 1.64 mmol) and propionic acid (6 g, 82 mmol) in water (5 mL) and MeCN (5 mL) were added (NH₄)₂S₂O₈ (936 mg, 4.10 mmol) and AgNO₃ (557 mg, 3.28 mmol). The mixture was stirred at 20 °C for 16 h. The mixture was then stirred at 50 °C for a further 4 h. The reaction mixture was diluted with water (10 mL) and extracted with EtOAc (3 × 8 mL). The combined organics were washed with brine (8 mL), dried over anhydrous Na₂SO₄, filtered, concentrated under reduced pressure and purified via silica gel chromatography. LCMS: *m*/*z =* 333.2 [M+H]⁺.

**2-[5-ethyl-4-(3-fluorophenyl)-6-oxo-3-propan-2-ylpyridazin-1-yl]-N-(5-fluoropyrimidin-4-**yl)acetamide: To a solution of methyl 2-(5-ethyl-4-(3-fluorophenyl)-3-isopropyl-6-oxopyridazin-1(6*H*)-yl)acetate (190 mg, 0.57 mmol) and 5-fluoropyrimidin-4-amine (194 mg, 1.71 mmol) in toluene (2 mL) and THF (2 mL) was added AlMe₃ (2 M in toluene, 0.86 mL) in one portion at 20 °C. The mixture was stirred at 110 °C for 4 h. The reaction mixture was poured into cold H₂O (8 mL) and extracted with EtOAc (3 × 5 mL). The combined organics were dried over Na₂SO₄, filtered, concentrated under reduced pressure and purified by reverse-phase preparative HPLC. LCMS: *m*/*z* = 414.2 [M+H]⁺. ¹H NMR (400 MHz, CDCl₃): δ 9.40 (br s, 1H), 8.80 (s, 1H), 8.50 (d, *J* = 2.0 Hz, 1H), 7.53-7.43 (m, 1H), 7.21-7.12 (m, 1H), 6.97 (br d, *J* = 7.6 Hz, 1H), 6.91 (br d, *J* = 8.8 Hz, 1H), 5.34 (s, 2H), 2.59 (m, 1H), 2.37 (m, 2H), 1.11-1.01 (m, 9H).

### Example 36

### 2-[4-(3-fluorophenyl)-5-methyl-6-oxo-3-propan-2-ylpyridazin-1-yl]-N-(5-fluoropyrimidin-4-yl)acetamide

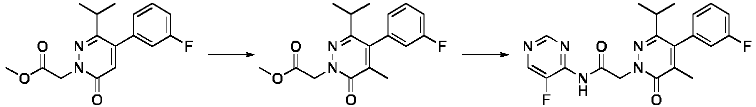

**methyl 2-(4-(3-fluorophenyl)-3-isopropyl-5-methyl-6-oxopyridazin-1(6*H*)-yl)acetate:** To a solution of methyl 2-(4-(3-fluorophenyl)-3-isopropyl-6-oxopyridazin-1(6*H*)-ylacetate (500 mg, 1.64 mmol) and AcOH (4.93 g, 82.2 mmol) in water (5 mL) and MeCN (5 mL) were added ammonia;sulfooxy hydrogen sulfate (937 mg, 4.11 mmol) and AgNO₃ (558 mg, 3.29 mmol). The reaction mixture was stirred at 20 °C for 16 h. The reaction mixture was poured into brine (30 mL) and extracted with EtOAc (3 × 10 mL). The combined organic layers were washed with brine (10 mL), dried over anhydrous Na₂SO₄, filtered, and concentrated under reduced pressure. The crude residue was purified by silica gel column chromatography. LCMS: *m*/*z* = 319.1 [M+H]⁺.

**2-[4-(3-fluorophenyl)-5-methyl-6-oxo-3-propan-2-ylpyridazin-1-yl]-N-(5-fluoropyrimidin-4-yl)acetamide:** To a solution of methyl 2-(4-(3-fluorophenyl)-3-isopropyl-5-methyl-6-oxopyridazin-1(6*H*)-yl)acetate (110 mg, 0.34 mmol) and 5-fluoropyrimidin-4-amine (117 mg, 1.04 mmol) in toluene (5 mL) was added AlMe₃ (2M in toluene, 0.5 mL, 1.04 mmol). The reaction mixture was stirred at 80 °C for 3 h. The reaction mixture was poured into saturated sat. aq. NH₄Cl (10 mL) and extracted with EtOAc (3 × 5 mL). The combined organic layers were washed with brine (5 mL), dried over anhydrous Na₂SO₄, filtered, and concentrated under reduced pressure. The crude residue was purified by reverse-phase preparative HPLC. LCMS: *m*/*z* = 400.1 [M+H]⁺. ¹H NMR (400 MHz, CDCl₃): δ 9.14 (s, 1H), 8.79 (s, 1H), 8.51 (s, 1H), 7.62-7.41 (m, 1H), 7.16 (t, *J* = 8.4 Hz, 1H), 6.96 (d, *J* = 7.6 Hz, 1H), 6.91 (d, *J* = 8.4 Hz, 1H), 5.37 (s, 2H), 2.65 (m, 1H), 1.96 (s, 3H), 1.08 (t, *J =* 6.8 Hz, 6H).

### Example 37

### 2-[5-(difluoromethyl)-4-(3-fluorophenyl)-6-oxo-3-propan-2-ylpyridazin-1-yl]-N-(5-fluoropyrimidin-4-yl)acetamide

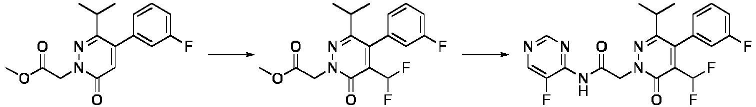

**methyl 2-(5-(difluoromethyl)-4-(3-fluorophenyl)-3-isopropyl-6-oxopyridazin-1(6*H*)-yl)acetate:** To a solution of methyl 2-(4-(3-fluorophenyl)-3-isopropyl-6-oxopyridazin-1(6*H*)-yl)acetate (1.0 g, 3.29 mmol) and 2,2-difluoroacetic acid (442 mg, 4.60 mmol) in water (6 mL) and MeCN (1.5 mL) were added TFA (75 mg, 0.66 mmol) and AgNO₃ (112 mg, 0.66 mmol) in water (2 mL) at 55 °C. To this mixture was added ammonia;sulfooxy hydrogen sulfate (1.2 g, 5.26 mmol) in water (6 mL). The reaction mixture was stirred at 70 °C for 1 h. The reaction mixture was poured into cold water (10 mL) and extracted with EtOAc (3 × 8 mL). The combined organic layers were washed with brine (10 mL), dried over anhydrous Na₂SO₄, filtered, and concentrated under reduced pressure. The crude residue was purified by silica gel column chromatography. ¹H NMR (400 MHz, CDCl₃): δ 7.48 (td, *J =* 8.0, 5.6 Hz, 1H), 7.17-7.23 (m, 1H), 7.05 (d, *J =* 7.6 Hz, 1H), 6.97-7.02 (m, 1H), 5.98 (t, *J* = 53.2 Hz, 1H), 5.01 (s, 2H), 3.85 (s, 3H), 2.64-2.73 (m, 1H), 1.08 (dd, *J =* 10.4, 6.8 Hz, 6H).

**2-[5-(difluoromethyl)-4-(3-fluorophenyl)-6-oxo-3-propan-2-ylpyridazin-1-yl]-N-(5-fluoropyrimidin-4-yl)acetamide:** To a solution of methyl 2-(5-(difluoromethyl)-4-(3-fluorophenyl)-3-isopropyl-6-oxopyridazin-1(6*H*)-yl)acetate (100 mg, 0.28 mmol) and 5-fluoropyrimidin-4-amine (96 mg, 0.85 mmol) in toluene (1 mL) and THF (0.5 mL) was added AlMe₃ (2 M in toluene, 0.42 mL). The reaction mixture was stirred at 110 °C for 5 h. The reaction mixture was diluted with water (5 mL) and extracted with EtOAc (3 × 5 mL). The combined organic layers were dried over anhydrous Na₂SO₄, filtered, and concentrated under reduced pressure. The crude residue was purified by reverse-phase preparative HPLC. LCMS: *m*/*z* = 436.2 [M+H]⁺. ¹H NMR (400 MHz, CDCl₃): δ 8.78 (d, *J* = 2.0 Hz, 1H), 8.53 (m, 2H), 7.44-7.52 (m, 1H), 7.20 (td, *J* = 8.4, 1.6 Hz, 1H), 7.06 (d, *J* = 7.6 Hz, 1H), 7.00 (br d, *J* = 8.8 Hz, 1H), 6.87 (t, *J* = 53.2 Hz, 1H), 5.53 (s, 2H), 2.64-2.73 (m, 1H), 1.09 (dd, *J =* 9.6, 6.8 Hz, 6H).

### Example 38

### 2-[4-(3,5-difluorophenyl)-5-methyl-6-oxo-3-propan-2-ylpyridazin-1-yl]-N-(5-fluoropyrimidin-4-yl)acetamide

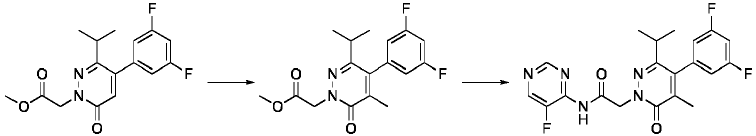

**methyl 2-(4-(3,5-difluorophenyl)-3-isopropyl-5-methyl-6-oxopyridazin-1(6*H*)-yl)acetate:** A mixture of methyl 2-(4-(3,5-difluorophenyl)-3-isopropyl-6-oxopyridazin-1(6*H*)-yl)acetate (300 mg, 0.93 mmol), AcOH (2.79 g, 46.54 mmol), (NH₄)₂S₂O₈ (531 mg, 2.33 mmol) and AgNO₃ (316 mg, 1.86 mmol) in water (3 mL) and MeCN (3 mL) was stirred at 20 °C for 16 h. The reaction mixture was poured into brine (8 mL) and the mixture was extracted with EtOAc (3 × 3 mL). The combined organics were washed with brine (8 mL), dried over Na₂SO₄, filtered, concentrated under reduced and purified by silica gel chromatography. LCMS: *m*/*z* = 337.2 [M+H]⁺.

**2-[4-(3,5-difluorophenyl)-5-methyl-6-oxo-3-propan-2-ylpyridazin-1-yl]-N-(5-fluoropyrimidin-4-yl)acetamide:** To a solution of methyl 2-(4-(3,5-difluorophenyl)-3-isopropyl-5-methyl-6-oxopyridazin-1(6*H*)-yl)acetate (60 mg, 0.18 mmol) in toluene (1 mL) and THF (1 mL) was added 5-fluoropyrimidin-4-amine (61 mg, 0.54 mmol) and AlMe₃ (2 M in toluene, 0.54 mmol, 0.27 mL) under N₂. The mixture was stirred at 110 °C for 3 h. The mixture was diluted with water (3 mL) and extracted with EtOAc (3 × 1 mL). The combined organics were washed with brine (3 mL), dried over Na₂SO₄, filtered, concentrated under reduced pressure and purified by reverse-phase preparative HPLC. LCMS: *m*/*z =* 418.1 [M+H]⁺.¹H NMR (400 MHz, CDCl₃): δ 8.78 (d, *J* = 1.6 Hz, 1H), 8.51 (d, *J* = 2.4 Hz, 1H), 6.96-6.87 (m, 1H), 6.77-6.70 (m, 2H), 5.39 (s, 2H), 2.66-2.58 (m, 1H), 1.97 (s, 3H), 1.10 (d, *J=* 6.8 Hz, 6H).

### Example 39

### N-[(3R)-1-cyclopropylpiperidin-3-yl]-2-[4-(3-fluorophenyl)-6-oxo-3-propan-2-ylpyridazin-1-yl]acetamide

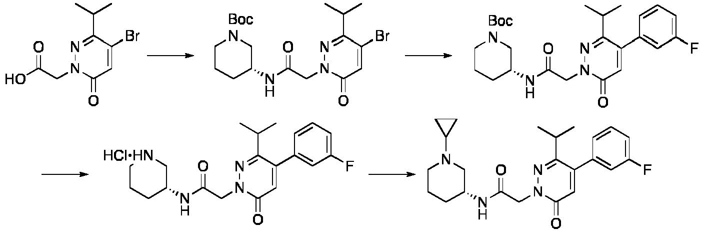

**(*R*)-*tert*-butyl 3-(2-(4-bromo-3-isopropyl-6-oxopyridazin-1(6*H*)-yl)acetamido)piperidine-1-carboxylate:** To a mixture of 2-(4-bromo-3-isopropyl-6-oxo-pyridazin-1-yl)acetic acid (0.7 g, 2.54 mmol) and *tert*-butyl (3*R*)-3-aminopiperidine-1-carboxylate (559 mg, 2.8 mmol) in EtOAc (7 mL) was added DIEA (1.3 g, 10.2 mmol) and T3P (3.02 mL, 5.1 mmol, 50% purity in EtOAc) at 0 °C. The mixture was stirred at 25 °C for 2 h. The reaction mixture was diluted with water (20 mL) and extracted with EtOAc (3 × 8 mL). The combined organics were washed with brine (8 mL), dried over anhydrous Na₂SO₄, filtered, and concentrated under reduced pressure. The residue was purified by silica gel column chromatography. LCMS: *m*/*z* = 357.1, 359.1 [M-99]⁺

**(*R*)-*tert-*butyl 3-(2-(4-(3-fluorophenyl)-3-isopropyl-6-oxopyridazin-1(6*H*)-yl)acetamido)piperidine-1-carboxylate:** To a mixture of (*R*)-*tert*-butyl 3-(2-(4-bromo-3-isopropyl-6-oxopyridazin-1(6*H*)-yl)acetamido)piperidine-1-carboxylate (300 mg, 0.65 mmol) and (3-fluorophenyl)boronic acid (110 mg, 0.78 mmol) in 1,4-dioxane (3 mL) and water (0.5 mL) was added a mixture of Cs₂CO₃ (427 mg, 1.3 mmol) and Pd(dppf)Cl₂ (48 mg, 0.065 mmol). The mixture was stirred at 100 °C for 3 h. The residue was diluted with water (10 mL) and extracted with EtOAc (3 × 5 mL). The combined organics were washed with brine (8 mL), dried over anhydrous Na₂SO₄, filtered, and concentrated under reduced pressure. The residue was purified by silica gel column chromatography. LCMS: *m*/*z* = 373.2 [M-99]⁺.

**(*R*)-2-(4-(3-fluorophenyl)-3-isopropyl-6-oxopyridazin-1(6*H*)-yl)-*N*-(piperidin-3-yl)acetamide HCl salt:** A solution of (*R*)-*tert*-butyl 3-(2-(4-(3-fluorophenyl)-3-isopropyl-6-oxopyridazin-1(6*H*)-yl)acetamido)piperidine-1-carboxylate (370 mg, 0.78 mmol) in HCl (4 M in EtOAc, 5 mL) was stirred for 2 h. The mixture was concentrated under reduced pressure and the residue was used directly. LCMS: *m*/*z =* 373.1 [M+H]⁺.

**N-[(3R)-1-cyclopropylpiperidin-3-yl]-2-[4-(3-fluorophenyl)-6-oxo-3-propan-2-ylpyridazin-1-yl]acetamide:** To a solution of (*R*)-2-(4-(3-fluorophenyl)-3-isopropyl-6-oxopyridazin-1(6*H*)-yl)*-N-*(piperidin-3-yl)acetamide HCl salt (40 mg, 0.1 mmol) in MeOH (2.5 mL) was added Et₃N (19.8 mg, 0.19 mmol). The reaction was stirred at 20 °C for 0.5 h followed by the addition of AcOH (0.5 mL), (1-ethoxycyclopropoxy)-trimethyl-silane (34.1 mg, 0.19 mmol), MgSO₄ (23.6 mg, 0.19 mmol), and NaBH₃CN (12.3 mg, 0.19 mmol). The reaction mixture was stirred at 60 °C for a further 12 h. The mixture was filtered and purified by reverse-phase preparative HPLC. LCMS: *m*/*z* = 413.2 [M+H]⁺. ¹H NMR (400 MHz, CDCl₃): δ 7.46 (m, 1H), 7.18 (dt, *J* = 2.0, 8.4 Hz, 1H), 7.09 (d, *J =* 7.6 Hz, 1H), 7.04 (m, 1H), 6.77 (s, 1H), 6.52 (br d, *J =* 6.6 Hz, 1H), 4.83 (d, *J =* 2.4 Hz, 2H), 4.06 (br s, 1H), 3.02-2.93 (m, 1H), 2.73-2.71 (m, 1H), 2.57 (br s, 2H), 2.35-2.33 (m, 1H), 1.64-1.45 (m, 4H), 1.11 (t, *J =* 6.2 Hz, 6H), 0.48-0.38 (m, 2H), 0.33-0.24 (m, 1H), 0.19-0.09 (m, 1H).

### Example 40

### 2-[3-cyclobutyl-4-(3-fluorophenyl)-6-oxopyridazin-1-yl]-N-[(3R)-1-ethylpiperidin-3-yl]acetamide

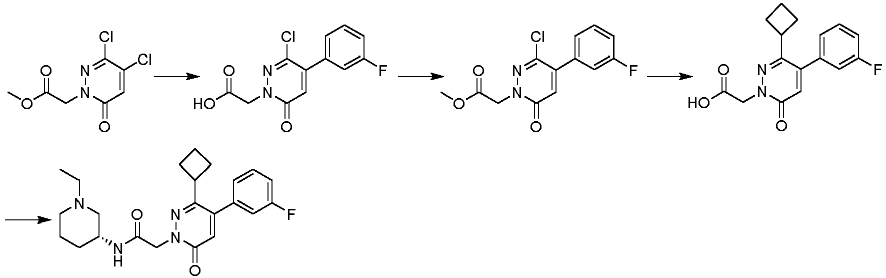

**2-(3-chloro-4-(3-fluorophenyl)-6-oxopyridazin-1(6*H*)-yl)acetic acid:** To a solution of methyl 2-(3,4-dichloro-6-oxopyridazin-1(6*H*)-yl)acetate (20 g, 59.1 mmol) in 1,4-dioxane (200 mL) and water (40 mL) were added (3-fluorophenyl)boronic acid (12.4 g, 88.6 mmol), Na₂CO₃ (12.5 g, 118 mmol), and Pd(PPh₃)₄ (3.4 g, 2.9 mmol). The mixture was stirred at 100 °C for 16 h. The mixture was poured into water (200 mL) and adjusted pH = 9 by NaHCO₃. The mixture extracted with EtOAc (150 mL). The aqueous phase was adjusted pH = 5 by aq. HCl (3 M) and extracted EtOAc (3 × 70 mL). The combined organic layers were washed with brine (200 mL), dried over anhydrous Na₂SO₄, filtered, and concentrated under reduced pressure. The crude residue was used directly. LCMS: *m*/*z* = 283.1, 285.1 [M+H]⁺.

**methyl 2-(3-chloro-4-(3-fluorophenyl)-6-oxopyridazin-1(6*H*)-yl)acetate:** To a solution of 2-(3-chloro-4-(3-fluorophenyl)-6-oxopyridazin-1(6*H*)-yl)acetic acid (2.0 g, 7.08 mmol) in MeCN (30 mL) was added diazomethyl(trimethyl)silane (2 M in *n*-hexane 7.08 mL) at 0 °C. The mixture was stirred at 20 °C for 16 h. The mixture was quenched by addition of aq. NH₄Cl (60 mL) and extracted with EtOAc (3 × 20 mL). The combined organics were washed with brine (20 mL), dried over anhydrous Na₂SO₄, filtered, concentrated under reduced pressure. The residue was purified by silica gel column chromatography. LCMS: *m*/*z* = 297.2, 299.2 [M+H]⁺.

**2-(3-cyclobutyl-4-(3-fluorophenyl)-6-oxopyridazin-1(6*H*)-yl)acetic acid:** To a solution of cyclobutylzinc(II) bromide (0.5 M in THF, 10.1 mL) in 1,4-dioxane (5 mL) were added Pd(dppf)Cl₂ (74 mg, 0.1 mmol) and methyl 2-(3-chloro-4-(3-fluorophenyl)-6-oxopyridazin-1(6*H*)-yl)acetate. The mixture was stirred at 80 °C for 16 h. The reaction mixture was diluted with water (2 mL) and concentrated under reduced pressure. The residue was purified by reverse-phase preparative HPLC. LCMS: *m*/*z* = 303.3 [M+H]⁺.

**2-[3-cyclobutyl-4-(3-fluorophenyl)-6-oxopyridazin-1-yl]-N-[(3R)-1-ethylpiperidin-3-yl]acetamide:** To a solution of 2-(3-cyclobutyl-4-(3-fluorophenyl)-6-oxopyridazin-1(6*H*)-yl)acetic acid (20 mg, 0.07 mmol) and (R)-1-ethylpiperidin-3-amine hydrochloride (16 mg, 0.10 mmol) in DMF (2 mL) was added DIPEA (26 mg, 0.20 mmol) and HATU (50 mg, 0.13 mmol). The mixture was stirred at 20 °C for 3 h. The reaction mixture was directly purified by reverse-phase preparative HPLC. LCMS: *m*/*z* = 413.1 [M+H]⁺.¹H NMR (400 MHz, CDCl₃): δ 7.43 (td, *J =* 8.0, 5.6 Hz, 1H), 7.16 (td, *J =* 8.4, 2.4 Hz, 1H), 7.03 (d, *J =* 7.6 Hz, 1H), 6.96 (br d, *J =* 9.2 Hz, 1H), 6.82 (br s, 1H), 6.76 (s, 1H), 4.82-4.93 (m, 2H), 4.11-4.13 (m, 1H), 3.42 (m, 1H), 2.33-2.65 (m, 5H), 2.16-2.31 (m, 3H), 1.89-2.06 (m, 5H), 1.64-1.78 (m, 3H), 1.03 (t, *J =* 7.2 Hz, 3H).

### Example 41

### 2-[4-(3-fluorophenyl)-6-oxo-3-propan-2-ylpyridazin-1-yl]-N-(3-fluoropyridin-2-yl)acetamide

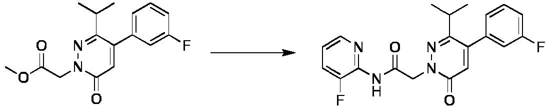

**2-[4-(3-fluorophenyl)-6-oxo-3-propan-2-ylpyridazin-1-yl]-N-(3-fluoropyridin-2-yl)acetamide:** To a mixture of methyl 2-(4-(3-fluorophenyl)-3-isopropyl-6-oxopyridazin-1(6*H*)-yl)acetate (100 mg, 0.33 mmol) and 3-fluoropyridin-2-amine (48 mg, 0.43 mmol) in toluene (3 mL) was added AlMe₃ (2 M in toluene, 0.49 mL). The mixture was stirred at 100 °C under N₂ for 3 h. The reaction mixture was diluted with water (10 mL) and extracted with EtOAc (3 × 5 mL). The combined organics were washed with brine (5 mL), dried over anhydrous Na₂SO₄, filtered, concentrated under reduced pressure, and purified by reverse-phase preparative HPLC. LCMS: *m*/*z* = 385.1 [M+H]⁺. ¹H NMR (400 MHz, CDCl₃) δ 8.66 (br s, 1H), 8.22 (br d, *J=* 4.4 Hz, 1H), 7.52-7.39 (m, 2H), 7.21-7.15 (m, 1H), 7.14-7.06 (m, 2H), 7.03 (br d, *J =* 8.8 Hz, 1H), 6.82 (s, 1H), 5.33 (br s, 2H), 2.97 (m, 1H), 1.13 (d, *J =* 6.8 Hz, 6H).

### Example 42

### 2-[4-(3,5-difluorophenyl)-6-oxo-3-propan-2-ylpyridazin-1-yl]-N-(5-fluoropyrimidin-4-yl)acetamide

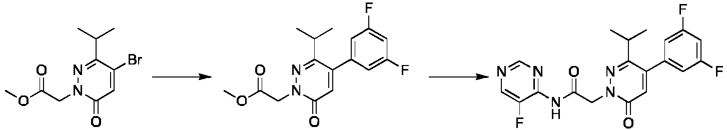

**methyl 2-(4-(3,5-difluorophenyl)-3-isopropyl-6-oxopyridazin-1(6*H*)-yl)acetate:** To a mixture of methyl 2-(4-bromo-3-isopropyl-6-oxopyridazin-1(6*H*)-yl)acetate (880 mg, 1.52 mmol), (3,5-difluorophenyl)boronic acid (200 mg, 1.27 mmol), CsF (577 mg, 3.80 mmol) in water (2.5 mL) and THF (5 mL) was added Pd(dppf)Cl₂ (93 mg, 0.13 mmol). The mixture was stirred at 100 °C for 16 h. The reaction mixture was diluted with water (10 mL) and extracted with EtOAc (3 × 5 mL). The combined organics were washed with brine (5 mL), dried over anhydrous Na₂SO₄, filtered, concentrated under reduced pressure, and purified by silica gel column chromatography. LCMS: *m*/*z* = 323.2 [M+H]⁺.

**2-[4-(3,5-difluorophenyl)-6-oxo-3-propan-2-ylpyridazin-1-yl]-N-(5-fluoropyrimidin-4-yl)acetamide:** To a mixture of methyl 2-(4-(3,5-difluorophenyl)-3-isopropyl-6-oxopyridazin-1(6*H*)-yl)acetate (100 mg, 0.31 mmol), 5-fluoropyrimidin-4-amine (105 mg, 0.93 mmol) in toluene (2 mL) and THF (2 mL) was added dropwise AlMe₃ (2 M in toluene, 0.5 mL) at 20 °C under N₂. The mixture was stirred for 3 h at 110 °C. The reaction mixture was poured into H₂O (10 mL) at 0 °C and extracted with EtOAc (4 × 5 mL). The combined organics were washed with brine (2 × 10 mL), dried over Na₂SO₄, filtered, concentrated under reduced pressure, and purified by reverse-phase preparative HPLC. LCMS: *m*/*z* = 404.0 [M+H]⁺.¹H NMR (400 MHz, CDCl₃): δ 9.03 (br s, 1H), 8.77 (d, *J* = 1.6 Hz, 1H), 8.51 (d, *J =* 2.0 Hz, 1H), 6.98-6.90 (m, 1H), 6.89-6.80 (m, 3H), 5.43 (s, 2H), 2.98-2.90 (m, 1H), 1.13 (d, *J =* 6.8 Hz, 6H).

### Example 43

### 2-[4-(3,5-difluorophenyl)-3-isopropyl-6-oxo-pyridazin-1-yl]-N-pyrimidin-2-yl-acetamide

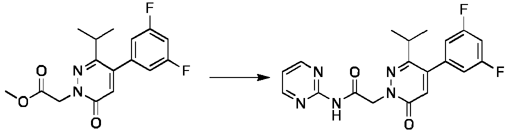

**2-[4-(3,5-difluorophenyl)-3-isopropyl-6-oxo-pyridazin-1-yl]-N-pyrimidin-2-yl-acetamide:** To a solution of methyl 2-(4-(3,5-difluorophenyl)-3-isopropyl-6-oxopyridazin-1(6*H*)-yl)acetate (80 mg, 0.25 mmol) and pyrimidin-2-amine (71 mg, 0.74 mmol) in toluene (2 mL) and THF (2 mL) was added AlMe₃ (2 M in toluene, 0.37 mL). The mixture was stirred at 100 °C for 8 h. The reaction mixture was poured into ice-cold water (5 mL) and extracted with EtOAc (3 × 5 mL). The combined organics were dried over anhydrous Na₂SO₄, filtered, concentrated under reduced pressure, and purified by reverse-phase preparative HPLC. LCMS: *m*/*z* = 386.1 [M+H]⁺. ¹H NMR (400 MHz, CDCl₃): δ 9.52 (br s, 1H), 8.66 (d, *J* = 4.8 Hz, 2H), 7.05 (t, *J* = 4.8 Hz, 1H), 6.93 (tt, *J* = 8.8, 2.4 Hz, 1H), 6.89-6.84 (m, 2H), 6.80 (s, 1H), 5.52 (s, 2H), 2.98-2.88 (m, 1H), 1.13 (d, *J* = 6.8 Hz, 6H).

### Example 44

### 2-[4-(3,5-difluorophenyl)-6-oxo-3-propan-2-ylpyridazin-1-yl]-N-(5-fluoropyrimidin-2-yl)acetamide

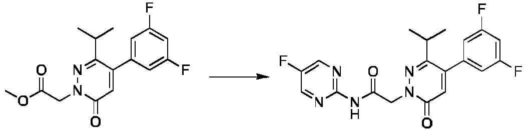

**2-[4-(3,5-difluorophenyl)-3-isopropyl-6-oxo-pyridazin-1-yl]-N-pyrimidin-2-yl-acetamide:** To a solution of methyl 2-(4-(3,5-difluorophenyl)-3-isopropyl-6-oxopyridazin-1(6*H*)-yl)acetate (100 mg, 0.31 mmol) and 5-fluoropyrimidin-2-amine (105 mg, 0.93 mmol) in toluene (1.5 mL) and THF (1.0 mL) was added AlMe₃ (2 M in toluene, 0.47 mL). The mixture was stirred at 100 °C for 3 h. The reaction mixture was poured into ice-cold water (5 mL) and extracted with EtOAc (3 × 5 mL). The combined organics were dried over anhydrous Na₂SO₄, filtered, concentrated under reduced pressure, and purified by reverse-phase preparative HPLC. LCMS: *m*/*z* = 404.0 [M+H]⁺. ¹H NMR (400 MHz, CDCl₃): δ 9.37 (br s, 1H), 8.51 (s, 2H), 6.93 (tt, *J =* 8.8, 2.4 Hz, 1H), 6.86 (br d, *J =* 5.4 Hz, 2H), 6.81 (s, 1H), 5.41 (s, 2H), 3.00-2.85 (m, 1H), 1.13 (d, *J =* 6.8 Hz, 6H).

### Example 45

### 2-[4-(3,5-difluorophenyl)-6-oxo-3-propan-2-ylpyridazin-1-yl]-N-(3,5-difluoropyridin-2-yl)acetamide

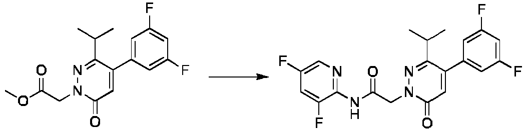

**2-[4-(3,5-difluorophenyl)-3-isopropyl-6-oxo-pyridazin-1-yl]-N-pyrimidin-2-yl-acetamide:** To a solution of methyl 2-(4-(3,5-difluorophenyl)-3-isopropyl-6-oxopyridazin-1(6*H*)-yl)acetate (100 mg, 0.31 mmol) and 3,5-difluoropyridin-2-amine (48 mg, 0.37 mmol) in toluene (3 mL) was added AlMe₃ (2 M in toluene, 0.2 mL). The mixture was stirred at 80 °C for 2 h. The reaction mixture was diluted with ice-cold water (5 mL) and extracted with EtOAc (3 × 5 mL). The combined organics were dried over anhydrous Na₂SO₄, filtered, concentrated under reduced pressure, and purified by reverse-phase preparative HPLC. LCMS: *m*/*z* = 421.1 [M+H]⁺. ¹H NMR (400 MHz, CDCl₃): δ 9.12 (br s, 1H), 8.12 (d, *J* = 1.6 Hz, 1H), 7.27 (s, 1H), 7.25-6.92 (m, 1H), 6.87-6.78 (m, 3H), 5.27 (br s, 2H), 2.98-2.88 (m, 1H), 1.12 (d, *J =* 6.8 Hz, 6H).

### Example 46

### 2-[4-(3-chloro-5-fluorophenyl)-6-oxo-3-propan-2-ylpyridazin-1-yl]-N-(5-fluoropyrimidin-4-yl)acetamide

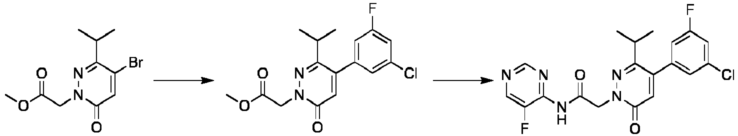

**methyl 2-(4-(3-chloro-5-fluorophenyl)-3-isopropyl-6-oxopyridazin-1(6*H*)-yl)acetate:** To a solution of methyl 2-(4-bromo-3-isopropyl-6-oxopyridazin-1(6*H*)-yl)acetate (200 mg, 0.69 mmol), (3-chloro-5-fluoro-phenyl)boronic acid (133 mg, 0.76 mmol) and CsF (315 mg, 2.08 mmol) in THF (2.0 mL) and water (1.0 mL) was added Pd(dppf)Cl₂ (51 mg, 0.07 mmol). The mixture was stirred at 110 °C for 12 h. The reaction mixture was quenched by addition of water (10 mL) and extracted with EtOAc (3 × 4 mL). The combined organics were washed with brine (4 mL), dried over anhydrous Na₂SO₄, filtered, concentrated under reduced pressure, and purified by silica gel column chromatography. LCMS: *m*/*z* = 339.1, 341.1 [M+H]⁺.

**2-[4-(3-chloro-5-fluorophenyl)-6-oxo-3-propan-2-ylpyridazin-1-yl]-N-(5-fluoropyrimidin-4-yl)acetamide:** To a solution of methyl 2-(4-(3-chloro-5-fluorophenyl)-3-isopropyl-6-oxopyridazin-1(6*H*)-yl)acetate (100 mg, 0.3 mmol) and 5-fluoropyrimidin-4-amine (50.1 mg, 0.44 mmol) in toluene (3.0 mL) was added AlMe₃ (2 M in toluene, 0.45 mL) The mixture was stirred at 50 °C for 3 h. The reaction mixture was quenched by addition of water (10 mL) and extracted with EtOAc (3 × 5 mL). The combined organics were washed with brine (5 mL), dried over anhydrous Na₂SO₄, filtered, concentrated under reduced pressure, and purified by reverse-phase preparative HPLC. LCMS: *m*/*z* = 420.1, 422.1 [M+H]⁺. ¹H NMR (400 MHz, CDCl₃) δ 8.91 (br s, 1H), 8.78 (d, *J* = 2.0 Hz, 1H), 8.51 (d, *J* = 2.4 Hz, 1H), 7.22 (td, *J* = 2.0, 8.4 Hz, 1H), 7.12 (s, 1H), 6.96 (dd, *J* = 1.2, 8.4 Hz, 1H), 6.82 (s, 1H), 5.43 (s, 2H), 2.98-2.89 (m, 1H), 1.14 (d, *J =* 6.8 Hz, 6H).

### Example 47

### 2-[4-[3-(2,2-difluorocyclopropyl)-5-fluorophenyl]-6-oxo-3-propan-2-ylpyridazin-1-yl]-N-(5-fluoropyrimidin-4-yl)acetamide

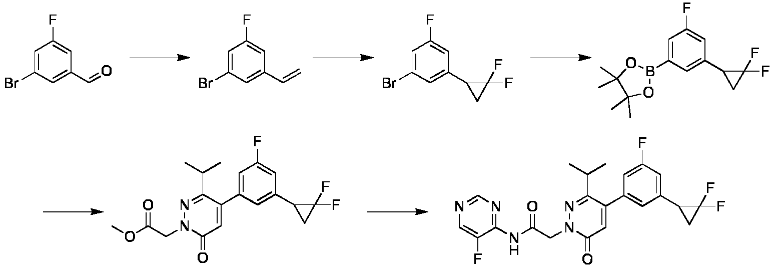

**1-bromo-3-fluoro-5-vinylbenzene:** To a solution of methyl(triphenyl)phosphonium bromide (12.3 g, 34.5 mmol) in THF (125 mL) was added t-BuOK (2.87 g, 25.6 mmol). The reaction mixture was stirred at 20 °C for 1 h. The reaction mixture was cooled to 0 °C. A solution of 3-bromo-5-fluorobenzaldehyde (5.0 g, 24.6 mmol) in THF (50 mL) was then added dropwise. The reaction mixture was stirred at 20 °C for further 12 h. The reaction mixture was cooled to 0 °C, diluted with saturated aqueous NH₄Cl (100 mL), and extracted with petroleum ether (3 × 50 mL). The combined organic layers were washed with brine (20 mL), dried over anhydrous Na₂SO₄, filtered, and concentrated under reduced pressure. The crude residue was purified by silica gel column chromatography.

**1-bromo-3-(2,2-difluorocyclopropyl)-5-fluorobenzene:** To a solution of 1-bromo-3-fluoro-5-vinylbenzene (50 mg, 0.25 mmol) in CH₃CN (2.0 mL) were added TMSCF₃ (354 mg, 2.49 mmol) and NaI (7 mg, 0.05 mmol). The reaction mixture was stirred at 110 °C for 2 h. The reaction mixture was diluted with water (10 mL) and extracted with MTBE (3 × 5 mL). The combined organic layers were washed with brine (5 mL), dried over anhydrous Na₂SO₄, filtered, and concentrated under reduced pressure to provide a residue that was used directly.

**2-(3-(2,2-difluorocyclopropyl)-5-fluorophenyl)-4,4,5,5-tetramethyl-1,3,2-dioxaborolane:** To a solution of 4,4,4',4',5,5,5',5'-octamethyl-2,2'-bi(1,3,2-dioxaborolane) (30 mg, 0.12 mmol), 1-bromo-3-(2,2-difluorocyclopropyl)-5-fluorobenzene (20 mg, 0.08 mmol), and KOAc (23 mg, 0.24 mmol) in 1,4-dioxane (2 mL) was added Pd(dppf)Cl₂•CH₂Cl₂ (20 mg, 0.02 mmol). The reaction mixture was stirred at 100 °C for 16 h. The reaction mixture was diluted with water (10 mL) and extracted with EtOAc (3 × 5 mL). The combined organic layers were washed with brine (5 mL), dried over anhydrous Na₂SO₄, filtered, and concentrated under reduced pressure to provide a residue that was used directly. LCMS: *m*/*z* = 299.2 [M+H]⁺.

**methyl 2-(4-(3-(2,2-difluorocyclopropyl)-5-fluorophenyl)-3-isopropyl-6-oxopyridazin-1(6*H*)-yl)acetate:** To a mixture of 2-(3-(2,2-difluorocyclopropyl)-5-fluorophenyl)-4,4,5,5-tetramethyl-1,3,2-dioxaborolane (100 mg, 0.11 mmol), methyl 2-(4-bromo-3-isopropyl-6-oxopyridazin-1(6*H*)-yl)acetate (42 mg, 0.14 mmol), and CsF (49 mg, 0.32 mmol) in water (1.0 mL) and THF (2.0 mL) was added Pd(dppf)Cl₂ (8 mg, 0.01 mmol). The reaction mixture was stirred at 100 °C for 16 h. The reaction mixture was diluted with water (10 mL) and extracted with EtOAc (3 × 5 mL). The combined organic layers were washed with brine (5 mL), dried over anhydrous Na₂SO₄, filtered, and concentrated under reduced pressure. The crude residue was purified by silica gel column chromatography. LCMS: *m*/*z* = 381.2 [M+H]⁺.

**2-[4-[3-(2,2-difluorocyclopropyl)-5-fluorophenyl]-6-oxo-3-propan-2-ylpyridazin-1-yl]-N-(5-fluoropyrimidin-4-yl)acetamide:** To a mixture of methyl 2-(4-(3-(2,2-difluorocyclopropyl)-5-fluorophenyl)-3-isopropyl-6-oxopyridazin-1(6*H*)-yl)acetate (10 mg, 0.03 mmol) and 5-fluoropyrimidin-4-amine (9 mg, 0.09 mmol) in toluene (1.0 mL) and THF (1.0 mL) was added AlMe₃ (2 M in toluene, 0.039 mL) dropwise. The reaction mixture was stirred for 3 h at 110 °C. The reaction mixture was cooled to 0 °C, diluted with water (10 mL), and extracted with EtOAc (4 × 5 mL). The combined organic layers were washed with brine (2 × 10 mL), dried over anhydrous Na₂SO₄, filtered, and concentrated under reduced pressure. The crude residue was purified by reverse-phase preparative HPLC. LCMS: *m*/*z* = 462.1 [M+H]⁺. ¹H NMR (400 MHz, CDCl₃): δ 9.02 (br s, 1H), 8.78 (d, *J* = 1.6 Hz, 1H), 8.51 (d, *J* = 2.4 Hz, 1H), 7.05 (br d, *J* = 9.2 Hz, 1H), 6.99-6.89 (m, 2H), 6.83 (s, 1H), 5.41 (s, 2H), 2.94 (m, 1H), 2.83-2.75 (m, 1H), 2.01-1.88 (m, 1H), 1.75-1.68 (m, 1H), 1.12 (d, *J =* 6.8 Hz, 6H).

### Example 48

### 2-[3-cyclopropyl-4-(3,5-difluorophenyl)-6-oxopyridazin-1-yl]-N-(5-fluoropyrimidin-4-yl)acetamide

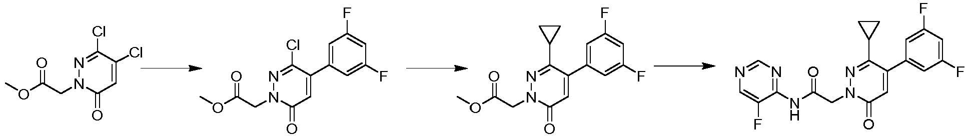

**methyl 2-(3-chloro-4-(3,5-difluorophenyl)-6-oxopyridazin-1(6*H*)-yl)acetate:** To a mixture of methyl 2-(3,4-dichloro-6-oxo-pyridazin-1-yl)acetate (600 mg, 2.53 mmol), (3,5-difluorophenyl)boronic acid (400 mg, 2.53 mmol), and CsF (1.15 g, 7.59 mmol) in 1,4-dioxane (6.0 mL) was added Pd(dppf)Cl₂ (93 mg, 0.13 mmol). The reaction mixture was stirred at 100 °C for 12 h. The reaction mixture was diluted with water (15 mL) and extracted with EtOAc (3 × 5 mL). The combined organic layers were washed with brine (5 mL), dried over anhydrous Na₂SO₄, filtered, and concentrated under reduced pressure. The crude residue was purified by silica gel column chromatography. LCMS: *m*/*z* = 315.1, 317.1 [M+H]⁺.

**2-(6-bromo-4-isopropyl-1-oxophthalazin-2(1*H*)-yl)-*N*-(5-fluoropyrimidin-4-yl)propanamide:** To a mixture of methyl 2-(3-chloro-4-(3,5-difluorophenyl)-6-oxopyridazin-1(6*H*)-yl)acetate (100 mg, 0.32 mmol), cyclopropylboronic acid (136 mg, 1.59 mmol), and CsF (144.82 mg, 0.953 mmol) in 1,4-dioxane (5.0 mL) was added Pd(dppf)Cl₂ (12 mg, 0.016 mmol). The reaction mixture was stirred at 80 °C for 3 h. The reaction mixture was diluted with water (15 mL) and extracted with EtOAc (3 × 5 mL). The combined organic layers were washed with brine (5 mL), dried over anhydrous Na₂SO₄, filtered, and concentrated under reduced pressure. The crude residue was purified by silica gel column chromatography. LCMS: *m*/*z* = 321.2 [M+H]⁺.

**2-[3-cyclopropyl-4-(3,5-difluorophenyl)-6-oxopyridazin-1-yl]-N-(5-fluoropyrimidin-4-yl)acetamide:** To a mixture of 2-(6-bromo-4-isopropyl-1-oxophthalazin-2(1*H*)-yl)-*N*-(5-fluoropyrimidin-4-yl)propanamide (130 mg, 0.41 mmol) and 5-fluoropyrimidin-4-amine (92 mg, 0.81 mmol) in toluene (5.0 mL) was added AlMe₃ (2 M in toluene, 0.30 mL). The reaction mixture was stirred at 80 °C for 5 h. The reaction mixture was diluted with water (10 mL) and filtered. The filtrate was extracted with EtOAc (3 × 5 mL). The combined organic layers were washed with brine (5 mL), dried over anhydrous Na₂SO₄, filtered, and concentrated under reduced pressure. The crude residue was purified by reverse-phase preparative HPLC. LCMS: *m*/*z* = 402.1 [M+H]⁺. ¹H NMR (400 MHz, CDCl₃): δ 8.88 (br s, 1H), 8.77 (d, *J=* 1.6 Hz, 1H), 8.51 *(d, J* = 2.4 Hz, 1H), 7.02 (br d, *J =* 5.6 Hz, 2H), 6.98-6.91 (m, 1H), 6.87 (s, 1H), 5.36 (s, 2H), 1.69-1.66 (m, 1H), 1.10-1.04 (m, 2H), 0.95-0.88 (m, 2H).

### Example 49

### 2-[3-(2,2-difluorocyclopropyl)-4-(3,5-difluorophenyl)-6-oxopyridazin-1-yl]-N-(5-fluoropyrimidin-4-yl)acetamide

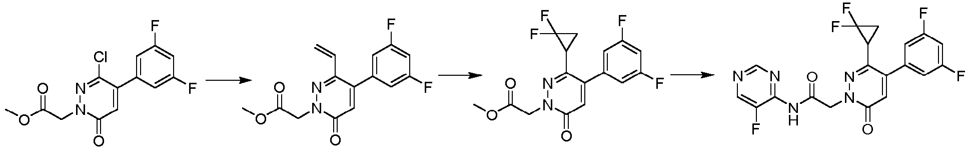

**methyl 2-(4-(3,5-difluorophenyl)-6-oxo-3-vinylpyridazin-1(6*H*)-yl)acetate:** To a solution of methyl 2-(3-chloro-4-(3,5-difluorophenyl)-6-oxopyridazin-1(6*H*)-yl)acetate (2.0 g, 6.36 mmol) in 1,4-dioxane (100 mL) were added potassium vinyltrifluoroborate (8.51 g, 63.6 mmol), CsF (2.90 g, 19.1 mmol), and Pd(dppf)Cl₂ (465 mg, 0.64 mmol). The reaction mixture was stirred at 100 °C for 16 h. The reaction mixture was concentrated under reduced pressure. The crude residue was purified by silica gel column chromatography. ¹H NMR (400 MHz, CDCl₃): δ 6.89-6.99 (m, 3H), 6.84 (s, 1H), 6.36 (dd, *J* = 17.2, 10.8 Hz, 1H), 6.02 (dd, *J* = 17.2, 1.2 Hz, 1H), 5.41 (dd, *J =* 10.8, 1.2 Hz, 1H), 4.98 (s, 2H), 3.83 (s, 3H).

**methyl 2-(3-(2,2-difluorocyclopropyl)-4-(3,5-difluorophenyl)-6-oxopyridazin-1(6*H*)-yl)acetate:** To a solution of methyl 2-(4-(3,5-difluorophenyl)-6-oxo-3-vinylpyridazin-1(6*H*)-yl)acetate (100 mg, 0.33 mmol) and NaI (10 mg, 0.07 mmol) in THF (1.0 mL) was added dropwise TMSCF₃ (929 mg, 6.53 mmol). The reaction mixture was stirred at 65 °C for 16 h. 5 batches of the proceeding reaction were run in parallel and combined for workup. The combined reaction mixtures were diluted with water (5 mL) and extracted with EtOAc (3 × 10 mL). The combined organic layers were washed with brine (5 mL), dried over anhydrous Na₂SO₄, filtered, and concentrated under reduced pressure. The crude residue was purified by silica gel column chromatography.

**2-[3-(2,2-difluorocyclopropyl)-4-(3,5-difluorophenyl)-6-oxopyridazin-1-yl]-N-(5-fluoropyrimidin-4-yl)acetamide:** To a solution of methyl 2-(3-(2,2-difluorocyclopropyl)-4-(3,5-difluorophenyl)-6-oxopyridazin-1(6*H*)-yl)acetate (100 mg, 0.28 mmol) and 5-fluoropyrimidin-4-amine (25.39 mg, 0.22 mmol) in toluene (1.0 mL) and THF (3.0 mL) was added AlMe₃ (2 M in toluene, 0.28 mL). The reaction mixture was stirred at 90 °C for 3 h. The reaction mixture was diluted with water (5 mL) and extracted with EtOAc (2 × 5 mL). The combined organic layers were washed with brine (5 mL), dried over anhydrous Na₂SO₄, filtered, and concentrated under reduced pressure. The crude residue was purified by reverse-phase preparative HPLC. LCMS: *m*/*z* = 438.1 [M+H]⁺. ¹H NMR (400 MHz, CDCl₃): δ 8.77 (d, *J* = 2.0 Hz, 1H), 8.49-8.63 (m, 2H), 6.92-7.01 (m, 4H), 5.62 (d, *J* = 16.4 Hz, 1H), 5.35 (d, *J* = 16.4 Hz, 1H), 2.44 (m, 1H), 2.22-2.34 (m, 1H), 1.69-1.77 (m, 1H).

### Example 50

### 2-[4-(3,5-difluorophenyl)-6-oxo-3-(2,2,2-trifluoroethyl)pyridazin-1-yl]-N-(5-fluoropyrimidin-4-yl)acetamide

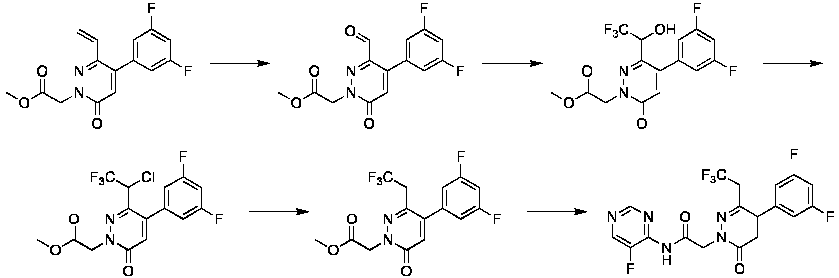

**methyl 2-(4-(3,5-difluorophenyl)-3-formyl-6-oxopyridazin-1(6*H*)-yl)acetate:** A solution of methyl 2-(4-(3,5-difluorophenyl)-6-oxo-3-vinylpyridazin-1(6*H*)-yl)acetate (1.45 g, 4.73 mmol) in DCM (20 mL) at -78 °C was bubbled with ozone for 0.5 h followed by the addition of Me₂S (3.97 g, 63.9 mmol). The reaction mixture was allowed to warm and stirred at 20 °C for 15.5 h. The reaction mixture was diluted with water (20 mL) and extracted with DCM (3 × 5 mL). The combined organic layers were washed with brine (20 mL), dried over anhydrous Na₂SO₄, filtered, and concentrated under reduced pressure to provide a residue that was used directly. LCMS: *m*/*z* = 309.1 [M+H]⁺.

**methyl 2-(4-(3,5-difluorophenyl)-6-oxo-3-(2,2,2-trifluoro-1-hydroxyethyl)pyridazin-1(6*H*)-yl)acetate:** To a solution of methyl 2-(4-(3,5-difluorophenyl)-3-formyl-6-oxopyridazin-1(6*H*)-yl)acetate (1.40 g, 4.54 mmol) in THF (30 mL) at 0 °C were added TMSCF₃ (3.23 g, 22.7 mmol) and TBAF (1 M in THF, 0.45 mmol). The reaction mixture was stirred at 20 °C for 2 h followed by the addition of TBAF (1 M in THF, 9.08 mmol). The reaction mixture was stirred at 20 °C for a further 4 h. The reaction mixture was diluted with water (30 mL) and extracted with EtOAc (3 × 10 mL). The combined organic layers were washed with brine (30 mL), dried over anhydrous Na₂SO₄, filtered, and concentrated under reduced pressure. The crude residue was purified by silica gel column chromatography. LCMS: *m*/*z* = 379.1 [M+H]⁺.

**methyl 2-(3-(1-chloro-2,2,2-trifluoroethyl)-4-(3,5-difluorophenyl)-6-oxopyridazin-1(6*H*)-yl)acetate:** To a mixture of methyl 2-(4-(3,5-difluorophenyl)-6-oxo-3-(2,2,2-trifluoro-1-hydroxyethyl)pyridazin-1(6*H*)-yl)acetate (250 mg, 0.66 mmol) in pyridine (105 mg, 1.32 mmol) at 0 °C were added SOCl₂ (87 mg, 0.73 mmol) and DMF (5 mg, 0.66 mmol). The reaction mixture was stirred at 50 °C for 2 h. The reaction mixture was diluted with water (5 mL) and extracted with EtOAc (3 × 2 mL). The combined organic layers were washed with brine (5 mL), dried over anhydrous Na₂SO₄, filtered, and concentrated under reduced pressure. The crude residue was purified by silica gel column chromatography. LCMS: *m*/*z =* 397.1, 399.1 [M+H]⁺.

**methyl 2-(4-(3,5-difluorophenyl)-6-oxo-3-(2,2,2-trifluoroethyl)pyridazin-1(6*H*)-yl)acetate:** To a solution of methyl 2-(3-(1-chloro-2,2,2-trifluoroethyl)-4-(3,5-difluorophenyl)-6-oxopyridazin-1(6H)-yl)acetate (100 mg, 0.25 mmol) in EtOAc (5.0 mL) was added Pd/C (20 mg, 10% purity). The reaction mixture was stirred at 20 °C for 2 h under an H₂ atmosphere (15 psi). The reaction mixture was filtered and the filtrate was concentrated under reduced pressure to provide a residue that was used directly. LCMS: *m*/*z* = 363.1 [M+H]⁺.

**2-[4-(3,5-difluorophenyl)-6-oxo-3-(2,2,2-trifluoroethyl)pyridazin-1-yl]-N-(5-fluoropyrimidin-4-yl)acetamide:** To a solution of methyl 2-(4-(3,5-difluorophenyl)-6-oxo-3-(2,2,2-trifluoroethyl)pyridazin-1(6*H*)-yl)acetate (40 mg, 0.11 mmol) in toluene (1.0 mL) and THF (1.0 mL) were added 5-fluoropyrimidin-4-amine (18 mg, 0.17 mmol) and AlMe₃ (2 M in toluene, 0.17 mL). The reaction mixture was stirred at 100 °C for 3 h. The reaction mixture was diluted with water (2 mL) and extracted with EtOAc (3 × 1 mL). The combined organic layers were washed with brine, dried over anhydrous Na₂SO₄, filtered, and concentrated under reduced pressure. The crude residue was purified by reverse-phase preparative HPLC. LCMS: *m*/*z* = 444.1 [M+H]⁺. ¹H NMR (400 MHz, CDCl₃): δ 8.77 (d, *J* = 2.0 Hz, 1H), 8.53 (d, *J* = 2.4 Hz, 1H), 8.43 (br s, 1H), 6.98 (tt, *J* = 2.4, 8.8 Hz, 1H), 6.90 (s, 1H), 6.89-6.83 (m, 2H), 5.58 (s, 2H), 3.42 (q, *J =* 9.6 Hz, 2H).

### Example 51

### 1-[4-(3,5-difluorophenyl)-3-isopropyl-6-oxo-pyridazin-1-yl]-N-pyrimidin-2-yl-cyclopropanecarboxamide

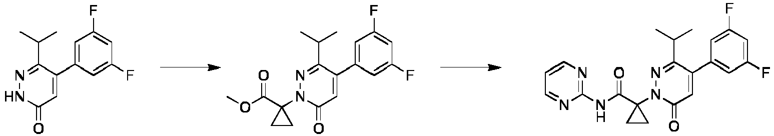

**Methyl 1-(4-(3,5-difluorophenyl)-3-isopropyl-6-oxopyridazin-1(6*H*)-yl)cyclopropane-carboxylate:** To a solution of 5-(3,5-difluorophenyl)-6- isopropylpyridazin-3(2*H*)-one (180 mg, 0.72 mmol) and methyl 2,4-dibromobutanoate (206 mg, 0.79 mmol) in DMF (4.5 mL) was added Cs₂CO₃ (937 mg, 2.88 mmol). The reaction mixture was stirred at 45 °C for 2 h. The reaction mixture was diluted with water (30 mL) and extracted with EtOAc (3 × 20 mL). The combined organic layers were washed with brine, dried over anhydrous Na₂SO₄, filtered, and concentrated under reduced pressure. The crude residue was purified by preparative TLC. LCMS: *m*/*z* = 349.0 [M+H]⁺.

**1-[4-(3,5-difluorophenyl)-3-isopropyl-6-oxo-pyridazin-1-yl]-N-pyrimidin-2-yl-cyclopropanecarboxamide:** To a solution of methyl 1-(4-(3,5-difluorophenyl)-3-isopropyl-6-oxopyridazin-1(6*H*)-yl)cyclopropanecarboxylate (80 mg, 0.23 mmol) and pyrimidin-2-amine (44 mg, 0.46 mmol) in THF (1.0 mL) and toluene (1.0 mL) was added AlMe₃ (2 M in toluene, 0.11 mL). The reaction mixture was stirred at 70 °C for 12 h. The reaction mixture was diluted with water (5 mL) and extracted with EtOAc (3 × 5 mL). The combined organic layers dried over anhydrous Na₂SO₄, filtered, and concentrated under reduced pressure. The crude residue was purified by reverse-phase preparative HPLC. LCMS: *m*/*z* = 412.2 [M+H]⁺. ¹H NMR (400 MHz, DMSO-*d*₆): δ 9.97 (br. s, 1H), 8.70 (d, *J* = 4.8 Hz, 2H), 7.41 (t, *J* = 9.6 Hz, 1H), 7.24 (m, 3H), 6.86 (s, 1H), 2.96-2.89 (m, 1H), 1.87-1.83 (m, 2H), 1.45-1.39 (m, 2H), 1.06 (d, *J =* 6.8 Hz, 6H).

### Example 52

### 2-[3-ethyl-4-(3-fluorophenyl)-5-methyl-6-oxopyridazin-1-yl]-N-(5-fluoropyrimidin-4-yl)acetamide

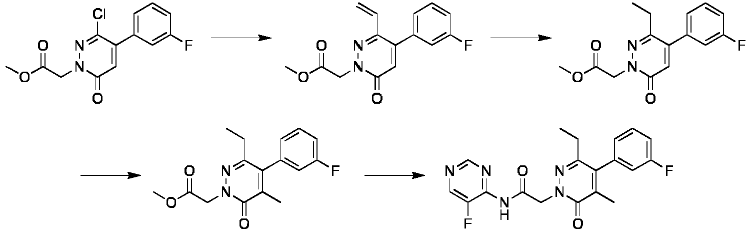

**methyl 2-(4-(3-fluorophenyl)-6-oxo-3-vinylpyridazin-1(6*H*)-yl)acetate:** To a solution of methyl 2-(3-chloro-4-(3-fluorophenyl)-6-oxopyridazin-1(6*H*)-yl)acetate (300 mg, 1.01 mmol) and potassium vinyltrifluoroborate (1.35 g, 10.11 mmol) in 1,4-dioxane (10 mL) were added CsF (307 mg, 2.02 mmol) and Pd(dppf)Cl₂ (74 mg, 0.1 mmol). The reaction mixture was stirred at 100 °C for 3 h. The reaction mixture was diluted with water (10 mL) and extracted with EtOAc (3 × 5 mL). The combined organic layers were washed with brine (5 mL), dried over anhydrous Na₂SO₄, filtered, and concentrated under reduced pressure. The crude residue was purified by silica gel column chromatography.

**methyl 2-(3-ethyl-4-(3-fluorophenyl)-6-oxopyridazin-1(6*H*)-yl)acetate:** To a solution of methyl 2-(4-(3-fluorophenyl)-6-oxo-3-vinylpyridazin-1(6H)-yl)acetate (280 mg, 0.98 mmol) in EtOAc (10 mL) was added Pd/C (100 mg, 10% purity). The mixture was degassed under vacuum and purged with H₂. The reaction mixture was stirred under an atmosphere of H₂ (15 psi) at 20 °C for 16 h. The reaction mixture was filtered and concentrated under reduced pressure to provide a residue that was used directly.

**methyl 2-(3-ethyl-4-(3-fluorophenyl)-5-methyl-6-oxopyridazin-1(6*H*)-yl)acetate:** To a solution of methyl 2-(3-ethyl-4-(3-fluorophenyl)-6-oxopyridazin-1(6*H*)-yl)acetate (140 mg, 0.48 mmol) and AcOH (1.45 g, 24.1 mmol) in water (5.0 mL) and MeCN (5.0 mL) were added ammonia;sulfooxy hydrogen sulfate (275 mg, 1.21 mmol) and AgNO₃ (164 mg, 0.96 mmol). The reaction mixture was stirred at 20 °C for 16 h. and then 50 °C for a further 4 h. The reaction mixture was diluted with water (10 mL) and extracted with EtOAc (3 × 10 mL). The combined organic layers were washed with brine (10 mL), dried over anhydrous Na₂SO₄, filtered, and concentrated under reduced pressure. The crude residue was purified by silica gel column chromatography LCMS: *m*/*z* = 305.1 [M+H]⁺.

**2-[3-ethyl-4-(3-fluorophenyl)-5-methyl-6-oxopyridazin-1-yl]-N-(5-fluoropyrimidin-4-yl)acetamide:** To a solution of methyl 2-(3-ethyl-4-(3-fluorophenyl)-5-methyl-6-oxopyridazin-1(6*H*)-yl)acetate (100 mg, 0.33 mmol) and 5-fluoropyrimidin-4-amine (37 mg, 0.33 mmol) in THF (1.0 mL) and toluene (1.0 mL) was added AlMe₃ (2 M in toluene, 0.50 mL). The reaction mixture was stirred at 90 °C for 3 h. The reaction mixture was diluted with water (10 mL) and extracted with EtOAc (3 × 10 mL). The combined organic layers were washed with brine (10 mL), dried over anhydrous Na₂SO₄, filtered, and concentrated under reduced pressure. The crude residue was purified by reverse-phase preparative HPLC. LCMS: *m*/*z* = 386.1 [M+H]⁺. ¹H NMR (400 MHz, CDCl₃): δ 9.25-8.92 (m, 1H), 8.86-8.72 (m, 1H), 8.50 (br s, 1H), 7.52-7.45 (m, 1H), 7.16 (dt, *J* = 2.0, 8.4 Hz, 1H), 6.96 (d, *J* = 7.6 Hz, 1H), 6.90 (br d, *J* = 8.4 Hz, 1H), 5.41 (s, 2H), 2.36 (q, *J* = 7.6 Hz, 2H), 1.98 (s, 3H), 1.08-1.02 (t, *J =* 8.0 Hz, 3H).

### Example 53

### 2-[3-(difluoromethyl)-4-(3,5-difluorophenyl)-6-oxopyridazin-1-yl]-N-(5-fluoropyrimidin-2-yl)acetamide

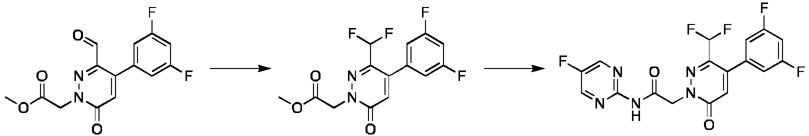

**methyl 2-(3-(difluoromethyl)-4-(3,5-difluorophenyl)-6-oxopyridazin-1(6*H*)-yl)acetate:** A solution of methyl 2-(4-(3,5-difluorophenyl)-3-formyl-6-oxopyridazin-1(6*H*)-yl)acetate (1.30 g, 4.22 mmol) in BAST (13.1 g, 59.4 mmol) was stirred at 20 °C for 12 h. The reaction mixture was poured into ice-cold aq. sat. NaHCO₃ (50 mL) and extracted with DCM (3 × 20 mL). The combined organic layers were washed with brine (15 mL), dried over anhydrous Na₂SO₄, filtered, and concentrated under reduced pressure. The crude residue was purified by silica gel column chromatography. ¹H NMR (400 MHz, CDCl₃): δ 6.98-6.94 (m, 3H), 6.90 (s, 1H), 6.42 (t, *J* = 53.6 Hz, 1H), 4.96 (s, 2H), 3.84 (s, 3H).

**2-[3-(difluoromethyl)-4-(3,5-difluorophenyl)-6-oxopyridazin-1-yl]-N-(5-fluoropyrimidin-2-yl)acetamide:** To a mixture of 5-fluoropyrimidin-2-amine (205 mg, 1.82 mmol) and methyl 2-(3-(difluoromethyl)-4-(3,5-difluorophenyl)-6-oxopyridazin-1(6*H*)-yl)acetate (200 mg, 0.61 mmol) in toluene (5.0 mL) was added trimethyl-(4-trimethylalumanuidyl-1,4-diazoniabicyclo[2.2.2]octan-1-yl)alumanuide (139 mg, 0.55 mmol). The reaction mixture was stirred for 12 h at 60 °C. The reaction mixture was cooled to 0 °C, diluted with water (10 mL), and extracted with EtOAc (4 × 5 mL). The combined organic layers were washed with brine (2 × 10 mL), dried over anhydrous Na₂SO₄, filtered, and concentrated under reduced pressure. The crude residue was purified by preparative TLC and followed by preparative SFC. LCMS: *m*/*z* = 412.1 [M+H]⁺. ¹H NMR (400 MHz, DMSO-*d*₆): δ 11.22 (s, 1H), 8.79 (s, 2H), 7.49-7.40 (m, 1H), 7.30 (br d, *J =* 6.0 Hz, 2H), 7.13 (s, 1H), 6.89 (t, *J* = 53.2 Hz, 1H), 5.21 (s, 2H).

### Example 54

### 2-[4-(5-fluoropyridin-3-yl)-6-oxo-3-propan-2-ylpyridazin-1-yl]-N-(5-fluoropyrimidin-2-yl)acetamide

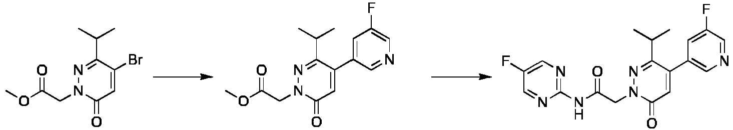

**methyl 2-(4-(5-fluoropyridin-3-yl)-3-isopropyl-6-oxopyridazin-1(6*H*)-yl)acetate:** To a solution of methyl 2-(4-bromo-3-isopropyl-6-oxopyridazin-1(6*H*)-yl)acetate (200 mg, 0.69 mmol) in 1,4-dioxane (2.0 mL) were added (5-fluoropyridin-3-yl)boronic acid (292 mg, 2.08 mmol), CsF (315 mg, 2.08 mmol), and Pd(dppf)Cl₂ (51 mg, 0.07 mmol). The reaction mixture was stirred at 90 °C for 4 h. The reaction mixture was diluted with water (5 mL) and extracted with EtOAc (3 × 2 mL). The combined organic layers were washed with brine (3 mL), dried over anhydrous Na₂SO₄, filtered, and concentrated under reduced pressure. The crude residue was purified by silica gel column chromatography. LCMS: *m*/*z* = 306.1 [M+H]⁺.

**2-[4-(5-fluoropyridin-3-yl)-6-oxo-3-propan-2-ylpyridazin-1-yl]-N-(5-fluoropyrimidin-2-yl)acetamide:** To a solution of methyl 2-(4-(5-fluoropyridin-3-yl)-3-isopropyl-6-oxopyridazin-1(6*H*)-yl)acetate (100 mg, 0.33 mmol) in toluene (1.0 mL) and THF (0.5 mL) were added 5-fluoropyrimidin-2-amine (111 mg, 0.98 mmol) and AlMe₃ (2 M in toluene, 0.49 mL). The reaction mixture was stirred at 90 °C for 6 h. The reaction mixture was diluted with water (5 mL) and extracted with EtOAc (3 × 2 mL). The combined organic layers were washed with brine (5 mL), dried over anhydrous Na₂SO₄, filtered, and concentrated under reduced pressure. The crude residue was purified by reverse-phase preparative HPLC. LCMS: *m*/*z* = 387.1 [M+H]⁺. ¹H NMR (400 MHz, CDCl₃): δ 9.08 (br s, 1H), 8.61 (d, *J* = 2.8 Hz, 1H), 8.51 (s, 2H), 8.44 (s, 1H), 7.42 (td, *J =* 2.4, 8.4 Hz, 1H), 6.85 (s, 1H), 5.42 (br s, 2H), 2.93-2.84 (m, 1H), 1.14 (d, *J* = 6.8 Hz, 6H).

### Example 55

### 2-[4-(3,5-difluorophenyl)-3-(1-fluoropropan-2-yl)-6-oxopyridazin-1-yl]-N-(5-fluoropyrimidin-2-yl)acetamide

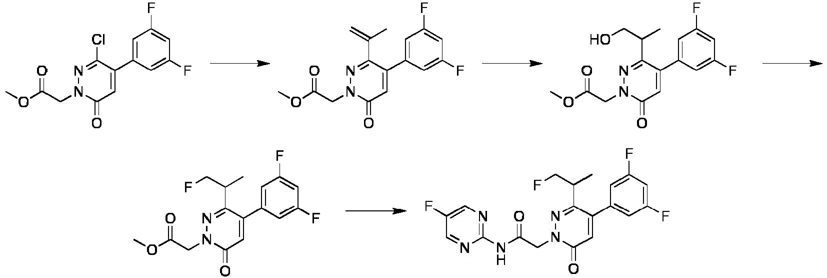

**methyl 2-(4-(3,5-difluorophenyl)-6-oxo-3-(prop-1-en-2-yl)pyridazin-1(6*H*)-yl)acetate:** To a solution of methyl 2-(3-chloro-4-(3,5-difluorophenyl)-6-oxopyridazin-1(6*H*)-yl)acetate (5.0 g, 15.9 mmol) and 2-isopropenyl-4,4,5,5-tetramethyl-1,3,2-dioxaborolane (26.7 g, 158.9 mmol) in 1,4-dioxane (100 mL) were added CsF (7.24 g, 47.7 mmol) and Pd(dppf)Cl₂ (1.16 g, 1.59 mmol). The reaction mixture was stirred at 100 °C for 16 h. The reaction mixture was filtered. The filtrate was diluted with water (150 mL) and extracted with EtOAc (3 × 50 mL). The combined organic layers were washed with brine (50 mL), dried over anhydrous Na₂SO₄, filtered, and concentrated under reduced pressure. The crude residue was purified by silica gel column chromatography. LCMS: *m*/*z* = 321.0 [M+H]⁺.

**methyl 2-(4-(3,5-difluorophenyl)-3-(1-hydroxypropan-2-yl)-6-oxopyridazin-1(6*H*)-yl)acetate:** To a solution of methyl 2-[4-(3,5-difluorophenyl)-3-isopropenyl-6-oxo-pyridazin-1-yl]acetate (1.80 g, 5.62 mmol) in DCM (30 mL) was added 9-BBN (0.5 M in THF, 39.3 mL). The reaction mixture was stirred at 50 °C for 64 h. The reaction mixture was diluted with water (60 mL) and adjusted to pH=8 by addition of sat. aq. Na₂CO₃. The reaction mixture was then cooled to 0 °C followed by the addition of H₂O₂ (2.55 g, 22.48 mmol, 36% purity). The reaction mixture was stirred at 15 °C for 1 h. The reaction mixture was quenched by the addition of sat. aq. Na₂S₂O₃ (20 mL) and extracted with EtOAc (3 × 20 mL). The combined organic layers were washed with brine (20 mL), dried over anhydrous Na₂SO₄, filtered, and concentrated under reduced pressure. The crude residue was purified by reverse-phase HPLC. LCMS: *m*/*z* = 339.0 [M+H]⁺.

**methyl 2-(4-(3,5-difluorophenyl)-3-(1-fluoropropan-2-yl)-6-oxopyridazin-1(6*H*)-yl)acetate:** To a solution of methyl 2-(4-(3,5-difluorophenyl)-3-(1-hydroxypropan-2-yl)-6-oxopyridazin-1(6*H*)-yl)acetate (300 mg, 0.887 mmol) in DCM (3 mL) at 0 °C was added a solution of BAST (1.96 g, 8.87 mmol, 1.94 mL) The reaction mixture was stirred at 25 °C for 16 h. The reaction mixture cooled to 0 °C, diluted with DCM (5 mL), and quenched by addition sat. aq. NaHCO₃ (10 mL). The resultant mixture was extracted with EtOAc (3 × 5 mL). The combined organic layers were washed with brine (5 mL), dried over anhydrous Na₂SO₄, filtered, and concentrated under reduced pressure. The crude residue was purified by silica gel column chromatography. LCMS: *m*/*z* = 341.0 [M+H]⁺.

**2-[4-(3,5-difluorophenyl)-3-(1-fluoropropan-2-yl)-6-oxopyridazin-1-yl]-N-(5-fluoropyrimidin-2-yl)acetamide:** To a solution of methyl 2-(4-(3,5-difluorophenyl)-3-(1-fluoropropan-2-yl)-6-oxopyridazin-1(6*H*)-yl)acetate (100 mg, 0.294 mmol) and 5-fluoropyrimidin-2-amine (37 mg, 0.32 mmol) in DCE (3.0 mL) was added trimethyl-(4-trimethylalumanuidyl-1,4-diazoniabicyclo[2.2.2]octan-1-yl)alumanuide (75 mg, 0.29 mmol). The reaction mixture was stirred at 80 °C for 24 h. The reaction mixture was filtered and the filtrated was concentrated under reduced pressure. The crude residue was purified by reverse-phase preparative HPLC. LCMS: *m*/*z* = 422.0 [M+H]⁺. ¹H NMR (400 MHz, CDCl₃): δ 9.13-8.84 (m, 1H), 8.62-8.45 (m, 2H), 7.04-6.79 (m, 4H), 5.58-5.24 (m, 2H), 4.74-4.30 (m, 2H), 3.33-3.09 (m, 1H), 1.13 (dd, *J=* 1.2, 6.8 Hz, 3H).

### Example 56

### 2-[3-cyclobutyl-4-(3,5-difluorophenyl)-6-oxopyridazin-1-yl]-N-(5-fluoropyrimidin-2-yl)acetamide

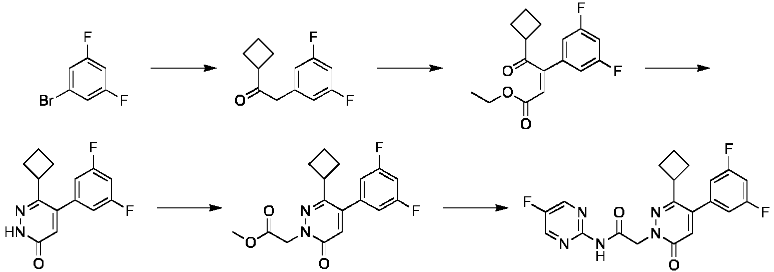

**1-cyclobutyl-2-(3,5-difluorophenyl)ethanone:** To a mixture of 1-bromo-3,5-difluoro-benzene (1.0 g, 5.18 mmol), (5-diphenylphosphanyl-9,9-dimethyl-xanthen-4-yl)-diphenyl-phosphane (60 mg, 0.11 mmol), Pd₂(dba)₃ (48 mg, 0.05 mmol), and t-BuONa (598 mg, 6.22 mmol) in THF (15 mL) was added 1-cyclobutylethanone (1.02 g, 10.4 mmol). The reaction mixture was stirred at 50 °C for 7 h. The reaction mixture was diluted with water (10 mL) and extracted with EtOAc (3 × 5 mL). The combined organic layers were washed with brine (5 mL), dried over anhydrous Na₂SO₄, filtered, and concentrated under reduced pressure. The crude residue was purified by silica gel column chromatography.

**(Z)-ethyl 4-cyclobutyl-3-(3,5-difluorophenyl)-4-oxobut-2-enoate:** To a mixture of 1-cyclobutyl-2-(3,5-difluorophenyl)ethanone (1.0 g, 4.76 mmol) and ethyl 2-oxoacetate (971 mg, 4.76 mmol) in toluene (10 mL) was added Et₃N (3.64 g, 35.9 mmol). The reaction mixture was stirred at 20 °C for 12 h. The reaction mixture was diluted with water (10 mL) and extracted with EtOAc (3 × 5 mL). The combined organic layers were washed with brine (5 mL), dried over anhydrous Na₂SO₄, filtered, and concentrated under reduced pressure. The crude residue was purified by silica gel column chromatography.

**6-cyclobutyl-5-(3,5-difluorophenyl)pyridazin-3(2*H*)-one:** To a mixture of ethyl (Z)-4-cyclobutyl-3-(3,5-difluorophenyl)-4-oxo-but-2-enoate (900 mg, 3.06 mmol) in EtOH (5.0 mL) was added NH₂NH₂•H₂O (1.56 g, 30.6 mmol). The reaction mixture was stirred at 80 °C for 2 h. The reaction mixture was diluted with water (10 mL) and extracted with EtOAc (3 × 5 mL). The combined organic layers were washed with brine (5 mL), dried over anhydrous Na₂SO₄, filtered, and concentrated under reduced pressure. The crude residue was purified by silica gel column chromatography. LCMS: m/z = 263.0 [M+H]⁺.

**methyl 2-(3-cyclobutyl-4-(3,5-difluorophenyl)-6-oxopyridazin-1(6*H*)-yl)acetate:** To a mixture of 6-cyclobutyl-5-(3,5-difluorophenyl)pyridazin-3(2H)-one (40 mg, 0.15 mmol) and methyl 2-bromoacetate (35 mg, 0.23 mmol) in DMF (1.0 mL) was added Cs₂CO₃ (76 mg, 0.23 mmol). The reaction mixture was stirred at 20 °C for 1 h. The reaction mixture was diluted with water (10 mL) and extracted with EtOAc (3 × 5 mL). The combined organic layers were washed with brine (5 mL), dried over anhydrous Na₂SO₄, filtered, and concentrated under reduced pressure. The crude residue was purified by preparative TLC. LCMS: *m*/*z* = 335.0 [M+H]⁺.

**2-[3-cyclobutyl-4-(3,5-difluorophenyl)-6-oxopyridazin-1-yl]-N-(5-fluoropyrimidin-2-yl)acetamide:** To a mixture of methyl 2-(3-cyclobutyl-4-(3,5-difluorophenyl)-6-oxopyridazin-1(6H)-yl)acetate (40 mg, 0.12 mmol) and 5-fluoropyrimidin-2-amine (41 mg, 0.36 mmol) in DCE (1.0 mL) was added AlMe₃ (2.0 M in toluene, 0.2 mL). The reaction mixture was stirred at 80 °C for 1 h. The reaction mixture was diluted with water (10 mL) and extracted with EtOAc (3 × 5 mL). The combined organic layers were washed with brine (5 mL), dried over anhydrous Na₂SO₄, filtered, and concentrated under reduced pressure. The crude residue was purified by reverse-phase preparative HPLC. LCMS: *m*/*z* = 416.0 [M+H]⁺. ¹H NMR (400 MHz, CDCl₃): δ 9.22 (br s, 1H), 8.52 (s, 2H), 6.92 (br t, *J =* 8.8 Hz, 1H), 6.86-6.78 (m, 3H), 5.43 (br s, 2H), 3.41 (quin, *J* = 8.4 Hz, 1H), 2.37-2.19 (m, 2H), 2.06-1.70 (m, 4H).

### Example 57

### 2-[4-(3,5-difluorophenyl)-3-methoxy-6-oxopyridazin-1-yl]-N-(5-fluoropyrimidin-2-yl)acetamide

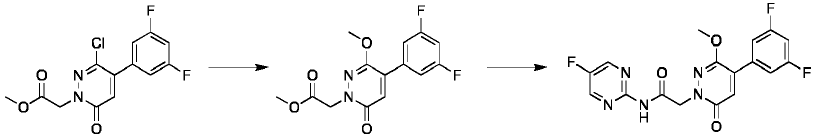

**methyl 2-(4-(3,5-difluorophenyl)-3-methoxy-6-oxopyridazin-1(6*H*)-yl)acetate:** To a mixture of methyl 2-(3-chloro-4-(3,5-difluorophenyl)-6-oxopyridazin-1(6*H*)-yl)acetate (500 mg, 1.59 mmol) in MeOH (10 mL) was added NaOMe (129 mg, 2.38 mmol). The reaction mixture was stirred at 35 °C for 12 h. The reaction mixture was concentrated under reduced pressure. The crude residue was purified by reverse-phase preparative HPLC. LCMS: *m*/*z* = 311.2 [M+H]⁺.

**2-[4-(3,5-difluorophenyl)-3-methoxy-6-oxopyridazin-1-yl]-N-(5-fluoropyrimidin-2-yl)acetamide:** To a mixture of methyl 2-(4-(3,5-difluorophenyl)-3-methoxy-6-oxopyridazin-1(6*H*)-yl)acetate (50 mg, 0.16 mmol) and 5-fluoropyrimidin-2-amine (37 mg, 0.32 mmol) in DCE (1.0 mL) was added trimethyl-(4-trimethylalumanuidyl-1,4-diazoniabicyclo[2.2.2]octan-1-yl)alumanuide (54 mg, 0.21 mmol). The reaction mixture was stirred at 80 °C for 12 h. The reaction mixture was diluted with water (10 mL) and extracted with EtOAc (3 × 5 mL). The combined organic layers were washed with brine (5 mL), dried over anhydrous Na₂SO₄, filtered, and concentrated under reduced pressure. The crude residue was purified by reverse-phase preparative HPLC followed by preparative SFC. LCMS: *m*/*z* = 392.0 [M+H]⁺. ¹H NMR (400 MHz, CDCl₃): δ 9.11 (br s, 1H), 8.50 (s, 2H), 7.16-7.04 (m, 2H), 7.01 (s, 1H), 6.93 (tt, *J* = 2.4, 8.8 Hz, 1H), 5.26 (br s, 2H), 3.89 (s, 3H).

### BIOLOGICAL EXAMPLE 1

### Biochemical Assay of the Compounds

Compounds as provided herein were tested in the following assay. Cell culture medium employed contained RPMI 1640 medium (89%), FBS (10%), Pen/Strep (1%), and 2-mercaptoethanol (0.05 mM). Freezing medium was made up of 90% FBS and 10% DMSO. THP-1 cells were removed from the liquid nitrogen and placed into a 37 °C water bath to thaw, until signs of ice dissipated. The cells were then added to 9 mL of warm cell culture medium and centrifuged for 5 minutes at 1000 rpm. The supernatant was discarded, and the cells were resuspended in new cell culture medium. The THP-1 cells were then split and cultured in the cell culture medium, being passaged every 2-3 days (5x10⁵ cells/mL passaged every two days, 3x10⁵ cells/mL passaged every three days). Cell density was maintained between 5x10⁵-1.5x10⁶ viable cells/mL.

To freeze, cells were resuspended with fresh freezing medium, adjusting the cell density to between 3x10⁶-10x10⁶ cells/mL. The cell suspension was partitioned into 1 mL aliquots per vial, and the vials were transferred to a -80 °C freezer. After one day at -80 °C, the cell vials were transferred to liquid nitrogen freezer for storage.

### Procedure for 96 well format plates

PBST solution was prepared by mixing 3600 mL of water, 400 mL of 10X PBS, and 4 mL of Tween 20. IFN-γ solution was reconstituted with 100 µL of water to obtain a 1 mg/mL solution, which was then diluted with 0.1 % BSA to provide a 100 µg/mL solution. The IFN-γ solution was stored at -20 °C. LPS was reconstituted with 1 mL of PBS solution to obtain a 1 mg/mL stock solution, which was further diluted to 50 ng/mL with serum-free medium. The LPS solution was stored at 4 °C.

### Day 1: THP-1 cells differentiated with IFN-γ

To suspension containing 1.0x10⁶ THP-1 cells/mL in cell culture medium was added IFN-γ (final concentration: 25 ng/mL). 100 µL of this suspension was distributed to each well of the desired 96 well plates. The 96 well plates were then incubated at 37 °C under a humidified atmosphere of 5% CO₂ for 24 h.

### Day 2: LPS induction and IL-1β detection kit

LPS induction: When IFN-γ treatment was completed, the supernatants were discarded by hand. 100 µL of a 1X solution of LPS in serum-free medium (50 ng/mL) was added. The plates were incubated in a 37 °C incubator under a humidified atmosphere of 5% CO₂ for 4 h. Sample compounds were dissolved in DMSO and the compound solutions were dispensed to the wells using a Tecan D300e digital dispenser. The final concentration of DMSO for each well was 0.5%. The plates were then incubated at 37 °C under a humidified atmosphere of 5% CO₂ for 1 h. 20 µL of a 6X solution of ATP was added (final concentration per well: 5 mM), and the plates incubated at 37 °C under a humidified atmosphere of 5% CO₂ for 1 h. The supernatants were then collected and analyzed using an IL-Iβ detection kit. If needed, the test samples could be stored at -20 °C until analyzed.

Compound dilution: Compound solution source plates (20 µL of a 10 mM solution of each compound was delivered to the specified wells and stored in a nitrogen cabinet until tested.

Coat Elisa plate: The captured antibody (mAb Mt175) was diluted in PBS to a concentration of 2 µg/mL, and then used to coat the ELISA plate (SIGMA-P6366) overnight at 4 °C.

### Day 3: IL-1β detection

The antibody coat was discarded and the plate was washed 4 times with PBST. The plate was blocked by adding 25 µL/well of blocking buffer (LiCor-927-40000) with 0.1% Tween 20, then incubated for 1 h at room temperature. The blocking buffer was then discarded and the plate was washed 4 times with PBST.

One hour before test sample addition, the sample plates were allowed to thaw at room temperature, centrifuged at 1000 rpm for 1 minute, and shaken for 30 seconds. 25 µL/well of the test samples were transferred to the ELISA plate, and the plate was incubated for 2 h at room temperature.

The test samples were discarded and the ELISA plate was washed 4 times with PBST. 15 µL/well of mAb7P10-biotin at 0.5 µg/mL (1:1000) in blocking buffer was then added to the ELISA plate and it was incubated for 1 h at room temperature.

The antibody was discarded and the ELISA plate was washed 4 times with PBST. Then 15 µL/well of streptavidin-HRP diluted 1: 1000 in blocking buffer was added to the ELISA plate and it was allowed to incubate for 1 h at room temperature.

The streptavidin-HRP was discarded and the plate was washed 4 times with PBST. 25 µL/well of HRP substrate was then added to the ELISA plate. The plate was incubated 1-2 minutes at room temperature, during which time the solution changed to blue. Then 25 µL/well of the liquid stop solution was added to the plate, and the solution changed to yellow. The plate was then read at 450 nm in a microplate reader. Percent inhibition was calculated as follows: $% inhibition rate = \left(treated samples-high control\right) / \left(low control-high control\right) \times 100$

Activity of the tested compounds is provided in Table 3 below as follows: +++ = IC₅₀ < 10 µM; ++ = IC₅₀ 10-15 µM; + = IC₅₀ > 15 µM.

**Table 3**

| **No.** | **Activity** |
|---|---|
| 1 | +++ |
| 2 | +++ |
| 3 | +++ |
| 4 | +++ |
| 5 | +++ |
| 6 | +++ |
| 7 | +++ |
| 8 | ++ |
| 9 | +++ |
| 10 | ++ |
| 11 | +++ |
| 12 | +++ |
| 13 | +++ |
| 14 | +++ |
| 15 | +++ |
| 16 | +++ |
| 17 | +++ |
| 18 | + |
| 19 | +++ |
| 20 | +++ |

### Procedure for IL-1β secretion assay in 384-well plates

PMA was dissolved in DMSO to make a stock solution at 5 mg/mL and stored in 10 µl aliquots at -20°C for single use. PMA is added to normal growth medium. LPS was diluted with 1 mL of water solution to provide a 1 mg/mL stock solution and stored in 15 µl aliquots at -20 °C for single use. Nigericin is diluted in ice cold 100% ethanol to 5 mg/mL (6.7 mM) and stored in 75 µL aliquots at -20 °C for single use. Serum-free media contains RPMI 1640 medium (99%), Pen/Strep (1%), and 2-mercaptoethanol (0.05 mM). The two control conditions used to qualify and normalize test compound dose-response curves were as follows: High Control = 25 ng/mL LPS, 5 µM Nigericin, 0.5% DMSO, Low Control = 25 ng/mL, LPS, 0.5% DMSO.

### Day 1: Differentiation with PMA

THP-1 cells were diluted to provide a suspension at a concentration of 1.0x10⁶ cells/mL with the total volume of suspension required to enable the desired number of assay plates. The growth media was supplemented with PMA (5 ng/mL final concentration) and the cells were incubated at 37 °C under a humidified atmosphere of 5% CO₂ for 40 h.

### Day 3: Plating with sequential LPS and nigericin stimulation

All media was carefully aspirated from each culture flask. The cells were washed carefully with 1x DPBS. The cells were then briefly digested with trypsin LE for 5 minutes at 23 °C and immediately resuspended in cell growth media. After resuspension, the cells were centrifuged at 1000 rpm for 3 minutes and the supernatant was discarded. The cells were resuspended in DPBS and once again centrifuged at 1000 rpm for 5 minutes. The supernatant was discarded and the cell pellet was resuspended in serum-free media supplemented with LPS (25 ng/mL final concentration) to enable the distribution of 30K THP-1 cells within 45 µL of media into each well of 384-well PDL-coated plates. The 384-well plates were then incubated at 37 °C under a humidified atmosphere of 5% CO₂ for 2 h. Following this period, test compounds were dispensed by Tecan across the desired concentration range with all wells normalized to a final 0.5 % DMSO concentration. The plates were then incubated at 37 °C under a humidified atmosphere of 5% CO₂ for 1 h. Following this period, 5 µL of the 5 mg/mL nigericin stock solution was added to each of the appropriate wells and plates were centrifuged at 1000 rpm for 30 seconds. The plates were the immediately reintroduced to the incubator at 37 °C under a humidified atmosphere of 5% CO₂ for 2 h. After this time, 35 µL/well of supernatant was collected and transferred into v-bottom plate and centrifuged at 1000 rpm for 1 minute. These supernatant aliquots were analyzed using an IL-Iβ detection kit as described below. If needed, the test samples could be snap frozen and stored at -80 °C until analyzed.

### IL-1β detection

To prepare each ELISA plate, capture antibody (mAb Mt175) was diluted with PBS to a final concentration of 2 µg/mL and then 20 µL of this solution was added to each well of the ELISA plate. Each plate was allowed to incubate overnight at 4 °C. The next day, the capture antibody solution was removed and discarded. Each ELISA plate was washed 4 times with PBST followed by the addition of 25 µL/well of blocking buffer (Licor-927-40010) supplemented with 0.1% Tween 20. Each ELISA plate was then allowed to incubate for 1 hour at 23 °C. After this time, the blocking buffer was removed and discarded. Each ELISA plate was washed 4 times with PBST. During this time, the v-bottomed plates containing the supernatant aliquots from the assay run were centrifuged at 300 g for 5 minutes before transferring 15 µL/well of the supernatant sample to each ELISA plate. Each ELISA plate was then allowed to incubate for 2 h at 23 °C. After this time, the supernatant samples were removed and discarded. Each ELISA plate was washed 4 times with PBST. To each ELISA plate was added 15 µL/well of mAb7P10-biotin at 0.5 µg/mL (1: 1000 diluted in blocking buffer). Each ELISA plate was then allowed to incubate for 1 h at 23 °C. After this time, the antibody solution was removed and discarded. Each ELISA plate was washed 4 times with PBST. To each ELISA plate was added 20 µL/well of streptavidin-HRP (1:2000 diluted in blocking buffer). Each ELISA plate was then allowed to incubate for 1 h at 23 °C. After this time, the buffer was removed and discarded. Each ELISA plate was washed 4 times with PBST. To each ELISA plate was added 20 µL/well of HRP substrate. Each ELISA plate was then allowed to incubate for 2 minutes at 23 °C. After this time, to each ELISA plate was added 40 µL/well of stop solution. Each ELISA plate was centrifuged at 1200 rpm for 30 seconds.

The plate was then read at 450 nm in a microplate reader. Percent inhibition was calculated as follows: $% inhibition rate = \left(treated samples-high control\right) / \left(low control-high control\right) \times 100$

Activity of the tested compounds is provided in Table 4 below.

**Table 4**

| **Ex.** | **Activity (µM)** |
|---|---|
| 1 | 3.32 |
| 2 | 1.49 |
| 3 | 6.77 |
| 4 | 5.91 |
| 5 | 0.091 |
| 6 | 0.535 |
| 7 | 7.06 |
| 8 | 24.5 |
| 9 | 2.27 |
| 10 | 50 |
| 11 | 2.88 |
| 12 | 1.66 |
| 13 | 21.8 |
| 14 | 1.49 |
| 15 | 3.51 |
| 16 | 1.15 |
| 17 | 19.5 |
| 18 | 50 |
| 19 | 21.7 |
| 20 | 24.0 |
| 21 | 0.902 |
| 22 | 1.57 |
| 23 | 1.89 |
| 24 | 1.90 |
| 25 | 1.82 |
| 26 | 6.66 |
| 27 | 1.91 |
| 28 | 8.47 |
| 29 | 0.874 |
| 30 | 1.30 |
| 31 | 6.98 |
| 32 | 0.649 |
| 33 | 3.21 |
| 34 | 29.5 |
| 35 | 35.3 |
| 36 | 1.00 |
| 37 | 30.1 |
| 38 | 0.812 |
| 39 | 0.490 |
| 40 | 1.44 |
| 41 | 7.21 |
| 42 | 0.320 |
| 43 | 0.289 |
| 44 | 0.236 |
| 45 | 0.596 |
| 46 | 0.637 |
| 47 | 8.45 |
| 48 | 1.64 |
| 49 | 3.24 |
| 50 | 1.64 |
| 51 | 1.14 |
| 52 | 4.36 |
| 53 | 2.77 |
| 54 | 6.98 |
| 55 | 1.06 |
| 56 | 0.713 |
| 57 | 1.96 |

Unless otherwise defined, all technical and scientific terms used herein have the same meaning as commonly understood by one of ordinary skill in the art to which this disclosure belongs.

The disclosure illustratively described herein may suitably be practiced in the absence of any element or elements, limitation or limitations, not specifically disclosed herein. Thus, for example, the terms "comprising," "including," "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the disclosure.

## Claims

1. A compound of Formula I: 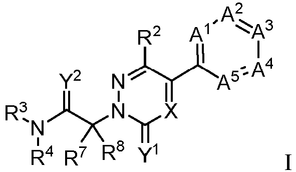 or a pharmaceutically acceptable salt, isotopically enriched analog, stereoisomer, or mixture of stereoisomers thereof, wherein:
each of A¹, A², A³, A⁴, and A⁵ is independently N or CR¹;
X is N or CR⁵;
each of Y¹ and Y² is independently O or S;
each R¹ is independently hydrogen, halo, cyano, hydroxy, -SH, -NH₂, -NO₂, -SF₅, C₁₋₆alkyl, C₂₋₆ alkenyl, C₂₋₆ alkynyl, C₁₋₆ haloalkyl, C₂₋₆ heteroalkyl, C₃₋₁₀ cycloalkyl, heterocyclyl, aryl, heteroaryl, -N(R¹¹)₂, -OR¹¹, -C(O)R¹¹, -C(O)OR¹¹, -S(O)₀₋₂R¹¹, -NR¹¹S(O)₀₋₂-R¹¹, -S(O)₀₋₂N(R¹¹)₂, -NR¹¹S(O)₀₋₂N(R¹¹)₂, -NR¹¹C(O)N(R¹¹)₂, -C(O)N(R¹¹)₂, -NR¹¹C(O)R¹¹, -OC(O)N(R¹¹)₂, or -NR¹¹C(O)OR¹¹; wherein each C₁₋₆alkyl, C₂₋₆alkenyl, C₂₋₆ alkynyl, C₁₋₆ haloalkyl, C₂₋₆ heteroalkyl, C₃₋₁₀ cycloalkyl, heterocyclyl, aryl, or heteroaryl is independently optionally substituted with one to five Z¹; and wherein any two adjacent R¹ groups can join to form a C₃₋₁₀ cycloalkyl, heterocyclyl, aryl, or heteroaryl ring, which C₃₋₁₀ cycloalkyl, heterocyclyl, aryl, or heteroaryl ring may further be independently optionally substituted with one to five Z¹;
R² is -C(R⁶)₂R¹⁰, -OR⁹, -N(R⁶)(R⁹), -SR⁹, -S(O)R⁹, -S(O)₂R⁹, - OC(O)N(R⁶)(R⁹), -NR⁶C(O)OR⁹, -NR⁶C(O)R⁹, C₃₋₁₀ cycloalkyl, heterocyclyl, or halo; wherein the C₃₋₁₀ cycloalkyl or heterocyclyl is independently optionally substituted with one to five Z¹;
R³ is hydrogen, C₁₋₆ alkyl, C₂₋₆ alkenyl, C₂₋₆ alkynyl, C₁₋₆ haloalkyl, C₁₋₆ alkoxy, C₂₋₆ heteroalkyl, C₃₋₁₀ cycloalkyl, heterocyclyl, aryl, or heteroaryl; wherein the C₁₋₆ alkyl, C₂₋₆ alkenyl, C₂₋₆ alkynyl, C₁₋₆ haloalkyl, C₁₋₆ alkoxy, C₂₋₆ heteroalkyl, C₃₋₁₀ cycloalkyl, heterocyclyl, aryl, or heteroaryl is independently optionally substituted with one to five Z¹;
R⁴ is hydrogen, C₁₋₆ alkyl, C₂₋₆ alkenyl, C₂₋₆ alkynyl, C₁₋₆ haloalkyl, C₁₋₆ alkoxy, C₂₋₆ heteroalkyl, C₃₋₁₀ cycloalkyl, heterocyclyl, aryl, or heteroaryl; wherein the C₁₋₆ alkyl, C₂₋₆ alkenyl, C₂₋₆ alkynyl, C₁₋₆ haloalkyl, C₁₋₆ alkoxy, C₂₋₆ heteroalkyl, C₃₋₁₀ cycloalkyl, heterocyclyl, aryl, or heteroaryl is independently optionally substituted with one to five Z¹;
or R³ and R⁴ join to form a heterocyclyl or heteroaryl ring; wherein the heterocyclyl or heteroaryl ring may further be independently optionally substituted with one to five Z¹;
R⁵ is hydrogen, halo, C₁₋₆ alkyl, C₁₋₆ haloalkyl, C₁₋₆ alkoxy, C₁₋₆ haloalkoxy, C₃₋₁₀ cycloalkyl, or heterocyclyl; wherein the C₁₋₆ alkyl, C₁₋₆ haloalkyl, C₁₋₆ alkoxy, C₁₋₆ haloalkoxy, C₃₋₁₀ cycloalkyl, or heterocyclyl is independently optionally substituted with one to five Z¹;
each R⁶ is independently hydrogen, halo, cyano, hydroxy, C₁₋₆ alkyl, C₂₋₆ alkenyl, C₂₋₆ alkynyl, C₁₋₆ haloalkyl, C₁₋₆ alkoxy, C₁₋₆ haloalkoxy, C₂₋₆ heteroalkyl, C₃₋₁₀ cycloalkyl, heterocyclyl, aryl, or heteroaryl;
R⁷ is hydrogen, halo, cyano, hydroxy, C₁₋₆ alkyl, C₂₋₆ alkenyl, C₂₋₆ alkynyl, C₁₋₆ haloalkyl, C₁₋₆ alkoxy, C₁₋₆ haloalkoxy, C₂₋₆ heteroalkyl, C₃₋₁₀ cycloalkyl, or heterocyclyl;
R⁸ is hydrogen, halo, cyano, hydroxy, C₁₋₆ alkyl, C₂₋₆ alkenyl, C₂₋₆ alkynyl, C₁₋₆ haloalkyl, C₁₋₆ alkoxy, C₁₋₆ haloalkoxy, C₂₋₆ heteroalkyl, C₃₋₁₀ cycloalkyl, or heterocyclyl;
or R⁷ and R⁸ join to form a C₃₋₁₀ cycloalkyl or heterocyclyl ring; wherein the C₃₋₁₀ cycloalkyl or heterocyclyl ring may further be independently optionally substituted with one to five Z^{1a};
R⁹ is C₁₋₆ alkyl, C₂₋₆ alkenyl, C₂₋₆ alkynyl, C₁₋₆ haloalkyl, C₂₋₆ heteroalkyl, C₁₋₆ alkoxy, C₁₋₆ haloalkoxy, C₃₋₁₀ cycloalkyl, heterocyclyl, aryl, or heteroaryl; wherein the C₁₋₆ alkyl, C₂₋₆ alkenyl, C₂₋₆ alkynyl, C₁₋₆ haloalkyl, C₂₋₆ heteroalkyl, C₁₋₆ alkoxy, C₁₋₆ haloalkoxy, C₃₋₁₀ cycloalkyl, heterocyclyl, aryl, or heteroaryl is independently optionally substituted with one to five Z¹; or
R¹⁰ is hydrogen, halo, hydroxy, C₁₋₆ alkyl, C₂₋₆ alkenyl, C₂₋₆ alkynyl, C₁₋₆ haloalkyl, C₂₋₆ heteroalkyl, C₃₋₁₀ cycloalkyl, heterocyclyl, aryl, or heteroaryl; wherein the C₁₋₆ alkyl, C₂₋₆ alkenyl, C₂₋₆ alkynyl, C₁₋₆ haloalkyl, C₂₋₆ heteroalkyl, C₃₋₁₀ cycloalkyl, heterocyclyl, aryl, or heteroaryl is independently optionally substituted with one to five Z¹;
each Z¹ is independently halo, cyano, hydroxy, -SH, -NH₂, -NO₂, -SF₅, C₁₋₆ alkyl, C₂₋₆ alkenyl, C₂₋₆ alkynyl, C₁₋₆ haloalkyl, C₂₋₆ heteroalkyl, C₃₋₁₀ cycloalkyl, heterocyclyl, aryl, heteroaryl, -N(R¹¹)₂, -OR¹¹, -C(O)R¹¹, -C(O)OR¹¹, -S(O)₀₋₂R¹¹, -NR¹¹S(O)₀₋₂-R¹¹, -S(O)₀₋₂N(R¹¹)₂, -NR¹¹S(O)₀₋₂N(R¹¹)₂, -NR¹¹C(0)N(R¹¹)₂, -C(O)N(R¹¹)₂, -NR¹¹C(O)R¹¹, -OC(O)N(R¹¹)₂, or -NR¹¹C(O)OR¹¹; wherein each C₁₋₆ alkyl, C₂₋₆ alkenyl, C₂₋₆ alkynyl, C₁₋₆ haloalkyl, C₂₋₆ heteroalkyl, C₃₋₁₀ cycloalkyl, heterocyclyl, aryl, or heteroaryl is independently optionally substituted with one to five Z^{1a};
each R¹¹ is independently hydrogen, C₁₋₆ alkyl, C₂₋₆ alkenyl, C₂₋₆ alkynyl, C₁₋₆ haloalkyl, C₂₋₆ heteroalkyl, C₃₋₁₀ cycloalkyl, heterocyclyl, aryl, or heteroaryl; wherein each C₁₋₆ alkyl, C₂₋₆ alkenyl, C₂₋₆ alkynyl, C₁₋₆ haloalkyl, C₂₋₆ heteroalkyl, C₃₋₁₀ cycloalkyl, heterocyclyl, aryl, or heteroaryl of R¹¹ is independently optionally substituted with one to five Z^{1a};
each Z^{1a} is independently hydroxy, halo, cyano, hydroxy, -SH, -NH₂, -NO₂, -SF₅, C₁₋₆ alkyl, C₂₋₆ alkenyl, C₂₋₆ alkynyl, C₁₋₆ haloalkyl, C₁₋₆ alkoxy, C₁₋₆ haloalkoxy, C₃₋₁₀ cycloalkyl, heterocyclyl, aryl, heteroaryl, -N(R¹²)₂, -OR¹², -C(O)R¹², -C(O)OR¹², -S(O)₀₋₂R¹², -NR¹²S(O)₀₋₂-R¹², -S(O)₀₋₂N(R¹²)₂, -NR¹²S(O)₀₋₂N(R¹²)₂, -NR¹²C(O)N(R¹²)₂, -C(O)N(R¹²)₂, -NR¹²C(O)R¹², -OC(O)N(R¹²)₂, or -NR¹²C(O)OR¹²; wherein each C₁₋₆ alkyl, C₂₋₆ alkenyl, C₂₋₆ alkynyl, C₁₋₆ haloalkyl, C₁₋₆ alkoxy, C₁₋₆ haloalkoxy, C₃₋₁₀ cycloalkyl, heterocyclyl, aryl, or heteroaryl is independently optionally substituted with one to five Z^{1b};
each R¹² is independently hydrogen, C₁₋₆ alkyl, C₂₋₆ alkenyl, C₂₋₆ alkynyl, C₁₋₆ haloalkyl, C₂₋₆ heteroalkyl, C₃₋₁₀ cycloalkyl, heterocyclyl, aryl, or heteroaryl; wherein each C₁₋₆ alkyl, C₂₋₆ alkenyl, C₂₋₆ alkynyl, C₁₋₆ haloalkyl, C₂₋₆ heteroalkyl, C₃₋₁₀ cycloalkyl, heterocyclyl, aryl, or heteroaryl of R¹² is independently optionally substituted with one to five Z^{1b};
each Z^{1b} is independently halo, cyano, hydroxy, -SH, -NH₂, -NO₂, -SF₅, C₁₋₆ alkyl, C₂₋₆ alkenyl, C₂₋₆ alkynyl, C₁₋₆ haloalkyl, C₁₋₆ alkoxy, C₂₋₆ heteroalkyl, C₁₋₆ haloalkoxy, C₃₋₁₀ cycloalkyl, heterocyclyl, aryl, heteroaryl, -L-C₁₋₆ alkyl, -L-C₂₋₆ alkenyl, -L-C₂₋₆ alkynyl, -L-C₁₋₆ haloalkyl, -L-C₃₋₁₀ cycloalkyl, -L-heterocyclyl, -L-aryl, or -L-heteroaryl; and
each L is independently -O-, -NH-, -S-, -S(O)-, -S(O)₂-, -N(C₁₋₆ alkyl)-, -N(C₂₋₆ alkenyl)-, -N(C₂₋₆ alkynyl)-, -N(C₁₋₆ haloalkyl)-, -N(C₃₋₁₀ cycloalkyl)-, -N(heterocyclyl)-, -N(aryl)-, - N(heteroaryl)-, -C(O)-, -C(O)O-, -C(O)NH-, -C(O)N(C₁₋₆ alkyl)-, -C(O)N(C₂₋₆ alkenyl)-, -C(O)N(C₂₋₆ alkynyl)-, -C(O)N(C₁₋₆ haloalkyl)-, -C(O)N(C₃₋₁₀ cycloalkyl)-, -C(O)N(heterocyclyl)-, -C(O)N(aryl)-, -C(O)N(heteroaryl)-, -NHC(O)-, -NHC(O)O-, -NHC(O)NH-, -NHS(O)-, or -S(O)₂NH-;
wherein each C₁₋₆ alkyl, C₂₋₆ alkenyl, C₂₋₆ alkynyl, C₁₋₆ haloalkyl, C₁₋₆ alkoxy, C₁₋₆ haloalkoxy, C₃₋₁₀ cycloalkyl, heterocyclyl, aryl, and heteroaryl of Z^{1b} and L is further independently optionally substituted with one to five hydroxy, halo, cyano, hydroxy, -SH, -NH₂, -NO₂, -SF₅, C₁₋₆ alkyl, C₂₋₆ alkenyl, C₂₋₆ alkynyl, C₁₋₆ haloalkyl, C₁₋₆ alkoxy, C₁₋₆ haloalkoxy, C₃₋₁₀ cycloalkyl, heterocyclyl, aryl, or heteroaryl;
provided the compound is not N-(4-bromophenyl)-2-[3-methyl-6-oxo-4-phenylpyridazin-1(6H)-yl]acetamide, N-(4-bromophenyl)-5-[(3-methoxyphenyl)methyl]-3-methyl-6-oxo-4-phenyl-1(6H)-pyridazineacetamide, 4-[[2-[4-(2-acetyl-5-chlorophenyl)-3-methoxy-6-oxo-1(6H)-pyridazinyl]-4-methyl-1-oxopentyl]amino]-benzoic acid 1,1-dimethylethyl ester, or 4-[[2-[4-(2-acetyl-5-chlorophenyl)-3-methoxy-6-oxo-1(6H)-pyridazinyl]-4-methyl-1-oxopentyl]amino]-benzoic acid.

2. The compound of claim 1, or a pharmaceutically acceptable salt, isotopically enriched analog, stereoisomer, or mixture of stereoisomers thereof, wherein:
(i) each of A¹, A², A³, A⁴, and A⁵ is independently CR¹;
(ii) one of A¹, A², A³, A⁴, and A⁵ is N and the remaining A¹, A², A³, A⁴, and A⁵ are independently CR¹; or
(iii) two of A¹, A², A³, A⁴, and A⁵ are N and the remaining A¹, A², A³, A⁴, and A⁵ are independently CR¹.

3. The compound of claims 1 or 2, or a pharmaceutically acceptable salt, isotopically enriched analog, stereoisomer, or mixture of stereoisomers thereof, wherein X is N.

4. The compound of any one of claims 1 or 2, or a pharmaceutically acceptable salt, isotopically enriched analog, stereoisomer, or mixture of stereoisomers thereof, wherein:
(i) X is CR⁵; and/or
(ii) R⁵ is hydrogen, C₁₋₆ alkyl, or C₁₋₆ haloalkyl.

5. The compound of any one of claims 1-4, or a pharmaceutically acceptable salt, isotopically enriched analog, stereoisomer, or mixture of stereoisomers thereof, wherein:
(i) each R¹ is independently hydrogen, halo, cyano, C₁₋₆ alkyl, C₁₋₆ alkoxy, C₁₋₆ haloalkyl, or C₃₋₁₀ cycloalkyl, or any two adjacent R¹ groups can join to form a aryl or heteroaryl ring;
(ii) each R¹ is independently hydrogen, fluoro, chloro, cyano, -CH₃, -OCH₃, -OCH₂CH₃, - CF₃, cyclopropyl, 2,2-difluorocyclopropyl, or cyclobutyl;
(iii) R² is -C(R⁶)₂R¹⁰, -OR⁹, C₃₋₁₀ cycloalkyl, or halo; wherein the C₃₋₁₀ cycloalkyl is independently optionally substituted with one to five Z¹;
(iv) R³ is C₃₋₁₀ cycloalkyl, heterocyclyl or heteroaryl; wherein the C₃₋₁₀ cycloalkyl, heterocyclyl or heteroaryl is independently optionally substituted with one to five Z¹;
(v) R³ is C₃₋₁₀ cycloalkyl, heterocyclyl, or heteroaryl; wherein the C₃₋₁₀ cycloalkyl, heterocyclyl, or heteroaryl is independently optionally substituted with one to five halo, hydroxy, C₁₋₆ alkyl, or C₃₋₁₀ cycloalkyl;
(vi) R³ is 5-fluoropyrimidin-4-yl, 1-cyclobutylpiperidin-3-yl, 1-ethylpiperidin-3-yl, 1-cyclopropylpiperidin-3-yl, 3-fluoropyridin-2-yl, 5-fluoropyrimidin-2-yl, 3,5-difluoropyridin-2-yl, or 3-hydroxy-3-methylcyclobutyl; and/or
(vii) R⁴ is hydrogen.

6. The compound of any one of claims 1-5, or a pharmaceutically acceptable salt, isotopically enriched analog, stereoisomer, or mixture of stereoisomers thereof, wherein R² is:
(i) -C(R⁶)₂R¹⁰; and at least one R⁶ is hydrogen; and/or
(ii) -C(R⁶)₂R¹⁰; and R¹⁰ is halo, C₁₋₆ alkyl, or C₁₋₆ haloalkyl.

7. The compound of any one of claims 1-5, or a pharmaceutically acceptable salt, isotopically enriched analog, stereoisomer, or mixture of stereoisomers thereof, wherein R² is -OR⁹; and R⁹ is C₁₋₆ alkyl.

8. The compound of any one of claims 1-7, or a pharmaceutically acceptable salt, isotopically enriched analog, stereoisomer, or mixture of stereoisomers thereof, wherein:
(i) R⁷ is hydrogen; and/or
(ii) R⁸ is hydrogen.

9. The compound of claim 1, or a pharmaceutically acceptable salt, isotopically enriched analog, stereoisomer, or mixture of stereoisomers thereof, wherein:
(i) each of A¹, A², A³, A⁴, and A⁵ is independently CR¹;
each R¹ is independently hydrogen, halo, cyano, C₁₋₆ alkyl, C₁₋₆ alkoxy, C₁₋₆ haloalkyl, or C₃₋₁₀ cycloalkyl, or any two adjacent R¹ groups can join to form a aryl or heteroaryl ring;
X is CR⁵;
R² is -C(R⁶)₂R¹⁰, -OR⁹, C₃₋₁₀ cycloalkyl, or halo; wherein the C₃₋₁₀ cycloalkyl is independently optionally substituted with one to five Z¹;
R³ is C₃₋₁₀ cycloalkyl, heterocyclyl, or heteroaryl; wherein the C₃₋₁₀ cycloalkyl, heterocyclyl, or heteroaryl is independently optionally substituted with one to five halo, hydroxy, C₁₋₆ alkyl, or C₃₋₁₀ cycloalkyl;
R⁴ is hydrogen;
R⁵ is hydrogen, C₁₋₆ alkyl, or C₁₋₆ haloalkyl;
at least one R⁶ is hydrogen;
R⁷ and R⁸ are hydrogen, or R⁷ and R⁸ join to form a C₃₋₁₀ cycloalkyl;
R⁹ is C₁₋₆ alkyl; and
R¹⁰ is halo, C₁₋₆ alkyl, or C₁₋₆ haloalkyl;
(i) one of A¹, A², A³, A⁴, and A⁵ is N and the remaining A¹, A², A³, A⁴, and A⁵ are independently CR¹;
each R¹ is independently hydrogen, halo, cyano, C₁₋₆ alkyl, C₁₋₆ alkoxy, C₁₋₆ haloalkyl, or C₃₋₁₀ cycloalkyl, or any two adjacent R¹ groups can join to form a aryl or heteroaryl ring;
X is CR⁵;
R² is -C(R⁶)₂R¹⁰, -OR⁹, C₃₋₁₀ cycloalkyl, or halo; wherein the C₃₋₁₀ cycloalkyl is independently optionally substituted with one to five Z¹;
R³ is C₃₋₁₀ cycloalkyl, heterocyclyl, or heteroaryl; wherein the C₃₋₁₀ cycloalkyl, heterocyclyl, or heteroaryl is independently optionally substituted with one to five halo, hydroxy, C₁₋₆ alkyl, or C₃₋₁₀ cycloalkyl;
R⁴ is hydrogen;
R⁵ is hydrogen, C₁₋₆ alkyl, or C₁₋₆ haloalkyl;
at least one R⁶ is hydrogen;
R⁷ and R⁸ are hydrogen, or R⁷ and R⁸ join to form a C₃₋₁₀ cycloalkyl;
R⁹ is C₁₋₆ alkyl; and
R¹⁰ is halo, C₁₋₆ alkyl, or C₁₋₆ haloalkyl;
(iii) two of A¹, A², A³, A⁴, and A⁵ are N and the remaining A¹, A², A³, A⁴, and A⁵ are independently CR¹;
each R¹ is independently hydrogen, halo, cyano, C₁₋₆ alkyl, C₁₋₆ alkoxy, C₁₋₆ haloalkyl, or C₃₋₁₀ cycloalkyl, or any two adjacent R¹ groups can join to form a aryl or heteroaryl ring;
X is CR⁵;
R² is -C(R⁶)₂R¹⁰, -OR⁹, C₃₋₁₀ cycloalkyl, or halo; wherein the C₃₋₁₀ cycloalkyl is independently optionally substituted with one to five Z¹;
R³ is C₃₋₁₀ cycloalkyl, heterocyclyl, or heteroaryl; wherein the C₃₋₁₀ cycloalkyl, heterocyclyl, or heteroaryl is independently optionally substituted with one to five halo, hydroxy, C₁₋₆ alkyl, or C₃₋₁₀ cycloalkyl;
R⁴ is hydrogen;
R⁵ is hydrogen, C₁₋₆ alkyl, or C₁₋₆ haloalkyl;
at least one R⁶ is hydrogen;
R⁷ and R⁸ are hydrogen;
R⁹ is C₁₋₆ alkyl; and
R¹⁰ is halo, C₁₋₆ alkyl, or C₁₋₆ haloalkyl;
(iv) each of A¹, A², A³, A⁴, and A⁵ is independently CR¹;
each R¹ is independently hydrogen, halo, cyano, C₁₋₆ alkyl, C₁₋₆ alkoxy, C₁₋₆ haloalkyl, or C₃₋₁₀ cycloalkyl, or any two adjacent R¹ groups can join to form a aryl or heteroaryl ring;
X is N;
R² is -C(R⁶)₂R¹⁰, -OR⁹, C₃₋₁₀ cycloalkyl, or halo; wherein the C₃₋₁₀ cycloalkyl is independently optionally substituted with one to five Z¹;
R³ is C₃₋₁₀ cycloalkyl, heterocyclyl, or heteroaryl; wherein the C₃₋₁₀ cycloalkyl, heterocyclyl, or heteroaryl is independently optionally substituted with one to five halo, hydroxy, C₁₋₆ alkyl, or C₃₋₁₀ cycloalkyl;
R⁴ is hydrogen;
at least one R⁶ is hydrogen;
R⁷ and R⁸ are hydrogen, or R⁷ and R⁸ join to form a C₃₋₁₀ cycloalkyl;
R⁹ is C₁₋₆ alkyl; and
R¹⁰ is halo, C₁₋₆ alkyl, or C₁₋₆ haloalkyl;
(v) one of A¹, A², A³, A⁴, and A⁵ is N and the remaining A¹, A², A³, A⁴, and A⁵ are independently CR¹;
each R¹ is independently hydrogen, halo, cyano, C₁₋₆ alkyl, C₁₋₆ alkoxy, C₁₋₆ haloalkyl, or C₃₋₁₀ cycloalkyl, or any two adjacent R¹ groups can join to form a aryl or heteroaryl ring;
X is N;
R² is -C(R⁶)₂R¹⁰, -OR⁹, C₃₋₁₀ cycloalkyl, or halo; wherein the C₃₋₁₀ cycloalkyl is independently optionally substituted with one to five Z¹;
R³ is C₃₋₁₀ cycloalkyl, heterocyclyl, or heteroaryl; wherein the C₃₋₁₀ cycloalkyl, heterocyclyl, or heteroaryl is independently optionally substituted with one to five halo, hydroxy, C₁₋₆ alkyl, or C₃₋₁₀ cycloalkyl;
R⁴ is hydrogen;
at least one R⁶ is hydrogen;
R⁷ and R⁸ are hydrogen, or R⁷ and R⁸ join to form a C₃₋₁₀ cycloalkyl;
R⁹ is C₁₋₆ alkyl; and
R¹⁰ is halo, C₁₋₆ alkyl, or C₁₋₆ haloalkyl; or
(vi) two of A¹, A², A³, A⁴, and A⁵ are N and the remaining A¹, A², A³, A⁴, and A⁵ are independently CR¹;
each R¹ is independently hydrogen, halo, cyano, C₁₋₆ alkyl, C₁₋₆ alkoxy, C₁₋₆ haloalkyl, or C₃₋₁₀ cycloalkyl, or any two adjacent R¹ groups can join to form a aryl or heteroaryl ring;
X is N;
R² is -C(R⁶)₂R¹⁰, -OR⁹, C₃₋₁₀ cycloalkyl, or halo; wherein the C₃₋₁₀ cycloalkyl is independently optionally substituted with one to five Z¹;
R³ is C₃₋₁₀ cycloalkyl, heterocyclyl, or heteroaryl; wherein the C₃₋₁₀ cycloalkyl, heterocyclyl, or heteroaryl is independently optionally substituted with one to five halo, hydroxy, C₁₋₆ alkyl, or C₃₋₁₀ cycloalkyl;
R⁴ is hydrogen;
at least one R⁶ is hydrogen;
R⁷ and R⁸ are hydrogen, or R⁷ and R⁸ join to form a C₃₋₁₀ cycloalkyl;
R⁹ is C₁₋₆ alkyl; and
R¹⁰ is halo, C₁₋₆ alkyl, or C₁₋₆ haloalkyl.

10. A compound or a pharmaceutically acceptable salt, isotopically enriched analog, stereoisomer, or mixture of stereoisomers thereof, selected from:
(i) Table 1; or
(ii) Table 2.

11. A pharmaceutical composition comprising a compound of any preceding claim, or a pharmaceutically acceptable salt, stereoisomer, or mixture of stereoisomers thereof, and a pharmaceutically acceptable carrier.

12. The pharmaceutical composition according to claim 11 for use in a method of treating a disease or condition mediated, at least in part, by NLRP3, the method comprising administering an effective amount of the pharmaceutical composition of claim 11 to a subject in need thereof, optionally wherein the disease or condition is:
(i) Alzheimer disease, atherosclerosis, asthma, allergic airway inflammation, cryopyrin-associated periodic syndromes, gout, inflammatory bowel disease and related disorders, nonalcoholic fatty liver disease (NAFLD), nonalcoholic steatohepatitis (NASH), hypertension, myocardial infarction, multiple sclerosis, experimental autoimmune encephalitis, oxalate-induced nephropathy, hyperinflammation following influenza infection, graft-versus-host disease, stroke, silicosis, type 1 diabetes, obesity-induced inflammation or insulin resistance, rheumatoid arthritis, myelodysplastic syndrome, contact hypersensitivity, joint inflammation triggered by chikungunya virus, or traumatic brain injury;
(ii) nonalcoholic fatty liver disease (NAFLD) or nonalcoholic steatohepatitis (NASH); or
(iii) Alzheimer's disease.

13. The compound of any one of claims 1-10, or a pharmaceutically acceptable salt, stereoisomer, or mixture of stereoisomers thereof, for use in treating a disease or condition mediated, at least in part, by NLRP3, optionally wherein the disease or condition is Alzheimer disease, atherosclerosis, asthma, allergic airway inflammation, cryopyrin-associated periodic syndromes, gout, inflammatory bowel disease and related disorders, nonalcoholic fatty liver disease (NAFLD), nonalcoholic steatohepatitis (NASH), hypertension, myocardial infarction, multiple sclerosis, experimental autoimmune encephalitis, oxalate-induced nephropathy, hyperinflammation following influenza infection, graft-versus-host disease, stroke, silicosis, type 1 diabetes, obesity-induced inflammation or insulin resistance, rheumatoid arthritis, myelodysplastic syndrome, contact hypersensitivity, joint inflammation triggered by chikungunya virus, or traumatic brain injury.

14. The compound of any one of claims 1-10, or a pharmaceutically acceptable salt, stereoisomer, or mixture of stereoisomers thereof, for use in:
(i) therapy;
(ii) treating Alzheimer disease; or
(iii) treating nonalcoholic fatty liver disease (NAFLD) or nonalcoholic steatohepatitis (NASH).

15. The use of a compound of claims 1-10, or a pharmaceutically acceptable salt, stereoisomer, or mixture of stereoisomers thereof, for the manufacture of a medicament for treating a neurodegenerative disease, treating Alzheimer disease, atherosclerosis, asthma, allergic airway inflammation, cryopyrin-associated periodic syndromes, gout, inflammatory bowel disease and related disorders, nonalcoholic fatty liver disease (NAFLD), nonalcoholic steatohepatitis (NASH), hypertension, myocardial infarction, multiple sclerosis, experimental autoimmune encephalitis, oxalate-induced nephropathy, hyperinflammation following influenza infection, graft-versus-host disease, stroke, silicosis, type 1 diabetes, obesity-induced inflammation or insulin resistance, rheumatoid arthritis, myelodysplastic syndrome, contact hypersensitivity, joint inflammation triggered by chikungunya virus, or traumatic brain injury.

## Patentansprüche

1. Verbindung von Formel I: 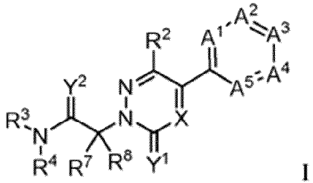
oder ein pharmazeutisch annehmbares Salz, isotopenangereichertes Analogon, Stereoisomer oder Gemisch von Stereoisomeren davon, wobei:
jedes von A¹, A², A³, A⁴ und A⁵ unabhängig N oder CR¹ ist;
X N oder CR⁵ ist;
jedes von Y¹ und Y² unabhängig O oder S ist;
jedes R¹ unabhängig Wasserstoff, Halogen, Cyano, Hydroxy, -SH, -NH₂, -NO₂, -SF₅, C₁₋₆-Alkyl, C₂₋₆-Alkenyl, C₂₋₆-Alkinyl, C₁₋₆-Halogenalkyl, C₂₋₆-Heteroalkyl, C₃₋₁₀-Cycloalkyl, Heterocyclyl, Aryl, Heteroaryl,
-N(R¹¹)₂, -OR¹¹, -C(O)R¹¹, -C(O)OR¹¹, -S(O)₀₋₂R¹¹, -NR¹¹S(O)₀₋₂-R¹¹, -S(O)₀₋₂N(R¹¹)₂,
-NR¹¹S(O)₀₋₂N(R¹¹)₂, -NR¹¹C(O)N(R¹¹)₂, -NR¹¹C(O)R¹¹, -OC(O)N(R¹¹)₂ oder
-NR¹¹C(O)OR¹¹ ist; wobei jedes C₁₋₆-Alkyl, C₂₋₆-Alkenyl, C₂₋₆-Alkinyl, C₁₋₆-Halogenalkyl, C₂₋₆-Heteroalkyl, C₃₋₁₀-Cycloalkyl, Heterocyclyl, Aryl oder Heteroaryl unabhängig optional mit einem bis fünf Z¹ substituiert ist; und wobei sich je zwei benachbarte R¹-Gruppen zu einem C₃₋₁₀-Cycloalkyl-, Heterocyclyl-, Aryl- oder Heteroarylring verbinden können, der C₃₋₁₀-Cycloalkyl-, Heterocyclyl-, Aryl- oder Heteroarylring ferner unabhängig optional mit einem bis fünf Z¹ substituiert werden kann;
R² -C(R⁶)₂R¹⁰, -OR⁹, -N(R⁶)(R⁹), -SR⁹, -S(O)R⁹, -S(O)₂R⁹, - OC(O)N(R⁶)(R⁹), -NR⁶C(O)OR⁹,
-NR⁶C(O)R⁹, C₃₋₁₀-Cycloalkyl, Heterocyclyl oder Halogen ist; wobei das C₃₋₁₀-Cycloalkyl oder Heterocyclyl unabhängig mit einem bis fünf Z¹ substituiert ist;
R³ Wasserstoff, C₁₋₆-Alkyl, C₂₋₆-Alkenyl, C₂₋₆-Alkinyl, C₁₋₆-Halogenalkyl, C₁₋₆-Alkoxy, C₂₋₆-Heteroalkyl, C₃₋₁₀-Cycloalkyl, Heterocyclyl, Aryl oder Heteroaryl ist; wobei das C₁₋₆-Alkyl, C₂₋₆-Alkenyl, C₂₋₆-Alkinyl,
C₁₋₆-Halogenalkyl, C₁₋₆-Alkoxy, C₂₋₆-Heteroalkyl, C₃₋₁₀-Cycloalkyl, Heterocyclyl, Aryl oder Heteroaryl unabhängig optional mit einem bis fünf Z¹ substituiert ist;
R⁴ Wasserstoff, C₁₋₆-Alkyl, C₂₋₆-Alkenyl, C₂₋₆-Alkinyl, C₁₋₆-Halogenalkyl, C₁₋₆-Alkoxy, C₂₋₆-Heteroalkyl, C₃₋₁₀-Cycloalkyl, Heterocyclyl, Aryl oder Heteroaryl ist; wobei das C₁₋₆-Alkyl, C₂₋₆-Alkenyl, C₂₋₆-Alkinyl,
C₁₋₆-Halogenalkyl, C₁₋₆-Alkoxy, C₂₋₆-Heteroalkyl, C₃₋₁₀-Cycloalkyl, Heterocyclyl, Aryl oder Heteroaryl unabhängig optional mit einem bis fünf Z¹ substituiert ist;
oder R³ und R⁴ sich zu einem Heterocyclyl- oder Heteroarylring verbinden; wobei der Heterocyclyl- oder Heteroarylring ferner unabhängig optional mit einem bis fünf Z¹ substituiert sein kann,
R⁵ Wasserstoff, Halogen, C₁₋₆-Alkyl, C₁₋₆-Halogenalkyl, C₁₋₆-Alkoxy, C₁₋₆-Halogenalkoxy, C₃₋₁₀-Cycloalkyl oder Heterocyclyl ist; wobei das C₁₋₆-Alkyl, C₁₋₆-Halogenalkyl, C₁₋₆-Alkoxy, C₁₋₆-Halogenalkoxy, C₃₋₁₀-Cycloalkyl oder Heterocyclyl unabhängig optional mit einem bis fünf Z¹ substituiert ist;
jedes R⁶ unabhängig Wasserstoff, Halogen, Cyano, Hydroxy, C₁₋₆-Alkyl, C₂₋₆-Alkenyl, C₂₋₆-Alkinyl, C₁₋₆-Halogenalkyl, C₁₋₆-Alkoxy, C₁₋₆-Halogenalkoxy, C₂₋₆-Heteroalkyl, C₃₋₁₀-Cycloalkyl, Heterocyclyl, Aryl oder Heteroaryl ist;
R⁷ Wasserstoff, Halogen, Cyano, Hydroxy, C₁₋₆-Alkyl, C₂₋₆-Alkenyl, C₂₋₆-Alkinyl, C₁₋₆-Halogenalkyl, C₁₋₆-Alkoxy, C₁₋₆-Halogenalkoxy, C₂₋₆-Heteroalkyl, C₃₋₁₀-Cycloalkyl oder Heterocyclyl ist;
R⁸ Wasserstoff, Halogen, Cyano, Hydroxy, C₁₋₆-Alkyl, C₂₋₆-Alkenyl, C₂₋₆-Alkinyl, C₁₋₆-Halogenalkyl, C₁₋₆-Alkoxy, C₁₋₆-Halogenalkoxy, C₂₋₆-Heteroalkyl, C₃₋₁₀-Cycloalkyl oder Heterocyclyl ist;
oder R⁷ und R⁸ sich zu einem C₃₋₁₀-Cycloalkyl- oder Heterocyclylring verbinden; wobei der C₃₋₁₀-Cycloalkyl- oder Heterocyclylring optional ferner unabhängig mit einem bis fünf Z^{1a} substituiert sein kann;
R⁹ C₁₋₆-Alkyl, C₂₋₆-Alkenyl, C₂₋₆-Alkinyl, C₁₋₆-Halogenalkyl, C₂₋₆-Heteroalkyl, C₁₋₆-Alkoxy, C₁₋₆-Halogenalkoxy, C₃₋₁₀-Cycloalkyl, Heterocyclyl, Aryl oder Heteroaryl ist; wobei das C₁₋₆-Alkyl, C₂₋₆-Alkenyl, C₂₋₆-Alkinyl, C₁₋₆-Halogenalkyl, C₂₋₆-Heteroalkyl, C₁₋₆-Alkoxy, C₁₋₆-Halogenalkoxy, C₃₋₁₀-Cycloalkyl, Heterocyclyl, Aryl oder Heteroaryl unabhängig optional mit einem bis fünf Z¹ substituiert ist; oder
R¹⁰ Wasserstoff, Halogen, Hydroxy, C₁₋₆-Alkyl, C₂₋₆-Alkenyl, C₂₋₆-Alkinyl, C₁₋₆-Halogenalkyl, C₂₋₆-Heteroalkyl, C₃₋₁₀-Cycloalkyl, Heterocyclyl, Aryl oder Heteroaryl ist; wobei das C₁₋₆-Alkyl-, C₂₋₆-Alkenyl-, C₂₋₆-Alkinyl-, C₁₋₆-Halogenalkyl-, C₂₋₆-Heteroalkyl-, C₃₋₁₀ - Cycloalkyl, -Heterocyclyl, -Aryl oder -Heteroaryl unabhängig optional mit einem bis fünf Z¹ substituiert ist;
jedes Z¹ unabhängig Halogen, Cyano, Hydroxy, -SH, -NH₂, -NO₂, -SF₅, C₁₋₆-Alkyl, C₂₋₆-Alkenyl, C₂₋₆-Alkinyl, C₁₋₆-Halogenalkyl, C₂₋₆-Heteroalkyl, C₃₋₁₀-Cycloalkyl, Heterocyclyl, Aryl, Heteroaryl, -N(R¹¹)₂, -OR¹¹, -C(O)R¹¹, -C(O)OR¹¹, -S(O)_{0.2}R¹¹, -NR¹¹S(O)_{0.2}-R¹¹, -S(O)_{0.2}N(R¹¹)₂, - NR¹¹S(O)_{0.2}N(R¹¹)₂, -NR¹¹C(O)N(R¹¹)₂, -C(O)N(R¹¹)₂, -NR¹¹C(O)R¹¹, -OC(O)N(R¹¹)₂, oder - NR¹¹C(O)OR¹¹; ist; wobei jedes C₁₋₆-Alkyl, C₂₋₆-Alkenyl, C₂₋₆-Alkinyl, C₁₋₆-Halogenalkyl, C₂₋₆-Heteroalkyl, C₃₋₁₀-Cycloalkyl, Heterocyclyl, Aryl oder Heteroaryl unabhängig optional mit einem bis fünf Z^{1a} substituiert ist;
jedes R¹¹ unabhängig Wasserstoff, C₁₋₆-Alkyl, C₂₋₆-Alkenyl, C₂₋₆-Alkinyl, C₁₋₆-Halogenalkyl, C₂₋₆-Heteroalkyl, C₃₋₁₀-Cycloalkyl, Heterocyclyl, Aryl oder Heteroaryl ist; wobei jedes C₁₋₆-Alkyl, C₂₋₆-Alkenyl,
C₂₋₆-Alkinyl, C₁₋₆-Halogenalkyl, C₂₋₆-Heteroalkyl, C₃₋₁₀-Cycloalkyl, Heterocyclyl, Aryl oder Heteroaryl von R¹¹ unabhängig optional mit einem bis fünf Z^{1a} substituiert ist;
jedes Z^{1a} unabhängig Hydroxy, Halogen, Cyano, Hydroxy, -SH, -NH₂, -NO₂, -SF₅, C₁₋₆-Alkyl, C₂₋₆-Alkenyl, C₂₋₆-Alkinyl, C₁₋₆-Halogenalkyl, C₁₋₆-Alkoxy, C₁₋₆-Halogenalkoxy, C₃₋₁₀-Cycloalkyl, Heterocyclyl, Aryl, Heteroaryl, -N(R¹²)₂, -OR¹², -C(O)R¹², -C(O)OR¹², -S(O)_{0.2}R¹², - NR¹²S(O)_{0.2}-R¹², -S(O)_{0.2}N(R¹²)₂, -NR¹²S(O)_{0.2}N(R¹²)₂, -NR¹²C(O)N(R¹²)₂, -C(O)N(R¹²)₂, - NR¹²C(O)R¹², -OC(O)N(R¹²)₂,oder
-NR¹²C(O)OR¹² ist; wobei jedes C₁₋₆-Alkyl, C₂₋₆-Alkenyl, C₂₋₆-Alkinyl, C₁₋₆-Halogenalkyl, C₁₋₆-Alkoxy, C₁₋₆-Halogenalkoxy, C₃₋₁₀-Cycloalkyl, Heterocyclyl, Aryl oder Heteroaryl unabhängig optional mit einem bis fünf Z^{1b} substituiert ist;
jedes R¹² unabhängig Wasserstoff, C₁₋₆-Alkyl, C₂₋₆-Alkenyl, C₂₋₆-Alkinyl, C₁₋₆-Halogenalkyl, C₂₋₆-Heteroalkyl, C₃₋₁₀-Cycloalkyl, Heterocyclyl, Aryl oder Heteroaryl ist; wobei jedes C₁₋₆-Alkyl, C₂₋₆-Alkenyl,
C₂₋₆-Alkinyl, C₁₋₆-Halogenalkyl, C₂₋₆-Heteroalkyl, C₃₋₁₀-Cycloalkyl, Heterocyclyl, Aryl oder Heteroaryl von R¹² unabhängig optional mit einem bis fünf Z^{1b} substituiert ist;
jedes Z^{1b} unabhängig Halogen, Cyano, Hydroxy, -SH, -NH₂, -NO₂, -SF₅, C₁₋₆-Alkyl, C₂₋₆-Alkenyl, C₂₋₆-Alkinyl, C₁₋₆-Halogenalkyl, C₁₋₆-Alkoxy, C₂₋₆-Heteroalkyl, C₁₋₆-Halogenalkoxy, C₃₋₁₀-Cycloalkyl, Heterocyclyl, Aryl, Heteroaryl, -L - C₁₋₆ Alkyl, -L - C₂₋₆ Alkenyl, -L - C₂₋₆ Alkinyl, -L - C₁₋₆ Halogenalkyl, -L - C₃₋₁₀ Cycloalkyl,
-L-Heterocyclyl, -L-Aryl oder -L-Heteroaryl ist; und
jedes L unabhängig -O-, -NH-, -S-, -S(O)-, -S(O)₂-, -N(C₁₋₆-Alkyl)-, -N(C₂₋₆-Alkenyl)-,-N(C₂₋₆-Alkinyl, )-, -N(C₁₋₆-Halogenalkyl)-, -N(C₃₋₁₀-Cycloalkyl)-, -N(Heterocyclyl)-, -N(Aryl)-, - N(Heteroaryl)-, -C(O)-, -C(O)O-, -C(O)NH-, -C(O)N(C₁₋₆-Alkyl)-, -C(O)N(C₂₋₆-Alkenyl)-, -C(O)N(C₂₋₆-Alkinyl)-,
-C(O)N(C₁₋₆-Halogenalkyl )-, -C(O)N(C₃₋₁₀-Cycloalkyl)-, -C(O)N(Heterocyclyl)-, - C(O)N(Aryl)-, -C(O)N(-Heteroaryl)-, -NHC(O)-, -NHC(O)O-, -NHC(O)NH-, -NHS(O)- oder -S(O)₂NH - ist;
wobei jedes C₁₋₆-Alkyl, C₂₋₆-Alkenyl, C₂₋₆-Alkinyl, C₁₋₆-Halogenalkyl, C₁₋₆-Alkoxy, C₁₋₆-Halogenalkoxy, C₃₋₁₀-Cycloalkyl, -Heterocyclyl, -Aryl und -Heteroaryl von Z^{1b} und L ferner unabhängig optional mit einem bis fünf Hydroxy, Halogen, Cyano, Hydroxy, -SH, -NH₂, -NO₂, -SF₅, C₁₋₆-Alkyl, C₂₋₆-Alkenyl, C₂₋₆-Alkinyl, C₁₋₆-Halogenalkyl, C₁₋₆-Alkoxy, C₁₋₆-Halogenalkoxy, C₃₋₁₀-Cycloalkyl, Heterocyclyl, Aryl oder Heteroaryl substituiert ist;
vorausgesetzt, dass die Verbindung nicht N-(4-Bromophenyl)-2-[3-methyl-6-oxo-4-phenylpyridazin-1(6H)-yl]acetamid, N-(4-Bromophenyl)-5-[(3-methoxyphenyl)methyl]-3-methyl-6-oxo-4-phenyl-1(6H)-pyridazinacetamid, 4-[[2-[4-(2-Acetyl-5-chlorophenyl)-3-methoxy-6-oxo-1(6H)-pyridazinyl]-4-methyl-1-oxopentyl]amino]-benzoesäure-1,1-dimethylethylester oder 4-[[2-[4-(2-Acetyl-5-chlorophenyl)-3-methoxy-6-oxo-1(6H)-pyridazinyl]-4-methyl-1-oxopentyl]amino]-benzoesäure ist.

2. Verbindung nach Anspruch 1 oder ein pharmazeutisch annehmbares Salz, isotopenangereichertes Analogon, Stereoisomer oder Gemisch von Stereoisomeren davon, wobei:
(i) jedes von A¹, A², A³, A⁴ und A⁵ unabhängig CR¹ ist;
(ii) eines von A¹, A², A³, A⁴ und A⁵ N ist und die verbleibenden A¹, A², A³, A⁴ und A⁵ unabhängig CR¹ sind; oder
(iii) zwei von A¹, A², A³, A⁴ und A⁵ N sind und die verbleibenden A¹, A², A³, A⁴ und A⁵ unabhängig CR¹ sind.

3. Verbindung nach Anspruch 1 oder 2 oder ein pharmazeutisch annehmbares Salz, isotopenangereichertes Analogon, Stereoisomer oder Gemisch von Stereoisomeren davon, wobei X N ist.

4. Verbindung nach einem der Ansprüche 1 oder 2 oder ein pharmazeutisch annehmbares Salz, isotopenangereichertes Analogon, Stereoisomer oder Gemisch von Stereoisomeren davon, wobei:
(i) X CR⁵ ist; und/oder
(ii) R⁵ Wasserstoff, C₁₋₆-Alkyl oder C₁₋₆-Halogenalkyl ist.

5. Verbindung nach einem der Ansprüche 1-4 oder ein pharmazeutisch annehmbares Salz, isotopenangereichertes Analogon, Stereoisomer oder Gemisch von Stereoisomeren davon, wobei:
(i) jedes R¹ unabhängig Wasserstoff-, Halogen, Cyano, C₁₋₆-Alkyl, C₁₋₆-Alkoxy, C₁₋₆-Halogenalkyl-oder C₃₋₁₀-Cycloalkyl ist oder zwei beliebige benachbarte R¹-Gruppen sich zu einem Aryl- oder Heteroarylring verbinden können;
(ii) jedes R¹ unabhängig Wasserstoff, Fluor, Chlor, Cyano, -CH₃, -OCH₃, -OCH₂CH₃, -CF₃, Cyclopropyl, 2,2-Difluorcyclopropyl oder Cyclobutyl ist;
(iii) R² -C(R⁶)₂R¹⁰, -OR⁹, C₃₋₁₀-Cycloalkyl oder Halogen ist; wobei das C₃₋₁₀-Cycloalkyl unabhängig optional mit einem bis fünf Z¹ substituiert ist;
(iv) R³ C₃₋₁₀-Cycloalkyl, Heterocyclyl oder Heteroaryl ist; wobei das C₃₋₁₀-Cycloalkyl, Heterocyclyl oder Heteroaryl unabhängig optional mit einem bis fünf Z¹ substituiert ist;
(v) R³ C₃₋₁₀-Cycloalkyl, Heterocyclyl oder Heteroaryl ist; wobei das C₃₋₁₀-Cycloalkyl, Heterocyclyl oder Heteroaryl unabhängig optional mit einem bis fünf Halogen, Hydroxy, C₁₋₆-Alkyl- oder C₃₋₁₀-Cycloalkyl substituiert ist;
(vi) R³ 5-Fluorpyrimidin-4-yl, 1-Cyclobutylpiperidin-3-yl, 1-Ethylpiperidin-3-yl, 1-Cyclopropylpiperidin-3-yl, 3-Fluorpyridin-2-yl, 5-Fluorpyrimidin-2-yl, 3,5-Difluorpyridin-2-yl oder 3-Hydroxy-3-methylcyclobutyl ist; und/oder
(vii) R⁴ Wasserstoff ist.

6. Verbindung nach einem der Ansprüche 1-5 oder ein pharmazeutisch annehmbares Salz, isotopenangereichertes Analogon, Stereoisomer oder Gemisch von Stereoisomeren davon, wobei R² ist:
(i) -C(R⁶)₂R¹⁰; und mindestens ein R⁶ Wasserstoff ist; und/oder
(ii) -C(R⁶)₂R¹⁰; und R¹⁰ Halogen, C₁₋₆-Alkyl oder C₁₋₆-Halogenalkyl ist.

7. Verbindung nach einem der Ansprüche 1 bis 5 oder ein pharmazeutisch annehmbares Salz, isotopenangereichertes Analogon, Stereoisomer oder Gemisch von Stereoisomeren davon, wobei R² -OR⁹ ist; und R⁹ C₁₋₆-Alkyl ist.

8. Verbindung nach einem der Ansprüche 1-7 oder ein pharmazeutisch annehmbares Salz, isotopenangereichertes Analogon, Stereoisomer oder Gemisch von Stereoisomeren davon, wobei:
(i) R⁷ Wasserstoff ist; und/oder
(ii) R⁸ Wasserstoff ist.

9. Verbindung nach Anspruch 1 oder ein pharmazeutisch annehmbares Salz, isotopenangereichertes Analogon, Stereoisomer oder Gemisch von Stereoisomeren davon, wobei:
(i) jedes von A¹, A², A³, A⁴ und A⁵ unabhängig CR¹ ist;
jedes R¹ unabhängig Wasserstoff, Halogen, Cyano, C₁₋₆-Alkyl, C₁₋₆-Alkoxy, C₁₋₆-Halogenalkyl oder C₃₋₁₀-Cycloalkyl ist oder zwei beliebige benachbarte R¹-Gruppen sich zu einem Aryl- oder Heteroarylring verbinden können;
X CR⁵ ist;
R² -C(R⁶)₂R¹⁰, -OR⁹, C₃₋₁₀-Cycloalkyl oder Halogen ist; wobei das C₃₋₁₀-Cycloalkyl unabhängig optional mit einem bis fünf Z¹ substituiert ist;
R³ C₃₋₁₀-Cycloalkyl, Heterocyclyl oder Heteroaryl ist; wobei das C₃₋₁₀-Cycloalkyl, Heterocyclyl oder Heteroaryl unabhängig optional mit einem bis fünf Halogen, Hydroxy, C₁₋₆-Alkyl- oder C₃₋₁₀-Cycloalkyl substituiert ist;
R⁴ Wasserstoff ist;
R⁵ Wasserstoff, C₁₋₆-Alkyl oder C₁₋₆-Haloalkyl ist;
mindestens ein R⁶ Wasserstoff ist;
R⁷ und R⁸ Wasserstoff sind oder R⁷ und R⁸ sich zu einem C₃₋₁₀-Cycloalkyl verbinden;
R⁹ C₁₋₆-Alkyl ist; und
R¹⁰ Halogen, C₁₋₆-Alkyl oder C₁₋₆-Halogenalkyl ist;
(i) eines von A¹, A², A³, A⁴ und A⁵ N ist und die verbleibenden A¹, A², A³, A⁴ und A⁵ unabhängig CR¹ sind;
jedes R¹ unabhängig Wasserstoff, Halogen, Cyano, C₁₋₆-Alkyl, C₁₋₆-Alkoxy, C₁₋₆-Halogenalkyl oder C₃₋₁₀-Cycloalkyl ist oder zwei beliebige benachbarte R¹-Gruppen sich zu einem Aryl- oder Heteroarylring verbinden können;
X CR⁵ ist;
R² -C(R⁶)₂R¹⁰, -OR⁹, C₃₋₁₀-Cycloalkyl oder Halogen ist; wobei das C₃₋₁₀-Cycloalkyl unabhängig optional mit einem bis fünf Z¹ substituiert ist;
R³ C₃₋₁₀-Cycloalkyl, Heterocyclyl oder Heteroaryl ist; wobei das C₃₋₁₀-Cycloalkyl, Heterocyclyl oder Heteroaryl unabhängig optional mit einem bis fünf Halogen, Hydroxy, C₁₋₆-Alkyl- oder C₃₋₁₀-Cycloalkyl substituiert ist;
R⁴ Wasserstoff ist;
R⁵ Wasserstoff, C₁₋₆-Alkyl oder C₁₋₆-Haloalkyl ist;
mindestens ein R⁶ Wasserstoff ist;
R⁷ und R⁸ Wasserstoff sind oder R⁷ und R⁸ sich zu einem C₃₋₁₀-Cycloalkyl verbinden;
R⁹ C₁₋₆-Alkyl ist; und
R¹⁰ Halogen, C₁₋₆-Alkyl oder C₁₋₆-Halogenalkyl ist;
(iii) zwei von A¹, A², A³, A⁴, und A⁵ N sind und die verbleibenden A¹, A², A³, A⁴ und A⁵ unabhängig CR¹ sind;
jedes R¹ unabhängig Wasserstoff, Halogen, Cyano, C₁₋₆-Alkyl, C₁₋₆-Alkoxy, C₁₋₆-Halogenalkyl oder C₃₋₁₀-Cycloalkyl ist oder zwei beliebige benachbarte R¹-Gruppen sich zu einem Aryl- oder Heteroarylring verbinden können;
X CR⁵ ist;
R² -C(R⁶)₂R¹⁰, -OR⁹, C₃₋₁₀-Cycloalkyl oder Halogen ist; wobei das C₃₋₁₀-Cycloalkyl unabhängig optional mit einem bis fünf *Z*¹ substituiert ist;
R³ C₃₋₁₀-Cycloalkyl, Heterocyclyl oder Heteroaryl ist; wobei das C₃₋₁₀-Cycloalkyl, Heterocyclyl oder Heteroaryl unabhängig optional mit einem bis fünf Halogen, Hydroxy, C₁₋₆-Alkyl- oder C₃₋₁₀-Cycloalkyl substituiert ist;
R⁴ Wasserstoff ist;
R⁵ Wasserstoff, C₁₋₆-Alkyl oder C₁₋₆-Haloalkyl ist;
mindestens ein R⁶ Wasserstoff ist;
R⁷ und R⁸ Wasserstoff sind;
R⁹ C₁₋₆-Alkyl ist; und
R¹⁰ Halogen, C₁₋₆-Alkyl oder C₁₋₆-Halogenalkyl ist;
(iv) jedes von A¹, A², A³, A⁴ und A⁵ unabhängig CR¹ ist;
jedes R¹ unabhängig Wasserstoff, Halogen, Cyano, C₁₋₆-Alkyl, C₁₋₆-Alkoxy, C₁₋₆-Halogenalkyl oder C₃₋₁₀-Cycloalkyl ist oder zwei beliebige benachbarte R¹-Gruppen sich zu einem Aryl- oder Heteroarylring verbinden können;
X N ist;
R² -C(R⁶)₂R¹⁰, -OR⁹, C₃₋₁₀-Cycloalkyl oder Halogen ist; wobei das C₃₋₁₀-Cycloalkyl unabhängig optional mit einem bis fünf *Z*¹ substituiert ist;
R³ C₃₋₁₀-Cycloalkyl, Heterocyclyl oder Heteroaryl ist; wobei das C₃₋₁₀-Cycloalkyl, Heterocyclyl oder Heteroaryl unabhängig optional mit einem bis fünf Halogen, Hydroxy, C₁₋₆-Alkyl- oder C₃₋₁₀-Cycloalkyl substituiert ist;
R⁴ Wasserstoff ist;
mindestens ein R⁶ Wasserstoff ist;
R⁷ und R⁸ Wasserstoff sind oder R⁷ und R⁸ sich zu einem C₃₋₁₀-Cycloalkyl verbinden;
R⁹ C₁₋₆-Alkyl ist; und
R¹⁰ Halogen, C₁₋₆-Alkyl oder C₁₋₆-Halogenalkyl ist;
(v) eines von A¹, A², A³, A⁴ und A⁵ N ist und die verbleibenden A¹, A², A³, A⁴ und A⁵ unabhängig CR¹ sind;
jedes R¹ unabhängig Wasserstoff, Halogen, Cyano, C₁₋₆-Alkyl, C₁₋₆-Alkoxy, C₁₋₆-Halogenalkyl oder C₃₋₁₀-Cycloalkyl ist oder zwei beliebige benachbarte R¹-Gruppen sich zu einem Aryl- oder Heteroarylring verbinden können;
X N ist;
R² -C(R⁶)₂R¹⁰, -OR⁹, C₃₋₁₀-Cycloalkyl oder Halogen ist; wobei das C₃₋₁₀-Cycloalkyl unabhängig optional mit einem bis fünf *Z*¹ substituiert ist;
R³ C₃₋₁₀-Cycloalkyl, Heterocyclyl oder Heteroaryl ist; wobei das C₃₋₁₀-Cycloalkyl, Heterocyclyl oder Heteroaryl unabhängig optional mit einem bis fünf Halogen, Hydroxy, C₁₋₆-Alkyl- oder C₃₋₁₀-Cycloalkyl substituiert ist;
R⁴ Wasserstoff ist;
mindestens ein R⁶ Wasserstoff ist;
R⁷ und R⁸ Wasserstoff sind oder R⁷ und R⁸ sich zu einem C₃₋₁₀-Cycloalkyl verbinden;
R⁹ C₁₋₆-Alkyl ist; und
R¹⁰ Halogen, C₁₋₆-Alkyl oder C₁₋₆-Halogenalkyl ist; oder
(vi) zwei von A¹, A², A³, A⁴ und A⁵ N sind und die verbleibenden A¹, A², A³, A⁴ und A⁵ unabhängig CR¹ sind;
jedes R¹ unabhängig Wasserstoff, Halogen, Cyano, C₁₋₆-Alkyl, C₁₋₆-Alkoxy, C₁₋₆-Halogenalkyl oder C₃₋₁₀-Cycloalkyl ist oder zwei beliebige benachbarte R¹-Gruppen sich zu einem Aryl- oder Heteroarylring verbinden können;
X N ist;
R² -C(R⁶)₂R¹⁰, -OR⁹, C₃₋₁₀-Cycloalkyl oder Halogen ist; wobei das C₃₋₁₀-Cycloalkyl unabhängig optional mit einem bis fünf *Z*¹ substituiert ist;
R³ C₃₋₁₀-Cycloalkyl, Heterocyclyl oder Heteroaryl ist; wobei das C₃₋₁₀-Cycloalkyl, Heterocyclyl oder Heteroaryl unabhängig optional mit einem bis fünf Halogen, Hydroxy, C₁₋₆-Alkyl- oder C₃₋₁₀-Cycloalkyl substituiert ist;
R⁴ Wasserstoff ist;
mindestens ein R⁶ Wasserstoff ist;
R⁷ und R⁸ Wasserstoff sind oder R⁷ und R⁸ sich zu einem C₃₋₁₀-Cycloalkyl verbinden;
R⁹ C₁₋₆-Alkyl ist; und
R¹⁰ Halogen, C₁₋₆-Alkyl oder C₁₋₆-Halogenalkyl ist.

10. Verbindung oder ein pharmazeutisch annehmbares Salz, isotopenangereichertes Analogon, Stereoisomer oder Gemisch von Stereoisomeren davon, ausgewählt aus:
(i) Tabelle 1; oder
(ii) Tabelle 2.

11. Pharmazeutische Zusammensetzung, umfassend eine Verbindung nach einem vorstehenden Anspruch oder ein pharmazeutisch annehmbares Salz, Stereoisomer oder Gemisch von Stereoisomeren davon, und einen pharmazeutisch annehmbaren Träger.

12. Pharmazeutische Zusammensetzung nach Anspruch 11 zur Verwendung in einem Verfahren zur Behandlung einer Krankheit oder eines Zustands, die mindestens teilweise durch NLRP3 vermittelt werden, wobei das Verfahren Verabreichen einer wirksamen Menge der pharmazeutischen Zusammensetzung nach Anspruch 11 an einen Patienten umfasst, der diese benötigt, optional wobei die Krankheit oder der Zustand ist:
(i) Alzheimer-Krankheit, Arteriosklerose, Asthma, allergische Atemwegsentzündung, Cryopyrin-assoziierte periodische Syndrome, Gicht, entzündliche Darmerkrankungen und verwandte Störungen, nichtalkoholische Fettlebererkrankung (NAFLD), nichtalkoholische Steatohepatitis (NASH), Bluthochdruck, Myokardinfarkt, Multiple Sklerose, experimentelle Autoimmunenzephalitis, Oxalat-induzierte Nephropathie, Hyperinflammation nach Influenza-Infektion, Graft-versus-Host-Reaktion, Schlaganfall, Silikose, Typ-1-Diabetes, Adipositas-bedingte Entzündung oder Insulinresistenz, rheumatoide Arthritis, myelodysplastisches Syndrom, Kontakthypersensitivität, durch das Chikungunya-Virus ausgelöste Gelenkentzündung oder traumatische Hirnverletzung;
(ii) nichtalkoholische Fettlebererkrankung (NAFLD) oder nichtalkoholische Steatohepatitis (NASH); oder
(iii) Alzheimer-Krankheit.

13. Verbindung nach einem der Ansprüche 1-10 oder ein pharmazeutisch annehmbares Salz, Stereoisomer oder ein Gemisch von Stereoisomeren davon, zur Verwendung bei der Behandlung einer Krankheit oder eines Zustands, die mindestens teilweise durch NLRP3 vermittelt werden, wobei die Krankheit oder der Zustand Alzheimer-Krankheit, Arteriosklerose, Asthma, allergische Atemwegsentzündung, Cryopyrin-assoziierte periodische Syndrome, Gicht, entzündliche Darmerkrankungen und verwandte Störungen, nichtalkoholische Fettlebererkrankung (NAFLD), nichtalkoholische Steatohepatitis (NASH), Bluthochdruck, Myokardinfarkt, Multiple Sklerose, experimentelle Autoimmunenzephalitis, Oxalat-induzierte Nephropathie, Hyperinflammation nach Influenza-Infektion, Graft-versus-Host-Reaktion, Schlaganfall, Silikose, Typ-1-Diabetes, Adipositas-bedingte Entzündung oder Insulinresistenz, rheumatoide Arthritis, myelodysplastisches Syndrom, Kontakthypersensitivität, durch das Chikungunya-Virus ausgelöste Gelenkentzündung oder traumatische Hirnverletzung ist.

14. Verbindung nach einem der Ansprüche 1-10 oder ein pharmazeutisch annehmbares Salz, Stereoisomer oder Gemisch von Stereoisomeren davon zur Verwendung in:
(i) Therapie;
(ii) Behandlung von Alzheimer-Krankheit; oder
(iii) Behandlung von nichtalkoholischer Fettlebererkrankung (NAFLD) oder nichtalkoholischer Steatohepatitis (NASH).

15. Verwendung einer Verbindung nach den Ansprüchen 1-10 oder eines pharmazeutisch akzeptablen Salzes, Stereoisomers oder eines Gemisches von Stereoisomeren davon zur Herstellung eines Arzneimittels zur Behandlung einer neurodegenerativen Erkrankung, Behandlung von Alzheimer-Krankheit, Arteriosklerose, Asthma, allergischer Atemwegsentzündung, Cryopyrinassoziierten periodischen Syndromen, Gicht, entzündlichen Darmerkrankungen und verwandten Störungen, nichtalkoholischer Fettlebererkrankung (NAFLD), nichtalkoholischer Steatohepatitis (NASH), Bluthochdruck, Myokardinfarkt, Multipler Sklerose, experimenteller Autoimmunenzephalitis, Oxalat-induzierter Nephropathie, Hyperinflammation nach Influenza-Infektion, Graft-versus-Host-Reaktion, Schlaganfall, Silikose, Typ-1-Diabetes, Adipositas-bedingter Entzündung oder Insulinresistenz, rheumatoider Arthritis, myelodysplastischem Syndrom, Kontakthypersensitivität, durch das Chikungunya-Virus ausgelöster Gelenkentzündung oder traumatischer Hirnverletzung.

## Revendications

1. Composé de Formule I : 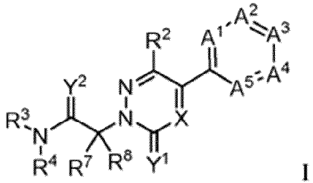
ou sel pharmaceutiquement acceptable, analogue enrichi isotopiquement, stéréoisomère ou mélange de stéréoisomères de celui-ci, dans lequel :
chacun parmi A¹, A², A³, A⁴ et A⁵ représente indépendamment N ou CR¹ ;
X représente N ou CR⁵ ;
chacun parmi Y¹ et Y² représente indépendamment O ou S ;
chaque R¹ représente indépendamment un hydrogène, un halo, un cyano, un hydroxy, un alkyle en -SH, -NH₂, -NO₂, -SF₅, C₁₋₆, un alcényle en C₂₋₆, un alcynyle en C₂₋₆, un haloalkyle en C₁₋₆, un hétéroalkyle en C₂₋₆, un cycloalkyle en C₃₋₁₀, un hétérocyclyle, un aryle, un hétéroaryle,
-N(R¹¹)₂, -OR¹¹, -C(O)R¹¹, -C(O)OR¹¹, -S(O)₀-₂R¹¹, -NR¹¹S(O)₀₋₂-R¹¹, -S(O)₀₋₂N(R¹¹)₂,
-NR¹¹S(O)₀₋₂N(R¹¹)₂, -NR¹¹C(O)N(R¹¹)₂, -C(O)N(R¹¹)₂, -NR¹¹C(O)R¹¹, -OC(O)N(R¹¹)₂ , ou
-NR¹¹C(O)OR¹¹ ; dans lequel chaque alkyle en C₁₋₆, alcényle en C₂₋₆, alcynyle en C₂₋₆, haloalkyle en C₁₋₆, hétéroalkyle en C₂₋₆, cycloalkyle en C₃₋₁₀, hétérocyclyle, aryle ou hétéroaryle est substitué indépendamment de manière facultative par un à cinq Z¹ ; et dans lequel deux groupes R¹ quelconques adjacents peuvent se lient pour former un cycle cycloalkyle en C₃₋₁₀, hétérocyclyle, aryle ou hétéroaryle, lequel cycle cycloalkyle en C₃₋₁₀, hétérocyclyle, aryle ou hétéroaryle peut être substitué en outre indépendamment de manière facultative par un à cinq Z¹ ;
R² représente -C(R⁶)₂R¹⁰, -OR⁹, -N(R⁶)(R⁹), -SR⁹, -S(O)R⁹, -S(O)₂R⁹, - OC(O)N(R⁶)(R⁹), -NR⁶C(O)OR⁹,
un cycloalkyle en -NR⁶C(O)R⁹, C₃₋₁₀, un hétérocyclyle ou un halo ; dans lequel le cycloalkyle en C₃₋₁₀ ou le hétérocyclyle est substitué indépendamment de manière facultative par un à cinq Z¹ ;
R³ représente un hydrogène, un alkyle en C₁₋₆, un alcényle en C₂₋₆, un alcynyle en C₂₋₆, un haloalkyle en C₁₋₆, un alcoxy en C₁₋₆, un hétéroalkyle en C₂₋₆, un cycloalkyle en C₃₋₁₀, un hétérocyclyle, un aryle ou un hétéroaryle ; dans lequel l'alkyle en C₁₋₆, l'alcényle en C₂₋₆, l'alcynyle en C₂₋₆,
un haloalkyle en C₁₋₆, un alcoxy en C₁₋₆, un hétéroalkyle en C₂₋₆, un cycloalkyle en C₃₋₁₀, un hétérocyclyle, un aryle ou un hétéroaryle est substitué indépendamment de manière facultative par un à cinq Z¹ ;
R⁴ représente un hydrogène, un alkyle en C₁₋₆, un alcényle en C₂₋₆, un alcynyle en C₂₋₆, un haloalkyle en C₁₋₆, un alcoxy en C₁₋₆, un hétéroalkyle en C₂₋₆, un cycloalkyle en C₃₋₁₀, un hétérocyclyle, un aryle ou un hétéroaryle ; dans lequel l'alkyle en C₁₋₆, l'alcényle en C₂₋₆, l'alcynyle en C₂₋₆,
un haloalkyle en C₁₋₆, un alcoxy en C₁₋₆, un hétéroalkyle en C₂₋₆, un cycloalkyle en C₃₋₁₀, un hétérocyclyle, un aryle ou un hétéroaryle est substitué indépendamment de manière facultative par un à cinq Z¹ ;
ou R³ et R⁴ se lient pour former un cycle hétérocyclyle ou hétéroaryle ; dans lequel le cycle hétérocyclyle ou hétéroaryle peut être substitué en outre indépendamment de manière facultative par un à cinq Z¹,
R⁵ représente un hydrogène, un halo, un alkyle en C₁₋₆, un haloalkyle en C₁₋₆, un alcoxy en C₁₋₆, un haloalcoxy en C₁₋₆, un cycloalkyle en C₃₋₁₀ ou un hétérocyclyle ; dans lequel l'alkyle en C₁₋₆, l'haloalkyle en C₁₋₆, l'alcoxy en C₁₋₆, l'haloalcoxy en C₁₋₆, le cycloalkyle en C₃₋₁₀ ou le hétérocyclyle est substitué indépendamment de manière facultative par un à cinq *Z*¹ ;
chaque R⁶ représente indépendamment un hydrogène, un halo, un cyano, un hydroxy, un alkyle en C₁₋₆, un alcényle en C₂₋₆, un alcynyle en C₂₋₆, un haloalkyle en C₁₋₆, un alcoxy en C₁₋₆, un haloalcoxy en C₁₋₆, un hétéroalkyle en C₂₋₆, un cycloalkyle en C₃₋₁₀, un hétérocyclyle, un aryle ou un hétéroaryle ;
R⁷ représente un hydrogène, un halo, un cyano, un hydroxy, un alkyle en C₁₋₆, un alcényle en C₂₋₆, un alcynyle en C₂₋₆, un haloalkyle en C₁₋₆, un alcoxy en C₁₋₆, un haloalcoxy en C₁₋₆, un hétéroalkyle en C₂₋₆, un cycloalkyle en C₃₋₁₀ ou un hétérocyclyle ;
R⁸ représente un hydrogène, un halo, un cyano, un hydroxy, un alkyle en C₁₋₆, un alcényle en C₂₋₆, un alcynyle en C₂₋₆, un haloalkyle en C₁₋₆, un alcoxy en C₁₋₆, un haloalcoxy en C₁₋₆, un hétéroalkyle en C₂₋₆, un cycloalkyle en C₃₋₁₀ ou un hétérocyclyle ;
ou R⁷ et R⁸ se lient pour former un cycle cycloalkyle en C₃₋₁₀ ou hétérocyclyle ; dans lequel le cycle cycloalkyle en C₃₋₁₀ ou hétérocyclyle peut être substitué en outre indépendamment de manière facultative par un à cinq *Z*^{1a} ;
R⁹ représente un alkyle en C₁₋₆, un alcényle en C₂₋₆, un alcynyle en C₂₋₆, un haloalkyle en C₁₋₆, un hétéroalkyle en C₂₋₆, un alcoxy en C₁₋₆, un haloalcoxy en C₁₋₆, un cycloalkyle en C₃₋₁₀, un hétérocyclyle, un aryle ou un hétéroaryle ; dans lequel l'alkyle en C₁₋₆, l'alcényle en C₂₋₆, l'alcynyle en C₂₋₆, l'haloalkyle en C₁₋₆, le hétéroalkyle en C₂₋₆, l'alcoxy en C₁₋₆, l'haloalcoxy en C₁₋₆, le cycloalkyle en C₃₋₁₀, le hétérocyclyle, l'aryle ou le hétéroaryle est substitué indépendamment de manière facultative par un à cinq groupes *Z*¹ ; ou
R¹⁰ représente un hydrogène, un halo, un hydroxy, un alkyle en C₁₋₆, un alcényle en C₂₋₆, un alcynyle en C₂₋₆, un haloalkyle en C₁₋₆, un hétéroalkyle en C₂₋₆, un cycloalkyle en C₃₋₁₀, un hétérocyclyle, un aryle ou un hétéroaryle ; dans lequel l'alkyle en C₁₋₆, l'alcényle en C₂₋₆, l'alcynyle en C₂₋₆, l'haloalkyle en C₁₋₆, le hétéroalkyle en C₂₋₆, le cycloalkyle en C₃₋₁₀, le hétérocyclyle, l'aryle ou le hétéroaryle est substitué indépendamment de manière facultative par un à cinq groupes *Z*¹; ou
chaque Z¹ représente indépendamment un halo, un cyano, un hydroxy, un alkyle en -SH, -NH₂, -NO₂, -SF₅, C₁₋₆, un alcényle en C₂₋₆, un alcynyle en C₂₋₆, un haloalkyle en C₁₋₆, un hétéroalkyle en C₂₋₆, un cycloalkyle en C₃₋₁₀, un hétérocyclyle, un aryle, un hétéroaryle, -N(R¹¹)₂, - OR¹¹, -C(O)R¹¹, -C(O)OR¹¹, -S(O)_{0·2}R¹¹, -NR¹¹S(O)_{0·2}-R¹¹, -S(O)_{0·2}N(R¹¹)₂, -NR¹¹S(O)_{0·2}N(R¹¹)₂, - NR¹¹C(O)N(R¹¹)₂, -C(O)N(R¹¹)₂, -NR¹¹C(O)R¹¹, -OC(O)N(R¹¹)₂, ou -NR¹¹C(O)OR¹¹ ; dans lequel chaque alkyle en C₁₋₆, alcényle en C₂₋₆, alcynyle en C₂₋₆, haloalkyle en C₁₋₆, hétéroalkyle en C₂₋₆, cycloalkyle en C₃₋₁₀, hétérocyclyle, aryle ou hétéroaryle est substitué indépendamment de manière facultative par un à cinq Z^{1a} ;
chaque R¹¹ représente indépendamment un hydrogène, un alkyle en C₁₋₆, un alcényle en C₂₋₆, un alcynyle en C₂₋₆, un haloalkyle enC₁₋₆, un hétéroalkyle en C₂₋₆, un cycloalkyle en C₃₋₁₀, un hétérocyclyle, un aryle ou un hétéroaryle ; dans lequel chaque alkyle en C₁₋₆, alcényle en C₂₋₆,
un alcynyle en C₂₋₆, un haloalkyle en C₁₋₆, un hétéroalkyle en C₂₋₆, un cycloalkyle en C₃₋₁₀, un hétérocyclyle, un aryle ou un hétéroaryle de R¹¹ est substitué indépendamment de manière facultative par un à cinq *Z*^{1a} ;
chaque Z^{1a} représente indépendamment un hydroxy, un halo, un cyano, un hydroxy, un alkyle en -SH, -NH₂, -NO₂, -SF₅, C₁₋₆, un alcényle en C₂₋₆, un alcynyle en C₂₋₆, un haloalkyle en C₁₋₆, un alcoxy en C₁₋₆, un haloalcoxy en C₁₋₆, un cycloalkyle en C₃₋₁₀, un hétérocyclyle, un aryle, un hétéroaryle, -N(R¹²)₂, -OR¹², -C(O)R¹², -C(O)OR¹², -S(O)_{0·2}R¹², -NR¹²S(O)_{0·2}-R¹², -S(O)_{0·2}N(R¹²)₂, - NR¹²S(O)_{0·2}N(R¹²)₂, -NR¹²C(O)N(R¹²)₂, -C(O)N(R¹²)₂, -NR¹²C(O)R¹², -OC(O)N(R¹²)₂,ou
-NR¹²C(O)OR¹² ; dans lequel chaque alkyle en C₁₋₆, alcényle enC₂₋₆, alcynyle en C₂₋₆, haloalkyle en C₁₋₆, alcoxy en C₁₋₆, haloalcoxy en C₁₋₆, cycloalkyle en C₃₋₁₀, hétérocyclyle, aryle ou hétéroaryle est substitué indépendamment de manière facultative par un à cinq Z^{1b} ;
chaque R¹² représente indépendamment un hydrogène, un alkyle en C₁₋₆, un alcényle en C₂₋₆, un alcynyle en C₂₋₆, un haloalkyle en C₁₋₆, un hétéroalkyle en C₂₋₆, un cycloalkyle en C₃₋₁₀, un hétérocyclyle, un aryle ou un hétéroaryle ; dans lequel chaque alkyle en C₁₋₆, alcényle en C₂₋₆,
un alcynyle en C₂₋₆, haloalkyle en C₁₋₆, un hétéroalkyle en C₂₋₆, un cycloalkyle en C₃₋₁₀, hétérocyclyle, un aryle ou un hétéroaryle de R¹² est substitué indépendamment de manière faculative par un à cinq Z^{1b} ;
chaque Z^{1b} représente indépendamment un halo, un cyano, un hydroxy, un alkyle en -SH, -NH₂, -NO₂, -SF₅, C₁₋₆, un alcényle en C₂₋₆, un alcynyle en C₂₋₆, un haloalkyle en C₁₋₆, un alcoxy en C₁₋₆, un hétéroalkyle en C₂₋₆, un haloalcoxy en C₁₋₆, un cycloalkyle en C₃₋₁₀, un hétérocyclyle, aryle, hétéroaryle, un alkyle en -L - C₁₋₆, un alcényle en -L - C₂₋₆, un alcynyle en -L - C₂₋₆, un haloalkyle en -L - C₁₋₆, un cycloalkyle en -L - C₃₋₁₀,
-L-hétérocyclyle, -L-aryle ou -L-hétéroaryle ; et
chaque L représente indépendamment -O-, -NH-, -S-, -S(O)-, -S(O)₂-, -N(alkyle en C₁₋₆)-, - N(alcényle en C₂₋₆)-, alcynyle en -N(C₂₋₆ haloalkyle en )-, -N(C₁₋₆)-, cycloalkyle en -N(C₃₋₁₀)-, - N(hétérocyclyle)-, -N(aryle)-, -N(hétéroaryle)-, -C(O)-, -C(O)O-, -C(O)NH-, -C(O)N( alkyle enC₁₋₆)-, - C(O)N(alcényle en C₂₋₆)-, -C(O)N(alcynyle en C₂₋₆)-,
haloalkyle en -C(O)N(C₁₋₆ cycloalkyle en )-, -C(O)N(C₃₋₁₀)-, -C(O)N(hétérocyclyle)-, - C(O)N(aryle)-, -C(O)N(hétéroaryle)-, -NHC(O)-, -NHC(O)O-, -NHC(O)NH-, -NHS(O)-, or -S(O)₂NH - ;
dans lequel chaque alkyle en C₁₋₆, alcényle en C₂₋₆, alcynyle en C₂₋₆, haloalkyle en C₁₋₆, alcoxy en C₁₋₆, haloalcoxy en C₁₋₆, cycloalkyle en C₃₋₁₀, hétérocyclyle, aryle et hétéroaryle de Z^{1b} et L est substitué en outre indépendamment de manière facultative par un à cinq hydroxy, halo, cyano, hydroxy, alkyle en -SH, -NH₂, -NO₂, -SF₅, C₁₋₆, alcényle en C₂₋₆, alcynyle en C₂₋₆, haloalkyle en C₁₋₆, alcoxy en C₁₋₆, haloalcoxy en C₁₋₆, cycloalkyle en C₃₋₁₀, hétérocyclyle, aryle ou hétéroaryle ;
à condition que le composé ne soit pas le N-(4-bromophényl)-2-[3-méthyl-6-oxo-4-phénylpyridazin-1(6H)-yl]acétamide, N-(4-bromophényl)-5-[(3-méthoxyphényl)méthyl]-3-méthyl-6-oxo-4-phényl-1(6H)-pyridazineacétamide, l'ester 1,1-diméthyléthyl de l'acide 4-[[2-[4-(2-acétyl-5-chlorophényl)-3-méthoxy-6-oxo-1(6H)-pyridazinyl]-4-méthyl-1-oxopentyl]amino] benzoïque ou l'acide 4-[[2-[4-(2-acétyl-5-chlorophényl)-3-méthoxy-6-oxo-1(6H)-pyridazinyl]-4-méthyl-1-oxopentyl]amino] benzoïque.

2. Composé selon la revendication 1, ou sel pharmaceutiquement acceptable, analogue enrichi isotopiquement, stéréoisomère ou mélange de stéréoisomères de celui-ci, dans lequel :
(i) chacun parmi A¹, A², A³, A⁴, et A⁵ représente indépendamment CR¹ ;
(ii) l'un parmi A¹, A², A³, A⁴, et A⁵ représente N et les autres A¹, A², A³, A⁴, et A⁵ représentent indépendamment CR¹; ou
(iii) deux parmi A¹, A², A³, A⁴, et A⁵ représentent N et les autres A¹, A², A³, A⁴, et A⁵ représentent indépendamment CR¹.

3. Composé selon les revendications 1 ou 2, ou sel pharmaceutiquement acceptable, analogue enrichi isotopiquement, stéréoisomère ou mélange de stéréoisomères de celui-ci, dans lequel X représente N.

4. Composé selon l'une quelconque des revendications 1 ou 2, ou sel pharmaceutiquement acceptable, analogue enrichi isotopiquement, stéréoisomère ou mélange de stéréoisomères de celui-ci, dans lequel :
(i) X représente CR⁵ ; et/ou
(ii) R⁵ représente un hydrogène, un alkyle en C₁₋₆ ou un haloalkyle en C₁₋₆.

5. Composé selon l'une quelconque des revendications 1-4, ou sel pharmaceutiquement acceptable, analogue enrichi isotopiquement, stéréoisomère ou mélange de stéréoisomères de celui-ci, dans lequel :
(i) chaque R¹ représente indépendamment un hydrogène, un halo, un cyano, un alkyle en C₁₋₆, un alcoxy en C₁₋₆, un haloalkyle en C₁₋₆ou un cycloalkyle en C₃₋₁₀, ou deux groupes R¹ quelconques adjacents peuvent se lier pour former un cycle aryle ou hétéroaryle ;
(ii) chaque R¹ représente indépendamment un hydrogène, un fluoro, un chloro, un cyano, -CH₃, -OCH₃, -OCH₂CH₃, -CF₃, un cyclopropyle, le 2,2-difluorocyclopropyle ou un cyclobutyle ;
(iii) R² représente un cycloalkyle en -C(R⁶)₂R¹⁰, -OR⁹, C₃₋₁₀ ou un halo ; dans lequel le cycloalkyle en C₃₋₁₀ est substitué indépendamment de manière facultative par un à cinq Z¹ ;
(iv) R³ représente un cycloalkyle en C₃₋₁₀, un hétérocyclyle ou un hétéroaryle ; dans lequel le cycloalkyle en C₃₋₁₀, le hétérocyclyle ou le hétéroaryle est substitué indépendamment de manière facultative par un à cinq Z¹ ;
(v) R³ représente un cycloalkyle en C₃₋₁₀, un hétérocyclyle ou un hétéroaryle ; dans lequel le cycloalkyle en C₃₋₁₀, le hétérocyclyle ou le hétéroaryle est substitué indépendamment de manière facultative par un à cinq halo, hydroxy, alkyle en C₁₋₆ou cycloalkyle en C₃₋₁₀ ;
(vi) R³ représente le 5-fluoropyrimidin-4-yle, le 1-cyclobutylpipéridin-3-yle, le 1-éthylpipéridin-3-yle, le 1-cyclopropylpipéridin-3-yle, le 3-fluoropyridin-2-yle, le 5-fluoropyrimidin-2-yle, le 3,5-difluoropyridin-2-yle ou le 3-hydroxy-3-méthylcyclobutyle ; et/ou
(vii) R⁴ représente un hydrogène ;

6. Composé selon l'une quelconque des revendications 1-5, ou sel pharmaceutiquement acceptable, analogue enrichi isotopiquement, stéréoisomère ou mélange de stéréoisomères de celui-ci, dans lequel R² représente :
(i) -C(R⁶)₂R¹⁰ ; et au moins un R⁶ représente un hydrogène ; et/ou
(ii) -C(R⁶)₂R¹⁰ ; et R¹⁰ représente un halo, un alkyle en C₁₋₆ ou un haloalkyle en C₁₋₆.

7. Composé selon l'une quelconque des revendications 1-5, ou sel pharmaceutiquement acceptable, analogue enrichi isotopiquement, stéréoisomère ou mélange de stéréoisomères de celui-ci, dans lequel R² représente -OR⁹ ; et R⁹ représente un alkyle en C₁₋₆.

8. Composé selon l'une quelconque des revendications 1-7, ou sel pharmaceutiquement acceptable, analogue enrichi isotopiquement, stéréoisomère ou mélange de stéréoisomères de celui-ci, dans lequel :
(i) R⁷ représente un hydrogène ; et/ou
(ii) R⁸ représente un hydrogène.

9. Composé selon la revendication 1, ou sel pharmaceutiquement acceptable, analogue enrichi isotopiquement, stéréoisomère ou mélange de stéréoisomères de celui-ci, dans lequel :
(i) chacun parmi A¹, A², A³, A⁴, et A⁵ représente indépendamment CR¹ ;
chaque R¹ représente indépendamment un hydrogène, un halo, un cyano, un alkyle en C₁₋₆, un alcoxy en C₁₋₆, un haloalkyle en C₁₋₆ ou un cycloalkyle en C₃₋₁₀ ou deux groupes R¹ quelconques adjacents peuvent se lier pour former un cycle aryle ou hétéroaryle ;
X représente CR⁵;
R² représente un cycloalkyle en -C(R⁶)₂R¹⁰, -OR⁹, C₃₋₁₀ ou un halo ; dans lequel le cycloalkyle en C₃₋₁₀ est substitué indépendamment de manière facultative par un à cinq Z¹ ;
R³ représente un cycloalkyle en C₃₋₁₀, un hétérocyclyle ou un hétéroaryle ; dans lequel le cycloalkyle en C₃₋₁₀, le hétérocyclyle ou le hétéroaryle est substitué indépendamment de manière facultative par un à cinq halo, hydroxy, alkyle en C₁₋₆ou cycloalkyle en C₃₋₁₀ ;
R⁴ représente un hydrogène ;
R⁵ représente un hydrogène, un alkyle en C₁₋₆ou un haloalkyle en C₁₋₆ ;
au moins un R⁶ représente un hydrogène ;
R⁷ et R⁸ représentent un hydrogène, ou R⁷ et R⁸ se lient pour former un cycloalkyle en C₃₋₁₀ ;
R⁹ représente un alkyle en C₁₋₆ ; et
R¹⁰ représente un halo, un alkyle en C₁₋₆ ou un haloalkyle en C₁₋₆ ;
(i) l'un parmi A¹, A², A³, A⁴, et A⁵ représente N et les autres A¹, A², A³, A⁴, et A⁵ représentent indépendamment CR¹ ;
chaque R¹ représente indépendamment un hydrogène, un halo, un cyano, un alkyle en C₁₋₆, un alcoxy en C₁₋₆, un haloalkyle en C₁₋₆ou un cycloalkyle en C₃₋₁₀, ou deux groupes R¹ quelconques adjacents peuvent se lier pour former un cycle aryle ou hétéroaryle ;
X représente CR⁵ ;
R² représente un cycloalkyle en -C(R⁶)₂R¹⁰, -OR⁹, C₃₋₁₀ ou un halo ; dans lequel le cycloalkyle en C₃₋₁₀ est substitué indépendamment de manière facultative par un à cinq *Z*¹;
R³ représente un cycloalkyle en C₃₋₁₀, un hétérocyclyle ou un hétéroaryle ; dans lequel le cycloalkyle en C₃₋₁₀, le hétérocyclyle ou le hétéroaryle est substitué indépendamment de manière facultative par un à cinq halo, hydroxy, alkyle en C₁₋₆ ou cycloalkyle en C₃₋₁₀ ;
R⁴ représente un hydrogène ;
R⁵ représente un hydrogène, un alkyle en C₁₋₆ ou un haloalkyle en C₁₋₆ ;
au moins un R⁶ représente un hydrogène ;
R⁷ et R⁸ représentent un hydrogène, ou R⁷ et R⁸ se lient pour former un cycloalkyle en C₃₋₁₀ ;
R⁹ représente un alkyle en C₁₋₆ ; et
R¹⁰ représente un halo, un alkyle en C₁₋₆ou un haloalkyle en C₁₋₆ ;
(iii) deux parmi A¹, A², A³, A⁴ et A⁵ représentent N et les autres A¹, A², A³, A⁴ et A⁵ représentent indépendamment CR¹ ;
chaque R¹ représente indépendamment un hydrogène, un halo, un cyano, un alkyle en C₁₋₆, un alcoxy en C₁₋₆, un haloalkyle en C₁₋₆ou un cycloalkyle en C₃₋₁₀, ou deux groupes R¹ quelconques adjacents peuvent se lier pour former un cycle aryle ou hétéroaryle ;
X représente CR⁵ ;
R² représente un cycloalkyle en -C(R⁶)₂R¹⁰, -OR⁹, C₃₋₁₀ ou un halo ; dans lequel le cycloalkyle en C₃₋₁₀ est substitué indépendamment de manière facultative par un à cinq *Z*¹ ;
R³ représente un cycloalkyle en C₃₋₁₀, un hétérocyclyle ou un hétéroaryle ; dans lequel le cycloalkyle en C₃₋₁₀, le hétérocyclyle ou le hétéroaryle est substitué indépendamment de manière facultative par un à cinq halo, hydroxy, alkyle en C₁₋₆ou cycloalkyle en C₃₋₁₀ ;
R⁴ représente un hydrogène ;
R⁵ représente un hydrogène, un alkyle en C₁₋₆ ou un haloalkyle en C₁₋₆ ;
au moins un R⁶ représente un hydrogène ;
R⁷ et R⁸ représentent un hydrogène ;
R⁹ représente un alkyle en C₁₋₆ ; et
R¹⁰ représente un halo, un alkyle en C₁₋₆ ou un haloalkyle en C₁₋₆ ;
(iv) chacun parmi A¹, A², A³, A⁴ et A⁵ représentent indépendamment CR¹ ;
chaque R¹ représente indépendamment un hydrogène, un halo, un cyano, un alkyle en C₁₋₆, un alcoxy en C₁₋₆, un haloalkyle en C₁₋₆ou un cycloalkyle en C₃₋₁₀ ou deux groupes R¹ quelconques adjacents peuvent se lier pour former un cycle aryle ou hétéroaryle ;
X représente N ;
R² représente un cycloalkyle en -C(R⁶)₂R¹⁰, -OR⁹, C₃₋₁₀ ou un halo ; dans lequel le cycloalkyle en C₃₋₁₀ est substitué indépendamment de manière facultative par un à cinq *Z*¹ ;
R³ représente un cycloalkyle en C₃₋₁₀, un hétérocyclyle ou un hétéroaryle ; dans lequel le cycloalkyle en C₃₋₁₀, le hétérocyclyle ou le hétéroaryle est substitué indépendamment de manière facultative par un à cinq halo, hydroxy, alkyle en C₁₋₆ ou cycloalkyle en C₃₋₁₀ ;
R⁴ représente un hydrogène ;
au moins un R⁶ représente un hydrogène ;
R⁷ et R⁸ représentent un hydrogène, ou R⁷ et R⁸ se lient pour former un cycloalkyle en C₃₋₁₀ ;
R⁹ représente un alkyle en C₁₋₆ ; et
R¹⁰ représente un halo, un alkyle en C₁₋₆ ou un haloalkyle en C₁₋₆ ;
(v) l'un parmi A¹, A², A³, A⁴ et A⁵ représente N et les autres A¹, A², A³, A⁴ et A⁵ représentent indépendamment CR¹ ;
chaque R¹ représente indépendamment un hydrogène, un halo, un cyano, un alkyle en C₁₋₆, un alcoxy en C₁₋₆, un haloalkyle en C₁₋₆ ou un cycloalkyle en C₃₋₁₀ ou deux groupes R¹ quelconques adjacents peuvent se lier pour former un cycle aryle ou hétéroaryle ;
X représente N ;
R² représente un cycloalkyle en -C(R⁶)₂R¹⁰, -OR⁹, C₃₋₁₀ ou un halo ; dans lequel le cycloalkyle en C₃₋₁₀ est substitué indépendamment de manière facultative par un à cinq *Z*¹ ;
R³ représente un cycloalkyle en C₃₋₁₀, un hétérocyclyle ou un hétéroaryle ; dans lequel le cycloalkyle en C₃₋₁₀, le hétérocyclyle ou le hétéroaryle est substitué indépendamment de manière facultative par un à cinq halo, hydroxy, alkyle en C₁₋₆ ou cycloalkyle en C₃₋₁₀ ;
R⁴ représente un hydrogène ;
au moins un R⁶ représente un hydrogène ;
R⁷ et R⁸ représentent un hydrogène, ou R⁷ et R⁸ se lient pour former un cycloalkyle en C₃₋₁₀ ;
R⁹ représente un alkyle en C₁₋₆ ; et
R¹⁰ représente un halo, un alkyle en C₁₋₆ou un haloalkyle en C₁₋₆ ; ou
(vi) deux parmi A¹, A², A³, A⁴ et A⁵ représentent N et les autres A¹, A², A³, A⁴ et A⁵ représentent indépendamment CR¹ ;
chaque R¹ représente indépendamment un hydrogène, un halo, un cyano, un alkyle en C₁₋₆, un alcoxy en C₁₋₆, un haloalkyle en C₁₋₆ ou un cycloalkyle en C₃₋₁₀, ou deux groupes R¹ quelconques adjacents peuvent se lier pour former un cycle aryle ou hétéroaryle ;
X représente N ;
R² représente un cycloalkyle en -C(R⁶)₂R¹⁰, -OR⁹, C₃₋₁₀ ou un halo ; dans lequel le cycloalkyle en C₃₋₁₀ est substitué indépendamment de manière facultative par un à cinq *Z*¹ ;
R³ représente un cycloalkyle en C₃₋₁₀, un hétérocyclyle ou un hétéroaryle ; dans lequel le cycloalkyle en C₃₋₁₀, le hétérocyclyle ou le hétéroaryle est substitué indépendamment de manière facultative par un à cinq halo, hydroxy, alkyle en C₁₋₆ou cycloalkyle en C₃₋₁₀ ;
R⁴ représente un hydrogène ;
au moins un R⁶ représente un hydrogène ;
R⁷ et R⁸ représentent un hydrogène, ou R⁷ et R⁸ se lient pour former un cycloalkyle en C₃₋₁₀ ;
R⁹ représente un alkyle en C₁₋₆ ; et
R¹⁰ représente un halo, un alkyle en C₁₋₆ ou un haloalkyle en C₁₋₆.

10. Composé ou sel pharmaceutiquement acceptable, analogue enrichi isotopiquement, stéréoisomère ou mélange de stéréoisomères de celui-ci, choisi parmi :
(i) le tableau 1 ; ou
(ii) le tableau 2.

11. Composition pharmaceutique comprenant un composé selon une quelconque revendication précédente ou sel pharmaceutiquement acceptable, stéréoisomère ou mélange de stéréoisomères de celui-ci, et vecteur pharmaceutiquement acceptable.

12. Composition pharmaceutique selon la revendication 11 destinée à être utilisée dans un procédé de traitement d'une maladie ou d'une affection induite, au moins en partie, par NLRP3, le procédé comprenant l'administration d'une quantité efficace de la composition pharmaceutique selon la revendication 11 à un sujet en ayant besoin, facultativement dans laquelle la maladie ou l'affection est l'une parmi :
(i) Maladie d'Alzheimer, athérosclérose, asthme, inflammation allergique des voies respiratoires, syndromes périodiques associés à la cryopyrine, goutte, maladies inflammatoires de l'intestin et troubles apparentés, stéatose hépatique non alcoolique (NAFLD), stéatohépatite non alcoolique (NASH), hypertension, infarctus du myocarde, sclérose en plaques, encéphalite auto-immune expérimentale, néphropathie induite par l'oxalate, hyperinflammation consécutive à une infection grippale, maladie du greffon contre l'hôte, accident vasculaire cérébral, silicose, diabète de type 1, inflammation ou résistance à l'insuline induite par l'obésité, polyarthrite rhumatoïde, syndrome myélodysplasique, hypersensibilité de contact, inflammation articulaire déclenchée par le virus chikungunya ou traumatisme crânien ;
(ii) stéatose hépatique non alcoolique (NAFLD) ou stéatohépatite non alcoolique (NASH) ; ou
(iii) Maladie d'Alzheimer.

13. Composé de l'une quelconque des revendications 1-10, ou sel pharmaceutiquement acceptable, stéréoisomère ou mélange de stéréoisomères de celui-ci, destiné à être utilisé dans le traitement d'une maladie ou d'une affection induite, au moins en partie, par NLRP3, facultativement dans lequel la maladie ou l'affection est l'une parmi : la maladie d'Alzheimer, l'athérosclérose, l'asthme, l'inflammation allergique des voies respiratoires, les syndromes périodiques associés à la cryopyrine, la goutte, les maladies inflammatoires de l'intestin et les troubles apparentés, la stéatose hépatique non alcoolique (NAFLD), la stéatohépatite non alcoolique (NASH), l'hypertension, l'infarctus du myocarde, la sclérose en plaques, l'encéphalite auto-immune expérimentale, la néphropathie induite par l'oxalate, l'hyperinflammation consécutive à une infection grippale, la réaction du greffon contre l'hôte, l'accident vasculaire cérébral, la silicose, le diabète de type 1, l'inflammation ou la résistance à l'insuline induite par l'obésité, la polyarthrite rhumatoïde, le syndrome myélodysplasique, l'hypersensibilité de contact, l'inflammation articulaire déclenchée par le virus chikungunya ou un traumatisme crânien.

14. Composé selon l'une quelconque des revendications 1-10, ou sel pharmaceutiquement acceptable, stéréoisomère ou n mélange de stéréoisomères de celui-ci, destiné à être utilisé dans :
(i) une thérapie ;
(ii) le traitement de la maladie d'Alzheimer ; ou
(iii) le traitement de la stéatose hépatique non alcoolique (NAFLD) ou la stéatohépatite non alcoolique (NASH).

15. Utilisation d'un composé selon les revendications 1-10, ou sel pharmaceutiquement acceptable, stéréoisomère ou mélange de stéréoisomères de celui-ci, pour la fabrication d'un médicament destiné au traitement d'une maladie neurodégénérative, au traitement de la maladie d'Alzheimer, de l'athérosclérose, de l'asthme, de l'inflammation allergique des voies respiratoires, des syndromes périodiques associés à la cryopyrine, de la goutte, des maladies inflammatoires de l'intestin et des troubles apparentés, de la stéatose hépatique non alcoolique (NAFLD), de la stéatohépatite non alcoolique (NASH), de l'hypertension, de l'infarctus du myocarde, de la sclérose en plaques, de l'encéphalite auto-immune expérimentale, de la néphropathie induite par l'oxalate, de l'hyperinflammation consécutive à une infection grippale, de la réaction du greffon contre l'hôte, de l'accident vasculaire cérébral, de la silicose, du diabète de type 1, de l'inflammation ou de la résistance à l'insuline induite par l'obésité, de la polyarthrite rhumatoïde, du syndrome myélodysplasique, de l'hypersensibilité de contact, de l'inflammation articulaire déclenchée par le virus chikungunya ou d'un traumatisme crânien.
